# EUROPEAN PATENT SPECIFICATION

(11) **EP 3 174 546 B1**
(45) Date of publication and mention of the grant of the patent: **30.10.2019**
(21) Application number: 15753252.4
(22) Date of filing: 31.07.2015
(51) Int. Cl.: C07K 16/28, A61K 39/00, A61K 35/17, C07K 14/705, A61K 39/395, A61K 31/436, C07K 14/725, C12N 5/0783

(54) **SUBSET-OPTIMIZED CHIMERIC ANTIGEN RECEPTOR-CONTAINING T-CELLS**
TEILMENGENOPTIMIERTE CHIMÄRE ANTIGENREZEPTORHALTIGE T-ZELLEN
SOUS-ENSEMBLE OPTIMISÉ DE LYMPHOCYTES T CONTENANT UN RÉCEPTEUR D'ANTIGÈNE CHIMÈRE

(30) Priority: 31.07.2014 US 201462031699 P
(43) Date of publication of application: 07.06.2017
(73) Proprietor: Novartis AG, 4056 Basel (CH); The Trustees of The University of Pennsylvania, Philadelphia, PA 19104 (US); Guedan Carrio, Sonia, Philadelphia, PA 19143 (US)
(72) Inventor: GUEDAN CARRIO, Sonia, Philadelphia, PA 19143 (US); JUNE, Carl H., Merion Station, PA 19066 (US); POSEY, Avery D., Philadelphia, PA 19106 (US); SCHOLLER, John, Narberth, PA 19072 (US)
(74) Representative: Wilding, James Roger
(86) International application number: PCT/US2015/043219
(87) International publication number: WO 2016/019300

(56) References cited:
- WO-A1-2014/059248
- WO-A1-2014/190273
- WO-A2-2014/144622
- MOELLER M ET AL: "Adoptive transfer of gene-engineered CD4+ helper T cells induces potent primary and secondary tumor rejection", BLOOD, AMERICAN SOCIETY OF HEMATOLOGY, US, vol. 106, no. 9, 1 November 2005 (2005-11-01), pages 2995-3003, XP003022406, ISSN: 0006-4971, DOI: 10.1182/BLOOD-2004-12-4906
- JOHN LIZA B ET AL: "Genetic modification of mouse effector and helper T lymphocytes expressing a chimeric antigen receptor", METHODS IN MOLECULAR BIOLOGY, HUMANA PRESS, INC, US, vol. 1139, 1 January 2014 (2014-01-01), pages 177-187, XP008177659, ISSN: 1940-6029, DOI: 10.1007/978-1-4939-0345-0_16 [retrieved on 2014-02-03]
- MARC CARTELLIERI ET AL: "Chimeric Antigen Receptor-Engineered T Cells for Immunotherapy of Cancer", JOURNAL OF BIOMEDICINE AND BIOTECHNOLOGY, HINDAWI PUBLISHING CORPORATION, US, vol. 2010, 1 January 2010 (2010-01-01), pages 956304-1, XP002736525, ISSN: 1110-7243, DOI: 10.1155/2010/956304
- XIULI WANG ET AL: "Phenotypic and Functional Attributes of Lentivirus-modified CD19-specific Human CD8+ Central Memory T Cells Manufactured at Clinical Scale", JOURNAL OF IMMUNOTHERAPY, vol. 35, no. 9, 1 January 2012 (2012-01-01), pages 689-701, XP55211902, ISSN: 1524-9557, DOI: 10.1097/CJI.0b013e318270dec7
- JENNIFER D. STONE ET AL: "Role of T Cell Receptor Affinity in the Efficacy and Specificity of Adoptive T Cell Therapies", FRONTIERS IN IMMUNOLOGY, vol. 4, 1 January 2013 (2013-01-01), XP55217565, ISSN: 1664-3224, DOI: 10.3389/fimmu.2013.00244
- I. LIADI ET AL: "Individual Motile CD4+ T Cells Can Participate in Efficient Multikilling through Conjugation to Multiple Tumor Cells", CANCER IMMUNOLOGY RESEARCH, vol. 3, no. 5, 1 May 2015 (2015-05-01), pages 473-482, XP055217529, ISSN: 2326-6066, DOI: 10.1158/2326-6066.CIR-14-0195
- P. S. ADUSUMILLI ET AL: "Regional delivery of mesothelin-targeted CAR T cell therapy generates potent and long-lasting CD4-dependent tumor immunity", SCIENCE TRANSLATIONAL MEDICINE, vol. 6, no. 261, 5 November 2014 (2014-11-05), pages 261ra151-261ra151, XP055217533, ISSN: 1946-6234, DOI: 10.1126/scitranslmed.3010162

### SEQUENCE LISTING

<110> NOVARTIS AG THE TRUSTEES OF THE UNIVERSITY OF PENNSYLVANIA
<120> SUBSET-OPTIMIZED CHIMERIC ANTIGEN RECEPTOR-CONTAINING CELLS
<130> N2067-70660WO
<140>
   <141>
<150> 62/031,699
   <151> 2014-07-31
<160> 109
<170> PatentIn version 3.5
<210> 1
   <211> 1184
   <212> DNA
   <213> Artificial Sequence
<220>
   <221> source
   <223> /note="Description of Artificial Sequence: Synthetic polynucleotide"
<400> 1
   cgtgaggctc cggtgcccgt cagtgggcag agcgcacatc gcccacagtc cccgagaagt      60
   tggggggagg ggtcggcaat tgaaccggtg cctagagaag gtggcgcggg gtaaactggg     120
   aaagtgatgt cgtgtactgg ctccgccttt ttcccgaggg tgggggagaa ccgtatataa     180
   gtgcagtagt cgccgtgaac gttctttttc gcaacgggtt tgccgccaga acacaggtaa     240
   gtgccgtgtg tggttcccgc gggcctggcc tctttacggg ttatggccct tgcgtgcctt     300
   gaattacttc cacctggctg cagtacgtga ttcttgatcc cgagcttcgg gttggaagtg     360
   ggtgggagag ttcgaggcct tgcgcttaag gagccccttc gcctcgtgct tgagttgagg     420
   cctggcctgg gcgctggggc cgccgcgtgc gaatctggtg gcaccttcgc gcctgtctcg     480
   ctgctttcga taagtctcta gccatttaaa atttttgatg acctgctgcg acgctttttt     540
   tctggcaaga tagtcttgta aatgcgggcc aagatctgca cactggtatt tcggtttttg     600
   gggccgcggg cggcgacggg gcccgtgcgt cccagcgcac atgttcggcg aggcggggcc     660
   tgcgagcgcg gccaccgaga atcggacggg ggtagtctca agctggccgg cctgctctgg     720
   tgcctggcct cgcgccgccg tgtatcgccc cgccctgggc ggcaaggctg gcccggtcgg     780
   caccagttgc gtgagcggaa agatggccgc ttcccggccc tgctgcaggg agctcaaaat     840
   ggaggacgcg gcgctcggga gagcgggcgg gtgagtcacc cacacaaagg aaaagggcct     900
   ttccgtcctc agccgtcgct tcatgtgact ccacggagta ccgggcgccg tccaggcacc     960
   tcgattagtt ctcgagcttt tggagtacgt cgtctttagg ttggggggag gggttttatg    1020
   cgatggagtt tccccacact gagtgggtgg agactgaagt taggccagct tggcacttga    1080
   tgtaattctc cttggaattt gccctttttg agtttggatc ttggttcatt ctcaagcctc    1140
   agacagtggt tcaaagtttt tttcttccat ttcaggtgtc gtga                      1184
<210> 2
   <211> 21
   <212> PRT
   <213> Artificial Sequence
<220>
   <221> source
   <223> /note="Description of Artificial Sequence: Synthetic peptide"
<400> 2
   Met Ala Leu Pro Val Thr Ala Leu Leu Leu Pro Leu Ala Leu Leu Leu
   1               5                   10                  15
   His Ala Ala Arg Pro
               20
<210> 3
   <211> 63
   <212> DNA
   <213> Artificial Sequence
<220>
   <221> source
   <223> /note="Description of Artificial Sequence: Synthetic oligonucleotide"
<400> 3
   atggccctgc ctgtgacagc cctgctgctg cctctggctc tgctgctgca tgccgctaga       60
   ccc                                                                     63
<210> 4
   <211> 45
   <212> PRT
   <213> Artificial Sequence
<220>
   <221> source
   <223> /note="Description of Artificial Sequence: Synthetic polypeptide"
<400> 4
   Thr Thr Thr Pro Ala Pro Arg Pro Pro Thr Pro Ala Pro Thr Ile Ala
   1               5                   10                  15
   Ser Gln Pro Leu Ser Leu Arg Pro Glu Ala Cys Arg Pro Ala Ala Gly
               20                  25                  30
   Gly Ala Val His Thr Arg Gly Leu Asp Phe Ala Cys Asp
           35                  40                  45
<210> 5
   <211> 135
   <212> DNA
   <213> Artificial Sequence
<220>
   <221> source
   <223> /note="Description of Artificial Sequence: Synthetic polynucleotide"
<400> 5
   accacgacgc cagcgccgcg accaccaaca ccggcgccca ccatcgcgtc gcagcccctg       60
   tccctgcgcc cagaggcgtg ccggccagcg gcggggggcg cagtgcacac gagggggctg      120
   gacttcgcct gtgat                                                       135
<210> 6
   <211> 230
   <212> PRT
   <213> Artificial Sequence
<220>
   <221> source
   <223> /note="Description of Artificial Sequence: Synthetic polypeptide"
<400> 6
   Glu Ser Lys Tyr Gly Pro Pro Cys Pro Pro Cys Pro Ala Pro Glu Phe
   1               5                   10                  15
   Leu Gly Gly Pro Ser Val Phe Leu Phe Pro Pro Lys Pro Lys Asp Thr
               20                  25                  30
   Leu Met Ile Ser Arg Thr Pro Glu Val Thr Cys Val Val Val Asp Val
           35                  40                  45
   Ser Gln Glu Asp Pro Glu Val Gln Phe Asn Trp Tyr Val Asp Gly Val
       50                  55                  60
   Glu Val His Asn Ala Lys Thr Lys Pro Arg Glu Glu Gln Phe Asn Ser
   65                  70                  75                  80
   Thr Tyr Arg Val Val Ser Val Leu Thr Val Leu His Gln Asp Trp Leu
                   85                  90                  95
   Asn Gly Lys Glu Tyr Lys Cys Lys Val Ser Asn Lys Gly Leu Pro Ser
               100                 105                 110
   Ser Ile Glu Lys Thr Ile Ser Lys Ala Lys Gly Gln Pro Arg Glu Pro
           115                 120                 125
   Gln Val Tyr Thr Leu Pro Pro Ser Gln Glu Glu Met Thr Lys Asn Gln
       130                 135                 140
   Val Ser Leu Thr Cys Leu Val Lys Gly Phe Tyr Pro Ser Asp Ile Ala
   145                 150                 155                 160
   Val Glu Trp Glu Ser Asn Gly Gln Pro Glu Asn Asn Tyr Lys Thr Thr
                   165                 170                 175
   Pro Pro Val Leu Asp Ser Asp Gly Ser Phe Phe Leu Tyr Ser Arg Leu
               180                 185                 190
   Thr Val Asp Lys Ser Arg Trp Gln Glu Gly Asn Val Phe Ser Cys Ser
           195                 200                 205
   Val Met His Glu Ala Leu His Asn His Tyr Thr Gln Lys Ser Leu Ser
       210                 215                 220
   Leu Ser Leu Gly Lys Met
   225                 230
<210> 7
   <211> 690
   <212> DNA
   <213> Artificial Sequence
<220>
   <221> source
   <223> /note="Description of Artificial Sequence: Synthetic polynucleotide"
<400> 7
   gagagcaagt acggccctcc ctgcccccct tgccctgccc ccgagttcct gggcggaccc        60
   agcgtgttcc tgttcccccc caagcccaag gacaccctga tgatcagccg gacccccgag       120
   gtgacctgtg tggtggtgga cgtgtcccag gaggaccccg aggtccagtt caactggtac       180
   gtggacggcg tggaggtgca caacgccaag accaagcccc gggaggagca gttcaatagc       240
   acctaccggg tggtgtccgt gctgaccgtg ctgcaccagg actggctgaa cggcaaggaa       300
   tacaagtgta aggtgtccaa caagggcctg cccagcagca tcgagaaaac catcagcaag       360
   gccaagggcc agcctcggga gccccaggtg tacaccctgc cccctagcca agaggagatg       420
   accaagaacc aggtgtccct gacctgcctg gtgaagggct tctaccccag cgacatcgcc       480
   gtggagtggg agagcaacgg ccagcccgag aacaactaca agaccacccc ccctgtgctg       540
   gacagcgacg gcagcttctt cctgtacagc cggctgaccg tggacaagag ccggtggcag       600
   gagggcaacg tctttagctg ctccgtgatg cacgaggccc tgcacaacca ctacacccag       660
   aagagcctga gcctgtccct gggcaagatg                                        690
<210> 8
   <211> 282
   <212> PRT
   <213> Artificial Sequence
<220>
   <221> source
   <223> /note="Description of Artificial Sequence: Synthetic polypeptide"
<400> 8
   Arg Trp Pro Glu Ser Pro Lys Ala Gln Ala Ser Ser Val Pro Thr Ala
   1               5                   10                  15
   Gln Pro Gln Ala Glu Gly Ser Leu Ala Lys Ala Thr Thr Ala Pro Ala
               20                  25                  30
   Thr Thr Arg Asn Thr Gly Arg Gly Gly Glu Glu Lys Lys Lys Glu Lys
           35                  40                  45
   Glu Lys Glu Glu Gln Glu Glu Arg Glu Thr Lys Thr Pro Glu Cys Pro
       50                  55                  60
   Ser His Thr Gln Pro Leu Gly Val Tyr Leu Leu Thr Pro Ala Val Gln
   65                  70                  75                  80
   Asp Leu Trp Leu Arg Asp Lys Ala Thr Phe Thr Cys Phe Val Val Gly
                   85                  90                  95
   Ser Asp Leu Lys Asp Ala His Leu Thr Trp Glu Val Ala Gly Lys Val
               100                 105                 110
   Pro Thr Gly Gly Val Glu Glu Gly Leu Leu Glu Arg His Ser Asn Gly
           115                 120                 125
   Ser Gln Ser Gln His Ser Arg Leu Thr Leu Pro Arg Ser Leu Trp Asn
       130                 135                 140
   Ala Gly Thr Ser Val Thr Cys Thr Leu Asn His Pro Ser Leu Pro Pro
   145                 150                 155                 160
   Gln Arg Leu Met Ala Leu Arg Glu Pro Ala Ala Gln Ala Pro Val Lys
                   165                 170                 175
   Leu Ser Leu Asn Leu Leu Ala Ser Ser Asp Pro Pro Glu Ala Ala Ser
               180                 185                 190
   Trp Leu Leu Cys Glu Val Ser Gly Phe Ser Pro Pro Asn Ile Leu Leu
           195                 200                 205
   Met Trp Leu Glu Asp Gln Arg Glu Val Asn Thr Ser Gly Phe Ala Pro
       210                 215                 220
   Ala Arg Pro Pro Pro Gln Pro Gly Ser Thr Thr Phe Trp Ala Trp Ser
   225                 230                 235                 240
   Val Leu Arg Val Pro Ala Pro Pro Ser Pro Gln Pro Ala Thr Tyr Thr
                   245                 250                 255
   Cys Val Val Ser His Glu Asp Ser Arg Thr Leu Leu Asn Ala Ser Arg
               260                 265                 270
   Ser Leu Glu Val Ser Tyr Val Thr Asp His
           275                 280
<210> 9
   <211> 847
   <212> DNA
   <213> Artificial Sequence
<220>
   <221> source
   <223> /note="Description of Artificial Sequence: Synthetic polynucleotide"
<400> 9
   aggtggcccg aaagtcccaa ggcccaggca tctagtgttc ctactgcaca gccccaggca      60
   gaaggcagcc tagccaaagc tactactgca cctgccacta cgcgcaatac tggccgtggc     120
   ggggaggaga agaaaaagga gaaagagaaa gaagaacagg aagagaggga gaccaagacc     180
   cctgaatgtc catcccatac ccagccgctg ggcgtctatc tcttgactcc cgcagtacag     240
   gacttgtggc ttagagataa ggccaccttt acatgtttcg tcgtgggctc tgacctgaag     300
   gatgcccatt tgacttggga ggttgccgga aaggtaccca cagggggggt tgaggaaggg     360
   ttgctggagc gccattccaa tggctctcag agccagcact caagactcac ccttccgaga     420
   tccctgtgga acgccgggac ctctgtcaca tgtactctaa atcatcctag cctgccccca     480
   cagcgtctga tggcccttag agagccagcc gcccaggcac cagttaagct tagcctgaat     540
   ctgctcgcca gtagtgatcc cccagaggcc gccagctggc tcttatgcga agtgtccggc     600
   tttagcccgc ccaacatctt gctcatgtgg ctggaggacc agcgagaagt gaacaccagc     660
   ggcttcgctc cagcccggcc cccaccccag ccgggttcta ccacattctg ggcctggagt     720
   gtcttaaggg tcccagcacc acctagcccc cagccagcca catacacctg tgttgtgtcc     780
   catgaagata gcaggaccct gctaaatgct tctaggagtc tggaggtttc ctacgtgact     840
   gaccatt                                                               847
<210> 10
   <211> 10
   <212> PRT
   <213> Artificial Sequence
<220>
   <221> source
   <223> /note="Description of Artificial Sequence: Synthetic peptide"
<400> 10
   Gly Gly Gly Gly Ser Gly Gly Gly Gly Ser
   1               5                   10
<210> 11
   <211> 30
   <212> DNA
   <213> Artificial Sequence
<220>
   <221> source
   <223> /note="Description of Artificial Sequence: Synthetic oligonucleotide"
<400> 11
   ggtggcggag gttctggagg tggaggttcc 30
<210> 12
   <211> 24
   <212> PRT
   <213> Artificial Sequence
<220>
   <221> source
   <223> /note="Description of Artificial Sequence: Synthetic peptide"
<400> 12
   Ile Tyr Ile Trp Ala Pro Leu Ala Gly Thr Cys Gly Val Leu Leu Leu
   1               5                   10                  15
   Ser Leu Val Ile Thr Leu Tyr Cys
               20
<210> 13
   <211> 72
   <212> DNA
   <213> Artificial Sequence
<220>
   <221> source
   <223> /note="Description of Artificial Sequence: Synthetic oligonucleotide"
<400> 13
   atctacatct gggcgccctt ggccgggact tgtggggtcc ttctcctgtc actggttatc 60
   accctttact gc 72
<210> 14
   <211> 42
   <212> PRT
   <213> Artificial Sequence
<220>
   <221> source
   <223> /note="Description of Artificial Sequence: Synthetic polypeptide"
<400> 14
   Lys Arg Gly Arg Lys Lys Leu Leu Tyr Ile Phe Lys Gln Pro Phe Met
   1 5 10 15
   Arg Pro Val Gln Thr Thr Gln Glu Glu Asp Gly Cys Ser Cys Arg Phe
   20 25 30
   Pro Glu Glu Glu Glu Gly Gly Cys Glu Leu
   35 40
<210> 15
   <211> 126
   <212> DNA
   <213> Artificial Sequence
<220>
   <221> source
   <223> /note="Description of Artificial Sequence: Synthetic polynucleotide"
<400> 15
   aaacggggca gaaagaaact cctgtatata ttcaaacaac catttatgag accagtacaa 60
   actactcaag aggaagatgg ctgtagctgc cgatttccag aagaagaaga aggaggatgt 120
   gaactg 126
<210> 16
   <211> 48
   <212> PRT
   <213> Artificial Sequence
<220>
   <221> source
   <223> /note="Description of Artificial Sequence: Synthetic polypeptide"
<400> 16
   Gln Arg Arg Lys Tyr Arg Ser Asn Lys Gly Glu Ser Pro Val Glu Pro
   1               5                   10                  15
   Ala Glu Pro Cys Arg Tyr Ser Cys Pro Arg Glu Glu Glu Gly Ser Thr
               20                  25                  30
   Ile Pro Ile Gln Glu Asp Tyr Arg Lys Pro Glu Pro Ala Cys Ser Pro
           35                  40                  45
<210> 17
   <211> 123
   <212> DNA
   <213> Artificial Sequence
<220>
   <221> source
   <223> /note="Description of Artificial Sequence: Synthetic polynucleotide"
<400> 17
   aggagtaaga ggagcaggct cctgcacagt gactacatga acatgactcc ccgccgcccc      60
   gggcccaccc gcaagcatta ccagccctat gccccaccac gcgacttcgc agcctatcgc     120
   tcc                                                                   123
<210> 18
   <211> 108
   <212> PRT
   <213> Artificial Sequence
<220>
   <221> source
   <223> /note="Description of Artificial Sequence: Synthetic polypeptide"
<400> 18
   Arg Val Lys Phe Ser Arg Ser Ala Asp Ala Pro Ala Tyr Lys Gln Gly
   1               5                   10                  15
   Gln Asn Gln Leu Tyr Asn Glu Leu Asn Leu Gly Arg Arg Glu Glu Tyr
               20                  25                  30
   Asp Val Leu Asp Lys Arg Arg Gly Arg Asp Pro Glu Met Gly Gly Lys
           35                  40                  45
   Pro Arg Arg Lys Asn Pro Gln Glu Gly Asn Glu Leu Gln Lys Asp Lys
       50                  55                  60
   Met Ala Glu Ala Tyr Ser Glu Ile Gly Met Lys Gly Glu Arg Arg Arg
   65                  70                  75                  80
   Gly Lys Gly His Asp Gly Gln Gly Leu Ser Thr Ala Thr Lys Asp Thr
                   85                  90                  95
   Tyr Asp Ala Leu His Met Gln Ala Leu Pro Pro Arg
               100                 105
<210> 19
   <211> 336
   <212> DNA
   <213> Artificial Sequence
<220>
   <221> source
   <223> /note="Description of Artificial Sequence: Synthetic polynucleotide"
<400> 19
   agagtgaagt tcagcaggag cgcagacgcc cccgcgtaca agcagggcca gaaccagctc      60
   tataacgagc tcaatctagg acgaagagag gagtacgatg ttttggacaa gagacgtggc     120
   cgggaccctg agatgggggg aaagccgaga aggaagaacc ctcaggaagg cctgtacaat     180
   gaactgcaga aagataagat ggcggaggcc tacagtgaga ttgggatgaa aggcgagcgc     240
   cggaggggca aggggcacga tggcctttac cagggtctca gtacagccac caaggacacc     300
   tacgacgccc ttcacatgca ggccctgccc cctcgc                               336
<210> 20
   <211> 108
   <212> PRT
   <213> Artificial Sequence
<220>
   <221> source
   <223> /note="Description of Artificial Sequence: Synthetic polypeptide"
<400> 20
   Arg Val Lys Phe Ser Arg Ser Ala Asp Ala Pro Ala Tyr Gln Gln Gly
   1               5                   10                  15
   Gln Asn Gln Leu Tyr Asn Glu Leu Asn Leu Gly Arg Arg Glu Glu Tyr
               20                  25                  30
   Asp Val Leu Asp Lys Arg Arg Gly Arg Asp Pro Glu Met Gly Gly Lys
           35                  40                  45
   Pro Arg Arg Lys Asn Pro Gln Glu Gly Asn Glu Leu Gln Lys Asp Lys
       50                  55                  60
   Met Ala Glu Ala Tyr Ser Glu Ile Gly Met Lys Gly Glu Arg Arg Arg
   65                  70                  75                  80
   Gly Lys Gly His Asp Gly Gln Gly Leu Ser Thr Ala Thr Lys Asp Thr
                   85                  90                  95
   Tyr Asp Ala Leu His Met Gln Ala Leu Pro Pro Arg
               100                 105
<210> 21
   <211> 336
   <212> DNA
   <213> Artificial Sequence
<220>
   <221> source
   <223> /note="Description of Artificial Sequence: Synthetic polynucleotide"
<400> 21
   agagtgaagt tcagcaggag cgcagacgcc cccgcgtacc agcagggcca gaaccagctc       60
   tataacgagc tcaatctagg acgaagagag gagtacgatg ttttggacaa gagacgtggc      120
   cgggaccctg agatgggggg aaagccgaga aggaagaacc ctcaggaagg cctgtacaat      180
   gaactgcaga aagataagat ggcggaggcc tacagtgaga ttgggatgaa aggcgagcgc      240
   cggaggggca aggggcacga tggcctttac cagggtctca gtacagccac caaggacacc      300
   tacgacgccc ttcacatgca ggccctgccc cctcgc                                336
<210> 22
   <211> 5
   <212> PRT
   <213> Artificial Sequence
<220>
   <221> source
   <223> /note="Description of Artificial Sequence: Synthetic peptide"
<400> 22
   Gly Gly Gly Gly Ser
   1 5
<210> 23
   <211> 30
   <212> DNA
   <213> Artificial Sequence
<220>
   <221> source
   <223> /note="Description of Artificial Sequence: Synthetic oligonucleotide"
<400> 23
   ggtggcggag gttctggagg tggaggttcc 30
<210> 24
   <211> 150
   <212> PRT
   <213> Artificial Sequence
<220>
   <221> source
   <223> /note="Description of Artificial Sequence: Synthetic polypeptide"
<400> 24
<210> 25
   <211> 450
   <212> DNA
   <213> Artificial Sequence
<220>
   <221> source
   <223> /note="Description of Artificial Sequence: Synthetic polynucleotide"
<400> 25
   cccggatggt ttctggactc tccggatcgc ccgtggaatc ccccaacctt ctcaccggca      60
   ctcttggttg tgactgaggg cgataatgcg accttcacgt gctcgttctc caacacctcc     120
   gaatcattcg tgctgaactg gtaccgcatg agcccgtcaa accagaccga caagctcgcc     180
   gcgtttccgg aagatcggtc gcaaccggga caggattgtc ggttccgcgt gactcaactg     240
   ccgaatggca gagacttcca catgagcgtg gtccgcgcta ggcgaaacga ctccgggacc     300
   tacctgtgcg gagccatctc gctggcgcct aaggcccaaa tcaaagagag cttgagggcc     360
   gaactgagag tgaccgagcg cagagctgag gtgccaactg cacatccatc cccatcgcct     420
   cggcctgcgg ggcagtttca gaccctggtc 450
<210> 26
   <211> 390
   <212> PRT
   <213> Artificial Sequence
<220>
   <221> source
   <223> /note="Description of Artificial Sequence: Synthetic polypeptide"
<400> 26
   Met Ala Leu Pro Val Thr Ala Leu Leu Leu Pro Leu Ala Leu Leu Leu
   1               5                   10                  15
   His Ala Ala Arg Pro Pro Gly Trp Phe Leu Asp Ser Pro Asp Arg Pro
               20                  25                  30
   Trp Asn Pro Pro Thr Phe Ser Pro Ala Leu Leu Val Val Thr Glu Gly
           35                  40                  45
   Asp Asn Ala Thr Phe Thr Cys Ser Phe Ser Asn Thr Ser Glu Ser Phe
       50                  55                  60
   Val Leu Asn Trp Tyr Arg Met Ser Pro Ser Asn Gln Thr Asp Lys Leu
   65                  70                  75                  80
   Ala Ala Phe Pro Glu Asp Arg Ser Gln Pro Gly Gln Asp Cys Arg Phe
                   85                  90                  95
   Arg Val Thr Gln Leu Pro Asn Gly Arg Asp Phe His Met Ser Val Val
               100                 105                 110
   Arg Ala Arg Arg Asn Asp Ser Gly Thr Tyr Leu Cys Gly Ala Ile Ser
           115                 120                 125
   Leu Ala Pro Lys Ala Gln Ile Lys Glu Ser Leu Arg Ala Glu Leu Arg
       130                 135                 140
   Val Thr Glu Arg Arg Ala Glu Val Pro Thr Ala His Pro Ser Pro Ser
   145                 150                 155                 160
   Pro Arg Pro Ala Gly Gln Phe Gln Thr Leu Val Thr Thr Thr Pro Ala
                   165                 170                 175
   Pro Arg Pro Pro Thr Pro Ala Pro Thr Ile Ala Ser Gln Pro Leu Ser
               180                 185                 190
   Leu Arg Pro Glu Ala Cys Arg Pro Ala Ala Gly Gly Ala Val His Thr
           195                 200                 205
   Arg Gly Leu Asp Phe Ala Cys Asp Ile Tyr Ile Trp Ala Pro Leu Ala
       210                 215                 220
   Gly Thr Cys Gly Val Leu Leu Leu Ser Leu Val Ile Thr Leu Tyr Cys
   225                 230                 235                 240
   Lys Arg Gly Arg Lys Lys Leu Leu Tyr Ile Phe Lys Gln Pro Phe Met
                   245                 250                 255
   Arg Pro Val Gln Thr Thr Gln Glu Glu Asp Gly Cys Ser Cys Arg Phe
               260                 265                 270
   Pro Glu Glu Glu Glu Gly Gly Cys Glu Leu Arg Val Lys Phe Ser Arg
           275                 280                 285
   Ser Ala Asp Ala Pro Ala Tyr Lys Gln Gly Gln Asn Gln Leu Tyr Asn
       290                 295                 300
   Glu Leu Asn Leu Gly Arg Arg Glu Glu Tyr Asp Val Leu Asp Lys Arg
   305                 310                 315                 320
   Arg Gly Arg Asp Pro Glu Met Gly Gly Lys Pro Arg Arg Lys Asn Pro
                   325                 330                 335
   Gln Glu Gly Asn Glu Leu Gln Lys Asp Lys Met Ala Glu Ala Tyr Ser
               340                 345                 350
   Glu Ile Gly Met Lys Gly Glu Arg Arg Arg Gly Lys Gly His Asp Gly
           355                 360                 365
   Gln Gly Leu Ser Thr Ala Thr Lys Asp Thr Tyr Asp Ala Leu His Met
       370                 375                 380
   Gln Ala Leu Pro Pro Arg
   385                 390
<210> 27
   <211> 1182
   <212> DNA
   <213> Artificial Sequence
<220>
   <221> source
   <223> /note="Description of Artificial Sequence: Synthetic polynucleotide"
<400> 27
   atggccctcc ctgtcactgc cctgcttctc cccctcgcac tcctgctcca cgccgctaga        60
   ccacccggat ggtttctgga ctctccggat cgcccgtgga atcccccaac cttctcaccg       120
   gcactcttgg ttgtgactga gggcgataat gcgaccttca cgtgctcgtt ctccaacacc       180
   tccgaatcat tcgtgctgaa ctggtaccgc atgagcccgt caaaccagac cgacaagctc       240
   gccgcgtttc cggaagatcg gtcgcaaccg ggacaggatt gtcggttccg cgtgactcaa       300
   ctgccgaatg gcagagactt ccacatgagc gtggtccgcg ctaggcgaaa cgactccggg       360
   acctacctgt gcggagccat ctcgctggcg cctaaggccc aaatcaaaga gagcttgagg       420
   gccgaactga gagtgaccga gcgcagagct gaggtgccaa ctgcacatcc atccccatcg       480
   cctcggcctg cggggcagtt tcagaccctg gtcacgacca ctccggcgcc gcgcccaccg       540
   actccggccc caactatcgc gagccagccc ctgtcgctga ggccggaagc atgccgccct       600
   gccgccggag gtgctgtgca tacccgggga ttggacttcg catgcgacat ctacatttgg       660
   gctcctctcg ccggaacttg tggcgtgctc cttctgtccc tggtcatcac cctgtactgc       720
   aagcggggtc ggaaaaagct tctgtacatt ttcaagcagc ccttcatgag gcccgtgcaa       780
   accacccagg aggaggacgg ttgctcctgc cggttccccg aagaggaaga aggaggttgc       840
   gagctgcgcg tgaagttctc ccggagcgcc gacgcccccg cctataagca gggccagaac       900
   cagctgtaca acgaactgaa cctgggacgg cgggaagagt acgatgtgct ggacaagcgg       960
   cgcggccggg accccgaaat gggcgggaag cctagaagaa agaaccctca ggaaggcctg      1020
   tataacgagc tgcagaagga caagatggcc gaggcctact ccgaaattgg gatgaaggga      1080
   gagcggcgga ggggaaaggg gcacgacggc ctgtaccaag gactgtccac cgccaccaag      1140
   gacacatacg atgccctgca catgcaggcc cttccccctc gc                         1182
<210> 28
   <211> 40
   <212> PRT
   <213> Artificial Sequence
<220>
   <221> source
   <223> /note="Description of Artificial Sequence: Synthetic polypeptide"
<220>
   <221> misc_feature
   <222> (1)..(40)
   <223> /note="This sequence may encompass 1-10 'Gly Gly Gly Ser' repeating units, wherein some positions may be absent"
<220>
   <221> source
   <223> /note="See specification as filed for detailed description of substitutions and preferred embodiments"
<400> 28
   Gly Gly Gly Ser Gly Gly Gly Ser Gly Gly Gly Ser Gly Gly Gly Ser
   1               5                   10                  15
   Gly Gly Gly Ser Gly Gly Gly Ser Gly Gly Gly Ser Gly Gly Gly Ser
               20                  25                  30
   Gly Gly Gly Ser Gly Gly Gly Ser
           35                  40
<210> 29
   <211> 20
   <212> PRT
   <213> Artificial Sequence
<220>
   <221> source
   <223> /note="Description of Artificial Sequence: Synthetic peptide"
<400> 29
   Gly Gly Gly Gly Ser Gly Gly Gly Gly Ser Gly Gly Gly Gly Ser Gly
   1               5                   10                  15
   Gly Gly Gly Ser
               20
<210> 30
   <211> 15
   <212> PRT
   <213> Artificial Sequence
<220>
   <221> source
   <223> /note="Description of Artificial Sequence: Synthetic peptide"
<400> 30
   Gly Gly Gly Gly Ser Gly Gly Gly Gly Ser Gly Gly Gly Gly Ser
   1               5                   10                  15
<210> 31
   <211> 4
   <212> PRT
   <213> Artificial Sequence
<220>
   <221> source
   <223> /note="Description of Artificial Sequence: Synthetic peptide"
<400> 31
   Gly Gly Gly Ser
   1
<210> 32
   <211> 2000
   <212> DNA
   <213> Artificial Sequence
<220>
   <221> source
   <223> /note="Description of Artificial Sequence: Synthetic polynucleotide"
<220>
   <221> misc_feature
   <222> (1)..(2000)
   <223> /note="This sequence may encompass 50-2,000 nucleotides, wherein some positions may be absent"
<400> 32
   aaaaaaaaaa aaaaaaaaaa aaaaaaaaaa aaaaaaaaaa aaaaaaaaaa aaaaaaaaaa 60
   aaaaaaaaaa aaaaaaaaaa aaaaaaaaaa aaaaaaaaaa aaaaaaaaaa aaaaaaaaaa 120
   aaaaaaaaaa aaaaaaaaaa aaaaaaaaaa aaaaaaaaaa aaaaaaaaaa aaaaaaaaaa 180
   aaaaaaaaaa aaaaaaaaaa aaaaaaaaaa aaaaaaaaaa aaaaaaaaaa aaaaaaaaaa 240
   aaaaaaaaaa aaaaaaaaaa aaaaaaaaaa aaaaaaaaaa aaaaaaaaaa aaaaaaaaaa 300
   aaaaaaaaaa aaaaaaaaaa aaaaaaaaaa aaaaaaaaaa aaaaaaaaaa aaaaaaaaaa 360
   aaaaaaaaaa aaaaaaaaaa aaaaaaaaaa aaaaaaaaaa aaaaaaaaaa aaaaaaaaaa 420
   aaaaaaaaaa aaaaaaaaaa aaaaaaaaaa aaaaaaaaaa aaaaaaaaaa aaaaaaaaaa 480
   aaaaaaaaaa aaaaaaaaaa aaaaaaaaaa aaaaaaaaaa aaaaaaaaaa aaaaaaaaaa 540
   aaaaaaaaaa aaaaaaaaaa aaaaaaaaaa aaaaaaaaaa aaaaaaaaaa aaaaaaaaaa 600
   aaaaaaaaaa aaaaaaaaaa aaaaaaaaaa aaaaaaaaaa aaaaaaaaaa aaaaaaaaaa 660
   aaaaaaaaaa aaaaaaaaaa aaaaaaaaaa aaaaaaaaaa aaaaaaaaaa aaaaaaaaaa 720
   aaaaaaaaaa aaaaaaaaaa aaaaaaaaaa aaaaaaaaaa aaaaaaaaaa aaaaaaaaaa 780
   aaaaaaaaaa aaaaaaaaaa aaaaaaaaaa aaaaaaaaaa aaaaaaaaaa aaaaaaaaaa 840
   aaaaaaaaaa aaaaaaaaaa aaaaaaaaaa aaaaaaaaaa aaaaaaaaaa aaaaaaaaaa 900
   aaaaaaaaaa aaaaaaaaaa aaaaaaaaaa aaaaaaaaaa aaaaaaaaaa aaaaaaaaaa 960
   aaaaaaaaaa aaaaaaaaaa aaaaaaaaaa aaaaaaaaaa aaaaaaaaaa aaaaaaaaaa 1020
   aaaaaaaaaa aaaaaaaaaa aaaaaaaaaa aaaaaaaaaa aaaaaaaaaa aaaaaaaaaa 1080
   aaaaaaaaaa aaaaaaaaaa aaaaaaaaaa aaaaaaaaaa aaaaaaaaaa aaaaaaaaaa 1140
   aaaaaaaaaa aaaaaaaaaa aaaaaaaaaa aaaaaaaaaa aaaaaaaaaa aaaaaaaaaa 1200
   aaaaaaaaaa aaaaaaaaaa aaaaaaaaaa aaaaaaaaaa aaaaaaaaaa aaaaaaaaaa 1260
   aaaaaaaaaa aaaaaaaaaa aaaaaaaaaa aaaaaaaaaa aaaaaaaaaa aaaaaaaaaa 1320
   aaaaaaaaaa aaaaaaaaaa aaaaaaaaaa aaaaaaaaaa aaaaaaaaaa aaaaaaaaaa 1380
   aaaaaaaaaa aaaaaaaaaa aaaaaaaaaa aaaaaaaaaa aaaaaaaaaa aaaaaaaaaa 1440
   aaaaaaaaaa aaaaaaaaaa aaaaaaaaaa aaaaaaaaaa aaaaaaaaaa aaaaaaaaaa    1500
   aaaaaaaaaa aaaaaaaaaa aaaaaaaaaa aaaaaaaaaa aaaaaaaaaa aaaaaaaaaa    1560
   aaaaaaaaaa aaaaaaaaaa aaaaaaaaaa aaaaaaaaaa aaaaaaaaaa aaaaaaaaaa    1620
   aaaaaaaaaa aaaaaaaaaa aaaaaaaaaa aaaaaaaaaa aaaaaaaaaa aaaaaaaaaa    1680
   aaaaaaaaaa aaaaaaaaaa aaaaaaaaaa aaaaaaaaaa aaaaaaaaaa aaaaaaaaaa    1740
   aaaaaaaaaa aaaaaaaaaa aaaaaaaaaa aaaaaaaaaa aaaaaaaaaa aaaaaaaaaa    1800
   aaaaaaaaaa aaaaaaaaaa aaaaaaaaaa aaaaaaaaaa aaaaaaaaaa aaaaaaaaaa    1860
   aaaaaaaaaa aaaaaaaaaa aaaaaaaaaa aaaaaaaaaa aaaaaaaaaa aaaaaaaaaa    1920
   aaaaaaaaaa aaaaaaaaaa aaaaaaaaaa aaaaaaaaaa aaaaaaaaaa aaaaaaaaaa    1980
   aaaaaaaaaa aaaaaaaaaa                                                2000
<210> 33
   <211> 150
   <212> DNA
   <213> Artificial Sequence
<220>
   <221> source
   <223> /note="Description of Artificial Sequence: Synthetic polynucleotide"
<400> 33
   aaaaaaaaaa aaaaaaaaaa aaaaaaaaaa aaaaaaaaaa aaaaaaaaaa aaaaaaaaaa      60
   aaaaaaaaaa aaaaaaaaaa aaaaaaaaaa aaaaaaaaaa aaaaaaaaaa aaaaaaaaaa     120
   aaaaaaaaaa aaaaaaaaaa aaaaaaaaaa                                      150
<210> 34
   <211> 5000
   <212> DNA
   <213> Artificial Sequence
<220>
   <221> source
   <223> /note="Description of Artificial Sequence: Synthetic polynucleotide"
<400> 34
   aaaaaaaaaa aaaaaaaaaa aaaaaaaaaa aaaaaaaaaa aaaaaaaaaa aaaaaaaaaa      60
   aaaaaaaaaa aaaaaaaaaa aaaaaaaaaa aaaaaaaaaa aaaaaaaaaa aaaaaaaaaa     120
   aaaaaaaaaa aaaaaaaaaa aaaaaaaaaa aaaaaaaaaa aaaaaaaaaa aaaaaaaaaa     180
   aaaaaaaaaa aaaaaaaaaa aaaaaaaaaa aaaaaaaaaa aaaaaaaaaa aaaaaaaaaa     240
   aaaaaaaaaa aaaaaaaaaa aaaaaaaaaa aaaaaaaaaa aaaaaaaaaa aaaaaaaaaa     300
   aaaaaaaaaa aaaaaaaaaa aaaaaaaaaa aaaaaaaaaa aaaaaaaaaa aaaaaaaaaa     360
   aaaaaaaaaa aaaaaaaaaa aaaaaaaaaa aaaaaaaaaa aaaaaaaaaa aaaaaaaaaa 420
   aaaaaaaaaa aaaaaaaaaa aaaaaaaaaa aaaaaaaaaa aaaaaaaaaa aaaaaaaaaa 480
   aaaaaaaaaa aaaaaaaaaa aaaaaaaaaa aaaaaaaaaa aaaaaaaaaa aaaaaaaaaa 540
   aaaaaaaaaa aaaaaaaaaa aaaaaaaaaa aaaaaaaaaa aaaaaaaaaa aaaaaaaaaa 600
   aaaaaaaaaa aaaaaaaaaa aaaaaaaaaa aaaaaaaaaa aaaaaaaaaa aaaaaaaaaa 660
   aaaaaaaaaa aaaaaaaaaa aaaaaaaaaa aaaaaaaaaa aaaaaaaaaa aaaaaaaaaa 720
   aaaaaaaaaa aaaaaaaaaa aaaaaaaaaa aaaaaaaaaa aaaaaaaaaa aaaaaaaaaa 780
   aaaaaaaaaa aaaaaaaaaa aaaaaaaaaa aaaaaaaaaa aaaaaaaaaa aaaaaaaaaa 840
   aaaaaaaaaa aaaaaaaaaa aaaaaaaaaa aaaaaaaaaa aaaaaaaaaa aaaaaaaaaa 900
   aaaaaaaaaa aaaaaaaaaa aaaaaaaaaa aaaaaaaaaa aaaaaaaaaa aaaaaaaaaa 960
   aaaaaaaaaa aaaaaaaaaa aaaaaaaaaa aaaaaaaaaa aaaaaaaaaa aaaaaaaaaa 1020
   aaaaaaaaaa aaaaaaaaaa aaaaaaaaaa aaaaaaaaaa aaaaaaaaaa aaaaaaaaaa 1080
   aaaaaaaaaa aaaaaaaaaa aaaaaaaaaa aaaaaaaaaa aaaaaaaaaa aaaaaaaaaa 1140
   aaaaaaaaaa aaaaaaaaaa aaaaaaaaaa aaaaaaaaaa aaaaaaaaaa aaaaaaaaaa 1200
   aaaaaaaaaa aaaaaaaaaa aaaaaaaaaa aaaaaaaaaa aaaaaaaaaa aaaaaaaaaa 1260
   aaaaaaaaaa aaaaaaaaaa aaaaaaaaaa aaaaaaaaaa aaaaaaaaaa aaaaaaaaaa 1320
   aaaaaaaaaa aaaaaaaaaa aaaaaaaaaa aaaaaaaaaa aaaaaaaaaa aaaaaaaaaa 1380
   aaaaaaaaaa aaaaaaaaaa aaaaaaaaaa aaaaaaaaaa aaaaaaaaaa aaaaaaaaaa 1440
   aaaaaaaaaa aaaaaaaaaa aaaaaaaaaa aaaaaaaaaa aaaaaaaaaa aaaaaaaaaa 1500
   aaaaaaaaaa aaaaaaaaaa aaaaaaaaaa aaaaaaaaaa aaaaaaaaaa aaaaaaaaaa 1560
   aaaaaaaaaa aaaaaaaaaa aaaaaaaaaa aaaaaaaaaa aaaaaaaaaa aaaaaaaaaa 1620
   aaaaaaaaaa aaaaaaaaaa aaaaaaaaaa aaaaaaaaaa aaaaaaaaaa aaaaaaaaaa 1680
   aaaaaaaaaa aaaaaaaaaa aaaaaaaaaa aaaaaaaaaa aaaaaaaaaa aaaaaaaaaa 1740
   aaaaaaaaaa aaaaaaaaaa aaaaaaaaaa aaaaaaaaaa aaaaaaaaaa aaaaaaaaaa 1800
   aaaaaaaaaa aaaaaaaaaa aaaaaaaaaa aaaaaaaaaa aaaaaaaaaa aaaaaaaaaa 1860
   aaaaaaaaaa aaaaaaaaaa aaaaaaaaaa aaaaaaaaaa aaaaaaaaaa aaaaaaaaaa 1920
   aaaaaaaaaa aaaaaaaaaa aaaaaaaaaa aaaaaaaaaa aaaaaaaaaa aaaaaaaaaa 1980
   aaaaaaaaaa aaaaaaaaaa aaaaaaaaaa aaaaaaaaaa aaaaaaaaaa aaaaaaaaaa 2040
   aaaaaaaaaa aaaaaaaaaa aaaaaaaaaa aaaaaaaaaa aaaaaaaaaa aaaaaaaaaa 2100
   aaaaaaaaaa aaaaaaaaaa aaaaaaaaaa aaaaaaaaaa aaaaaaaaaa aaaaaaaaaa 2160
   aaaaaaaaaa aaaaaaaaaa aaaaaaaaaa aaaaaaaaaa aaaaaaaaaa aaaaaaaaaa 2220
   aaaaaaaaaa aaaaaaaaaa aaaaaaaaaa aaaaaaaaaa aaaaaaaaaa aaaaaaaaaa 2280
   aaaaaaaaaa aaaaaaaaaa aaaaaaaaaa aaaaaaaaaa aaaaaaaaaa aaaaaaaaaa 2340
   aaaaaaaaaa aaaaaaaaaa aaaaaaaaaa aaaaaaaaaa aaaaaaaaaa aaaaaaaaaa 2400
   aaaaaaaaaa aaaaaaaaaa aaaaaaaaaa aaaaaaaaaa aaaaaaaaaa aaaaaaaaaa 2460
   aaaaaaaaaa aaaaaaaaaa aaaaaaaaaa aaaaaaaaaa aaaaaaaaaa aaaaaaaaaa 2520
   aaaaaaaaaa aaaaaaaaaa aaaaaaaaaa aaaaaaaaaa aaaaaaaaaa aaaaaaaaaa 2580
   aaaaaaaaaa aaaaaaaaaa aaaaaaaaaa aaaaaaaaaa aaaaaaaaaa aaaaaaaaaa 2640
   aaaaaaaaaa aaaaaaaaaa aaaaaaaaaa aaaaaaaaaa aaaaaaaaaa aaaaaaaaaa 2700
   aaaaaaaaaa aaaaaaaaaa aaaaaaaaaa aaaaaaaaaa aaaaaaaaaa aaaaaaaaaa 2760
   aaaaaaaaaa aaaaaaaaaa aaaaaaaaaa aaaaaaaaaa aaaaaaaaaa aaaaaaaaaa 2820
   aaaaaaaaaa aaaaaaaaaa aaaaaaaaaa aaaaaaaaaa aaaaaaaaaa aaaaaaaaaa 2880
   aaaaaaaaaa aaaaaaaaaa aaaaaaaaaa aaaaaaaaaa aaaaaaaaaa aaaaaaaaaa 2940
   aaaaaaaaaa aaaaaaaaaa aaaaaaaaaa aaaaaaaaaa aaaaaaaaaa aaaaaaaaaa 3000
   aaaaaaaaaa aaaaaaaaaa aaaaaaaaaa aaaaaaaaaa aaaaaaaaaa aaaaaaaaaa 3060
   aaaaaaaaaa aaaaaaaaaa aaaaaaaaaa aaaaaaaaaa aaaaaaaaaa aaaaaaaaaa 3120
   aaaaaaaaaa aaaaaaaaaa aaaaaaaaaa aaaaaaaaaa aaaaaaaaaa aaaaaaaaaa 3180
   aaaaaaaaaa aaaaaaaaaa aaaaaaaaaa aaaaaaaaaa aaaaaaaaaa aaaaaaaaaa 3240
   aaaaaaaaaa aaaaaaaaaa aaaaaaaaaa aaaaaaaaaa aaaaaaaaaa aaaaaaaaaa 3300
   aaaaaaaaaa aaaaaaaaaa aaaaaaaaaa aaaaaaaaaa aaaaaaaaaa aaaaaaaaaa 3360
   aaaaaaaaaa aaaaaaaaaa aaaaaaaaaa aaaaaaaaaa aaaaaaaaaa aaaaaaaaaa 3420
   aaaaaaaaaa aaaaaaaaaa aaaaaaaaaa aaaaaaaaaa aaaaaaaaaa aaaaaaaaaa 3480
   aaaaaaaaaa aaaaaaaaaa aaaaaaaaaa aaaaaaaaaa aaaaaaaaaa aaaaaaaaaa 3540
   aaaaaaaaaa aaaaaaaaaa aaaaaaaaaa aaaaaaaaaa aaaaaaaaaa aaaaaaaaaa 3600
   aaaaaaaaaa aaaaaaaaaa aaaaaaaaaa aaaaaaaaaa aaaaaaaaaa aaaaaaaaaa 3660
   aaaaaaaaaa aaaaaaaaaa aaaaaaaaaa aaaaaaaaaa aaaaaaaaaa aaaaaaaaaa 3720
   aaaaaaaaaa aaaaaaaaaa aaaaaaaaaa aaaaaaaaaa aaaaaaaaaa aaaaaaaaaa 3780
   aaaaaaaaaa aaaaaaaaaa aaaaaaaaaa aaaaaaaaaa aaaaaaaaaa aaaaaaaaaa 3840
   aaaaaaaaaa aaaaaaaaaa aaaaaaaaaa aaaaaaaaaa aaaaaaaaaa aaaaaaaaaa 3900
   aaaaaaaaaa aaaaaaaaaa aaaaaaaaaa aaaaaaaaaa aaaaaaaaaa aaaaaaaaaa 3960
   aaaaaaaaaa aaaaaaaaaa aaaaaaaaaa aaaaaaaaaa aaaaaaaaaa aaaaaaaaaa 4020
   aaaaaaaaaa aaaaaaaaaa aaaaaaaaaa aaaaaaaaaa aaaaaaaaaa aaaaaaaaaa 4080
   aaaaaaaaaa aaaaaaaaaa aaaaaaaaaa aaaaaaaaaa aaaaaaaaaa aaaaaaaaaa 4140
   aaaaaaaaaa aaaaaaaaaa aaaaaaaaaa aaaaaaaaaa aaaaaaaaaa aaaaaaaaaa 4200
   aaaaaaaaaa aaaaaaaaaa aaaaaaaaaa aaaaaaaaaa aaaaaaaaaa aaaaaaaaaa 4260
   aaaaaaaaaa aaaaaaaaaa aaaaaaaaaa aaaaaaaaaa aaaaaaaaaa aaaaaaaaaa 4320
   aaaaaaaaaa aaaaaaaaaa aaaaaaaaaa aaaaaaaaaa aaaaaaaaaa aaaaaaaaaa 4380
   aaaaaaaaaa aaaaaaaaaa aaaaaaaaaa aaaaaaaaaa aaaaaaaaaa aaaaaaaaaa 4440
   aaaaaaaaaa aaaaaaaaaa aaaaaaaaaa aaaaaaaaaa aaaaaaaaaa aaaaaaaaaa 4500
   aaaaaaaaaa aaaaaaaaaa aaaaaaaaaa aaaaaaaaaa aaaaaaaaaa aaaaaaaaaa 4560
   aaaaaaaaaa aaaaaaaaaa aaaaaaaaaa aaaaaaaaaa aaaaaaaaaa aaaaaaaaaa 4620
   aaaaaaaaaa aaaaaaaaaa aaaaaaaaaa aaaaaaaaaa aaaaaaaaaa aaaaaaaaaa 4680
   aaaaaaaaaa aaaaaaaaaa aaaaaaaaaa aaaaaaaaaa aaaaaaaaaa aaaaaaaaaa 4740
   aaaaaaaaaa aaaaaaaaaa aaaaaaaaaa aaaaaaaaaa aaaaaaaaaa aaaaaaaaaa 4800
   aaaaaaaaaa aaaaaaaaaa aaaaaaaaaa aaaaaaaaaa aaaaaaaaaa aaaaaaaaaa 4860
   aaaaaaaaaa aaaaaaaaaa aaaaaaaaaa aaaaaaaaaa aaaaaaaaaa aaaaaaaaaa 4920
   aaaaaaaaaa aaaaaaaaaa aaaaaaaaaa aaaaaaaaaa aaaaaaaaaa aaaaaaaaaa 4980
   aaaaaaaaaa aaaaaaaaaa 5000
<210> 35
   <211> 100
   <212> DNA
   <213> Artificial Sequence
<220>
   <221> source
   <223> /note="Description of Artificial Sequence: Synthetic polynucleotide"
<400> 35
   tttttttttt tttttttttt tttttttttt tttttttttt tttttttttt tttttttttt 60
   tttttttttt tttttttttt tttttttttt tttttttttt 100
<210> 36
   <211> 500
   <212> DNA
   <213> Artificial Sequence
<220>
   <221> source
   <223> /note="Description of Artificial Sequence: Synthetic polynucleotide"
<400> 36
   tttttttttt tttttttttt tttttttttt tttttttttt tttttttttt tttttttttt 60
   tttttttttt tttttttttt tttttttttt tttttttttt tttttttttt tttttttttt 120
   tttttttttt tttttttttt tttttttttt tttttttttt tttttttttt tttttttttt 180
   tttttttttt tttttttttt tttttttttt tttttttttt tttttttttt tttttttttt 240
   tttttttttt tttttttttt tttttttttt tttttttttt tttttttttt tttttttttt 300
   tttttttttt tttttttttt tttttttttt tttttttttt tttttttttt tttttttttt 360
   tttttttttt tttttttttt tttttttttt tttttttttt tttttttttt tttttttttt 420
   tttttttttt tttttttttt tttttttttt tttttttttt tttttttttt tttttttttt 480
   tttttttttt tttttttttt 500
<210> 37
   <211> 64
   <212> DNA
   <213> Artificial Sequence
<220>
   <221> source
   <223> /note="Description of Artificial Sequence: Synthetic oligonucleotide"
<400> 37
   aaaaaaaaaa aaaaaaaaaa aaaaaaaaaa aaaaaaaaaa aaaaaaaaaa aaaaaaaaaa 60
   aaaa 64
<210> 38
   <211> 400
   <212> DNA
   <213> Artificial Sequence
<220>
   <221> source
   <223> /note="Description of Artificial Sequence: Synthetic polynucleotide"
<220>
   <221> misc_feature
   <222> (1)..(400)
   <223> /note="This sequence may encompass 100-400 nucleotides, wherein some positions may be absent"
<220>
   <221> source
   <223> /note="See specification as filed for detailed description of substitutions and preferred embodiments"
<400> 38
   aaaaaaaaaa aaaaaaaaaa aaaaaaaaaa aaaaaaaaaa aaaaaaaaaa aaaaaaaaaa 60
   aaaaaaaaaa aaaaaaaaaa aaaaaaaaaa aaaaaaaaaa aaaaaaaaaa aaaaaaaaaa 120
   aaaaaaaaaa aaaaaaaaaa aaaaaaaaaa aaaaaaaaaa aaaaaaaaaa aaaaaaaaaa 180
   aaaaaaaaaa aaaaaaaaaa aaaaaaaaaa aaaaaaaaaa aaaaaaaaaa aaaaaaaaaa 240
   aaaaaaaaaa aaaaaaaaaa aaaaaaaaaa aaaaaaaaaa aaaaaaaaaa aaaaaaaaaa 300
   aaaaaaaaaa aaaaaaaaaa aaaaaaaaaa aaaaaaaaaa aaaaaaaaaa aaaaaaaaaa 360
   aaaaaaaaaa aaaaaaaaaa aaaaaaaaaa aaaaaaaaaa 400
<210> 39
   <211> 369
   <212> PRT
   <213> Artificial Sequence
<220>
   <221> source
   <223> /note="Description of Artificial Sequence: Synthetic polypeptide"
<400> 39
   Pro Gly Trp Phe Leu Asp Ser Pro Asp Arg Pro Trp Asn Pro Pro Thr
   1               5                   10                  15
   Phe Ser Pro Ala Leu Leu Val Val Thr Glu Gly Asp Asn Ala Thr Phe
               20                  25                  30
   Thr Cys Ser Phe Ser Asn Thr Ser Glu Ser Phe Val Leu Asn Trp Tyr
           35                  40                  45
   Arg Met Ser Pro Ser Asn Gln Thr Asp Lys Leu Ala Ala Phe Pro Glu
       50                  55                  60
   Asp Arg Ser Gln Pro Gly Gln Asp Cys Arg Phe Arg Val Thr Gln Leu
   65                  70                  75                  80
   Pro Asn Gly Arg Asp Phe His Met Ser Val Val Arg Ala Arg Arg Asn
                   85                  90                  95
   Asp Ser Gly Thr Tyr Leu Cys Gly Ala Ile Ser Leu Ala Pro Lys Ala
               100                 105                 110
   Gln Ile Lys Glu Ser Leu Arg Ala Glu Leu Arg Val Thr Glu Arg Arg
           115                 120                 125
   Ala Glu Val Pro Thr Ala His Pro Ser Pro Ser Pro Arg Pro Ala Gly
       130                 135                 140
   Gln Phe Gln Thr Leu Val Thr Thr Thr Pro Ala Pro Arg Pro Pro Thr
   145                 150                 155                 160
   Pro Ala Pro Thr Ile Ala Ser Gln Pro Leu Ser Leu Arg Pro Glu Ala
                   165                 170                 175
   Cys Arg Pro Ala Ala Gly Gly Ala Val His Thr Arg Gly Leu Asp Phe
               180                 185                 190
   Ala Cys Asp Ile Tyr Ile Trp Ala Pro Leu Ala Gly Thr Cys Gly Val
           195                 200                 205
   Leu Leu Leu Ser Leu Val Ile Thr Leu Tyr Cys Lys Arg Gly Arg Lys
       210                 215                 220
   Lys Leu Leu Tyr Ile Phe Lys Gln Pro Phe Met Arg Pro Val Gln Thr
   225                 230                 235                 240
   Thr Gln Glu Glu Asp Gly Cys Ser Cys Arg Phe Pro Glu Glu Glu Glu
                   245                 250                 255
   Gly Gly Cys Glu Leu Arg Val Lys Phe Ser Arg Ser Ala Asp Ala Pro
               260                 265                 270
   Ala Tyr Lys Gln Gly Gln Asn Gln Leu Tyr Asn Glu Leu Asn Leu Gly
           275                 280                 285
   Arg Arg Glu Glu Tyr Asp Val Leu Asp Lys Arg Arg Gly Arg Asp Pro
       290                 295                 300
   Glu Met Gly Gly Lys Pro Arg Arg Lys Asn Pro Gln Glu Gly Asn Glu
   305                 310                 315                 320
   Leu Gln Lys Asp Lys Met Ala Glu Ala Tyr Ser Glu Ile Gly Met Lys
                   325                 330                 335
   Gly Glu Arg Arg Arg Gly Lys Gly His Asp Gly Gln Gly Leu Ser Thr
               340                 345                 350
   Ala Thr Lys Asp Thr Tyr Asp Ala Leu His Met Gln Ala Leu Pro Pro
           355                 360                 365
   Arg
<210> 40
   <211> 35
   <212> PRT
   <213> Homo sapiens
<400> 40
   Thr Lys Lys Lys Tyr Ser Ser Ser Val His Asp Pro Asn Gly Glu Tyr
   1               5                   10                  15
   Met Phe Met Arg Ala Val Asn Thr Ala Lys Lys Ser Arg Leu Thr Asp
               20                  25                  30
   Val Thr Leu
   35
<210> 41
   <211> 105
   <212> DNA
   <213> Artificial Sequence
<220>
   <221> source
   <223> /note="Description of Artificial Sequence: Synthetic polynucleotide"
<400> 41
   acaaaaaaga agtattcatc cagtgtgcac gaccctaacg gtgaatacat gttcatgaga 60
   gcagtgaaca cagccaaaaa atccagactc acagatgtga cccta 105
<210> 42
   <211> 69
   <212> PRT
   <213> Artificial Sequence
<220>
   <221> source
   <223> /note="Description of Artificial Sequence: Synthetic polypeptide"
<400> 42
   Thr Thr Thr Pro Ala Pro Arg Pro Pro Thr Pro Ala Pro Thr Ile Ala
   1 5 10 15
   Ser Gln Pro Leu Ser Leu Arg Pro Glu Ala Cys Arg Pro Ala Ala Gly
   20 25 30
   Gly Ala Val His Thr Arg Gly Leu Asp Phe Ala Cys Asp Phe Trp Leu
   35 40 45
   Pro Ile Gly Cys Ala Ala Phe Val Val Val Cys Ile Leu Gly Cys Ile
   50 55 60
   Leu Ile Cys Trp Leu
   65
<210> 43
   <211> 207
   <212> DNA
   <213> Artificial Sequence
<220>
   <221> source
   <223> /note="Description of Artificial Sequence: Synthetic polynucleotide"
<400> 43
   accacgacgc cagcgccgcg accaccaaca ccggcgccca ccatcgcgtc gcagcccctg 60
   tccctgcgcc cagaggcgtg ccggccagcg gcggggggcg cagtgcacac gagggggctg 120
   gacttcgcct gtgatttctg gttacccata ggatgtgcag cctttgttgt agtctgcatt 180
   ttgggatgca tacttatttg ttggctt 207
<210> 44
   <211> 41
   <212> PRT
   <213> Artificial Sequence
<220>
   <221> source
   <223> /note="Description of Artificial Sequence: Synthetic polypeptide"
<400> 44
   Arg Ser Lys Arg Ser Arg Leu Leu His Ser Asp Tyr Met Asn Met Thr
   1 5 10 15
   Pro Arg Arg Pro Gly Pro Thr Arg Lys His Tyr Gln Pro Tyr Ala Pro
   20 25 30
   Pro Arg Asp Phe Ala Ala Tyr Arg Ser
   35 40
<210> 45
   <211> 123
   <212> DNA
   <213> Artificial Sequence
<220>
   <221> source
   <223> /note="Description of Artificial Sequence: Synthetic polynucleotide"
<400> 45
   aggagtaaga ggagcaggct cctgcacagt gactacatga acatgactcc ccgccgcccc 60
   gggcccaccc gcaagcatta ccagccctat gccccaccac gcgacttcgc agcctatcgc 120
   tcc 123
<210> 46
   <211> 35
   <212> PRT
   <213> Artificial Sequence
<220>
   <221> source
   <223> /note="Description of Artificial Sequence: Synthetic polypeptide"
<400> 46
   Thr Lys Lys Lys Tyr Ser Ser Ser Val His Asp Pro Asn Gly Glu Phe
   1               5                   10                  15
   Met Phe Met Arg Ala Val Asn Thr Ala Lys Lys Ser Arg Leu Thr Asp
               20                  25                  30
   Val Thr Leu
           35
<210> 47
   <211> 4
   <212> PRT
   <213> Artificial Sequence
<220>
   <221> source
   <223> /note="Description of Artificial Sequence: Synthetic peptide"
<400> 47
   Phe Met Phe Met
   1
<210> 48
   <211> 242
   <212> PRT
   <213> Artificial Sequence
<220>
   <221> source
   <223> /note="Description of Artificial Sequence: Synthetic polypeptide"
<400> 48
   Glu Ile Val Met Thr Gln Ser Pro Ala Thr Leu Ser Leu Ser Pro Gly
   1               5                   10                  15
   Glu Arg Ala Thr Leu Ser Cys Arg Ala Ser Gln Asp Ile Ser Lys Tyr
               20                  25                  30
   Leu Asn Trp Tyr Gln Gln Lys Pro Gly Gln Ala Pro Arg Leu Leu Ile
           35                  40                  45
   Tyr His Thr Ser Arg Leu His Ser Gly Ile Pro Ala Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Val Tyr Phe Cys Gln Gln Gly Asn Thr Leu Pro Tyr
                   85                  90                  95
   Thr Phe Gly Gln Gly Thr Lys Leu Glu Ile Lys Gly Gly Gly Gly Ser
               100                 105                 110
   Gly Gly Gly Gly Ser Gly Gly Gly Gly Ser Gln Val Gln Leu Gln Glu
           115                 120                 125
   Ser Gly Pro Gly Leu Val Lys Pro Ser Glu Thr Leu Ser Leu Thr Cys
       130                 135                 140
   Thr Val Ser Gly Val Ser Leu Pro Asp Tyr Gly Val Ser Trp Ile Arg
   145                 150                 155                 160
   Gln Pro Pro Gly Lys Gly Leu Glu Trp Ile Gly Val Ile Trp Gly Ser
                   165                 170                 175
   Glu Thr Thr Tyr Tyr Ser Ser Ser Leu Lys Ser Arg Val Thr Ile Ser
               180                 185                 190
   Lys Asp Asn Ser Lys Asn Gln Val Ser Leu Lys Leu Ser Ser Val Thr
           195                 200                 205
   Ala Ala Asp Thr Ala Val Tyr Tyr Cys Ala Lys His Tyr Tyr Tyr Gly
       210                 215                 220
   Gly Ser Tyr Ala Met Asp Tyr Trp Gly Gln Gly Thr Leu Val Thr Val
   225                 230                 235                 240
   Ser Ser
<210> 49
   <211> 242
   <212> PRT
   <213> Artificial Sequence
<220>
   <221> source
   <223> /note="Description of Artificial Sequence: Synthetic polypeptide"
<400> 49
   Glu Ile Val Met Thr Gln Ser Pro Ala Thr Leu Ser Leu Ser Pro Gly
   1               5                   10                  15
   Glu Arg Ala Thr Leu Ser Cys Arg Ala Ser Gln Asp Ile Ser Lys Tyr
               20                  25                  30
   Leu Asn Trp Tyr Gln Gln Lys Pro Gly Gln Ala Pro Arg Leu Leu Ile
           35                  40                  45
   Tyr His Thr Ser Arg Leu His Ser Gly Ile Pro Ala Arg Phe Ser Gly
       50                  55                  60
   Ser Gly Ser Gly Thr Asp Tyr Thr Leu Thr Ile Ser Ser Leu Gln Pro
   65 70 75 80
   Glu Asp Phe Ala Val Tyr Phe Cys Gln Gln Gly Asn Thr Leu Pro Tyr
   85 90 95
   Thr Phe Gly Gln Gly Thr Lys Leu Glu Ile Lys Gly Gly Gly Gly Ser
   100 105 110
   Gly Gly Gly Gly Ser Gly Gly Gly Gly Ser Gln Val Gln Leu Gln Glu
   115 120 125
   Ser Gly Pro Gly Leu Val Lys Pro Ser Glu Thr Leu Ser Leu Thr Cys
   130 135 140
   Thr Val Ser Gly Val Ser Leu Pro Asp Tyr Gly Val Ser Trp Ile Arg
   145 150 155 160
   Gln Pro Pro Gly Lys Gly Leu Glu Trp Ile Gly Val Ile Trp Gly Ser
   165 170 175
   Glu Thr Thr Tyr Tyr Gln Ser Ser Leu Lys Ser Arg Val Thr Ile Ser
   180 185 190
   Lys Asp Asn Ser Lys Asn Gln Val Ser Leu Lys Leu Ser Ser Val Thr
   195 200 205
   Ala Ala Asp Thr Ala Val Tyr Tyr Cys Ala Lys His Tyr Tyr Tyr Gly
   210 215 220
   Gly Ser Tyr Ala Met Asp Tyr Trp Gly Gln Gly Thr Leu Val Thr Val
   225 230 235 240
   Ser Ser
<210> 50
   <211> 242
   <212> PRT
   <213> Artificial Sequence
<220>
   <221> source
   <223> /note="Description of Artificial Sequence: Synthetic polypeptide"
<400> 50
   Gln Val Gln Leu Gln Glu Ser Gly Pro Gly Leu Val Lys Pro Ser Glu
   1 5 10 15
   Thr Leu Ser Leu Thr Cys Thr Val Ser Gly Val Ser Leu Pro Asp Tyr
   20 25 30
   Gly Val Ser Trp Ile Arg Gln Pro Pro Gly Lys Gly Leu Glu Trp Ile
   35 40 45
   Gly Val Ile Trp Gly Ser Glu Thr Thr Tyr Tyr Ser Ser Ser Leu Lys
   50 55 60
   Ser Arg Val Thr Ile Ser Lys Asp Asn Ser Lys Asn Gln Val Ser Leu
   65 70 75 80
   Lys Leu Ser Ser Val Thr Ala Ala Asp Thr Ala Val Tyr Tyr Cys Ala
   85 90 95
   Lys His Tyr Tyr Tyr Gly Gly Ser Tyr Ala Met Asp Tyr Trp Gly Gln
   100 105 110
   Gly Thr Leu Val Thr Val Ser Ser Gly Gly Gly Gly Ser Gly Gly Gly
   115 120 125
   Gly Ser Gly Gly Gly Gly Ser Glu Ile Val Met Thr Gln Ser Pro Ala
   130 135 140
   Thr Leu Ser Leu Ser Pro Gly Glu Arg Ala Thr Leu Ser Cys Arg Ala
   145 150 155 160
   Ser Gln Asp Ile Ser Lys Tyr Leu Asn Trp Tyr Gln Gln Lys Pro Gly
   165 170 175
   Gln Ala Pro Arg Leu Leu Ile Tyr His Thr Ser Arg Leu His Ser Gly
   180 185 190
   Ile Pro Ala Arg Phe Ser Gly Ser Gly Ser Gly Thr Asp Tyr Thr Leu
   195 200 205
   Thr Ile Ser Ser Leu Gln Pro Glu Asp Phe Ala Val Tyr Phe Cys Gln
   210 215 220
   Gln Gly Asn Thr Leu Pro Tyr Thr Phe Gly Gln Gly Thr Lys Leu Glu
   225 230 235 240
   Ile Lys
<210> 51
   <211> 242
   <212> PRT
   <213> Artificial Sequence
<220>
   <221> source
   <223> /note="Description of Artificial Sequence: Synthetic polypeptide"
<400> 51
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Val Ser Leu Pro Asp Tyr
               20                  25                  30
   Gly Val Ser Trp Ile Arg Gln Pro Pro Gly Lys Gly Leu Glu Trp Ile
           35                  40                  45
   Gly Val Ile Trp Gly Ser Glu Thr Thr Tyr Tyr Gln Ser Ser Leu Lys
       50                  55                  60
   Ser Arg Val Thr Ile Ser Lys Asp Asn Ser Lys Asn Gln Val Ser Leu
   65                  70                  75                  80
   Lys Leu Ser Ser Val Thr Ala Ala Asp Thr Ala Val Tyr Tyr Cys Ala
                   85                  90                  95
   Lys His Tyr Tyr Tyr Gly Gly Ser Tyr Ala Met Asp Tyr Trp Gly Gln
               100                 105                 110
   Gly Thr Leu Val Thr Val Ser Ser Gly Gly Gly Gly Ser Gly Gly Gly
           115                 120                 125
   Gly Ser Gly Gly Gly Gly Ser Glu Ile Val Met Thr Gln Ser Pro Ala
       130                 135                 140
   Thr Leu Ser Leu Ser Pro Gly Glu Arg Ala Thr Leu Ser Cys Arg Ala
   145                 150                 155                 160
   Ser Gln Asp Ile Ser Lys Tyr Leu Asn Trp Tyr Gln Gln Lys Pro Gly
                   165                 170                 175
   Gln Ala Pro Arg Leu Leu Ile Tyr His Thr Ser Arg Leu His Ser Gly
               180                 185                 190
   Ile Pro Ala Arg Phe Ser Gly Ser Gly Ser Gly Thr Asp Tyr Thr Leu
           195                 200                 205
   Thr Ile Ser Ser Leu Gln Pro Glu Asp Phe Ala Val Tyr Phe Cys Gln
       210                 215                 220
   Gln Gly Asn Thr Leu Pro Tyr Thr Phe Gly Gln Gly Thr Lys Leu Glu
   225                 230                 235                 240
   Ile Lys
<210> 52
   <211> 247
   <212> PRT
   <213> Artificial Sequence
<220>
   <221> source
   <223> /note="Description of Artificial Sequence: Synthetic polypeptide"
<400> 52
   Glu Ile Val Met Thr Gln Ser Pro Ala Thr Leu Ser Leu Ser Pro Gly
   1               5                   10                  15
   Glu Arg Ala Thr Leu Ser Cys Arg Ala Ser Gln Asp Ile Ser Lys Tyr
               20                  25                  30
   Leu Asn Trp Tyr Gln Gln Lys Pro Gly Gln Ala Pro Arg Leu Leu Ile
           35                  40                  45
   Tyr His Thr Ser Arg Leu His Ser Gly Ile Pro Ala Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Val Tyr Phe Cys Gln Gln Gly Asn Thr Leu Pro Tyr
                   85                  90                  95
   Thr Phe Gly Gln Gly Thr Lys Leu Glu Ile Lys Gly Gly Gly Gly Ser
               100                 105                 110
   Gly Gly Gly Gly Ser Gly Gly Gly Gly Ser Gly Gly Gly Gly Ser Gln
           115                 120                 125
   Val Gln Leu Gln Glu Ser Gly Pro Gly Leu Val Lys Pro Ser Glu Thr
       130                 135                 140
   Leu Ser Leu Thr Cys Thr Val Ser Gly Val Ser Leu Pro Asp Tyr Gly
   145                 150                 155                 160
   Val Ser Trp Ile Arg Gln Pro Pro Gly Lys Gly Leu Glu Trp Ile Gly
                   165                 170                 175
   Val Ile Trp Gly Ser Glu Thr Thr Tyr Tyr Ser Ser Ser Leu Lys Ser
               180                 185                 190
   Arg Val Thr Ile Ser Lys Asp Asn Ser Lys Asn Gln Val Ser Leu Lys
           195                 200                 205
   Leu Ser Ser Val Thr Ala Ala Asp Thr Ala Val Tyr Tyr Cys Ala Lys
       210                 215                 220
   His Tyr Tyr Tyr Gly Gly Ser Tyr Ala Met Asp Tyr Trp Gly Gln Gly
   225                 230                 235                 240
   Thr Leu Val Thr Val Ser Ser
                   245
<210> 53
   <211> 247
   <212> PRT
   <213> Artificial Sequence
<220>
   <221> source
   <223> /note="Description of Artificial Sequence: Synthetic polypeptide"
<400> 53
   Glu Ile Val Met Thr Gln Ser Pro Ala Thr Leu Ser Leu Ser Pro Gly
   1               5                   10                  15
   Glu Arg Ala Thr Leu Ser Cys Arg Ala Ser Gln Asp Ile Ser Lys Tyr
               20                  25                  30
   Leu Asn Trp Tyr Gln Gln Lys Pro Gly Gln Ala Pro Arg Leu Leu Ile
           35                  40                  45
   Tyr His Thr Ser Arg Leu His Ser Gly Ile Pro Ala Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Val Tyr Phe Cys Gln Gln Gly Asn Thr Leu Pro Tyr
                   85                  90                  95
   Thr Phe Gly Gln Gly Thr Lys Leu Glu Ile Lys Gly Gly Gly Gly Ser
               100                 105                 110
   Gly Gly Gly Gly Ser Gly Gly Gly Gly Ser Gly Gly Gly Gly Ser Gln
           115                 120                 125
   Val Gln Leu Gln Glu Ser Gly Pro Gly Leu Val Lys Pro Ser Glu Thr
       130                 135                 140
   Leu Ser Leu Thr Cys Thr Val Ser Gly Val Ser Leu Pro Asp Tyr Gly
   145                 150                 155                 160
   Val Ser Trp Ile Arg Gln Pro Pro Gly Lys Gly Leu Glu Trp Ile Gly
                   165                 170                 175
   Val Ile Trp Gly Ser Glu Thr Thr Tyr Tyr Gln Ser Ser Leu Lys Ser
               180                 185                 190
   Arg Val Thr Ile Ser Lys Asp Asn Ser Lys Asn Gln Val Ser Leu Lys
           195                 200                 205
   Leu Ser Ser Val Thr Ala Ala Asp Thr Ala Val Tyr Tyr Cys Ala Lys
       210                 215                 220
   His Tyr Tyr Tyr Gly Gly Ser Tyr Ala Met Asp Tyr Trp Gly Gln Gly
   225                 230                 235                 240
   Thr Leu Val Thr Val Ser Ser
                   245
<210> 54
   <211> 247
   <212> PRT
   <213> Artificial Sequence
<220>
   <221> source
   <223> /note="Description of Artificial Sequence: Synthetic polypeptide"
<400> 54
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Val Ser Leu Pro Asp Tyr
               20                  25                  30
   Gly Val Ser Trp Ile Arg Gln Pro Pro Gly Lys Gly Leu Glu Trp Ile
           35                  40                  45
   Gly Val Ile Trp Gly Ser Glu Thr Thr Tyr Tyr Ser Ser Ser Leu Lys
       50                  55                  60
   Ser Arg Val Thr Ile Ser Lys Asp Asn Ser Lys Asn Gln Val Ser Leu
   65                  70                  75                  80
   Lys Leu Ser Ser Val Thr Ala Ala Asp Thr Ala Val Tyr Tyr Cys Ala
                   85                  90                  95
   Lys His Tyr Tyr Tyr Gly Gly Ser Tyr Ala Met Asp Tyr Trp Gly Gln
               100                 105                 110
   Gly Thr Leu Val Thr Val Ser Ser Gly Gly Gly Gly Ser Gly Gly Gly
           115                 120                 125
   Gly Ser Gly Gly Gly Gly Ser Gly Gly Gly Gly Ser Glu Ile Val Met
       130                 135                 140
   Thr Gln Ser Pro Ala Thr Leu Ser Leu Ser Pro Gly Glu Arg Ala Thr
   145                 150                 155                 160
   Leu Ser Cys Arg Ala Ser Gln Asp Ile Ser Lys Tyr Leu Asn Trp Tyr
                   165                 170                 175
   Gln Gln Lys Pro Gly Gln Ala Pro Arg Leu Leu Ile Tyr His Thr Ser
               180                 185                 190
   Arg Leu His Ser Gly Ile Pro Ala Arg Phe Ser Gly Ser Gly Ser Gly
           195                 200                 205
   Thr Asp Tyr Thr Leu Thr Ile Ser Ser Leu Gln Pro Glu Asp Phe Ala
       210                 215                 220
   Val Tyr Phe Cys Gln Gln Gly Asn Thr Leu Pro Tyr Thr Phe Gly Gln
   225                 230                 235                 240
   Gly Thr Lys Leu Glu Ile Lys
                   245
<210> 55
   <211> 247
   <212> PRT
   <213> Artificial Sequence
<220>
   <221> source
   <223> /note="Description of Artificial Sequence: Synthetic polypeptide"
<400> 55
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Val Ser Leu Pro Asp Tyr
               20                  25                  30
   Gly Val Ser Trp Ile Arg Gln Pro Pro Gly Lys Gly Leu Glu Trp Ile
           35                  40                  45
   Gly Val Ile Trp Gly Ser Glu Thr Thr Tyr Tyr Gln Ser Ser Leu Lys
       50                  55                  60
   Ser Arg Val Thr Ile Ser Lys Asp Asn Ser Lys Asn Gln Val Ser Leu
   65                  70                  75                  80
   Lys Leu Ser Ser Val Thr Ala Ala Asp Thr Ala Val Tyr Tyr Cys Ala
                   85                  90                  95
   Lys His Tyr Tyr Tyr Gly Gly Ser Tyr Ala Met Asp Tyr Trp Gly Gln
               100                 105                 110
   Gly Thr Leu Val Thr Val Ser Ser Gly Gly Gly Gly Ser Gly Gly Gly
           115                 120                 125
   Gly Ser Gly Gly Gly Gly Ser Gly Gly Gly Gly Ser Glu Ile Val Met
       130                 135                 140
   Thr Gln Ser Pro Ala Thr Leu Ser Leu Ser Pro Gly Glu Arg Ala Thr
   145                 150                 155                 160
   Leu Ser Cys Arg Ala Ser Gln Asp Ile Ser Lys Tyr Leu Asn Trp Tyr
                   165                 170                 175
   Gln Gln Lys Pro Gly Gln Ala Pro Arg Leu Leu Ile Tyr His Thr Ser
               180                 185                 190
   Arg Leu His Ser Gly Ile Pro Ala Arg Phe Ser Gly Ser Gly Ser Gly
           195                 200                 205
   Thr Asp Tyr Thr Leu Thr Ile Ser Ser Leu Gln Pro Glu Asp Phe Ala
       210                 215                 220
   Val Tyr Phe Cys Gln Gln Gly Asn Thr Leu Pro Tyr Thr Phe Gly Gln
   225                 230                 235                 240
   Gly Thr Lys Leu Glu Ile Lys
                   245
<210> 56
   <211> 247
   <212> PRT
   <213> Artificial Sequence
<220>
   <221> source
   <223> /note="Description of Artificial Sequence: Synthetic polypeptide"
<400> 56
   Glu Ile Val Met Thr Gln Ser Pro Ala Thr Leu Ser Leu Ser Pro Gly
   1               5                   10                  15
   Glu Arg Ala Thr Leu Ser Cys Arg Ala Ser Gln Asp Ile Ser Lys Tyr
               20                  25                  30
   Leu Asn Trp Tyr Gln Gln Lys Pro Gly Gln Ala Pro Arg Leu Leu Ile
           35                  40                  45
   Tyr His Thr Ser Arg Leu His Ser Gly Ile Pro Ala Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Val Tyr Phe Cys Gln Gln Gly Asn Thr Leu Pro Tyr
                   85                  90                  95
   Thr Phe Gly Gln Gly Thr Lys Leu Glu Ile Lys Gly Gly Gly Gly Ser
               100                 105                 110
   Gly Gly Gly Gly Ser Gly Gly Gly Gly Ser Gly Gly Gly Gly Ser Gln
           115                 120                 125
   Val Gln Leu Gln Glu Ser Gly Pro Gly Leu Val Lys Pro Ser Glu Thr
       130                 135                 140
   Leu Ser Leu Thr Cys Thr Val Ser Gly Val Ser Leu Pro Asp Tyr Gly
   145                 150                 155                 160
   Val Ser Trp Ile Arg Gln Pro Pro Gly Lys Gly Leu Glu Trp Ile Gly
                   165                 170                 175
   Val Ile Trp Gly Ser Glu Thr Thr Tyr Tyr Asn Ser Ser Leu Lys Ser
               180                 185                 190
   Arg Val Thr Ile Ser Lys Asp Asn Ser Lys Asn Gln Val Ser Leu Lys
           195                 200                 205
   Leu Ser Ser Val Thr Ala Ala Asp Thr Ala Val Tyr Tyr Cys Ala Lys
       210                 215                 220
   His Tyr Tyr Tyr Gly Gly Ser Tyr Ala Met Asp Tyr Trp Gly Gln Gly
   225                 230                 235                 240
   Thr Leu Val Thr Val Ser Ser
                   245
<210> 57
   <211> 247
   <212> PRT
   <213> Artificial Sequence
<220>
   <221> source
   <223> /note="Description of Artificial Sequence: Synthetic polypeptide"
<400> 57
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Val Ser Leu Pro Asp Tyr
               20                  25                  30
   Gly Val Ser Trp Ile Arg Gln Pro Pro Gly Lys Gly Leu Glu Trp Ile
           35                  40                  45
   Gly Val Ile Trp Gly Ser Glu Thr Thr Tyr Tyr Asn Ser Ser Leu Lys
       50                  55                  60
   Ser Arg Val Thr Ile Ser Lys Asp Asn Ser Lys Asn Gln Val Ser Leu
   65                  70                  75                  80
   Lys Leu Ser Ser Val Thr Ala Ala Asp Thr Ala Val Tyr Tyr Cys Ala
                   85                  90                  95
   Lys His Tyr Tyr Tyr Gly Gly Ser Tyr Ala Met Asp Tyr Trp Gly Gln
               100                 105                 110
   Gly Thr Leu Val Thr Val Ser Ser Gly Gly Gly Gly Ser Gly Gly Gly
           115                 120                 125
   Gly Ser Gly Gly Gly Gly Ser Gly Gly Gly Gly Ser Glu Ile Val Met
       130                 135                 140
   Thr Gln Ser Pro Ala Thr Leu Ser Leu Ser Pro Gly Glu Arg Ala Thr
   145                 150                 155                 160
   Leu Ser Cys Arg Ala Ser Gln Asp Ile Ser Lys Tyr Leu Asn Trp Tyr
                   165                 170                 175
   Gln Gln Lys Pro Gly Gln Ala Pro Arg Leu Leu Ile Tyr His Thr Ser
               180                 185                 190
   Arg Leu His Ser Gly Ile Pro Ala Arg Phe Ser Gly Ser Gly Ser Gly
           195                 200                 205
   Thr Asp Tyr Thr Leu Thr Ile Ser Ser Leu Gln Pro Glu Asp Phe Ala
       210                 215                 220
   Val Tyr Phe Cys Gln Gln Gly Asn Thr Leu Pro Tyr Thr Phe Gly Gln
   225                 230                 235                 240
   Gly Thr Lys Leu Glu Ile Lys
                   245
<210> 58
   <211> 242
   <212> PRT
   <213> Artificial Sequence
<220>
   <221> source
   <223> /note="Description of Artificial Sequence: Synthetic polypeptide"
<400> 58
   Glu Ile Val Met Thr Gln Ser Pro Ala Thr Leu Ser Leu Ser Pro Gly
   1               5                   10                  15
   Glu Arg Ala Thr Leu Ser Cys Arg Ala Ser Gln Asp Ile Ser Lys Tyr
               20                  25                  30
   Leu Asn Trp Tyr Gln Gln Lys Pro Gly Gln Ala Pro Arg Leu Leu Ile
           35                  40                  45
   Tyr His Thr Ser Arg Leu His Ser Gly Ile Pro Ala Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Val Tyr Phe Cys Gln Gln Gly Asn Thr Leu Pro Tyr
                   85                  90                  95
   Thr Phe Gly Gln Gly Thr Lys Leu Glu Ile Lys Gly Gly Gly Gly Ser
               100                 105                 110
   Gly Gly Gly Gly Ser Gly Gly Gly Gly Ser Gln Val Gln Leu Gln Glu
           115                 120                 125
   Ser Gly Pro Gly Leu Val Lys Pro Ser Glu Thr Leu Ser Leu Thr Cys
       130                 135                 140
   Thr Val Ser Gly Val Ser Leu Pro Asp Tyr Gly Val Ser Trp Ile Arg
   145 150 155 160
   Gln Pro Pro Gly Lys Gly Leu Glu Trp Ile Gly Val Ile Trp Gly Ser
   165 170 175
   Glu Thr Thr Tyr Tyr Asn Ser Ser Leu Lys Ser Arg Val Thr Ile Ser
   180 185 190
   Lys Asp Asn Ser Lys Asn Gln Val Ser Leu Lys Leu Ser Ser Val Thr
   195 200 205
   Ala Ala Asp Thr Ala Val Tyr Tyr Cys Ala Lys His Tyr Tyr Tyr Gly
   210 215 220
   Gly Ser Tyr Ala Met Asp Tyr Trp Gly Gln Gly Thr Leu Val Thr Val
   225 230 235 240
   Ser Ser
<210> 59
   <211> 242
   <212> PRT
   <213> Artificial Sequence
<220>
   <221> source
   <223> /note="Description of Artificial Sequence: Synthetic polypeptide"
<400> 59
   Gln Val Gln Leu Gln Glu Ser Gly Pro Gly Leu Val Lys Pro Ser Glu
   1 5 10 15
   Thr Leu Ser Leu Thr Cys Thr Val Ser Gly Val Ser Leu Pro Asp Tyr
   20 25 30
   Gly Val Ser Trp Ile Arg Gln Pro Pro Gly Lys Gly Leu Glu Trp Ile
   35 40 45
   Gly Val Ile Trp Gly Ser Glu Thr Thr Tyr Tyr Asn Ser Ser Leu Lys
   50 55 60
   Ser Arg Val Thr Ile Ser Lys Asp Asn Ser Lys Asn Gln Val Ser Leu
   65 70 75 80
   Lys Leu Ser Ser Val Thr Ala Ala Asp Thr Ala Val Tyr Tyr Cys Ala
   85 90 95
   Lys His Tyr Tyr Tyr Gly Gly Ser Tyr Ala Met Asp Tyr Trp Gly Gln
               100                 105                 110
   Gly Thr Leu Val Thr Val Ser Ser Gly Gly Gly Gly Ser Gly Gly Gly
           115                 120                 125
   Gly Ser Gly Gly Gly Gly Ser Glu Ile Val Met Thr Gln Ser Pro Ala
       130                 135                 140
   Thr Leu Ser Leu Ser Pro Gly Glu Arg Ala Thr Leu Ser Cys Arg Ala
   145                 150                 155                 160
   Ser Gln Asp Ile Ser Lys Tyr Leu Asn Trp Tyr Gln Gln Lys Pro Gly
                   165                 170                 175
   Gln Ala Pro Arg Leu Leu Ile Tyr His Thr Ser Arg Leu His Ser Gly
               180                 185                 190
   Ile Pro Ala Arg Phe Ser Gly Ser Gly Ser Gly Thr Asp Tyr Thr Leu
           195                 200                 205
   Thr Ile Ser Ser Leu Gln Pro Glu Asp Phe Ala Val Tyr Phe Cys Gln
       210                 215                 220
   Gln Gly Asn Thr Leu Pro Tyr Thr Phe Gly Gln Gly Thr Lys Leu Glu
   225                 230                 235                 240
   Ile Lys
<210> 60
   <211> 242
   <212> PRT
   <213> Artificial Sequence
<220>
   <221> source
   <223> /note="Description of Artificial Sequence: Synthetic polypeptide"
<400> 60
   Asp Ile Gln Met Thr Gln Thr Thr Ser Ser Leu Ser Ala Ser Leu Gly
   1               5                   10                  15
   Asp Arg Val Thr Ile Ser Cys Arg Ala Ser Gln Asp Ile Ser Lys Tyr
               20                  25                  30
   Leu Asn Trp Tyr Gln Gln Lys Pro Asp Gly Thr Val Lys Leu Leu Ile
           35                  40                  45
   Tyr His Thr Ser Arg Leu His Ser Gly Val Pro Ser Arg Phe Ser Gly
       50                  55                  60
   Ser Gly Ser Gly Thr Asp Tyr Ser Leu Thr Ile Ser Asn Leu Glu Gln
   65                  70                  75                  80
   Glu Asp Ile Ala Thr Tyr Phe Cys Gln Gln Gly Asn Thr Leu Pro Tyr
                   85                  90                  95
   Thr Phe Gly Gly Gly Thr Lys Leu Glu Ile Thr Gly Gly Gly Gly Ser
               100                 105                 110
   Gly Gly Gly Gly Ser Gly Gly Gly Gly Ser Glu Val Lys Leu Gln Glu
           115                 120                 125
   Ser Gly Pro Gly Leu Val Ala Pro Ser Gln Ser Leu Ser Val Thr Cys
       130                 135                 140
   Thr Val Ser Gly Val Ser Leu Pro Asp Tyr Gly Val Ser Trp Ile Arg
   145                 150                 155                 160
   Gln Pro Pro Arg Lys Gly Leu Glu Trp Leu Gly Val Ile Trp Gly Ser
                   165                 170                 175
   Glu Thr Thr Tyr Tyr Asn Ser Ala Leu Lys Ser Arg Leu Thr Ile Ile
               180                 185                 190
   Lys Asp Asn Ser Lys Ser Gln Val Phe Leu Lys Met Asn Ser Leu Gln
           195                 200                 205
   Thr Asp Asp Thr Ala Ile Tyr Tyr Cys Ala Lys His Tyr Tyr Tyr Gly
       210                 215                 220
   Gly Ser Tyr Ala Met Asp Tyr Trp Gly Gln Gly Thr Ser Val Thr Val
   225                 230                 235                 240
   Ser Ser
<210> 61
   <211> 244
   <212> PRT
   <213> Artificial Sequence
<220>
   <221> source
   <223> /note="Description of Artificial Sequence: Synthetic polypeptide"
<400> 61
   Asp Ile Val Leu Thr Gln Ser Pro Ala Ser Leu Ala Val Ser Leu Gly
   1               5                   10                  15
   Gln Arg Ala Thr Ile Ser Cys Arg Ala Ser Glu Ser Val Asp Asn Tyr
               20                  25                  30
   Gly Asn Thr Phe Met His Trp Tyr Gln Gln Lys Pro Gly Gln Pro Pro
           35                  40                  45
   Lys Leu Leu Ile Tyr Arg Ala Ser Asn Leu Glu Ser Gly Ile Pro Ala
       50                  55                  60
   Arg Phe Ser Gly Ser Gly Ser Arg Thr Asp Phe Thr Leu Thr Ile Asn
   65                  70                  75                  80
   Pro Val Glu Ala Asp Asp Val Ala Thr Tyr Tyr Cys Gln Gln Ser Asn
                   85                  90                  95
   Glu Asp Pro Pro Thr Phe Gly Ala Gly Thr Lys Leu Glu Leu Lys Gly
               100                 105                 110
   Gly Gly Gly Ser Gly Gly Gly Gly Ser Ser Gly Gly Gly Ser Gln Ile
           115                 120                 125
   Gln Leu Val Gln Ser Gly Pro Glu Leu Lys Lys Pro Gly Glu Thr Val
       130                 135                 140
   Lys Ile Ser Cys Lys Ala Ser Gly Tyr Ile Phe Thr Asn Tyr Gly Met
   145                 150                 155                 160
   Asn Trp Val Lys Gln Ala Pro Gly Lys Ser Phe Lys Trp Met Gly Trp
                   165                 170                 175
   Ile Asn Thr Tyr Thr Gly Glu Ser Thr Tyr Ser Ala Asp Phe Lys Gly
               180                 185                 190
   Arg Phe Ala Phe Ser Leu Glu Thr Ser Ala Ser Thr Ala Tyr Leu His
           195                 200                 205
   Ile Asn Asp Leu Lys Asn Glu Asp Thr Ala Thr Tyr Phe Cys Ala Arg
       210                 215                 220
   Ser Gly Gly Tyr Asp Pro Met Asp Tyr Trp Gly Gln Gly Thr Ser Val
   225                 230                 235                 240
   Thr Val Ser Ser
<210> 62
   <211> 237
   <212> PRT
   <213> Artificial Sequence
<220>
   <221> source
   <223> /note="Description of Artificial Sequence: Synthetic polypeptide"
<400> 62
   Asp Val Gln Ile Thr Gln Ser Pro Ser Tyr Leu Ala Ala Ser Pro Gly
   1               5                   10                  15
   Glu Thr Ile Thr Ile Asn Cys Arg Ala Ser Lys Ser Ile Ser Lys Asp
               20                  25                  30
   Leu Ala Trp Tyr Gln Glu Lys Pro Gly Lys Thr Asn Lys Leu Leu Ile
           35                  40                  45
   Tyr Ser Gly Ser Thr Leu Gln Ser Gly Ile Pro Ser Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Met Tyr Tyr Cys Gln Gln His Asn Lys Tyr Pro Tyr
                   85                  90                  95
   Thr Phe Gly Gly Gly Thr Lys Leu Glu Ile Lys Gly Gly Gly Gly Ser
               100                 105                 110
   Gly Gly Gly Gly Ser Ser Gly Gly Gly Ser Gln Val Gln Leu Gln Gln
           115                 120                 125
   Pro Gly Ala Glu Leu Val Arg Pro Gly Ala Ser Val Lys Leu Ser Cys
       130                 135                 140
   Lys Ala Ser Gly Tyr Thr Phe Thr Ser Tyr Trp Met Asn Trp Val Lys
   145                 150                 155                 160
   Gln Arg Pro Asp Gln Gly Leu Glu Trp Ile Gly Arg Ile Asp Pro Tyr
                   165                 170                 175
   Asp Ser Glu Thr His Tyr Asn Gln Lys Phe Lys Asp Lys Ala Ile Leu
               180                 185                 190
   Thr Val Asp Lys Ser Ser Ser Thr Ala Tyr Met Gln Leu Ser Ser Leu
   195 200 205
   Thr Ser Glu Asp Ser Ala Val Tyr Tyr Cys Ala Arg Gly Asn Trp Asp
   210 215 220
   Asp Tyr Trp Gly Gln Gly Thr Thr Leu Thr Val Ser Ser
   225 230 235
<210> 63
   <211> 249
   <212> PRT
   <213> Artificial Sequence
<220>
   <221> source
   <223> /note="Description of Artificial Sequence: Synthetic polypeptide"
<400> 63
   Asp Ile Val Leu Thr Gln Ser Pro Asp Ser Leu Ala Val Ser Leu Gly
   1               5                   10                  15
   Glu Arg Ala Thr Ile Asn Cys Arg Ala Ser Glu Ser Val Asp Asn Tyr
               20                  25                  30
   Gly Asn Thr Phe Met His Trp Tyr Gln Gln Lys Pro Gly Gln Pro Pro
           35                  40                  45
   Lys Leu Leu Ile Tyr Arg Ala Ser Asn Leu Glu Ser Gly Val Pro Asp
       50                  55                  60
   Arg Phe Ser Gly Ser Gly Ser Arg Thr Asp Phe Thr Leu Thr Ile Ser
   65                  70                  75                  80
   Ser Leu Gln Ala Glu Asp Val Ala Val Tyr Tyr Cys Gln Gln Ser Asn
                   85                  90                  95
   Glu Asp Pro Pro Thr Phe Gly Gln Gly Thr Lys Leu Glu Ile Lys Gly
               100                 105                 110
   Gly Gly Gly Ser Gly Gly Gly Gly Ser Gly Gly Gly Gly Ser Gly Gly
           115                 120                 125
   Gly Gly Ser Gln Ile Gln Leu Val Gln Ser Gly Ser Glu Leu Lys Lys
       130                 135                 140
   Pro Gly Ala Ser Val Lys Val Ser Cys Lys Ala Ser Gly Tyr Ile Phe
   145                 150                 155                 160
   Thr Asn Tyr Gly Met Asn Trp Val Arg Gln Ala Pro Gly Gln Gly Leu
                   165                 170                 175
   Glu Trp Met Gly Trp Ile Asn Thr Tyr Thr Gly Glu Ser Thr Tyr Ser
               180                 185                 190
   Ala Asp Phe Lys Gly Arg Phe Val Phe Ser Leu Asp Thr Ser Val Ser
           195                 200                 205
   Thr Ala Tyr Leu Gln Ile Asn Ala Leu Lys Ala Glu Asp Thr Ala Val
       210                 215                 220
   Tyr Tyr Cys Ala Arg Ser Gly Gly Tyr Asp Pro Met Asp Tyr Trp Gly
   225                 230                 235                 240
   Gln Gly Thr Thr Val Thr Val Ser Ser
                   245
<210> 64
   <211> 249
   <212> PRT
   <213> Artificial Sequence
<220>
   <221> source
   <223> /note="Description of Artificial Sequence: Synthetic polypeptide"
<400> 64
   Asp Ile Val Leu Thr Gln Ser Pro Asp Ser Leu Ala Val Ser Leu Gly
   1               5                   10                  15
   Glu Arg Ala Thr Ile Asn Cys Arg Ala Ser Glu Ser Val Asp Asn Tyr
               20                  25                  30
   Gly Asn Thr Phe Met His Trp Tyr Gln Gln Lys Pro Gly Gln Pro Pro
           35                  40                  45
   Lys Leu Leu Ile Tyr Arg Ala Ser Asn Leu Glu Ser Gly Val Pro Asp
       50                  55                  60
   Arg Phe Ser Gly Ser Gly Ser Arg Thr Asp Phe Thr Leu Thr Ile Ser
   65                  70                  75                  80
   Ser Leu Gln Ala Glu Asp Val Ala Val Tyr Tyr Cys Gln Gln Ser Asn
                   85                  90                  95
   Glu Asp Pro Pro Thr Phe Gly Gln Gly Thr Lys Leu Glu Ile Lys Gly
               100                 105                 110
   Gly Gly Gly Ser Gly Gly Gly Gly Ser Gly Gly Gly Gly Ser Gly Gly
           115                 120                 125
   Gly Gly Ser Gln Ile Gln Leu Val Gln Ser Gly Ala Glu Val Lys Lys
       130                 135                 140
   Pro Gly Ala Ser Val Lys Val Ser Cys Lys Ala Ser Gly Tyr Ile Phe
   145                 150                 155                 160
   Thr Asn Tyr Gly Met Asn Trp Val Arg Gln Ala Pro Gly Gln Arg Leu
                   165                 170                 175
   Glu Trp Met Gly Trp Ile Asn Thr Tyr Thr Gly Glu Ser Thr Tyr Ser
               180                 185                 190
   Ala Asp Phe Lys Gly Arg Val Thr Ile Thr Leu Asp Thr Ser Ala Ser
           195                 200                 205
   Thr Ala Tyr Met Glu Leu Ser Ser Leu Arg Ser Glu Asp Thr Ala Val
       210                 215                 220
   Tyr Tyr Cys Ala Arg Ser Gly Gly Tyr Asp Pro Met Asp Tyr Trp Gly
   225                 230                 235                 240
   Gln Gly Thr Thr Val Thr Val Ser Ser
                   245
<210> 65
   <211> 249
   <212> PRT
   <213> Artificial Sequence
<220>
   <221> source
   <223> /note="Description of Artificial Sequence: Synthetic polypeptide"
<400> 65
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Glu Ser Val Asp Asn Tyr
               20                  25                  30
   Gly Asn Thr Phe Met His Trp Tyr Gln Gln Lys Pro Gly Gln Ala Pro
           35                  40                  45
   Arg Leu Leu Ile Tyr Arg Ala Ser Asn Leu Glu Ser Gly Ile Pro Ala
       50                  55                  60
   Arg Phe Ser Gly Ser Gly Ser Arg Thr Asp Phe Thr Leu Thr Ile Ser
   65 70 75 80
   Ser Leu Glu Pro Glu Asp Val Ala Val Tyr Tyr Cys Gln Gln Ser Asn
   85 90 95
   Glu Asp Pro Pro Thr Phe Gly Gln Gly Thr Lys Leu Glu Ile Lys Gly
   100 105 110
   Gly Gly Gly Ser Gly Gly Gly Gly Ser Gly Gly Gly Gly Ser Gly Gly
   115 120 125
   Gly Gly Ser Gln Ile Gln Leu Val Gln Ser Gly Ser Glu Leu Lys Lys
   130 135 140
   Pro Gly Ala Ser Val Lys Val Ser Cys Lys Ala Ser Gly Tyr Ile Phe
   145 150 155 160
   Thr Asn Tyr Gly Met Asn Trp Val Arg Gln Ala Pro Gly Gln Gly Leu
   165 170 175
   Glu Trp Met Gly Trp Ile Asn Thr Tyr Thr Gly Glu Ser Thr Tyr Ser
   180 185 190
   Ala Asp Phe Lys Gly Arg Phe Val Phe Ser Leu Asp Thr Ser Val Ser
   195 200 205
   Thr Ala Tyr Leu Gln Ile Asn Ala Leu Lys Ala Glu Asp Thr Ala Val
   210 215 220
   Tyr Tyr Cys Ala Arg Ser Gly Gly Tyr Asp Pro Met Asp Tyr Trp Gly
   225 230 235 240
   Gln Gly Thr Thr Val Thr Val Ser Ser
   245
<210> 66
   <211> 249
   <212> PRT
   <213> Artificial Sequence
<220>
   <221> source
   <223> /note="Description of Artificial Sequence: Synthetic polypeptide"
<400> 66
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Glu Ser Val Asp Asn Tyr
               20                  25                  30
   Gly Asn Thr Phe Met His Trp Tyr Gln Gln Lys Pro Gly Gln Ala Pro
           35                  40                  45
   Arg Leu Leu Ile Tyr Arg Ala Ser Asn Leu Glu Ser Gly Ile Pro Ala
       50                  55                  60
   Arg Phe Ser Gly Ser Gly Ser Arg Thr Asp Phe Thr Leu Thr Ile Ser
   65                  70                  75                  80
   Ser Leu Glu Pro Glu Asp Val Ala Val Tyr Tyr Cys Gln Gln Ser Asn
                   85                  90                  95
   Glu Asp Pro Pro Thr Phe Gly Gln Gly Thr Lys Leu Glu Ile Lys Gly
               100                 105                 110
   Gly Gly Gly Ser Gly Gly Gly Gly Ser Gly Gly Gly Gly Ser Gly Gly
           115                 120                 125
   Gly Gly Ser Gln Ile Gln Leu Val Gln Ser Gly Ala Glu Val Lys Lys
       130                 135                 140
   Pro Gly Ala Ser Val Lys Val Ser Cys Lys Ala Ser Gly Tyr Ile Phe
   145                 150                 155                 160
   Thr Asn Tyr Gly Met Asn Trp Val Arg Gln Ala Pro Gly Gln Arg Leu
                   165                 170                 175
   Glu Trp Met Gly Trp Ile Asn Thr Tyr Thr Gly Glu Ser Thr Tyr Ser
               180                 185                 190
   Ala Asp Phe Lys Gly Arg Val Thr Ile Thr Leu Asp Thr Ser Ala Ser
           195                 200                 205
   Thr Ala Tyr Met Glu Leu Ser Ser Leu Arg Ser Glu Asp Thr Ala Val
       210                 215                 220
   Tyr Tyr Cys Ala Arg Ser Gly Gly Tyr Asp Pro Met Asp Tyr Trp Gly
   225                 230                 235                 240
   Gln Gly Thr Thr Val Thr Val Ser Ser
                   245
<210> 67
   <211> 249
   <212> PRT
   <213> Artificial Sequence
<220>
   <221> source
   <223> /note="Description of Artificial Sequence: Synthetic polypeptide"
<400> 67
   Gln Ile Gln Leu Val Gln Ser Gly Ser Glu Leu Lys Lys Pro Gly Ala
   1               5                   10                  15
   Ser Val Lys Val Ser Cys Lys Ala Ser Gly Tyr Ile Phe Thr Asn Tyr
               20                  25                  30
   Gly Met Asn Trp Val Arg Gln Ala Pro Gly Gln Gly Leu Glu Trp Met
           35                  40                  45
   Gly Trp Ile Asn Thr Tyr Thr Gly Glu Ser Thr Tyr Ser Ala Asp Phe
       50                  55                  60
   Lys Gly Arg Phe Val Phe Ser Leu Asp Thr Ser Val Ser Thr Ala Tyr
   65                  70                  75                  80
   Leu Gln Ile Asn Ala Leu Lys Ala Glu Asp Thr Ala Val Tyr Tyr Cys
                   85                  90                  95
   Ala Arg Ser Gly Gly Tyr Asp Pro Met Asp Tyr Trp Gly Gln Gly Thr
               100                 105                 110
   Thr Val Thr Val Ser Ser Gly Gly Gly Gly Ser Gly Gly Gly Gly Ser
           115                 120                 125
   Gly Gly Gly Gly Ser Gly Gly Gly Gly Ser Asp Ile Val Leu Thr Gln
       130                 135                 140
   Ser Pro Asp Ser Leu Ala Val Ser Leu Gly Glu Arg Ala Thr Ile Asn
   145                 150                 155                 160
   Cys Arg Ala Ser Glu Ser Val Asp Asn Tyr Gly Asn Thr Phe Met His
                   165                 170                 175
   Trp Tyr Gln Gln Lys Pro Gly Gln Pro Pro Lys Leu Leu Ile Tyr Arg
               180                 185                 190
   Ala Ser Asn Leu Glu Ser Gly Val Pro Asp Arg Phe Ser Gly Ser Gly
           195                 200                 205
   Ser Arg Thr Asp Phe Thr Leu Thr Ile Ser Ser Leu Gln Ala Glu Asp
       210                 215                 220
   Val Ala Val Tyr Tyr Cys Gln Gln Ser Asn Glu Asp Pro Pro Thr Phe
   225                 230                 235                 240
   Gly Gln Gly Thr Lys Leu Glu Ile Lys
                   245
<210> 68
   <211> 249
   <212> PRT
   <213> Artificial Sequence
<220>
   <221> source
   <223> /note="Description of Artificial Sequence: Synthetic polypeptide"
<400> 68
   Gln Ile Gln Leu Val Gln Ser Gly Ser Glu Leu Lys Lys Pro Gly Ala
   1               5                   10                  15
   Ser Val Lys Val Ser Cys Lys Ala Ser Gly Tyr Ile Phe Thr Asn Tyr
               20                  25                  30
   Gly Met Asn Trp Val Arg Gln Ala Pro Gly Gln Gly Leu Glu Trp Met
           35                  40                  45
   Gly Trp Ile Asn Thr Tyr Thr Gly Glu Ser Thr Tyr Ser Ala Asp Phe
       50                  55                  60
   Lys Gly Arg Phe Val Phe Ser Leu Asp Thr Ser Val Ser Thr Ala Tyr
   65                  70                  75                  80
   Leu Gln Ile Asn Ala Leu Lys Ala Glu Asp Thr Ala Val Tyr Tyr Cys
                   85                  90                  95
   Ala Arg Ser Gly Gly Tyr Asp Pro Met Asp Tyr Trp Gly Gln Gly Thr
               100                 105                 110
   Thr Val Thr Val Ser Ser Gly Gly Gly Gly Ser Gly Gly Gly Gly Ser
           115                 120                 125
   Gly Gly Gly Gly Ser Gly Gly Gly Gly Ser Glu Ile Val Leu Thr Gln
       130                 135                 140
   Ser Pro Ala Thr Leu Ser Leu Ser Pro Gly Glu Arg Ala Thr Leu Ser
   145                 150                 155                 160
   Cys Arg Ala Ser Glu Ser Val Asp Asn Tyr Gly Asn Thr Phe Met His
                   165                 170                 175
   Trp Tyr Gln Gln Lys Pro Gly Gln Ala Pro Arg Leu Leu Ile Tyr Arg
   180 185 190
   Ala Ser Asn Leu Glu Ser Gly Ile Pro Ala Arg Phe Ser Gly Ser Gly
   195 200 205
   Ser Arg Thr Asp Phe Thr Leu Thr Ile Ser Ser Leu Glu Pro Glu Asp
   210 215 220
   Val Ala Val Tyr Tyr Cys Gln Gln Ser Asn Glu Asp Pro Pro Thr Phe
   225 230 235 240
   Gly Gln Gly Thr Lys Leu Glu Ile Lys
   245
<210> 69
   <211> 249
   <212> PRT
   <213> Artificial Sequence
<220>
   <221> source
   <223> /note="Description of Artificial Sequence: Synthetic polypeptide"
<400> 69
   Gln Ile Gln Leu Val Gln Ser Gly Ala Glu Val Lys Lys Pro Gly Ala
   1 5 10 15
   Ser Val Lys Val Ser Cys Lys Ala Ser Gly Tyr Ile Phe Thr Asn Tyr
   20 25 30
   Gly Met Asn Trp Val Arg Gln Ala Pro Gly Gln Arg Leu Glu Trp Met
   35 40 45
   Gly Trp Ile Asn Thr Tyr Thr Gly Glu Ser Thr Tyr Ser Ala Asp Phe
   50 55 60
   Lys Gly Arg Val Thr Ile Thr Leu Asp Thr Ser Ala Ser Thr Ala Tyr
   65 70 75 80
   Met Glu Leu Ser Ser Leu Arg Ser Glu Asp Thr Ala Val Tyr Tyr Cys
   85 90 95
   Ala Arg Ser Gly Gly Tyr Asp Pro Met Asp Tyr Trp Gly Gln Gly Thr
   100 105 110
   Thr Val Thr Val Ser Ser Gly Gly Gly Gly Ser Gly Gly Gly Gly Ser
   115 120 125
   Gly Gly Gly Gly Ser Gly Gly Gly Gly Ser Asp Ile Val Leu Thr Gln
   130 135 140
   Ser Pro Asp Ser Leu Ala Val Ser Leu Gly Glu Arg Ala Thr Ile Asn
   145 150 155 160
   Cys Arg Ala Ser Glu Ser Val Asp Asn Tyr Gly Asn Thr Phe Met His
   165 170 175
   Trp Tyr Gln Gln Lys Pro Gly Gln Pro Pro Lys Leu Leu Ile Tyr Arg
   180 185 190
   Ala Ser Asn Leu Glu Ser Gly Val Pro Asp Arg Phe Ser Gly Ser Gly
   195 200 205
   Ser Arg Thr Asp Phe Thr Leu Thr Ile Ser Ser Leu Gln Ala Glu Asp
   210 215 220
   Val Ala Val Tyr Tyr Cys Gln Gln Ser Asn Glu Asp Pro Pro Thr Phe
   225 230 235 240
   Gly Gln Gly Thr Lys Leu Glu Ile Lys
   245
<210> 70
   <211> 249
   <212> PRT
   <213> Artificial Sequence
<220>
   <221> source
   <223> /note="Description of Artificial Sequence: Synthetic polypeptide"
<400> 70
   Gln Ile Gln Leu Val Gln Ser Gly Ala Glu Val Lys Lys Pro Gly Ala
   1 5 10 15
   Ser Val Lys Val Ser Cys Lys Ala Ser Gly Tyr Ile Phe Thr Asn Tyr
   20 25 30
   Gly Met Asn Trp Val Arg Gln Ala Pro Gly Gln Arg Leu Glu Trp Met
   35 40 45
   Gly Trp Ile Asn Thr Tyr Thr Gly Glu Ser Thr Tyr Ser Ala Asp Phe
   50 55 60
   Lys Gly Arg Val Thr Ile Thr Leu Asp Thr Ser Ala Ser Thr Ala Tyr
   65 70 75 80
   Met Glu Leu Ser Ser Leu Arg Ser Glu Asp Thr Ala Val Tyr Tyr Cys
   85 90 95
   Ala Arg Ser Gly Gly Tyr Asp Pro Met Asp Tyr Trp Gly Gln Gly Thr
   100 105 110
   Thr Val Thr Val Ser Ser Gly Gly Gly Gly Ser Gly Gly Gly Gly Ser
   115 120 125
   Gly Gly Gly Gly Ser Gly Gly Gly Gly Ser Glu Ile Val Leu Thr Gln
   130 135 140
   Ser Pro Ala Thr Leu Ser Leu Ser Pro Gly Glu Arg Ala Thr Leu Ser
   145 150 155 160
   Cys Arg Ala Ser Glu Ser Val Asp Asn Tyr Gly Asn Thr Phe Met His
   165 170 175
   Trp Tyr Gln Gln Lys Pro Gly Gln Ala Pro Arg Leu Leu Ile Tyr Arg
   180 185 190
   Ala Ser Asn Leu Glu Ser Gly Ile Pro Ala Arg Phe Ser Gly Ser Gly
   195 200 205
   Ser Arg Thr Asp Phe Thr Leu Thr Ile Ser Ser Leu Glu Pro Glu Asp
   210 215 220
   Val Ala Val Tyr Tyr Cys Gln Gln Ser Asn Glu Asp Pro Pro Thr Phe
   225 230 235 240
   Gly Gln Gly Thr Lys Leu Glu Ile Lys
   245
<210> 71
   <211> 246
   <212> PRT
   <213> Artificial Sequence
<220>
   <221> source
   <223> /note="Description of Artificial Sequence: Synthetic polypeptide"
<400> 71
   Glu Ile Gln Leu Val Gln Ser Gly Ala Glu Val Lys Lys Pro Gly Ala
   1 5 10 15
   Thr Val Lys Ile Ser Cys Lys Gly Ser Gly Phe Asn Ile Glu Asp Tyr
   20 25 30
   Tyr Ile His Trp Val Gln Gln Ala Pro Gly Lys Gly Leu Glu Trp Met
           35                  40                  45
   Gly Arg Ile Asp Pro Glu Asn Asp Glu Thr Lys Tyr Gly Pro Ile Phe
       50                  55                  60
   Gln Gly Arg Val Thr Ile Thr Ala Asp Thr Ser Thr Asn Thr Val Tyr
   65                  70                  75                  80
   Met Glu Leu Ser Ser Leu Arg Ser Glu Asp Thr Ala Val Tyr Tyr Cys
                   85                  90                  95
   Ala Phe Arg Gly Gly Val Tyr Trp Gly Gln Gly Thr Thr Val Thr Val
               100                 105                 110
   Ser Ser Gly Gly Gly Gly Ser Gly Gly Gly Gly Ser Gly Gly Gly Gly
           115                 120                 125
   Ser Gly Gly Gly Gly Ser Asp Val Val Met Thr Gln Ser Pro Asp Ser
       130                 135                 140
   Leu Ala Val Ser Leu Gly Glu Arg Ala Thr Ile Asn Cys Lys Ser Ser
   145                 150                 155                 160
   Gln Ser Leu Leu Asp Ser Asp Gly Lys Thr Tyr Leu Asn Trp Leu Gln
                   165                 170                 175
   Gln Lys Pro Gly Gln Pro Pro Lys Arg Leu Ile Ser Leu Val Ser Lys
               180                 185                 190
   Leu Asp Ser Gly Val Pro Asp Arg Phe Ser Gly Ser Gly Ser Gly Thr
           195                 200                 205
   Asp Phe Thr Leu Thr Ile Ser Ser Leu Gln Ala Glu Asp Val Ala Val
       210                 215                 220
   Tyr Tyr Cys Trp Gln Gly Thr His Phe Pro Gly Thr Phe Gly Gly Gly
   225                 230                 235                 240
   Thr Lys Val Glu Ile Lys
                   245
<210> 72
   <211> 246
   <212> PRT
   <213> Artificial Sequence
<220>
   <221> source
   <223> /note="Description of Artificial Sequence: Synthetic polypeptide"
<400> 72
   Asp Val Val Met Thr Gln Ser Pro Asp Ser Leu Ala Val Ser Leu Gly
   1               5                   10                  15
   Glu Arg Ala Thr Ile Asn Cys Lys Ser Ser Gln Ser Leu Leu Asp Ser
               20                  25                  30
   Asp Gly Lys Thr Tyr Leu Asn Trp Leu Gln Gln Lys Pro Gly Gln Pro
           35                  40                  45
   Pro Lys Arg Leu Ile Ser Leu Val Ser Lys Leu Asp Ser Gly Val Pro
       50                  55                  60
   Asp Arg Phe Ser Gly Ser Gly Ser Gly Thr Asp Phe Thr Leu Thr Ile
   65                  70                  75                  80
   Ser Ser Leu Gln Ala Glu Asp Val Ala Val Tyr Tyr Cys Trp Gln Gly
                   85                  90                  95
   Thr His Phe Pro Gly Thr Phe Gly Gly Gly Thr Lys Val Glu Ile Lys
               100                 105                 110
   Gly Gly Gly Gly Ser Gly Gly Gly Gly Ser Gly Gly Gly Gly Ser Gly
           115                 120                 125
   Gly Gly Gly Ser Glu Ile Gln Leu Val Gln Ser Gly Ala Glu Val Lys
       130                 135                 140
   Lys Pro Gly Ala Thr Val Lys Ile Ser Cys Lys Gly Ser Gly Phe Asn
   145                 150                 155                 160
   Ile Glu Asp Tyr Tyr Ile His Trp Val Gln Gln Ala Pro Gly Lys Gly
                   165                 170                 175
   Leu Glu Trp Met Gly Arg Ile Asp Pro Glu Asn Asp Glu Thr Lys Tyr
               180                 185                 190
   Gly Pro Ile Phe Gln Gly Arg Val Thr Ile Thr Ala Asp Thr Ser Thr
           195                 200                 205
   Asn Thr Val Tyr Met Glu Leu Ser Ser Leu Arg Ser Glu Asp Thr Ala
       210                 215                 220
   Val Tyr Tyr Cys Ala Phe Arg Gly Gly Val Tyr Trp Gly Gln Gly Thr
   225                 230                 235                 240
   Thr Val Thr Val Ser Ser
                   245
<210> 73
   <211> 246
   <212> PRT
   <213> Artificial Sequence
<220>
   <221> source
   <223> /note="Description of Artificial Sequence: Synthetic polypeptide"
<400> 73
   Glu Ile Gln Leu Val Gln Ser Gly Ala Glu Val Lys Lys Pro Gly Glu
   1               5                   10                  15
   Ser Leu Arg Ile Ser Cys Lys Gly Ser Gly Phe Asn Ile Glu Asp Tyr
               20                  25                  30
   Tyr Ile His Trp Val Arg Gln Met Pro Gly Lys Gly Leu Glu Trp Met
           35                  40                  45
   Gly Arg Ile Asp Pro Glu Asn Asp Glu Thr Lys Tyr Gly Pro Ile Phe
       50                  55                  60
   Gln Gly His Val Thr Ile Ser Ala Asp Thr Ser Ile Asn Thr Val Tyr
   65                  70                  75                  80
   Leu Gln Trp Ser Ser Leu Lys Ala Ser Asp Thr Ala Met Tyr Tyr Cys
                   85                  90                  95
   Ala Phe Arg Gly Gly Val Tyr Trp Gly Gln Gly Thr Thr Val Thr Val
               100                 105                 110
   Ser Ser Gly Gly Gly Gly Ser Gly Gly Gly Gly Ser Gly Gly Gly Gly
           115                 120                 125
   Ser Gly Gly Gly Gly Ser Asp Val Val Met Thr Gln Ser Pro Leu Ser
       130                 135                 140
   Leu Pro Val Thr Leu Gly Gln Pro Ala Ser Ile Ser Cys Lys Ser Ser
   145                 150                 155                 160
   Gln Ser Leu Leu Asp Ser Asp Gly Lys Thr Tyr Leu Asn Trp Leu Gln
                   165                 170                 175
   Gln Arg Pro Gly Gln Ser Pro Arg Arg Leu Ile Ser Leu Val Ser Lys
               180                 185                 190
   Leu Asp Ser Gly Val Pro Asp Arg Phe Ser Gly Ser Gly Ser Gly Thr
   195 200 205
   Asp Phe Thr Leu Lys Ile Ser Arg Val Glu Ala Glu Asp Val Gly Val
   210 215 220
   Tyr Tyr Cys Trp Gln Gly Thr His Phe Pro Gly Thr Phe Gly Gly Gly
   225 230 235 240
   Thr Lys Val Glu Ile Lys
   245
<210> 74
   <211> 246
   <212> PRT
   <213> Artificial Sequence
<220>
   <221> source
   <223> /note="Description of Artificial Sequence: Synthetic polypeptide"
<400> 74
   Asp Val Val Met Thr Gln Ser Pro Leu Ser Leu Pro Val Thr Leu Gly
   1 5 10 15
   Gln Pro Ala Ser Ile Ser Cys Lys Ser Ser Gln Ser Leu Leu Asp Ser
   20 25 30
   Asp Gly Lys Thr Tyr Leu Asn Trp Leu Gln Gln Arg Pro Gly Gln Ser
   35 40 45
   Pro Arg Arg Leu Ile Ser Leu Val Ser Lys Leu Asp Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Trp Gln Gly
   85 90 95
   Thr His Phe Pro Gly Thr Phe Gly Gly Gly Thr Lys Val Glu Ile Lys
   100 105 110
   Gly Gly Gly Gly Ser Gly Gly Gly Gly Ser Gly Gly Gly Gly Ser Gly
   115 120 125
   Gly Gly Gly Ser Glu Ile Gln Leu Val Gln Ser Gly Ala Glu Val Lys
   130 135 140
   Lys Pro Gly Glu Ser Leu Arg Ile Ser Cys Lys Gly Ser Gly Phe Asn
   145 150 155 160
   Ile Glu Asp Tyr Tyr Ile His Trp Val Arg Gln Met Pro Gly Lys Gly
   165 170 175
   Leu Glu Trp Met Gly Arg Ile Asp Pro Glu Asn Asp Glu Thr Lys Tyr
   180 185 190
   Gly Pro Ile Phe Gln Gly His Val Thr Ile Ser Ala Asp Thr Ser Ile
   195 200 205
   Asn Thr Val Tyr Leu Gln Trp Ser Ser Leu Lys Ala Ser Asp Thr Ala
   210 215 220
   Met Tyr Tyr Cys Ala Phe Arg Gly Gly Val Tyr Trp Gly Gln Gly Thr
   225 230 235 240
   Thr Val Thr Val Ser Ser
   245
<210> 75
   <211> 246
   <212> PRT
   <213> Artificial Sequence
<220>
   <221> source
   <223> /note="Description of Artificial Sequence: Synthetic polypeptide"
<400> 75
   Glu Ile Gln Leu Val Gln Ser Gly Ala Glu Val Lys Lys Pro Gly Ala
   1 5 10 15
   Thr Val Lys Ile Ser Cys Lys Gly Ser Gly Phe Asn Ile Glu Asp Tyr
   20 25 30
   Tyr Ile His Trp Val Gln Gln Ala Pro Gly Lys Gly Leu Glu Trp Met
   35 40 45
   Gly Arg Ile Asp Pro Glu Asn Asp Glu Thr Lys Tyr Gly Pro Ile Phe
   50 55 60
   Gln Gly Arg Val Thr Ile Thr Ala Asp Thr Ser Thr Asn Thr Val Tyr
   65 70 75 80
   Met Glu Leu Ser Ser Leu Arg Ser Glu Asp Thr Ala Val Tyr Tyr Cys
   85 90 95
   Ala Phe Arg Gly Gly Val Tyr Trp Gly Gln Gly Thr Thr Val Thr Val
               100                 105                 110
   Ser Ser Gly Gly Gly Gly Ser Gly Gly Gly Gly Ser Gly Gly Gly Gly
           115                 120                 125
   Ser Gly Gly Gly Gly Ser Asp Val Val Met Thr Gln Ser Pro Leu Ser
       130                 135                 140
   Leu Pro Val Thr Leu Gly Gln Pro Ala Ser Ile Ser Cys Lys Ser Ser
   145                 150                 155                 160
   Gln Ser Leu Leu Asp Ser Asp Gly Lys Thr Tyr Leu Asn Trp Leu Gln
                   165                 170                 175
   Gln Arg Pro Gly Gln Ser Pro Arg Arg Leu Ile Ser Leu Val Ser Lys
               180                 185                 190
   Leu Asp Ser Gly Val Pro Asp Arg Phe Ser Gly Ser Gly Ser Gly Thr
           195                 200                 205
   Asp Phe Thr Leu Lys Ile Ser Arg Val Glu Ala Glu Asp Val Gly Val
       210                 215                 220
   Tyr Tyr Cys Trp Gln Gly Thr His Phe Pro Gly Thr Phe Gly Gly Gly
   225                 230                 235                 240
   Thr Lys Val Glu Ile Lys
                   245
<210> 76
   <211> 246
   <212> PRT
   <213> Artificial Sequence
<220>
   <221> source
   <223> /note="Description of Artificial Sequence: Synthetic polypeptide"
<400> 76
   Glu Ile Gln Leu Val Gln Ser Gly Ala Glu Val Lys Lys Pro Gly Glu
   1               5                   10                  15
   Ser Leu Arg Ile Ser Cys Lys Gly Ser Gly Phe Asn Ile Glu Asp Tyr
               20                  25                  30
   Tyr Ile His Trp Val Arg Gln Met Pro Gly Lys Gly Leu Glu Trp Met
           35                  40                  45
   Gly Arg Ile Asp Pro Glu Asn Asp Glu Thr Lys Tyr Gly Pro Ile Phe
       50                  55                  60
   Gln Gly His Val Thr Ile Ser Ala Asp Thr Ser Ile Asn Thr Val Tyr
   65                  70                  75                  80
   Leu Gln Trp Ser Ser Leu Lys Ala Ser Asp Thr Ala Met Tyr Tyr Cys
                   85                  90                  95
   Ala Phe Arg Gly Gly Val Tyr Trp Gly Gln Gly Thr Thr Val Thr Val
               100                 105                 110
   Ser Ser Gly Gly Gly Gly Ser Gly Gly Gly Gly Ser Gly Gly Gly Gly
           115                 120                 125
   Ser Gly Gly Gly Gly Ser Asp Val Val Met Thr Gln Ser Pro Asp Ser
       130                 135                 140
   Leu Ala Val Ser Leu Gly Glu Arg Ala Thr Ile Asn Cys Lys Ser Ser
   145                 150                 155                 160
   Gln Ser Leu Leu Asp Ser Asp Gly Lys Thr Tyr Leu Asn Trp Leu Gln
                   165                 170                 175
   Gln Lys Pro Gly Gln Pro Pro Lys Arg Leu Ile Ser Leu Val Ser Lys
               180                 185                 190
   Leu Asp Ser Gly Val Pro Asp Arg Phe Ser Gly Ser Gly Ser Gly Thr
           195                 200                 205
   Asp Phe Thr Leu Thr Ile Ser Ser Leu Gln Ala Glu Asp Val Ala Val
       210                 215                 220
   Tyr Tyr Cys Trp Gln Gly Thr His Phe Pro Gly Thr Phe Gly Gly Gly
   225                 230                 235                 240
   Thr Lys Val Glu Ile Lys
                   245
<210> 77
   <211> 246
   <212> PRT
   <213> Artificial Sequence
<220>
   <221> source
   <223> /note="Description of Artificial Sequence: Synthetic polypeptide"
<400> 77
   Asp Val Val Met Thr Gln Ser Pro Asp Ser Leu Ala Val Ser Leu Gly
   1 5 10 15
   Glu Arg Ala Thr Ile Asn Cys Lys Ser Ser Gln Ser Leu Leu Asp Ser
   20 25 30
   Asp Gly Lys Thr Tyr Leu Asn Trp Leu Gln Gln Lys Pro Gly Gln Pro
   35 40 45
   Pro Lys Arg Leu Ile Ser Leu Val Ser Lys Leu Asp Ser Gly Val Pro
   50 55 60
   Asp Arg Phe Ser Gly Ser Gly Ser Gly Thr Asp Phe Thr Leu Thr Ile
   65 70 75 80
   Ser Ser Leu Gln Ala Glu Asp Val Ala Val Tyr Tyr Cys Trp Gln Gly
   85 90 95
   Thr His Phe Pro Gly Thr Phe Gly Gly Gly Thr Lys Val Glu Ile Lys
   100 105 110
   Gly Gly Gly Gly Ser Gly Gly Gly Gly Ser Gly Gly Gly Gly Ser Gly
   115 120 125
   Gly Gly Gly Ser Glu Ile Gln Leu Val Gln Ser Gly Ala Glu Val Lys
   130 135 140
   Lys Pro Gly Glu Ser Leu Arg Ile Ser Cys Lys Gly Ser Gly Phe Asn
   145 150 155 160
   Ile Glu Asp Tyr Tyr Ile His Trp Val Arg Gln Met Pro Gly Lys Gly
   165 170 175
   Leu Glu Trp Met Gly Arg Ile Asp Pro Glu Asn Asp Glu Thr Lys Tyr
   180 185 190
   Gly Pro Ile Phe Gln Gly His Val Thr Ile Ser Ala Asp Thr Ser Ile
   195 200 205
   Asn Thr Val Tyr Leu Gln Trp Ser Ser Leu Lys Ala Ser Asp Thr Ala
   210 215 220
   Met Tyr Tyr Cys Ala Phe Arg Gly Gly Val Tyr Trp Gly Gln Gly Thr
   225 230 235 240
   Thr Val Thr Val Ser Ser
   245
<210> 78
   <211> 246
   <212> PRT
   <213> Artificial Sequence
<220>
   <221> source
   <223> /note="Description of Artificial Sequence: Synthetic polypeptide"
<400> 78
   Asp Val Val Met Thr Gln Ser Pro Leu Ser Leu Pro Val Thr Leu Gly
   1               5                   10                  15
   Gln Pro Ala Ser Ile Ser Cys Lys Ser Ser Gln Ser Leu Leu Asp Ser
               20                  25                  30
   Asp Gly Lys Thr Tyr Leu Asn Trp Leu Gln Gln Arg Pro Gly Gln Ser
           35                  40                  45
   Pro Arg Arg Leu Ile Ser Leu Val Ser Lys Leu Asp Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Trp Gln Gly
                   85                  90                  95
   Thr His Phe Pro Gly Thr Phe Gly Gly Gly Thr Lys Val Glu Ile Lys
               100                 105                 110
   Gly Gly Gly Gly Ser Gly Gly Gly Gly Ser Gly Gly Gly Gly Ser Gly
           115                 120                 125
   Gly Gly Gly Ser Glu Ile Gln Leu Val Gln Ser Gly Ala Glu Val Lys
       130                 135                 140
   Lys Pro Gly Ala Thr Val Lys Ile Ser Cys Lys Gly Ser Gly Phe Asn
   145                 150                 155                 160
   Ile Glu Asp Tyr Tyr Ile His Trp Val Gln Gln Ala Pro Gly Lys Gly
                   165                 170                 175
   Leu Glu Trp Met Gly Arg Ile Asp Pro Glu Asn Asp Glu Thr Lys Tyr
               180                 185                 190
   Gly Pro Ile Phe Gln Gly Arg Val Thr Ile Thr Ala Asp Thr Ser Thr
           195                 200                 205
   Asn Thr Val Tyr Met Glu Leu Ser Ser Leu Arg Ser Glu Asp Thr Ala
       210                 215                 220
   Val Tyr Tyr Cys Ala Phe Arg Gly Gly Val Tyr Trp Gly Gln Gly Thr
   225                 230                 235                 240
   Thr Val Thr Val Ser Ser
                   245
<210> 79
   <211> 243
   <212> PRT
   <213> Artificial Sequence
<220>
   <221> source
   <223> /note="Description of Artificial Sequence: Synthetic polypeptide"
<400> 79
   Glu Ile Gln Leu Gln Gln Ser Gly Ala Glu Leu Val Lys Pro Gly Ala
   1               5                   10                  15
   Ser Val Lys Leu Ser Cys Thr Gly Ser Gly Phe Asn Ile Glu Asp Tyr
               20                  25                  30
   Tyr Ile His Trp Val Lys Gln Arg Thr Glu Gln Gly Leu Glu Trp Ile
           35                  40                  45
   Gly Arg Ile Asp Pro Glu Asn Asp Glu Thr Lys Tyr Gly Pro Ile Phe
       50                  55                  60
   Gln Gly Arg Ala Thr Ile Thr Ala Asp Thr Ser Ser Asn Thr Val Tyr
   65                  70                  75                  80
   Leu Gln Leu Ser Ser Leu Thr Ser Glu Asp Thr Ala Val Tyr Tyr Cys
                   85                  90                  95
   Ala Phe Arg Gly Gly Val Tyr Trp Gly Pro Gly Thr Thr Leu Thr Val
               100                 105                 110
   Ser Ser Gly Gly Gly Gly Ser Gly Gly Gly Gly Ser Gly Gly Gly Gly
           115                 120                 125
   Ser His Met Asp Val Val Met Thr Gln Ser Pro Leu Thr Leu Ser Val
       130                 135                 140
   Ala Ile Gly Gln Ser Ala Ser Ile Ser Cys Lys Ser Ser Gln Ser Leu
   145                 150                 155                 160
   Leu Asp Ser Asp Gly Lys Thr Tyr Leu Asn Trp Leu Leu Gln Arg Pro
   165 170 175
   Gly Gln Ser Pro Lys Arg Leu Ile Ser Leu Val Ser Lys Leu Asp Ser
   180 185 190
   Gly Val Pro Asp Arg Phe Thr Gly Ser Gly Ser Gly Thr Asp Phe Thr
   195 200 205
   Leu Arg Ile Ser Arg Val Glu Ala Glu Asp Leu Gly Ile Tyr Tyr Cys
   210 215 220
   Trp Gln Gly Thr His Phe Pro Gly Thr Phe Gly Gly Gly Thr Lys Leu
   225 230 235 240
   Glu Ile Lys
<210> 80
   <211> 239
   <212> PRT
   <213> Artificial Sequence
<220>
   <221> source
   <223> /note="Description of Artificial Sequence: Synthetic polypeptide"
<400> 80
   Gln Val Gln Leu Gln Gln Ser Gly Pro Glu Leu Glu Lys Pro Gly Ala
   1 5 10 15
   Ser Val Lys Ile Ser Cys Lys Ala Ser Gly Tyr Ser Phe Thr Gly Tyr
   20 25 30
   Thr Met Asn Trp Val Lys Gln Ser His Gly Lys Ser Leu Glu Trp Ile
   35 40 45
   Gly Leu Ile Thr Pro Tyr Asn Gly Ala Ser Ser Tyr Asn Gln Lys Phe
   50 55 60
   Arg Gly Lys Ala Thr Leu Thr Val Asp Lys Ser Ser Ser Thr Ala Tyr
   65 70 75 80
   Met Asp Leu Leu Ser Leu Thr Ser Glu Asp Ser Ala Val Tyr Phe Cys
   85 90 95
   Ala Arg Gly Gly Tyr Asp Gly Arg Gly Phe Asp Tyr Trp Gly Gln Gly
   100 105 110
   Thr Thr Val Thr Val Ser Ser Gly Gly Gly Gly Ser Gly Gly Gly Gly
   115 120 125
   Ser Gly Gly Gly Gly Ser Asp Ile Glu Leu Thr Gln Ser Pro Ala Ile
   130 135 140
   Met Ser Ala Ser Pro Gly Glu Lys Val Thr Met Thr Cys Ser Ala Ser
   145 150 155 160
   Ser Ser Val Ser Tyr Met His Trp Tyr Gln Gln Lys Ser Gly Thr Ser
   165 170 175
   Pro Lys Arg Trp Ile Tyr Asp Thr Ser Lys Leu Ala Ser Gly Val Pro
   180 185 190
   Gly Arg Phe Ser Gly Ser Gly Ser Gly Asn Ser Tyr Ser Leu Thr Ile
   195 200 205
   Ser Ser Val Glu Ala Glu Asp Asp Ala Thr Tyr Tyr Cys Gln Gln Trp
   210 215 220
   Ser Gly Tyr Pro Leu Thr Phe Gly Ala Gly Thr Lys Leu Glu Ile
   225 230 235
<210> 81
   <211> 256
   <212> PRT
   <213> Artificial Sequence
<220>
   <221> source
   <223> /note="Description of Artificial Sequence: Synthetic polypeptide"
<400> 81
   Ser Arg Ala Ala Gln Pro Ala Met Ala Gln Val Gln Leu Gln Gln Ser
   1 5 10 15
   Gly Ala Glu Leu Val Lys Pro Gly Ala Ser Val Leu Ile Ser Cys Lys
   20 25 30
   Ala Ser Gly Tyr Ser Phe Thr Gly Tyr Phe Met Asn Trp Val Lys Gln
   35 40 45
   Ser His Gly Lys Ser Leu Glu Trp Ile Gly Arg Ile His Pro Tyr Asp
   50 55 60
   Gly Asp Thr Phe Tyr Asn Gln Asn Phe Lys Asp Lys Ala Thr Leu Thr
   65 70 75 80
   Val Asp Lys Ser Ser Asn Thr Ala His Met Glu Leu Leu Ser Leu Thr
   85 90 95
   Ser Glu Asp Phe Ala Val Tyr Tyr Cys Thr Arg Tyr Asp Gly Ser Arg
   100 105 110
   Ala Met Asp Tyr Trp Gly Gln Gly Thr Thr Val Thr Val Ser Ser Gly
   115 120 125
   Gly Gly Gly Ser Gly Gly Gly Gly Ser Gly Gly Gly Gly Ser Asp Ile
   130 135 140
   Glu Leu Thr Gln Ser Pro Ala Ser Leu Ala Val Ser Leu Gly Gln Arg
   145 150 155 160
   Ala Ile Ile Ser Cys Lys Ala Ser Gln Ser Val Ser Phe Ala Gly Thr
   165 170 175
   Ser Leu Met His Trp Tyr His Gln Lys Pro Gly Gln Gln Pro Lys Leu
   180 185 190
   Leu Ile Tyr Arg Ala Ser Asn Leu Glu Ala Gly Val Pro Thr Arg Phe
   195 200 205
   Ser Gly Ser Gly Ser Lys Thr Asp Phe Thr Leu Asn Ile His Pro Val
   210 215 220
   Glu Glu Glu Asp Ala Ala Thr Tyr Tyr Cys Gln Gln Ser Arg Glu Tyr
   225 230 235 240
   Pro Tyr Thr Phe Gly Gly Gly Thr Lys Leu Glu Ile Lys Arg Ala Ala
   245 250 255
<210> 82
   <211> 119
   <212> PRT
   <213> Artificial Sequence
<220>
   <221> source
   <223> /note="Description of Artificial Sequence: Synthetic polypeptide"
<400> 82
   Gln Val Gln Leu Leu Glu Ser Gly Ala Glu Leu Val Arg Pro Gly Ser
   1 5 10 15
   Ser Val Lys Ile Ser Cys Lys Ala Ser Gly Tyr Ala Phe Ser Ser Tyr
   20 25 30
   Trp Met Asn Trp Val Lys Gln Arg Pro Gly Gln Gly Leu Glu Trp Ile
   35 40 45
   Gly Gln Ile Tyr Pro Gly Asp Gly Asp Thr Asn Tyr Asn Gly Lys Phe
   50 55 60
   Lys Gly Gln Ala Thr Leu Thr Ala Asp Lys Ser Ser Ser Thr Ala Tyr
   65 70 75 80
   Met Gln Leu Ser Gly Leu Thr Ser Glu Asp Ser Ala Val Tyr Ser Cys
   85 90 95
   Ala Arg Lys Thr Ile Ser Ser Val Val Asp Phe Tyr Phe Asp Tyr Trp
   100 105 110
   Gly Gln Gly Thr Thr Val Thr
   115
<210> 83
   <211> 111
   <212> PRT
   <213> Artificial Sequence
<220>
   <221> source
   <223> /note="Description of Artificial Sequence: Synthetic polypeptide"
<400> 83
   Glu Leu Val Leu Thr Gln Ser Pro Lys Phe Met Ser Thr Ser Val Gly
   1 5 10 15
   Asp Arg Val Ser Val Thr Cys Lys Ala Ser Gln Asn Val Gly Thr Asn
   20 25 30
   Val Ala Trp Tyr Gln Gln Lys Pro Gly Gln Ser Pro Lys Pro Leu Ile
   35 40 45
   Tyr Ser Ala Thr Tyr Arg Asn Ser Gly Val Pro Asp Arg Phe Thr Gly
   50 55 60
   Ser Gly Ser Gly Thr Asp Phe Thr Leu Thr Ile Thr Asn Val Gln Ser
   65 70 75 80
   Lys Asp Leu Ala Asp Tyr Phe Tyr Phe Cys Gln Tyr Asn Arg Tyr Pro
   85 90 95
   Tyr Thr Ser Gly Gly Gly Thr Lys Leu Glu Ile Lys Arg Arg Ser
   100 105 110
<210> 84
   <211> 4
   <212> PRT
   <213> Artificial Sequence
<220>
   <221> source
   <223> /note="Description of Artificial Sequence: Synthetic peptide"
<400> 84
   Arg Gly Asp Ser
   1
<210> 85
   <211> 4
   <212> PRT
   <213> Artificial Sequence
<220>
   <221> source
   <223> /note="Description of Artificial Sequence: Synthetic peptide"
<400> 85
   Tyr Met Phe Met
   1
<210> 86
   <211> 18
   <212> PRT
   <213> Artificial Sequence
<220>
   <221> source
   <223> /note="Description of Artificial Sequence: Synthetic peptide"
<400> 86
   Gly Ser Thr Ser Gly Ser Gly Lys Pro Gly Ser Gly Glu Gly Ser Thr
   1               5                   10                  15
   Lys Gly
<210> 87
   <211> 5000
   <212> DNA
   <213> Artificial Sequence
<220>
   <221> source
   <223> /note="Description of Artificial Sequence: Synthetic polynucleotide"
<220>
   <221> misc_feature
   <222> (1)..(5000)
   <223> /note="This sequence may encompass 50-5,000 nucleotides, wherein some positions may be absent"
<220>
   <221> source
   <223> /note="See specification as filed for detailed description of substitutions and preferred embodiments"
<400> 87
   tttttttttt tttttttttt tttttttttt tttttttttt tttttttttt tttttttttt 60
   tttttttttt tttttttttt tttttttttt tttttttttt tttttttttt tttttttttt 120
   tttttttttt tttttttttt tttttttttt tttttttttt tttttttttt tttttttttt 180
   tttttttttt tttttttttt tttttttttt tttttttttt tttttttttt tttttttttt 240
   tttttttttt tttttttttt tttttttttt tttttttttt tttttttttt tttttttttt 300
   tttttttttt tttttttttt tttttttttt tttttttttt tttttttttt tttttttttt 360
   tttttttttt tttttttttt tttttttttt tttttttttt tttttttttt tttttttttt 420
   tttttttttt tttttttttt tttttttttt tttttttttt tttttttttt tttttttttt 480
   tttttttttt tttttttttt tttttttttt tttttttttt tttttttttt tttttttttt 540
   tttttttttt tttttttttt tttttttttt tttttttttt tttttttttt tttttttttt 600
   tttttttttt tttttttttt tttttttttt tttttttttt tttttttttt tttttttttt 660
   tttttttttt tttttttttt tttttttttt tttttttttt tttttttttt tttttttttt 720
   tttttttttt tttttttttt tttttttttt tttttttttt tttttttttt tttttttttt 780
   tttttttttt tttttttttt tttttttttt tttttttttt tttttttttt tttttttttt 840
   tttttttttt tttttttttt tttttttttt tttttttttt tttttttttt tttttttttt 900
   tttttttttt tttttttttt tttttttttt tttttttttt tttttttttt tttttttttt 960
   tttttttttt tttttttttt tttttttttt tttttttttt tttttttttt tttttttttt 1020
   tttttttttt tttttttttt tttttttttt tttttttttt tttttttttt tttttttttt 1080
   tttttttttt tttttttttt tttttttttt tttttttttt tttttttttt tttttttttt 1140
   tttttttttt tttttttttt tttttttttt tttttttttt tttttttttt tttttttttt 1200
   tttttttttt tttttttttt tttttttttt tttttttttt tttttttttt tttttttttt 1260
   tttttttttt tttttttttt tttttttttt tttttttttt tttttttttt tttttttttt 1320
   tttttttttt tttttttttt tttttttttt tttttttttt tttttttttt tttttttttt 1380
   tttttttttt tttttttttt tttttttttt tttttttttt tttttttttt tttttttttt 1440
   tttttttttt tttttttttt tttttttttt tttttttttt tttttttttt tttttttttt 1500
   tttttttttt tttttttttt tttttttttt tttttttttt tttttttttt tttttttttt 1560
   tttttttttt tttttttttt tttttttttt tttttttttt tttttttttt tttttttttt 1620
   tttttttttt tttttttttt tttttttttt tttttttttt tttttttttt tttttttttt 1680
   tttttttttt tttttttttt tttttttttt tttttttttt tttttttttt tttttttttt 1740
   tttttttttt tttttttttt tttttttttt tttttttttt tttttttttt tttttttttt 1800
   tttttttttt tttttttttt tttttttttt tttttttttt tttttttttt tttttttttt 1860
   tttttttttt tttttttttt tttttttttt tttttttttt tttttttttt tttttttttt 1920
   tttttttttt tttttttttt tttttttttt tttttttttt tttttttttt tttttttttt 1980
   tttttttttt tttttttttt tttttttttt tttttttttt tttttttttt tttttttttt 2040
   tttttttttt tttttttttt tttttttttt tttttttttt tttttttttt tttttttttt 2100
   tttttttttt tttttttttt tttttttttt tttttttttt tttttttttt tttttttttt 2160
   tttttttttt tttttttttt tttttttttt tttttttttt tttttttttt tttttttttt 2220
   tttttttttt tttttttttt tttttttttt tttttttttt tttttttttt tttttttttt 2280
   tttttttttt tttttttttt tttttttttt tttttttttt tttttttttt tttttttttt 2340
   tttttttttt tttttttttt tttttttttt tttttttttt tttttttttt tttttttttt 2400
   tttttttttt tttttttttt tttttttttt tttttttttt tttttttttt tttttttttt 2460
   tttttttttt tttttttttt tttttttttt tttttttttt tttttttttt tttttttttt 2520
   tttttttttt tttttttttt tttttttttt tttttttttt tttttttttt tttttttttt 2580
   tttttttttt tttttttttt tttttttttt tttttttttt tttttttttt tttttttttt 2640
   tttttttttt tttttttttt tttttttttt tttttttttt tttttttttt tttttttttt 2700
   tttttttttt tttttttttt tttttttttt tttttttttt tttttttttt tttttttttt 2760
   tttttttttt tttttttttt tttttttttt tttttttttt tttttttttt tttttttttt 2820
   tttttttttt tttttttttt tttttttttt tttttttttt tttttttttt tttttttttt 2880
   tttttttttt tttttttttt tttttttttt tttttttttt tttttttttt tttttttttt 2940
   tttttttttt tttttttttt tttttttttt tttttttttt tttttttttt tttttttttt 3000
   tttttttttt tttttttttt tttttttttt tttttttttt tttttttttt tttttttttt 3060
   tttttttttt tttttttttt tttttttttt tttttttttt tttttttttt tttttttttt 3120
   tttttttttt tttttttttt tttttttttt tttttttttt tttttttttt tttttttttt 3180
   tttttttttt tttttttttt tttttttttt tttttttttt tttttttttt tttttttttt 3240
   tttttttttt tttttttttt tttttttttt tttttttttt tttttttttt tttttttttt 3300
   tttttttttt tttttttttt tttttttttt tttttttttt tttttttttt tttttttttt 3360
   tttttttttt tttttttttt tttttttttt tttttttttt tttttttttt tttttttttt 3420
   tttttttttt tttttttttt tttttttttt tttttttttt tttttttttt tttttttttt 3480
   tttttttttt tttttttttt tttttttttt tttttttttt tttttttttt tttttttttt 3540
   tttttttttt tttttttttt tttttttttt tttttttttt tttttttttt tttttttttt 3600
   tttttttttt tttttttttt tttttttttt tttttttttt tttttttttt tttttttttt 3660
   tttttttttt tttttttttt tttttttttt tttttttttt tttttttttt tttttttttt 3720
   tttttttttt tttttttttt tttttttttt tttttttttt tttttttttt tttttttttt 3780
   tttttttttt tttttttttt tttttttttt tttttttttt tttttttttt tttttttttt 3840
   tttttttttt tttttttttt tttttttttt tttttttttt tttttttttt tttttttttt 3900
   tttttttttt tttttttttt tttttttttt tttttttttt tttttttttt tttttttttt 3960
   tttttttttt tttttttttt tttttttttt tttttttttt tttttttttt tttttttttt 4020
   tttttttttt tttttttttt tttttttttt tttttttttt tttttttttt tttttttttt 4080
   tttttttttt tttttttttt tttttttttt tttttttttt tttttttttt tttttttttt 4140
   tttttttttt tttttttttt tttttttttt tttttttttt tttttttttt tttttttttt 4200
   tttttttttt tttttttttt tttttttttt tttttttttt tttttttttt tttttttttt 4260
   tttttttttt tttttttttt tttttttttt tttttttttt tttttttttt tttttttttt 4320
   tttttttttt tttttttttt tttttttttt tttttttttt tttttttttt tttttttttt 4380
   tttttttttt tttttttttt tttttttttt tttttttttt tttttttttt tttttttttt 4440
   tttttttttt tttttttttt tttttttttt tttttttttt tttttttttt tttttttttt 4500
   tttttttttt tttttttttt tttttttttt tttttttttt tttttttttt tttttttttt 4560
   tttttttttt tttttttttt tttttttttt tttttttttt tttttttttt tttttttttt 4620
   tttttttttt tttttttttt tttttttttt tttttttttt tttttttttt tttttttttt 4680
   tttttttttt tttttttttt tttttttttt tttttttttt tttttttttt tttttttttt 4740
   tttttttttt tttttttttt tttttttttt tttttttttt tttttttttt tttttttttt 4800
   tttttttttt tttttttttt tttttttttt tttttttttt tttttttttt tttttttttt 4860
   tttttttttt tttttttttt tttttttttt tttttttttt tttttttttt tttttttttt 4920
   tttttttttt tttttttttt tttttttttt tttttttttt tttttttttt tttttttttt 4980
   tttttttttt tttttttttt 5000
<210> 88
   <211> 5000
   <212> DNA
   <213> Artificial Sequence
<220>
   <221> source
   <223> /note="Description of Artificial Sequence: Synthetic polynucleotide"
<220>
   <221> misc_feature
   <222> (1)..(5000)
   <223> /note="This sequence may encompass 100-5,000 nucleotides, wherein some positions may be absent"
<220>
   <221> source
   <223> /note="See specification as filed for detailed description of substitutions and preferred embodiments"
<400> 88
   aaaaaaaaaa aaaaaaaaaa aaaaaaaaaa aaaaaaaaaa aaaaaaaaaa aaaaaaaaaa      60
   aaaaaaaaaa aaaaaaaaaa aaaaaaaaaa aaaaaaaaaa aaaaaaaaaa aaaaaaaaaa     120
   aaaaaaaaaa aaaaaaaaaa aaaaaaaaaa aaaaaaaaaa aaaaaaaaaa aaaaaaaaaa     180
   aaaaaaaaaa aaaaaaaaaa aaaaaaaaaa aaaaaaaaaa aaaaaaaaaa aaaaaaaaaa     240
   aaaaaaaaaa aaaaaaaaaa aaaaaaaaaa aaaaaaaaaa aaaaaaaaaa aaaaaaaaaa     300
   aaaaaaaaaa aaaaaaaaaa aaaaaaaaaa aaaaaaaaaa aaaaaaaaaa aaaaaaaaaa     360
   aaaaaaaaaa aaaaaaaaaa aaaaaaaaaa aaaaaaaaaa aaaaaaaaaa aaaaaaaaaa     420
   aaaaaaaaaa aaaaaaaaaa aaaaaaaaaa aaaaaaaaaa aaaaaaaaaa aaaaaaaaaa     480
   aaaaaaaaaa aaaaaaaaaa aaaaaaaaaa aaaaaaaaaa aaaaaaaaaa aaaaaaaaaa     540
   aaaaaaaaaa aaaaaaaaaa aaaaaaaaaa aaaaaaaaaa aaaaaaaaaa aaaaaaaaaa     600
   aaaaaaaaaa aaaaaaaaaa aaaaaaaaaa aaaaaaaaaa aaaaaaaaaa aaaaaaaaaa     660
   aaaaaaaaaa aaaaaaaaaa aaaaaaaaaa aaaaaaaaaa aaaaaaaaaa aaaaaaaaaa     720
   aaaaaaaaaa aaaaaaaaaa aaaaaaaaaa aaaaaaaaaa aaaaaaaaaa aaaaaaaaaa     780
   aaaaaaaaaa aaaaaaaaaa aaaaaaaaaa aaaaaaaaaa aaaaaaaaaa aaaaaaaaaa     840
   aaaaaaaaaa aaaaaaaaaa aaaaaaaaaa aaaaaaaaaa aaaaaaaaaa aaaaaaaaaa     900
   aaaaaaaaaa aaaaaaaaaa aaaaaaaaaa aaaaaaaaaa aaaaaaaaaa aaaaaaaaaa     960
   aaaaaaaaaa aaaaaaaaaa aaaaaaaaaa aaaaaaaaaa aaaaaaaaaa aaaaaaaaaa    1020
   aaaaaaaaaa aaaaaaaaaa aaaaaaaaaa aaaaaaaaaa aaaaaaaaaa aaaaaaaaaa    1080
   aaaaaaaaaa aaaaaaaaaa aaaaaaaaaa aaaaaaaaaa aaaaaaaaaa aaaaaaaaaa    1140
   aaaaaaaaaa aaaaaaaaaa aaaaaaaaaa aaaaaaaaaa aaaaaaaaaa aaaaaaaaaa    1200
   aaaaaaaaaa aaaaaaaaaa aaaaaaaaaa aaaaaaaaaa aaaaaaaaaa aaaaaaaaaa    1260
   aaaaaaaaaa aaaaaaaaaa aaaaaaaaaa aaaaaaaaaa aaaaaaaaaa aaaaaaaaaa    1320
   aaaaaaaaaa aaaaaaaaaa aaaaaaaaaa aaaaaaaaaa aaaaaaaaaa aaaaaaaaaa    1380
   aaaaaaaaaa aaaaaaaaaa aaaaaaaaaa aaaaaaaaaa aaaaaaaaaa aaaaaaaaaa    1440
   aaaaaaaaaa aaaaaaaaaa aaaaaaaaaa aaaaaaaaaa aaaaaaaaaa aaaaaaaaaa    1500
   aaaaaaaaaa aaaaaaaaaa aaaaaaaaaa aaaaaaaaaa aaaaaaaaaa aaaaaaaaaa 1560
   aaaaaaaaaa aaaaaaaaaa aaaaaaaaaa aaaaaaaaaa aaaaaaaaaa aaaaaaaaaa 1620
   aaaaaaaaaa aaaaaaaaaa aaaaaaaaaa aaaaaaaaaa aaaaaaaaaa aaaaaaaaaa 1680
   aaaaaaaaaa aaaaaaaaaa aaaaaaaaaa aaaaaaaaaa aaaaaaaaaa aaaaaaaaaa 1740
   aaaaaaaaaa aaaaaaaaaa aaaaaaaaaa aaaaaaaaaa aaaaaaaaaa aaaaaaaaaa 1800
   aaaaaaaaaa aaaaaaaaaa aaaaaaaaaa aaaaaaaaaa aaaaaaaaaa aaaaaaaaaa 1860
   aaaaaaaaaa aaaaaaaaaa aaaaaaaaaa aaaaaaaaaa aaaaaaaaaa aaaaaaaaaa 1920
   aaaaaaaaaa aaaaaaaaaa aaaaaaaaaa aaaaaaaaaa aaaaaaaaaa aaaaaaaaaa 1980
   aaaaaaaaaa aaaaaaaaaa aaaaaaaaaa aaaaaaaaaa aaaaaaaaaa aaaaaaaaaa 2040
   aaaaaaaaaa aaaaaaaaaa aaaaaaaaaa aaaaaaaaaa aaaaaaaaaa aaaaaaaaaa 2100
   aaaaaaaaaa aaaaaaaaaa aaaaaaaaaa aaaaaaaaaa aaaaaaaaaa aaaaaaaaaa 2160
   aaaaaaaaaa aaaaaaaaaa aaaaaaaaaa aaaaaaaaaa aaaaaaaaaa aaaaaaaaaa 2220
   aaaaaaaaaa aaaaaaaaaa aaaaaaaaaa aaaaaaaaaa aaaaaaaaaa aaaaaaaaaa 2280
   aaaaaaaaaa aaaaaaaaaa aaaaaaaaaa aaaaaaaaaa aaaaaaaaaa aaaaaaaaaa 2340
   aaaaaaaaaa aaaaaaaaaa aaaaaaaaaa aaaaaaaaaa aaaaaaaaaa aaaaaaaaaa 2400
   aaaaaaaaaa aaaaaaaaaa aaaaaaaaaa aaaaaaaaaa aaaaaaaaaa aaaaaaaaaa 2460
   aaaaaaaaaa aaaaaaaaaa aaaaaaaaaa aaaaaaaaaa aaaaaaaaaa aaaaaaaaaa 2520
   aaaaaaaaaa aaaaaaaaaa aaaaaaaaaa aaaaaaaaaa aaaaaaaaaa aaaaaaaaaa 2580
   aaaaaaaaaa aaaaaaaaaa aaaaaaaaaa aaaaaaaaaa aaaaaaaaaa aaaaaaaaaa 2640
   aaaaaaaaaa aaaaaaaaaa aaaaaaaaaa aaaaaaaaaa aaaaaaaaaa aaaaaaaaaa 2700
   aaaaaaaaaa aaaaaaaaaa aaaaaaaaaa aaaaaaaaaa aaaaaaaaaa aaaaaaaaaa 2760
   aaaaaaaaaa aaaaaaaaaa aaaaaaaaaa aaaaaaaaaa aaaaaaaaaa aaaaaaaaaa 2820
   aaaaaaaaaa aaaaaaaaaa aaaaaaaaaa aaaaaaaaaa aaaaaaaaaa aaaaaaaaaa 2880
   aaaaaaaaaa aaaaaaaaaa aaaaaaaaaa aaaaaaaaaa aaaaaaaaaa aaaaaaaaaa 2940
   aaaaaaaaaa aaaaaaaaaa aaaaaaaaaa aaaaaaaaaa aaaaaaaaaa aaaaaaaaaa 3000
   aaaaaaaaaa aaaaaaaaaa aaaaaaaaaa aaaaaaaaaa aaaaaaaaaa aaaaaaaaaa 3060
   aaaaaaaaaa aaaaaaaaaa aaaaaaaaaa aaaaaaaaaa aaaaaaaaaa aaaaaaaaaa 3120
   aaaaaaaaaa aaaaaaaaaa aaaaaaaaaa aaaaaaaaaa aaaaaaaaaa aaaaaaaaaa 3180
   aaaaaaaaaa aaaaaaaaaa aaaaaaaaaa aaaaaaaaaa aaaaaaaaaa aaaaaaaaaa 3240
   aaaaaaaaaa aaaaaaaaaa aaaaaaaaaa aaaaaaaaaa aaaaaaaaaa aaaaaaaaaa 3300
   aaaaaaaaaa aaaaaaaaaa aaaaaaaaaa aaaaaaaaaa aaaaaaaaaa aaaaaaaaaa 3360
   aaaaaaaaaa aaaaaaaaaa aaaaaaaaaa aaaaaaaaaa aaaaaaaaaa aaaaaaaaaa 3420
   aaaaaaaaaa aaaaaaaaaa aaaaaaaaaa aaaaaaaaaa aaaaaaaaaa aaaaaaaaaa 3480
   aaaaaaaaaa aaaaaaaaaa aaaaaaaaaa aaaaaaaaaa aaaaaaaaaa aaaaaaaaaa 3540
   aaaaaaaaaa aaaaaaaaaa aaaaaaaaaa aaaaaaaaaa aaaaaaaaaa aaaaaaaaaa 3600
   aaaaaaaaaa aaaaaaaaaa aaaaaaaaaa aaaaaaaaaa aaaaaaaaaa aaaaaaaaaa 3660
   aaaaaaaaaa aaaaaaaaaa aaaaaaaaaa aaaaaaaaaa aaaaaaaaaa aaaaaaaaaa 3720
   aaaaaaaaaa aaaaaaaaaa aaaaaaaaaa aaaaaaaaaa aaaaaaaaaa aaaaaaaaaa 3780
   aaaaaaaaaa aaaaaaaaaa aaaaaaaaaa aaaaaaaaaa aaaaaaaaaa aaaaaaaaaa 3840
   aaaaaaaaaa aaaaaaaaaa aaaaaaaaaa aaaaaaaaaa aaaaaaaaaa aaaaaaaaaa 3900
   aaaaaaaaaa aaaaaaaaaa aaaaaaaaaa aaaaaaaaaa aaaaaaaaaa aaaaaaaaaa 3960
   aaaaaaaaaa aaaaaaaaaa aaaaaaaaaa aaaaaaaaaa aaaaaaaaaa aaaaaaaaaa 4020
   aaaaaaaaaa aaaaaaaaaa aaaaaaaaaa aaaaaaaaaa aaaaaaaaaa aaaaaaaaaa 4080
   aaaaaaaaaa aaaaaaaaaa aaaaaaaaaa aaaaaaaaaa aaaaaaaaaa aaaaaaaaaa 4140
   aaaaaaaaaa aaaaaaaaaa aaaaaaaaaa aaaaaaaaaa aaaaaaaaaa aaaaaaaaaa 4200
   aaaaaaaaaa aaaaaaaaaa aaaaaaaaaa aaaaaaaaaa aaaaaaaaaa aaaaaaaaaa 4260
   aaaaaaaaaa aaaaaaaaaa aaaaaaaaaa aaaaaaaaaa aaaaaaaaaa aaaaaaaaaa 4320
   aaaaaaaaaa aaaaaaaaaa aaaaaaaaaa aaaaaaaaaa aaaaaaaaaa aaaaaaaaaa 4380
   aaaaaaaaaa aaaaaaaaaa aaaaaaaaaa aaaaaaaaaa aaaaaaaaaa aaaaaaaaaa 4440
   aaaaaaaaaa aaaaaaaaaa aaaaaaaaaa aaaaaaaaaa aaaaaaaaaa aaaaaaaaaa 4500
   aaaaaaaaaa aaaaaaaaaa aaaaaaaaaa aaaaaaaaaa aaaaaaaaaa aaaaaaaaaa 4560
   aaaaaaaaaa aaaaaaaaaa aaaaaaaaaa aaaaaaaaaa aaaaaaaaaa aaaaaaaaaa 4620
   aaaaaaaaaa aaaaaaaaaa aaaaaaaaaa aaaaaaaaaa aaaaaaaaaa aaaaaaaaaa 4680
   aaaaaaaaaa aaaaaaaaaa aaaaaaaaaa aaaaaaaaaa aaaaaaaaaa aaaaaaaaaa 4740
   aaaaaaaaaa aaaaaaaaaa aaaaaaaaaa aaaaaaaaaa aaaaaaaaaa aaaaaaaaaa 4800
   aaaaaaaaaa aaaaaaaaaa aaaaaaaaaa aaaaaaaaaa aaaaaaaaaa aaaaaaaaaa 4860
   aaaaaaaaaa aaaaaaaaaa aaaaaaaaaa aaaaaaaaaa aaaaaaaaaa aaaaaaaaaa 4920
   aaaaaaaaaa aaaaaaaaaa aaaaaaaaaa aaaaaaaaaa aaaaaaaaaa aaaaaaaaaa 4980
   aaaaaaaaaa aaaaaaaaaa 5000
<210> 89
   <211> 486
   <212> PRT
   <213> Artificial Sequence
<220>
   <221> source
   <223> /note="Description of Artificial Sequence: Synthetic polypeptide"
<400> 89
   Met Ala Leu Pro Val Thr Ala Leu Leu Leu Pro Leu Ala Leu Leu Leu
   1               5                   10                  15
   His Ala Ala Arg Pro Asp Ile Gln Met Thr Gln Thr Thr Ser Ser Leu
               20                  25                  30
   Ser Ala Ser Leu Gly Asp Arg Val Thr Ile Ser Cys Arg Ala Ser Gln
           35                  40                  45
   Asp Ile Ser Lys Tyr Leu Asn Trp Tyr Gln Gln Lys Pro Asp Gly Thr
       50                  55                  60
   Val Lys Leu Leu Ile Tyr His Thr Ser Arg Leu His Ser Gly Val Pro
   65                  70                  75                  80
   Ser Arg Phe Ser Gly Ser Gly Ser Gly Thr Asp Tyr Ser Leu Thr Ile
                   85                  90                  95
   Ser Asn Leu Glu Gln Glu Asp Ile Ala Thr Tyr Phe Cys Gln Gln Gly
               100                 105                 110
   Asn Thr Leu Pro Tyr Thr Phe Gly Gly Gly Thr Lys Leu Glu Ile Thr
           115                 120                 125
   Gly Gly Gly Gly Ser Gly Gly Gly Gly Ser Gly Gly Gly Gly Ser Glu
       130                 135                 140
   Val Lys Leu Gln Glu Ser Gly Pro Gly Leu Val Ala Pro Ser Gln Ser
   145                 150                 155                 160
   Leu Ser Val Thr Cys Thr Val Ser Gly Val Ser Leu Pro Asp Tyr Gly
                   165                 170                 175
   Val Ser Trp Ile Arg Gln Pro Pro Arg Lys Gly Leu Glu Trp Leu Gly
               180                 185                 190
   Val Ile Trp Gly Ser Glu Thr Thr Tyr Tyr Asn Ser Ala Leu Lys Ser
           195                 200                 205
   Arg Leu Thr Ile Ile Lys Asp Asn Ser Lys Ser Gln Val Phe Leu Lys
       210                 215                 220
   Met Asn Ser Leu Gln Thr Asp Asp Thr Ala Ile Tyr Tyr Cys Ala Lys
   225                 230                 235                 240
   His Tyr Tyr Tyr Gly Gly Ser Tyr Ala Met Asp Tyr Trp Gly Gln Gly
                   245                 250                 255
   Thr Ser Val Thr Val Ser Ser Thr Thr Thr Pro Ala Pro Arg Pro Pro
               260                 265                 270
   Thr Pro Ala Pro Thr Ile Ala Ser Gln Pro Leu Ser Leu Arg Pro Glu
           275                 280                 285
   Ala Cys Arg Pro Ala Ala Gly Gly Ala Val His Thr Arg Gly Leu Asp
       290                 295                 300
   Phe Ala Cys Asp Ile Tyr Ile Trp Ala Pro Leu Ala Gly Thr Cys Gly
   305                 310                 315                 320
   Val Leu Leu Leu Ser Leu Val Ile Thr Leu Tyr Cys Lys Arg Gly Arg
                   325                 330                 335
   Lys Lys Leu Leu Tyr Ile Phe Lys Gln Pro Phe Met Arg Pro Val Gln
               340                 345                 350
   Thr Thr Gln Glu Glu Asp Gly Cys Ser Cys Arg Phe Pro Glu Glu Glu
           355                 360                 365
   Glu Gly Gly Cys Glu Leu Arg Val Lys Phe Ser Arg Ser Ala Asp Ala
       370                 375                 380
   Pro Ala Tyr Lys Gln Gly Gln Asn Gln Leu Tyr Asn Glu Leu Asn Leu
   385                 390                 395                 400
   Gly Arg Arg Glu Glu Tyr Asp Val Leu Asp Lys Arg Arg Gly Arg Asp
                   405                 410                 415
   Pro Glu Met Gly Gly Lys Pro Arg Arg Lys Asn Pro Gln Glu Gly Leu
               420                 425                 430
   Tyr Asn Glu Leu Gln Lys Asp Lys Met Ala Glu Ala Tyr Ser Glu Ile
           435                 440                 445
   Gly Met Lys Gly Glu Arg Arg Arg Gly Lys Gly His Asp Gly Leu Tyr
       450                 455                 460
   Gln Gly Leu Ser Thr Ala Thr Lys Asp Thr Tyr Asp Ala Leu His Met
   465                 470                 475                 480
   Gln Ala Leu Pro Pro Arg
   485
<210> 90
   <211> 132
   <212> PRT
   <213> Artificial Sequence
<220>
   <221> source
   <223> /note="Description of Artificial Sequence: Synthetic polypeptide"
<400> 90
   Asp Val Pro Asp Tyr Ala Ser Leu Gly Gly Pro Ser Ser Pro Lys Lys
   1               5                   10                  15
   Lys Arg Lys Val Ser Arg Gly Val Gln Val Glu Thr Ile Ser Pro Gly
               20                  25                  30
   Asp Gly Arg Thr Phe Pro Lys Arg Gly Gln Thr Cys Val Val His Tyr
           35                  40                  45
   Thr Gly Met Leu Glu Asp Gly Lys Lys Phe Asp Ser Ser Arg Asp Arg
       50                  55                  60
   Asn Lys Pro Phe Lys Phe Met Leu Gly Lys Gln Glu Val Ile Arg Gly
   65                  70                  75                  80
   Trp Glu Glu Gly Val Ala Gln Met Ser Val Gly Gln Arg Ala Lys Leu
                   85                  90                  95
   Thr Ile Ser Pro Asp Tyr Ala Tyr Gly Ala Thr Gly His Pro Gly Ile
               100                 105                 110
   Ile Pro Pro His Ala Thr Leu Val Phe Asp Val Glu Leu Leu Lys Leu
           115                 120                 125
   Glu Thr Ser Tyr
       130
<210> 91
   <211> 108
   <212> PRT
   <213> Artificial Sequence
<220>
   <221> source
   <223> /note="Description of Artificial Sequence: Synthetic polypeptide"
<400> 91
   Val Gln Val Glu Thr Ile Ser Pro Gly Asp Gly Arg Thr Phe Pro Lys
   1               5                   10                  15
   Arg Gly Gln Thr Cys Val Val His Tyr Thr Gly Met Leu Glu Asp Gly
               20                  25                  30
   Lys Lys Phe Asp Ser Ser Arg Asp Arg Asn Lys Pro Phe Lys Phe Met
           35                  40                  45
   Leu Gly Lys Gln Glu Val Ile Arg Gly Trp Glu Glu Gly Val Ala Gln
       50                  55                  60
   Met Ser Val Gly Gln Arg Ala Lys Leu Thr Ile Ser Pro Asp Tyr Ala
   65                  70                  75                  80
   Tyr Gly Ala Thr Gly His Pro Gly Ile Ile Pro Pro His Ala Thr Leu
                   85                  90                  95
   Val Phe Asp Val Glu Leu Leu Lys Leu Glu Thr Ser
               100                 105
<210> 92
   <211> 93
   <212> PRT
   <213> Homo sapiens
<400> 92
<210> 93
   <211> 95
   <212> PRT
   <213> Artificial Sequence
<220>
   <221> source
   <223> /note="Description of Artificial Sequence: Synthetic polypeptide"
<400> 93
   Ile Leu Trp His Glu Met Trp His Glu Gly Leu Ile Glu Ala Ser Arg
   1               5                   10                  15
   Leu Tyr Phe Gly Glu Arg Asn Val Lys Gly Met Phe Glu Val Leu Glu
               20                  25                  30
   Pro Leu His Ala Met Met Glu Arg Gly Pro Gln Thr Leu Lys Glu Thr
           35                  40                  45
   Ser Phe Asn Gln Ala Tyr Gly Arg Asp Leu Met Glu Ala Gln Glu Trp
       50                  55                  60
   Cys Arg Lys Tyr Met Lys Ser Gly Asn Val Lys Asp Leu Thr Gln Ala
   65                  70                  75                  80
   Trp Asp Leu Tyr Tyr His Val Phe Arg Arg Ile Ser Lys Thr Ser
                   85                  90                  95
<210> 94
   <211> 95
   <212> PRT
   <213> Artificial Sequence
<220>
   <221> source
   <223> /note="Description of Artificial Sequence: Synthetic polypeptide"
<400> 94
   Ile Leu Trp His Glu Met Trp His Glu Gly Leu Leu Glu Ala Ser Arg
   1               5                   10                  15
   Leu Tyr Phe Gly Glu Arg Asn Val Lys Gly Met Phe Glu Val Leu Glu
               20                  25                  30
   Pro Leu His Ala Met Met Glu Arg Gly Pro Gln Thr Leu Lys Glu Thr
           35                  40                  45
   Ser Phe Asn Gln Ala Tyr Gly Arg Asp Leu Met Glu Ala Gln Glu Trp
       50                  55                  60
   Cys Arg Lys Tyr Met Lys Ser Gly Asn Val Lys Asp Leu Thr Gln Ala
   65                  70                  75                  80
   Trp Asp Leu Tyr Tyr His Val Phe Arg Arg Ile Ser Lys Thr Ser
                   85                  90                  95
<210> 95
   <211> 95
   <212> PRT
   <213> Artificial Sequence
<220>
   <221> source
   <223> /note="Description of Artificial Sequence: Synthetic polypeptide"
<400> 95
   Ile Leu Trp His Glu Met Trp His Glu Gly Leu Glu Glu Ala Ser Arg
   1               5                   10                  15
   Leu Tyr Phe Gly Glu Arg Asn Val Lys Gly Met Phe Glu Val Leu Glu
               20                  25                  30
   Pro Leu His Ala Met Met Glu Arg Gly Pro Gln Thr Leu Lys Glu Thr
           35                  40                  45
   Ser Phe Asn Gln Ala Tyr Gly Arg Asp Leu Met Glu Ala Gln Glu Trp
       50                  55                  60
   Cys Arg Lys Tyr Met Lys Ser Gly Asn Val Lys Asp Leu Leu Gln Ala
   65                  70                  75                  80
   Trp Asp Leu Tyr Tyr His Val Phe Arg Arg Ile Ser Lys Thr Ser
                   85                  90                  95
<210> 96
   <211> 95
   <212> PRT
   <213> Artificial Sequence
<220>
   <221> source
   <223> /note="Description of Artificial Sequence: Synthetic polypeptide"
<220>
   <221> MOD_RES
   <222> (12)..(12)
   <223> Any amino acid
<220>
   <221> MOD_RES
   <222> (78)..(78)
   <223> Any amino acid
<400> 96
   Ile Leu Trp His Glu Met Trp His Glu Gly Leu Xaa Glu Ala Ser Arg
   1               5                   10                  15
   Leu Tyr Phe Gly Glu Arg Asn Val Lys Gly Met Phe Glu Val Leu Glu
               20                  25                  30
   Pro Leu His Ala Met Met Glu Arg Gly Pro Gln Thr Leu Lys Glu Thr
           35                  40                  45
   Ser Phe Asn Gln Ala Tyr Gly Arg Asp Leu Met Glu Ala Gln Glu Trp
       50                  55                  60
   Cys Arg Lys Tyr Met Lys Ser Gly Asn Val Lys Asp Leu Xaa Gln Ala
   65                  70                  75                  80
   Trp Asp Leu Tyr Tyr His Val Phe Arg Arg Ile Ser Lys Thr Ser
                   85                  90                  95
<210> 97
   <211> 95
   <212> PRT
   <213> Artificial Sequence
<220>
   <221> source
   <223> /note="Description of Artificial Sequence: Synthetic polypeptide"
<400> 97
   Ile Leu Trp His Glu Met Trp His Glu Gly Leu Ile Glu Ala Ser Arg
   1               5                   10                  15
   Leu Tyr Phe Gly Glu Arg Asn Val Lys Gly Met Phe Glu Val Leu Glu
               20                  25                  30
   Pro Leu His Ala Met Met Glu Arg Gly Pro Gln Thr Leu Lys Glu Thr
           35                  40                  45
   Ser Phe Asn Gln Ala Tyr Gly Arg Asp Leu Met Glu Ala Gln Glu Trp
       50                  55                  60
   Cys Arg Lys Tyr Met Lys Ser Gly Asn Val Lys Asp Leu Leu Gln Ala
   65                  70                  75                  80
   Trp Asp Leu Tyr Tyr His Val Phe Arg Arg Ile Ser Lys Thr Ser
                   85                  90                  95
<210> 98
   <211> 95
   <212> PRT
   <213> Artificial Sequence
<220>
   <221> source
   <223> /note="Description of Artificial Sequence: Synthetic polypeptide"
<400> 98
   Ile Leu Trp His Glu Met Trp His Glu Gly Leu Leu Glu Ala Ser Arg
   1               5                   10                  15
   Leu Tyr Phe Gly Glu Arg Asn Val Lys Gly Met Phe Glu Val Leu Glu
               20                  25                  30
   Pro Leu His Ala Met Met Glu Arg Gly Pro Gln Thr Leu Lys Glu Thr
           35                  40                  45
   Ser Phe Asn Gln Ala Tyr Gly Arg Asp Leu Met Glu Ala Gln Glu Trp
       50                  55                  60
   Cys Arg Lys Tyr Met Lys Ser Gly Asn Val Lys Asp Leu Leu Gln Ala
   65                  70                  75                  80
   Trp Asp Leu Tyr Tyr His Val Phe Arg Arg Ile Ser Lys Thr Ser
                   85                  90                  95
<210> 99
   <211> 521
   <212> **DNA**
   <213> Homo sapiens
<400> 99
   acccctctct ccagccacta agccagttgc tccctcggct gacggctgca cgcgaggcct 60
   ccgaacgtct tacgccttgt ggcgcgcccg tccttgtccc gggtgtgatg gcggggtgtg 120
   gggcggaggg cgtggcgggg aagggccggc gacgagagcc gcgcgggacg actcgtcggc 180
   gataaccggt gtcgggtagc gccagccgcg cgacggtaac gagggaccgc gacaggcaga 240
   cgctcccatg atcactctgc acgccgaagg caaatagtgc aggccgtgcg gcgcttggcg 300
   ttccttggaa gggctgaatc cccgcctcgt ccttcgcagc ggccccccgg gtgttcccat 360
   cgccgcttct aggcccactg cgacgcttgc ctgcacttct tacacgctct gggtcccagc 420
   cgcggcgacg caaagggcct tggtgcgggt ctcgtcggcg cagggacgcg tttgggtccc 480
   gacggaacct tttccgcgtt ggggttgggg caccataagc t 521
<210> 100
   <211> 118
   <212> DNA
   <213> Artificial Sequence
<220>
   <221> source
   <223> /note="Description of Artificial Sequence: Synthetic polynucleotide"
<400> 100
   acccctctct ccagccacta agccagttgc tccctcggct gacggctgca cgcgaggcct 60
   ccgaacgtct tacgccttgt ggcgcgcccg tccttgtccc gggtgtgatg gcggggtg 118
<210> 101
   <211> 221
   <212> DNA
   <213> Artificial Sequence
<220>
   <221> source
   <223> /note="Description of Artificial Sequence: Synthetic polynucleotide"
<400> 101
   acccctctct ccagccacta agccagttgc tccctcggct gacggctgca cgcgaggcct 60
   ccgaacgtct tacgccttgt ggcgcgcccg tccttgtccc gggtgtgatg gcggggtgtg 120
   gggcggaggg cgtggcgggg aagggccggc gacgagagcc gcgcgggacg actcgtcggc 180
   gataaccggt gtcgggtagc gccagccgcg cgacggtaac g 221
<210> 102
   <211> 324
   <212> DNA
   <213> Artificial Sequence
<220>
   <221> source
   <223> /note="Description of Artificial Sequence: Synthetic polynucleotide"
<400> 102
   acccctctct ccagccacta agccagttgc tccctcggct gacggctgca cgcgaggcct 60
   ccgaacgtct tacgccttgt ggcgcgcccg tccttgtccc gggtgtgatg gcggggtgtg 120
   gggcggaggg cgtggcgggg aagggccggc gacgagagcc gcgcgggacg actcgtcggc 180
   gataaccggt gtcgggtagc gccagccgcg cgacggtaac gagggaccgc gacaggcaga 240
   cgctcccatg atcactctgc acgccgaagg caaatagtgc aggccgtgcg gcgcttggcg 300
   ttccttggaa gggctgaatc cccg 324
<210> 103
   <211> 422
   <212> DNA
   <213> Artificial Sequence
<220>
   <221> source
   <223> /note="Description of Artificial Sequence: Synthetic polynucleotide"
<400> 103
   acccctctct ccagccacta agccagttgc tccctcggct gacggctgca cgcgaggcct 60
   ccgaacgtct tacgccttgt ggcgcgcccg tccttgtccc gggtgtgatg gcggggtgtg 120
   gggcggaggg cgtggcgggg aagggccggc gacgagagcc gcgcgggacg actcgtcggc 180
   gataaccggt gtcgggtagc gccagccgcg cgacggtaac gagggaccgc gacaggcaga 240
   cgctcccatg atcactctgc acgccgaagg caaatagtgc aggccgtgcg gcgcttggcg 300
   ttccttggaa gggctgaatc cccgcctcgt ccttcgcagc ggcccccggg gtgttcccat 360
   cgccgcttct aggcccactg cgacgcttgc ctgcacttct tacacgctct gggtcccagc 420
   cg 422
<210> 104
   <211> 21
   <212> PRT
   <213> Artificial Sequence
<220>
   <221> source
   <223> /note="Description of Artificial Sequence: Synthetic peptide"
<400> 104
   Gly Ser Gly Glu Gly Arg Gly Ser Leu Leu Thr Cys Gly Asp Val Glu
   1               5                   10                  15
   Glu Asn Pro Gly Pro
               20
<210> 105
   <211> 22
   <212> PRT
   <213> Artificial Sequence
<220>
   <221> source
   <223> /note="Description of Artificial Sequence: Synthetic peptide"
<400> 105
   Gly Ser Gly Ala Thr Asn Phe Ser Leu Leu Lys Gln Ala Gly Asp Val
   1               5                   10                  15
   Glu Glu Asn Pro Gly Pro
               20
<210> 106
   <211> 23
   <212> PRT
   <213> Artificial Sequence
<220>
   <221> source
   <223> /note="Description of Artificial Sequence: Synthetic peptide"
<400> 106
   Gly Ser Gly Gln Cys Thr Asn Tyr Ala Leu Leu Lys Leu Ala Gly Asp
   1               5                   10                  15
   Val Glu Ser Asn Pro Gly Pro
               20
<210> 107
   <211> 25
   <212> PRT
   <213> Artificial Sequence
<220>
   <221> source
   <223> /note="Description of Artificial Sequence: Synthetic peptide"
<400> 107
   Gly Ser Gly Val Lys Gln Thr Leu Asn Phe Asp Leu Leu Lys Leu Ala
   1               5                   10                  15
   Gly Asp Val Glu Ser Asn Pro Gly Pro
               20                  25
<210> 108
   <211> 1132
   <212> PRT
   <213> Homo sapiens
<400> 108
<210> 109
   <211> 4027
   <212> DNA
   <213> Homo sapiens
<400> 109
   caggcagcgt ggtcctgctg cgcacgtggg aagccctggc cccggccacc cccgcgatgc        60
   cgcgcgctcc ccgctgccga gccgtgcgct ccctgctgcg cagccactac cgcgaggtgc       120
   tgccgctggc cacgttcgtg cggcgcctgg ggccccaggg ctggcggctg gtgcagcgcg       180
   gggacccggc ggctttccgc gcgctggtgg cccagtgcct ggtgtgcgtg ccctgggacg       240
   cacggccgcc ccccgccgcc ccctccttcc gccaggtgtc ctgcctgaag gagctggtgg       300
   cccgagtgct gcagaggctg tgcgagcgcg gcgcgaagaa cgtgctggcc ttcggcttcg       360
   cgctgctgga cggggcccgc gggggccccc ccgaggcctt caccaccagc gtgcgcagct       420
   acctgcccaa cacggtgacc gacgcactgc gggggagcgg ggcgtggggg ctgctgttgc       480
   gccgcgtggg cgacgacgtg ctggttcacc tgctggcacg ctgcgcgctc tttgtgctgg       540
   tggctcccag ctgcgcctac caggtgtgcg ggccgccgct gtaccagctc ggcgctgcca       600
   ctcaggcccg gcccccgcca cacgctagtg gaccccgaag gcgtctggga tgcgaacggg       660
   cctggaacca tagcgtcagg gaggccgggg tccccctggg cctgccagcc ccgggtgcga       720
   ggaggcgcgg gggcagtgcc agccgaagtc tgccgttgcc caagaggccc aggcgtggcg       780
   ctgcccctga gccggagcgg acgcccgttg ggcaggggtc ctgggcccac ccgggcagga       840
   cgcgtggacc gagtgaccgt ggtttctgtg tggtgtcacc tgccagaccc gccgaagaag       900
   ccacctcttt ggagggtgcg ctctctggca cgcgccactc ccacccatcc gtgggccgcc       960
   agcaccacgc gggcccccca tccacatcgc ggccaccacg tccctgggac acgccttgtc      1020
   ccccggtgta cgccgagacc aagcacttcc tctactcctc aggcgacaag gagcagctgc      1080
   ggccctcctt cctactcagc tctctgaggc ccagcctgac tggcgctcgg aggctcgtgg      1140
   agaccatctt tctgggttcc aggccctgga tgccagggac tccccgcagg ttgccccgcc      1200
   tgccccagcg ctactggcaa atgcggcccc tgtttctgga gctgcttggg aaccacgcgc      1260
   agtgccccta cggggtgctc ctcaagacgc actgcccgct gcgagctgcg gtcaccccag      1320
   cagccggtgt ctgtgcccgg gagaagcccc agggctctgt ggcggccccc gaggaggagg      1380
   acacagaccc ccgtcgcctg gtgcagctgc tccgccagca cagcagcccc tggcaggtgt      1440
   acggcttcgt gcgggcctgc ctgcgccggc tggtgccccc aggcctctgg ggctccaggc      1500
   acaacgaacg ccgcttcctc aggaacacca agaagttcat ctccctgggg aagcatgcca      1560
   agctctcgct gcaggagctg acgtggaaga tgagcgtgcg gggctgcgct tggctgcgca      1620
   ggagcccagg ggttggctgt gttccggccg cagagcaccg tctgcgtgag gagatcctgg 1680
   ccaagttcct gcactggctg atgagtgtgt acgtcgtcga gctgctcagg tctttctttt 1740
   atgtcacgga gaccacgttt caaaagaaca ggctcttttt ctaccggaag agtgtctgga 1800
   gcaagttgca aagcattgga atcagacagc acttgaagag ggtgcagctg cgggagctgt 1860
   cggaagcaga ggtcaggcag catcgggaag ccaggcccgc cctgctgacg tccagactcc 1920
   gcttcatccc caagcctgac gggctgcggc cgattgtgaa catggactac gtcgtgggag 1980
   ccagaacgtt ccgcagagaa aagagggccg agcgtctcac ctcgagggtg aaggcactgt 2040
   tcagcgtgct caactacgag cgggcgcggc gccccggcct cctgggcgcc tctgtgctgg 2100
   gcctggacga tatccacagg gcctggcgca ccttcgtgct gcgtgtgcgg gcccaggacc 2160
   cgccgcctga gctgtacttt gtcaaggtgg atgtgacggg cgcgtacgac accatccccc 2220
   aggacaggct cacggaggtc atcgccagca tcatcaaacc ccagaacacg tactgcgtgc 2280
   gtcggtatgc cgtggtccag aaggccgccc atgggcacgt ccgcaaggcc ttcaagagcc 2340
   acgtctctac cttgacagac ctccagccgt acatgcgaca gttcgtggct cacctgcagg 2400
   agaccagccc gctgagggat gccgtcgtca tcgagcagag ctcctccctg aatgaggcca 2460
   gcagtggcct cttcgacgtc ttcctacgct tcatgtgcca ccacgccgtg cgcatcaggg 2520
   gcaagtccta cgtccagtgc caggggatcc cgcagggctc catcctctcc acgctgctct 2580
   gcagcctgtg ctacggcgac atggagaaca agctgtttgc ggggattcgg cgggacgggc 2640
   tgctcctgcg tttggtggat gatttcttgt tggtgacacc tcacctcacc cacgcgaaaa 2700
   ccttcctcag gaccctggtc cgaggtgtcc ctgagtatgg ctgcgtggtg aacttgcgga 2760
   agacagtggt gaacttccct gtagaagacg aggccctggg tggcacggct tttgttcaga 2820
   tgccggccca cggcctattc ccctggtgcg gcctgctgct ggatacccgg accctggagg 2880
   tgcagagcga ctactccagc tatgcccgga cctccatcag agccagtctc accttcaacc 2940
   gcggcttcaa ggctgggagg aacatgcgtc gcaaactctt tggggtcttg cggctgaagt 3000
   gtcacagcct gtttctggat ttgcaggtga acagcctcca gacggtgtgc accaacatct 3060
   acaagatcct cctgctgcag gcgtacaggt ttcacgcatg tgtgctgcag ctcccatttc 3120
   atcagcaagt ttggaagaac cccacatttt tcctgcgcgt catctctgac acggcctccc 3180
   tctgctactc catcctgaaa gccaagaacg cagggatgtc gctgggggcc aagggcgccg 3240
   ccggccctct gccctccgag gccgtgcagt ggctgtgcca ccaagcattc ctgctcaagc 3300
   tgactcgaca ccgtgtcacc tacgtgccac tcctggggtc actcaggaca gcccagacgc 3360
   agctgagtcg gaagctcccg gggacgacgc tgactgccct ggaggccgca gccaacccgg 3420
   cactgccctc agacttcaag accatcctgg actgatggcc acccgcccac agccaggccg 3480
   agagcagaca ccagcagccc tgtcacgccg ggctctacgt cccagggagg gaggggcggc     3540
   ccacacccag gcccgcaccg ctgggagtct gaggcctgag tgagtgtttg gccgaggcct     3600
   gcatgtccgg ctgaaggctg agtgtccggc tgaggcctga gcgagtgtcc agccaagggc     3660
   tgagtgtcca gcacacctgc cgtcttcact tccccacagg ctggcgctcg gctccacccc     3720
   agggccagct tttcctcacc aggagcccgg cttccactcc ccacatagga atagtccatc     3780
   cccagattcg ccattgttca cccctcgccc tgccctcctt tgccttccac ccccaccatc     3840
   caggtggaga ccctgagaag gaccctggga gctctgggaa tttggagtga ccaaaggtgt     3900
   gccctgtaca caggcgagga ccctgcacct ggatgggggt ccctgtgggt caaattgggg     3960
   ggaggtgctg tgggagtaaa atactgaata tatgagtttt tcagttttga aaaaaaaaaa     4020
   aaaaaaa                                                                4027

## Description

This application claims priority to U.S. Serial No. 62/031,699 filed July 31, 2014.

### SEQUENCE LISTING

The instant application contains a Sequence Listing which has been submitted electronically in ASCII format and is hereby incorporated by reference in its entirety. Said ASCII copy, created on July 28, 2015, is named N2067-70660WO_SL.txt and is 174,225 bytes in size.

### FIELD OF THE INVENTION

The present invention relates generally to the use of T cells engineered to express a Chimeric Antigen Receptor (CAR) to treat a disease, e.g., a disease associated with the expression of a tumor antigen, e.g., wherein the CAR is optimized for T cells of the T cell subset in which is is provided.

### BACKGROUND OF THE INVENTION

The development of T cells which are genetically modified to express a chimeric antigen receptor (CAR) has opened the door for many new potential therapies for diseases, e.g., cancers. Generally, CARs comprise an extracellular antigen binding domain and an intracellular domain. The exact composition of the intracellular domain can provide unique characteristics to the CAR and to the cell population expression the CAR.

T cells redirected to express CARs have shown remarkable efficacy in treating some B cell malignancies. Targeting of different cancers using redirected T cells has shown some promising anti-tumor activity, but the activity was limited by poor persistence of the infused CAR T cell product. Thus, there is a need for methods and compositions for increasing the persistence and the anti-tumor activity of the redirected CAR T cells for treatment of disease.

Moeller et al. Blood (2005);106(9):2995-3003 relates to adoptive transfer of gene-engineered CD4⁺ helper T cells with induction of tumor rejection.

### SUMMARY OF THE INVENTION

The present disclosure is based on the surprising discovery that when subsets of T cells, e.g., CD4⁺ and CD8⁺ T cells, are engineered to express CARs containing different intracellular signaling domains, the persistence and anti-tumor activity of the infused CAR-expressing T cells can be modulated. Accordingly, the present disclosure describes methods and compositions of CD4⁺ and CD8⁺ T cells that express CARs containing specific combinations of intracellular signaling domains can be used to increase persistence and anti-tumor activity of the infused CAR-expressing T cells for treating a subject having a disease, e.g., a cancer.

In an aspect, described herein is a CD4⁺ T cell for use in the treatment of a subject having cancer, wherein the CD4⁺ T cell comprises a CAR (the CAR^{CD4+}) comprising:
an antigen binding domain;
a transmembrane domain; and
an intracellular signaling domain;
wherein the subject has received, is receiving or is about to receive:
a CD8⁺ T cell comprising a CAR (the CAR^{CD8+}) comprising:
   an antigen binding domain;
   a transmembrane domain; and
   an intracellular signaling domain;
wherein the CAR^{CD4+} and the CAR^{CD8+} differ from one another.

In a related aspect, described herein is a CD8⁺ T cell for use in the treatment of a subject having cancer, wherein the CD8⁺ T cell comprises a CAR (the CAR^{CD8+}) comprising:
an antigen binding domain;
a transmembrane domain; and
an intracellular signaling domain;
wherein the subject has received, is receiving or is about to receive:
a CD4⁺ T cell comprising a CAR (the CAR^{CD4+}) comprising:
   an antigen binding domain;
   a transmembrane domain; and
   an intracellular signaling domain;
wherein the CAR^{CD4+} and the CAR^{CD8+} differ from one another.

In a related aspect, described herein is a method of treating a subject, e.g., a subject having cancer, comprising, administering to said subject, an effective amount of:
1) a CD4⁺ T cell comprising a CAR (the CAR^{CD4+})comprising:
   an antigen binding domain;
   a transmembrane domain; and
   an intracellular signaling domain; and
2) a CD8⁺ T cell comprising a CAR (the CAR^{CD8+}) comprising:
   an antigen binding domain;
   a transmembrane domain; and
   an intracellular signaling domain;
   wherein the CAR^{CD4+} and the CAR^{CD8+} differ from one another.
   In an instance, the intracellular signaling domain of the CAR^{CD4+} differs from the CAR^{CD8+} intracellular signaling domain. In an instance, the CAR^{CD4+} comprises a first costimulatory signaling domain not present on the the CAR^{CD8+}. In an instance, the CAR^{CD4+} comprises a first costimulatory signaling domain and the CAR^{CD8+} comprises a second costimulatory signaling domain. In an instance, the the CAR^{CD4+} comprises a first costimulatory signaling domain not present on the CAR^{CD8+} and the CAR^{CD8+} comprises a second costimulatory signaling domain not present on the CAR^{CD4+}. In an instance, the antigen binding domain of the CAR^{CD4+} differs from the CAR^{CD8+} antigen binding domain, e.g., they are different antigen binding domains, to the same, or to different antigens.
   In an instance, the method of treating a subject, e.g., a subject having cancer, further comprises administering to the subject,
3) a second CD8⁺ T cell comprising a CAR (the second CAR^{CD8+}) comprising:
   an antigen binding domain;
   a transmembrane domain; and
   an intracellular signaling domain,
   wherein the CAR^{CD4+}, the CAR^{CD8+}, and the second CAR^{CD8+} differ from one another.

In an instance, the intracellular signaling domain of the CAR^{CD8+} differs from the second CAR^{CD8+} intracellular signaling domain. In an instance, the CAR^{CD8+} comprises a first costimulatory signaling domain not present on the second CAR^{CD8+}. In an instance, the CAR^{CD8+} comprises a first costimulatory signaling domain and the second CAR^{CD8+} comprises a second costimulatory signaling domain. In an instance, the CAR^{CD4+} comprises a first costimulatory signaling domain not present on the CAR^{CD8+} and the CAR^{CD8+} comprises a second costimulatory signaling domain not present on the CAR^{CD4+}. In an instance, the antigen binding domain of the CAR^{CD8+} differs from the second CAR^{CD8+} antigen binding domain, e.g., they are different antigen binding domains, to the same, or to different antigens. In an instance, the intracellular signaling domain of the CAR^{CD4+}, the intracellular signaling domain of the CAR^{CD8+}, and the intracellular signaling domain of the second CAR^{CD8+}, differ from one another. In an instance, each of the CAR^{CD4+}, CAR^{CD8+}, and second CAR^{CD8}, comprises a costimulatory signaling domain not present in either of the others. In an instance, the CAR^{CD4+} comprises an ICOS domain. In an instance, the ICOS domain comprises the sequence of SEQ ID NO: 46.

In an aspect, described herein is a method of treating a subject, e.g., a subject having cancer, comprising, administering to said subject, an effective amount of:
1) a CD4⁺ T cell comprising a CAR (the CAR^{CD4+}) comprising:
   an antigen binding domain;
   a transmembrane domain; and
   an ICOS domain; and
2) a CD8⁺ T cell comprising a CAR (the CAR^{CD8+}) comprising:
   an antigen binding domain;
   a transmembrane domain; and
   an intracellular signaling domain which differ from an intracellualar signaling domain in CAR^{CD4+}, and, optionally, wherein the CAR^{CD8+} does not comprise an ICOS domain; and optionally,
3) a second CD8⁺ T cell comprising a CAR (the second CAR^{CD8+}) comprising:
   an antigen binding domain;
   a transmembrane domain; and
   an intracellular signaling domain, wherein the second CAR^{CD8+} comprises an intracellular signaling domain, e.g., a costimulatory signaling domain, not present on the CAR^{CD8+}, and, optionally, does not comprise an ICOS signaling domain.

In an instance, the CAR^{CD8+} does not comprise an ICOS domain.

In an instance, treating a subject, e.g., a subject having cancer, comprises providing an anti-tumor immunity in the subject.

In an instance, treating a subject, e.g., a subject having cancer, comprises stimulating a T cell-mediated immune response to a target cell population or tissue in the subject. In an instance, the subject is treated for a disease, disorder or condition associated with, expression, e.g., elevated expression, of a tumor antigen.

In an instance, the method of treating a subject, e.g., a subject having cancer, further comprises administering to the subject,
3) a second CD8⁺ T cell comprising a CAR (the second CAR^{CD8+}) comprising:
an antigen binding domain;
a transmembrane domain; and
an intracellular signaling domain, wherin the second CAR^{CD8+} does not comprise an ICOS signaling domain and comprises an intracellular signaling domain not present on the CAR^{CD8+}.

In an instance, the method comprises administering:
a second CD8⁺ T cell comprising a CAR (the second CAR^{CD8+}) comprising:
an antigen binding domain;
a transmembrane domain; and
an intracellular signaling domain, wherein the second CAR^{CD8+} comprises an intracellular signaling domain not present on the CAR^{CD8+}, and, optionally, does not comprise an ICOS domain.

In an instance, the method of treating a subject comprises administering
a CD4⁺ T cell comprising a CAR^{CD4+} comprising:
   an antigen binding domain;
   a transmembrane domain; and
   a primary intracellular signaling domain, e.g., a CD3zeta domain (and an ICOS domain);
a CD8⁺ T cell comprising a CAR^{CD8+} comprising:
   an antigen binding domain;
   a transmembrane domain; and
   a primary intracellular signaling domain, e.g., a CD3zeta domain, and a first costimulatory signaling domain, e.g., a 4-1BB domain; and
a second CD8⁺ T cell comprising a second CAR^{CD8+} comprising:
   an antigen binding domain;
   a transmembrane domain; and
   a primary intracellular signaling domain, e.g., a CD3zeta domain, and a second costimulatory signaling domain, e.g.,a CD28 domain, wherein the second CAR^{CD8+} does not comprise an ICOS signaling domain
   wherein said first and second costimulatory signaling domains are different.

In an instance, the CD4⁺ T cell comprising a CAR^{CD4+} persists in the subject for at least 10, 15, 30, 45, 60, or 90 days after administration.

In an instance, the CD8+ T cell comprising a CAR^{CD8+} persists in the subject for at least 10, 15, 30, 45, 60, or 90 days after administration.

In an instance, the method further comprises administering a second CD8+ T cell comprising a second CAR^{CD8+}, wherein the second CD8+ T cell persists in the subject for at least 10, 15, 30, 45, 60, or 90 days after administration.

In an instance, the method of treating a subject comprises administering a preparation of T cells to the subject, wherein at least 10, 20, 30, 40, 50, 60, 70, or 80 % of the T cells, or cells, in the preparation are CD4⁺ T cells comprising a CAR^{CD4+}.

In an instance, the method of treating a subject comprises administering a preparation of T cells to the subject, wherein at least 10, 20, 30, 40, 50, 60, 70, or 80 % of the T cells, or cells, in the preparation are CD8⁺ T cells comprising a CAR^{CD8+}.

In an instance, the method of treating a subject comprises administering a preparation of T cells to the subject, wherein at least 10, 20, 30, 40, 50, 60, 70, or 80 % of the T cells, or cells, in the preparation are CD4⁺ T cells comprising a CAR^{CD4+}; and at least 10, 20, 30, 40, 50, 60, 70, or 80% of the T cells, or cells, in the preparation are CD8⁺ T cells comprising a CAR^{CD8+} or second CAR^{CD8+} (it being understood that the total cells do not exceed 100%).

In an instance, the method of treating a subject comprises administering a preparation of T cells to the subject, wherein 10 to 70% of the T cells, or cells, in the preparation are CD4⁺ T cells comprising a CAR^{CD4+}; and 10 to 70% of the T cells, or cells, in the preparation are CD8⁺ T cells comprising a CAR^{CD8+} or second CAR^{CD8+} (it being understood that the total cells do not exceed 100%).

In an instance, the method of treating a subject comprises administering a preparation of T cells to the subject, wherein at least 10, 20, 30, 40, 50, 60, 70, or 80 % of the T cells, or cells, in the preparation are CD4⁺ T cells comprising a CAR^{CD4+}; at least 10, 20, 30, 40, 50, 60, 70, or 80 % of the T cells, or cells, in the preparation are CD8⁺ T cells comprising a CAR^{CD8+}; and at least 10, 20, 30, 40, 50, 60, 70, or 80 % of the T cells, or cells, in the preparation are CD8⁺ T cells comprising a second CAR^{CD8+} (it being understood that the total cells do not exceed 100%).

In an instance, the CD4⁺ T cell comprising the CAR^{CD4+} is an autologous T cell. In an instance, the CD8+ T cell comprising the CAR^{CD8+} is an autologous T cell. In an instance, the CD4⁺ T cell comprising the CAR^{CD4+} and the CD8⁺ T cell comprising the CAR^{CD8+} are autologous T cells. In an instance, administering a second CD8+ T cell comprising a second CAR^{CD8+}, wherein the second CD8⁺T cell is an autologous T cell.

In an instance, the CD4⁺ T cell comprising the CAR^{CD4+} is an allogeneic T cell. In an instance, the CD8⁺T cell comprising the CAR^{CD8+} is an allogeneic T cell. In an instance, the CD4⁺ T cell comprising the CAR^{CD4+} and the CD8⁺T cell comprising the CAR^{CD8+} are allogeneic T cells. In an instance, the method further comprises administering a second CD8⁺ T cell comprising a second CAR^{CD8+}, wherein the second CD8⁺T cell is an allogeneic T cell.

In an instance, the CD4⁺T cell comprising the CAR^{CD4+} is an autologous Tcell and the CD8+ T cell comprising the CAR^{CD8+} is an allogeneic T cell.

In an instance, the CD4⁺ T cell comprising the CAR^{CD4+} is an allogeneic Tcell and the CD8⁺ T cell comprising the CAR^{CD8+} is an autologous T cell.

In an instance, the method further comprises evaluating the subject for a side effect of said treatment. In an instance, said side effect comprises acute respiratory distress syndrome, febrile neutropenia, hypotension, encephalopathy, hepatic transaminitis, seizure, or macrophage activation syndrome.

In an instance, the method further comprises treating the subject having a side effect with anti-cytokine agent, e.g., a tumor necrosis factor antagonist, e.g., a TNF-Ig fusion, e.g., etanercept, an IL-6 antagonist, e.g., an IL-6 receptor antagonist, e.g., an anti-IL6 receptor antibody, e.g., tocilizumab, or a corticosteroid. In an instance, treating the subject having a side effect comprises administering an anti-IL6 receptor antibody to the subject.

In an instance, the subject is a human.

The CAR-expressing CD4⁺ T cells and CD8⁺ T cells described herein can be administered simultaneously, in the same or in separate compositions, or sequentially. In an instance, the CAR-expressing CD4⁺ T cells are administered before the CAR-expressing CD8⁺ T cells, and in an instance, the CAR-expressing CD8⁺ T cells are administered before the CAR-expressing CD4⁺ T cells.

The following instances describe any of the CAR^{CD4+}, or CD4+ T cell comprising the CAR^{CD4+}, the CAR^{CD8+}, or CD8+ T cell comprising the CAR^{CD8+}, or the second CAR^{CD8+}, or the second CD8+ T cell comprising the second CAR^{CD8+}, encompassed in any of the methods, compositions, or kits disclosed herein.

In an instance, the CAR^{CD4+} does not include a 4-1BB domain.

In an instance, the CAR^{CD4+} does not include a CD28 domain.

In an instance, the CAR^{CD4+} does not include a costimulatory domain other than the ICOS domain.

In an instance, the CAR^{CD4+} comprises a primary intracellular signaling domain, e.g., a primary signal domain from Table 4, e.g., a CD3zeta domain. In an instance, the CAR^{CD4+} comprises a CD3zeta domain but does not include a costimulatory domain other than the ICOS domain.

In an instance, the CAR^{CD4+} comprises a second costimulatory signaling domain, e.g., from Table 5.

In an instance, the CAR^{CD8+} comprises a primary intracellular signaling domain, e.g., a primary signal domain from Table 4, e.g., a CD3zeta domain and a costimulatory signaling domain, e.g.,from Table 5.

In an instance, the CAR^{CD8+} comprises a second costimulatory signaling domain, e.g., from Table 5.

In an instance, the CAR^{CD8+} comprises a CD3zeta domain.

In an instance, the CAR^{CD8+} comprises a costimulatory signaling domain other than ICOS, e.g., from Table 5, e.g., 4-1BB or CD28.

In an instance, the CAR^{CD8+} comprises a 4-1BB domain.

In an instance, the CAR^{CD8+} comprises a CD3zeta domain and a 4-1BB domain.

In an instance, the CAR^{CD8+} comprises a CD28 domain.

In an instance, the CAR^{CD8+} comprises a CD3zeta domain and a CD28 domain.

In an instance, the CAR^{CD8+} comprises a CD3zeta domain, a 4-1BB domain and a CD28 domain.

In an instance, the CAR^{CD4+} comprises a CD3zeta domain; and the CAR^{CD8+} comprises a CD3zeta domain and a 4-1BB domain.

In an instance, the CAR^{CD4+} comprises a CD3zeta domain; and the CAR^{CD8+} comprises a CD3zeta domain and a CD28 domain.

In an instance, the CAR^{CD4+} comprises a CD3zeta domain (and an ICOS domain); and the CAR^{CD8+} comprises a CD3zeta domain, a 4-1BB domain, and a CD28 domain.

In an instance, the CAR^{CD4+} comprises a CD3zeta domain (e.g., according to SEQ ID NO: 18 or SEQ ID NO: 20) and an ICOS domain (e.g., according to SEQ ID NO: 40 or SEQ ID NO: 46); and the CAR^{CD8+} comprises a CD3zeta domain (e.g., according to SEQ ID NO: 18 or SEQ ID NO: 20) and a 4-1BB domain (e.g., according to SEQ ID NO: 14). In an instance, the CAR^{CD4+} comprises a CD3zeta domain (e.g., according to SEQ ID NO: 18 or SEQ ID NO: 20); and the CAR^{CD8+} comprises a CD3zeta domain (e.g., according to SEQ ID NO: 18 or SEQ ID NO: 20). In an instance, the CAR^{CD4+} comprises an ICOS domain (e.g., according to SEQ ID NO: 40 or SEQ ID NO: 46); and the CAR^{CD8+} comprises a 4-1BB domain (e.g., according to SEQ ID NO: 14). In an instance, the CAR^{CD4+} comprises a CD3zeta domain (e.g., according to SEQ ID NO: 18 or SEQ ID NO: 20); and the CAR^{CD8+} comprises a CD3zeta domain (e.g., according to SEQ ID NO: 18 or SEQ ID NO: 20) and a 4-1BB domain (e.g., according to SEQ ID NO: 14). In an instance, the CAR^{CD4+} comprises an ICOS domain (e.g., according to SEQ ID NO: 40 or SEQ ID NO: 46); and the CAR^{CD8+} comprises a CD3zeta domain (e.g., according to SEQ ID NO: 18 or SEQ ID NO: 20) and a 4-1BB domain (e.g., according to SEQ ID NO: 14). In an instance, the CAR^{CD4+} comprises a CD3zeta domain (e.g., according to SEQ ID NO: 18 or SEQ ID NO: 20) and an ICOS domain (e.g., according to SEQ ID NO: 40 or SEQ ID NO: 46); and the CAR^{CD8+} comprises a CD3zeta domain (e.g., according to SEQ ID NO: 18 or SEQ ID NO: 20). In an instance, the CAR^{CD4+} comprises a CD3zeta domain (e.g., according to SEQ ID NO: 18 or SEQ ID NO: 20) and an ICOS domain (e.g., according to SEQ ID NO: 40 or SEQ ID NO: 46); and the CAR^{CD8+} comprises a 4-1BB domain (e.g., according to SEQ ID NO: 14).

In an instance, the second CAR^{CD8+} does not comprise an ICOS domain.

In an instance, the second CAR^{CD8+} comprises a primary intracellular signaling domain, e.g., a primary signal domain from Table 4, e.g., a CD3zeta domain.

In an instance, the second CAR^{CD8+} comprises a costimulatory signaling domain other than ICOS, e.g., from Table 5, e.g., a 4-1BB domain.

In an instance, the second CAR^{CD8+} comprises a costimulatory signaling domain other than ICOS, e.g., from Table 5, e.g., a CD28 domain.

In an instance, the second CAR^{CD8+} comprises a CD3zeta domain and a 4-1BB domain.

In an instance, the second CAR^{CD8+} comprises a CD3zeta domain and a CD28 domain.

In an instance, the CAR^{CD4+} comprises a CD3zeta domain (and an ICOS domain); the CAR^{CD8+} comprises a CD3zeta domain and a 4-1BB domain; and the second CAR^{CD8+} comprises a CD3zeta domain and a CD28 domain.

In an instance, the CD4⁺ cell is a Th17 polarized cell. In an instance, the CD4⁺ cell produces IL-17A and/or IFNγ. In an instance, the CD4⁺ cell expresses IL-23R and/or CD161 on the cell surface.

In an instance, the CAR^{CD4+} comprises an antibody variable domain, an scFv, or a nanobody, or an antigen binding fragment thereof. In an instance, the antigen binding domain specific for a tumor antigen selected from CD19, CD20, CD22, ROR1, mesothelin, CD33/IL3Ra, c-Met, PSMA, Glycolipid F77, EGFRvIII, GD-2, NY-ESO-1 TCR, MAGE A3 TCR, and any combination thereof. In an instance, the CAR^{CD4+} comprises an antigen binding domain which binds an antigen described herein, e.g., an antigen listed in Table 2. In an instance, the CAR^{CD4+} comprises an antigen binding domain from Table 2.

In an instance, the CAR^{CD8+} comprises an antibody variable domain, an scFv, or a nanobody, or an antigen binding fragment thereof. In an instance, the CAR^{CD8+} comprises an antigen binding domain specific for a tumor antigen selected from CD19, CD20, CD22, ROR1, mesothelin, CD33/IL3Ra, c-Met, PSMA, Glycolipid F77, EGFRvIII, GD-2, NY-ESO-1 TCR, MAGE A3 TCR, and any combination thereof. In an instance, the CAR^{CD8+} comprises an antigen binding domain which binds a cancer cell, e.g., which binds an antigen described in Table 2. In an instance, the CAR^{CD8+} comprises an antigen binding domain from Table 2.

In an instance, the CAR^{CD4+} and the CAR^{CD8+} each comprises an antigen binding domain which binds a cancer cell, e.g., which binds an antigen described in Table 2. In an instance, each of the CAR^{CD4+} and CAR^{CD8+} comprises an antigen binding domain which binds the same antigen. In an instance, the CAR^{CD4+} and the CAR^{CD8+} each comprises an antigen binding domain from Table 2. In an instance, each of the CAR^{CD4+} and CAR^{CD8+} comprises the same antigen binding domain.

In an instance, a second CD8+ T cell comprising a second CAR^{CD8+} is administered, and the second CAR^{CD8+} comprises an antigen binding domain which binds a cancer cell, e.g., which binds an antigen described in Table 2.

In an instance, a second CD8+ T cell comprising a second CAR^{CD8+} is administered, and the second CAR^{CD8+} comprises an antigen binding domain from Table 2.

In an instance, a second CD8+ T cell comprising a second CAR^{CD8+} is administered, and the CAR^{CD4+}, CAR^{CD8+}, and the second CAR^{CD8+} each comprises an antigen binding domain which binds a cancer cell, e.g., which binds an antigen described in Table 2.

In an instance, a second CD8+ T cell comprising a second CAR^{CD8+} is administered, and each of the CAR^{CD4+}, CAR^{CD8+}, and second CAR^{CD8+} comprises an antigen binding domain which binds the same antigen.

In an instance, the CAR^{CD4+}, CAR^{CD8+} and the second CAR^{CD8+} each comprises an antigen binding domain from Table 2.

In an instance, a second CD8+ T cell comprising a second CAR^{CD8+} is administered, and wherein each of the CAR^{CD4+}, CAR^{CD8+}, and second CAR^{CD8+} comprises the same antigen binding domain.

In an instance, the CAR^{CD4+} comprises an ICOS domain. In an instance, the CAR^{CD8+} does not comprise an ICOS domain. In an instance, the second CAR^{CD8+} does not comprise an ICOS signaling domain and comprises an intracellular signaling domain not present on the CAR^{CD8+}.

In instances, the intracellular signaling domain of a CAR molecule described herein comprises a costimulatory domain. In instances, the intracellular signaling domain of the CAR molecule comprises a primary signaling domain. In instances, the intracellular signaling domain of the isolated CAR molecule a costimulatory domain and a primary signaling domain.

In one instance, the costimulatory domain comprises a functional signaling domain of a protein selected from the group consisting of MHC class I molecule, TNF receptor proteins, Immunoglobulin-like proteins, cytokine receptors, integrins, signaling lymphocytic activation molecules (SLAM proteins), activating NK cell receptors, BTLA, a Toll ligand receptor, OX40, CD2, CD7, CD27, CD28, CD30, CD40, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, and a ligand that specifically binds with CD83. In instances, the costimulatory domain comprises 4-1BB, CD27, CD28, or ICOS.

In one instance, the 4-1BB costimulatory domain comprises a sequence of SEQ ID NO: 14. In one instance, the 4-1BB costimulatory domain comprises an amino acid sequence having at least one, two or three modifications (e.g., substitutions) but not more than 20, 10 or 5 modifications (e.g., substitutions) of an amino acid sequence of SEQ ID NO: 14, or a sequence with 95-99% identity to an amino acid sequence of SEQ ID NO:14. In one instance, the 4-1BB costimulatory domain is encoded by a nucleic acid sequence of SEQ ID NO: 15, or a sequence with with 95-99% identity thereof.

In one instance, the CD27 costimulatory domain comprises a sequence of SEQ ID NO: 16. In one instance, the CD27 costimulatory domain comprises an amino acid sequence having at least one, two or three modifications (e.g., substitutions) but not more than 20, 10 or 5 modifications (e.g., substitutions) of an amino acid sequence of SEQ ID NO: 16, or a sequence with 95-99% identity to an amino acid sequence of SEQ ID NO:16. In one instance, the CD27 costimulatory domain is encoded by a nucleic acid sequence of SEQ ID NO: 17, or a sequence with with 95-99% identity thereof.

In one instance, the CD28 costimulatory domain comprises a sequence of SEQ ID NO: 44. In one instance, the CD28 costimulatory domain comprises an amino acid sequence having at least one, two or three modifications (e.g., substitutions) but not more than 20, 10 or 5 modifications (e.g., substitutions) of an amino acid sequence of SEQ ID NO: 44, or a sequence with 95-99% identity to an amino acid sequence of SEQ ID NO:44. In one instance, the CD28 costimulatory domain is encoded by a nucleic acid sequence of SEQ ID NO:45, or a sequence with with 95-99% identity thereof.

In one instance, the wild-type ICOS costimulatory domain comprises a sequence of SEQ ID NO: 40. In one instance, the wild-type ICOS costimulatory domain comprises an amino acid sequence having at least one, two or three modifications (e.g., substitutions) but not more than 20, 10 or 5 modifications (e.g., substitutions) of an amino acid sequence of SEQ ID NO: 40, or a sequence with 95-99% identity to an amino acid sequence of SEQ ID NO:40. In one instance, the wild-type ICOS costimulatory domain is encoded by a nucleic acid sequence of SEQ ID NO:41, or a sequence with with 95-99% identity thereof.

In one instance, the Y to F mutant ICOS costimulatory domain comprises a sequence of SEQ ID NO: 46. In one instance, the Y to F mutant ICOS costimulatory domain comprises an amino acid sequence having at least one, two or three modifications (e.g., substitutions) but not more than 20, 10 or 5 modifications (e.g., substitutions) of an amino acid sequence of SEQ ID NO: 46, or a sequence with 95-99% identity to an amino acid sequence of SEQ ID NO:46. In one instance, the Y to F mutant ICOS costimulatory domain is encoded by a nucleic acid sequence with 95-99% identity to a nucleic acid sequence of SEQ ID NO:41 (wherein SEQ ID NO: 41 encodes wild-type ICOS).

In instances, the primary signaling domain comprises a functional signaling domain of CD3 zeta. In instances, the functional signaling domain of CD3 zeta comprises SEQ ID NO: 18 (mutant CD3 zeta) or SEQ ID NO: 20 (wild-type human CD3 zeta).

In one instance, the intracellular signaling domain of a CAR, e.g., a CAR^{CD4+} or a CAR^{CD8+}, comprises a functional signaling domain of CD27 and/or a functional signaling domain of CD3 zeta. In one instance, the intracellular signaling domain of a CAR, e.g., a CAR^{CD4+} or a CAR^{CD8+}, comprises a functional signaling domain of CD28 and/or a functional signaling domain of CD3 zeta. In one instance, the intracellular signaling domain of a CAR, e.g., a CAR^{CD4+} or a CAR^{CD8+}, comprises a functional signaling domain of ICOS and/or a functional signaling domain of CD3 zeta. In one instance, the intracellular signaling domain of a CAR, e.g., a CAR^{CD4+} or a CAR^{CD8+}, comprises a functional signaling domain of 4-1BB and/or a functional signaling domain of CD3 zeta.

In one instance, a CART cell described herein displays an enhanced persistence compared to a control T cell, e.g., a T cell of a different type (e.g., CD8+ or CD4+) expressing the same CAR. In some instances, a CD4+ T cell comprises a CAR described herein, which CAR comprises an intracellular signaling domain suitable for (e.g., optimized for, e.g., leading to enhanced persistence in) a CD4+ T cell, e.g., an ICOS domain. In some instances, a CD8+ T cell comprises a CAR described herein, which CAR comprises an intracellular signaling domain suitable for (e.g., optimized for, e.g., leading to enhanced persistence of) a CD8+ T cell, e.g., a 4-1BB domain, a CD28 domain, or another costimulatory domain other than an ICOS domain.

In an aspect, disclosed herein is a composition, e.g., a pharmaceutically acceptable composition, or set of compositions, e.g., a set of pharmaceutically acceptable compositions, comprising one or more of the following components:
1) a CD4⁺ T cell comprising a CAR (the CAR^{CD4+}) comprising:
   an antigen binding domain;
   a transmembrane domain; and
   an intracellular signaling domain; and
2) a CD8⁺ T cell comprising a CAR (the CAR^{CD8+}) comprising:
   an antigen binding domain;
   a transmembrane domain; and
   an intracellular signaling domain; and,
   wherein the the CAR^{CD4+} and the CAR^{CD8+} differ from one another.
   In an instance, the composition, or set of compositions, further comprises:
3) a second CD8⁺ T cell comprising a CAR (the second CAR^{CD8+}) comprising:
   an antigen binding domain;
   a transmembrane domain; and
   an intracellular signaling domain,
   wherein the second CAR^{CD8+} differs from the CAR^{CD4+} and the the CAR^{CD8+}.

In an instance, the composition, or set of compositions, comprises a plurality of components 1), 2), and 3).

In an instance, the composition, or set of compositions, comprises a composition comprising component 1).

In an instance, the composition, or set of compositions, comprises a composition comprising component 2).

In an instance, the composition, or set of compositions, comprises a composition comprising component 3).

In an instance, the composition, or set of compositions, comprises a composition comprising components 1) and 2).

In an instance, the composition, or set of compositions, comprises a composition comprising components 1) and 3).

In an instance, the composition, or set of compositions, comprises a composition comprising 2) and 3).

In an instance, the composition, or set of compositions, comprises a composition comprising components 1), 2) and 3).

In an instance, the composition, or set of compositions, comprises a set of compositions, the set comprising a composition comprising component 1) and a composition comprising component 2).

In an instance, the composition, or set of compositions, comprises a set of compositions, the set comprising a composition comprising component 1) and a composition comprising component 3).

In an instance, the composition, or set of compositions, comprises a set of compositions, the set comprising a composition comprising component 2), and a composition comprising component 3).

In an instance, the composition, or set of compositions, comprises a set of compositions, the set comprising a composition comprising component 1), a composition comprising component 2), and a composition comprising component 3).

In an instance, the composition is a pharmaceutically acceptable composition. In an instance, the set of compositions comprises one or more (e.g., all) pharmaceutically acceptable compositions.

In an aspect, disclosed herein is a composition, e.g., a pharmaceutically acceptable composition, or set of compositions, e.g., a set of pharmaceutically acceptable compositions, comprising one, or more, or all of a CD4⁺ T cell comprising a CAR^{CD4+}, a CD8⁺ T cell comprising a CAR^{CD8+}, and a second CD8⁺ T cell comprising a second CAR^{CD8+}, for use as a medicament.

In an aspect, disclosed herein is a composition, e.g., a pharmaceutically acceptable composition, or set of compositions, e.g., a set of pharmaceutically acceptable compositions, comprising one, or more, or all of a CD4⁺ T cell comprising a CAR^{CD4+}, a CD8⁺ T cell comprising a CAR^{CD8+}, and a second CD8⁺ T cell comprising a second CAR^{CD8+}, for use as a medicament in the treatment of a disorder described herein, e.g., cancer.

Any of the CAR^{CD4+}, CAR^{CD8+}, or second CAR^{CD8+} disclosed herein can be used in the compositions or set of compositions described herein.

In an aspect, disclosed herein is a kit comprising, one, or more, or all of the following components:
1) a nucleic acid comprising sequence encoding a CAR^{CD4+} comprising:
   an antigen binding domain;
   a transmembrane domain; and
   an intracellular signaling domain; and
2) a nucleic acid comprising sequence encoding a CAR^{CD8+} comprising:
   an antigen binding domain;
   a transmembrane domain; and
   an intracellular signaling domain; and,
   wherein the the CAR^{CD4+} and the CAR^{CD8+} differ from one another.
   In an instance, the CAR^{CD4+} comprises an ICOS domain. In an instance, the CAR^{CD8+} does not comprise an ICOS domain.
   In an instance, the kit further further comprises:
3) a nucleic acid comprising sequence encoding a second CAR^{CD8+} comprising:
   an antigen binding domain;
   a transmembrane domain; and
   an intracellular signaling domain,
   wherein the second CAR^{CD8+} differs from the CAR^{CD4+} and the the CAR^{CD8+}. In an instance, the second CAR^{CD8+} does not comprise an ICOS signaling domain and comprises an intracellular signaling domain not present on the CAR^{CD8+}.

In an instance, the kit comprises component 1).

In an instance, the kit comprises component 2).

In an instance, the kit comprises component 3).

In an instance, the kit comprises a plurality of components 1), 2), and 3).

In an instance, the kit comprises components 1) and 2).

In an instance, the kit comprises 1) and 3).

In an instance, the kit comprises 1), 2) and 3).

In an instance, the kit comprises a plurality of components 1), 2) and 3) and each component of the plurality is disposed in a separate container.

In an instance, the kit further comprises a buffer.

In an instance, the kit further comprises a reagent useful for introducing one of the components into a T cell, e.g., a buffer or transfection reagent.

In an instance, component 1) comprises a viral vector, e.g., a lenti viral vector.

In an instance, component 2) comprises a viral vector, e.g., a lenti viral vector.

In an instance, component 3) comprises a viral vector, e.g., a lenti viral vector.

In an aspect, the kit disclosed herein comprises one, or more, or all of the following components:
1) a CD4⁺ T cell, or preparation thereof, comprising a CAR (the CAR^{CD4+}) comprising:
   an antigen binding domain;
   a transmembrane domain; and
   an intracellular signaling domain; and
2) a CD8⁺ T cell, or preparation thereof, comprising a CAR (the CAR^{CD8+}) comprising:
   an antigen binding domain;
   a transmembrane domain; and
   an intracellular signaling domain; and,
   wherein the CAR^{CD4+} and the CAR^{CD8+} differ from one another. In an instance, the CAR^{CD4+} comprises an ICOS domain. In an instance, the CAR^{CD8+} does not comprise an ICOS domain.
   In an instance, the kit further comprises
3) a second CD8⁺ T cell, or preparation thereof, comprising a CAR (the second CAR^{CD8+}) comprising:
   an antigen binding domain;
   a transmembrane domain; and
   an intracellular signaling domain,
   wherein the second CAR^{CD8+} differs from the CAR^{CD4+} and the the CAR^{CD8+}. In an instance, the second CAR^{CD8+} does not comprise an ICOS signaling domain and comprises an intracellular signaling domain not present on the CAR^{CD8+}.

In an instance, the CD4⁺ T cell, or preparation thereof, and the CD8⁺ T cell, or preparation thereof, are disposed in a container.

In an instance, the CD4⁺ T cell, or preparation thereof, is disposed in a first conatainer and the CD8⁺ T cell, or preparation thereof, is disposed in a second container.

In an instance, the kit comprises component 1).

In an instance, the kit comprises component 2).

In an instance, the kit comprises component 3).

In an instance, the kit comprises a plurality of components 1), 2) and 3).

In an instance, the kit comprises components 1) and 2).

In an instance, the kit comprises components 1) and 3).

In an instance, the kit comprises components 1), 2) and 3).

In an instance, each component of the kit described herein is disposed in a separate container.

In an aspect, disclosed herein is a kit comprising, one, or more, or all of, a nucleic acid comprising sequence encoding a CAR^{CD4+}, a nucleic acid comprising sequence encoding a CAR^{CD8+}, and a nucleic acid comprising sequence encoding a second CAR^{CD8+}, for use as a medicament.

In an aspect, disclosed herein is a kit comprising, one, or more, or all of, a nucleic acid comprising sequence encoding a CAR^{CD4+}, a nucleic acid comprising sequence encoding a CAR^{CD8+}, and a nucleic acid comprising sequence encoding a second CAR^{CD8+}, for use as a medicament in the treatment of a disorder described herein, e.g., cancer.

In an aspect, disclosed herein is a kit comprising one, or more, or all of a CD4⁺ T cell, or preparation thereof, comprising a CAR^{CD4+}, a CD8⁺ T cell, or preparation thereof, comprising a CAR^{CD8+}, and a second CD8⁺ T cell, or preparation thereof, comprising a second CAR^{CD8+}, for use as a medicament.

In an aspect, disclosed herein is a kit comprising one, or more, or all of a CD4⁺ T cell, or preparation thereof, comprising a CAR^{CD4+}, a CD8⁺ T cell, or preparation thereof, comprising a CAR^{CD8+}, and a second CD8⁺ T cell, or preparation thereof, comprising a second CAR^{CD8+}, for use as a medicament in the treatment of a disorder described herein, e.g., cancer.

Any of the CAR^{CD4+}, CAR^{CD8+}, or second CAR^{CD8+} disclosed herein can be used in the kit, or the components of the kit, described herein.

In an instance, at least 10, 20, 30, 40, 50, 60, 70, or 80 % of the T cells, or a preparation thereof, in the composition or kit described herein are CD4⁺ T cells comprising a CAR^{CD4+}.

In an instance, at least 10, 20, 30, 40, 50, 60, 70, or 80 % of the T cells, or a prepration thereof, in the composition or kit described herein are CD8⁺ T cells comprising a CAR^{CD8+}.

In an instance, at least 10, 20, 30, 40, 50, 60, 70, or 80 % of the T cells , or a preparation thereof, in the composition or kit described herein are CD4⁺ T cells comprising a CAR^{CD4+}; and at least 10, 20, 30, 40, 50, 60, 70, or 80% of the T cells, or a preparation thereof, in the composition or a kit described herein are CD8⁺ T cells comprising a CAR^{CD8+} or second CAR^{CD8+} (it being understood that the total cells do not exceed 100%).

In an instance, at least 10 to 70% of the T cells, or a preparation thereof, in the composition or kit described herein are CD4⁺ T cells comprising a CAR^{CD4+}; and at least 10 to 70% of the T cells, or a preparation thereof, in the composition or kit described herein are CD8⁺ T cells comprising a CAR^{CD8+} or second CAR^{CD8+} (it being understood that the total cells do not exceed 100%).

In an instance, at least 10, 20, 30, 40, 50, 60, 70, or 80 % of the T cells, or a preparation thereof, in the composition or kit described herein are CD4⁺ T cells comprising a CAR^{CD4+}; at least 10, 20, 30, 40, 50, 60, 70, or 80 % of the T cells, or a preparation thereof, in the composition or kit described herein are CD8⁺ T cells comprising a CAR^{CD8+}; and at least 10, 20, 30, 40, 50, 60, 70, or 80 % of the T cells, or a preparation thereof, in the composition or kit described herein are CD8⁺ T cells comprising a second CAR^{CD8+} (it being understood that the total cells do not exceed 100%).

In an aspect, disclosed herein is a method of making a CD4⁺ T cell comprising a CAR (a CAR^{CD4+}) and a CD8⁺ T cell comprising a CAR (a CAR^{CD8+}) (or a kit or composition comprising the same), the method comprising:
providing a CD4⁺ T cell comprising a CAR^{CD4+}, e.g., by introducing a nucleic acid sequence that encodes a CAR^{CD4+} into a CD4⁺ T cell;
providing a CD8⁺ T cell comprising a CAR^{CD8+}, e.g., by introducing a nucleic acid sequence that encodes a CAR^{CD8+} into a CD8+ T cell;
   wherein,
   the CAR^{CD4+} comprises:
      an antigen binding domain;
      a transmembrane domain; and
      an intracellular signaling domain; and
   the CAR^{CD8+} comprises:
      an antigen binding domain;
      a transmembrane domain; and
      an intracellular signaling domain,
      wherein the CAR^{CD4+} and the CAR^{CD4+} are different. In an instance, the CAR^{CD4+} comprises an ICOS domain. In an instance, the CAR^{CD8+} does not comprise an ICOS domain.

In an instance, the method of making a CD4⁺ T cell comprising a CAR (a CAR^{CD4+}) and a CD8⁺ T cell comprising a CAR (a CAR^{CD8+}) (or a kit or composition comprising the same) comprises:
a) providing a CD4⁺ T cell;
b) introducing a nucleic acid sequence that encodes a CAR^{CD4+} into the CD4⁺ T cell to provide a CD4⁺ T cell comprising a CAR^{CD4+};
c) providing a CD8⁺ T cell;
d) introducing a nucleic acid sequence that encodes a CAR^{CD8+} into the CD8+ T cell to provide a CD8⁺ T cell comprising a CAR^{CD8+}. In an instance, c) is performed before a). In an instance, step a) comprises selecting a CD4⁺ T cell, e.g., by negative selection or by cell sorting. In an instance, step c) comprises selecting a CD8⁺ T cell, e.g., by negative selection or by cell sorting. In an instance, a) and c) are performed simultaneously.

In an instance, the method of making a CD4⁺ T cell comprising a CAR (a CAR^{CD4+}) and a CD8⁺ T cell comprising a CAR (a CAR^{CD8+}) (or a kit or composition comprising the same) further comprises e) Th 17 polarizing the CD4⁺ T cell or the CD4⁺ T cell comprising a CAR^{CD4+}.

In an instance, the method further comprises expanding the CD4⁺ T cell to provide progeny CD4⁺ T cells. In an instance, the method further comprises expanding the CD4⁺ T cell comprising a CAR^{CD4+} to provide progeny CD4⁺ T cells comprising a CAR^{CD4+}.

In an instance, the method further comprises expanding the Th 17 polarized CD4⁺ T cell to provide progeny Th17 polarized CD4⁺ T cells. In an instance, the method further comprises expanding the Th 17 polarized CD4⁺ T cell comprising a CAR^{CD4+} to provide Th 17 polarized progeny CD4⁺ T cells comprising a CAR^{CD4+}.

In an instance, the method further comprises expanding the CD8+ T cell to provide progeny CD8⁺ T cells. In an instance, the method further comprises expanding the CD8⁺ T cell comprising a CAR^{CD8+} to provide progeny CD8⁺ T cells comprising a CAR^{CD8+}.

In an instance, the method further comprises providing a second CD8⁺ T cell comprising a second CAR^{CD8+}, e.g., by introducing a nucleic acid sequence that encodes a CAR^{CD8+} into a CD8⁺ T cell;
wherein,
the second CAR^{CD8+} comprises:
   an antigen binding domain;
   a transmembrane domain; and
   an intracellular signaling domain,
wherein the second CAR^{CD8+} differs from the CAR^{CD4+}.

In an instance, the second CAR^{CD8+} does not comprise an ICOS signaling domain and comprises an intracellular signaling domain not present on the CAR^{CD8+}.

In an instance, the method further comprises
f) providing a second CD8⁺ T cell;
g) introducing a nucleic acid sequence that encodes a second CAR^{CD8+} into the CD8+ T cell to provide a CD8⁺ T cell comprising a CAR^{CD8+}. In an instance, f) is performed before a).

In an instance, f) comprises selecting a second CD8⁺ T cell, e.g., by negative selection or by cell sorting.

In an instance, the method further comprises expanding the second CD8⁺ T cell to provide progeny second CD8⁺ T cells. In an instance, the method further comprises expanding the second CD8⁺ T cell comprising a CAR^{CD8+} to provide progeny second CD8⁺ T cells comprising a second CAR^{CD8+}.

In some instances, the methosd of making disclosed herein further comprises contacting the population of CD4+ cells, CD8+ cells, or both CD4+ and CD8+ cells, with a nucleic acid encoding a telomerase subunit, e.g., hTERT. The nucleic acid encoding the telomerase subunit can be DNA.

In some instances, the method of making disclosed herein further comprises culturing the population of of CD4+ cells, CD8+ cells, or both CD4+ and CD8+ cells, in serum comprising 2% hAB serum.

Headings, sub-headings or numbered or lettered elements, e.g., (a), (b), (i) etc, are presented merely for ease of reading. The use of headings or numbered or lettered elements in this document does not require the steps or elements be performed in alphabetical order or that the steps or elements are necessarily discrete from one another.

Other features, objects, and advantages of the invention will be apparent from the description and drawings, and from the claims.

Certain instances of the present disclosure provide embodiments of the present invention. More particularly, the present invention is as defined in the appended claims.

### BRIEF DESCRIPTION OF THE DRAWINGS

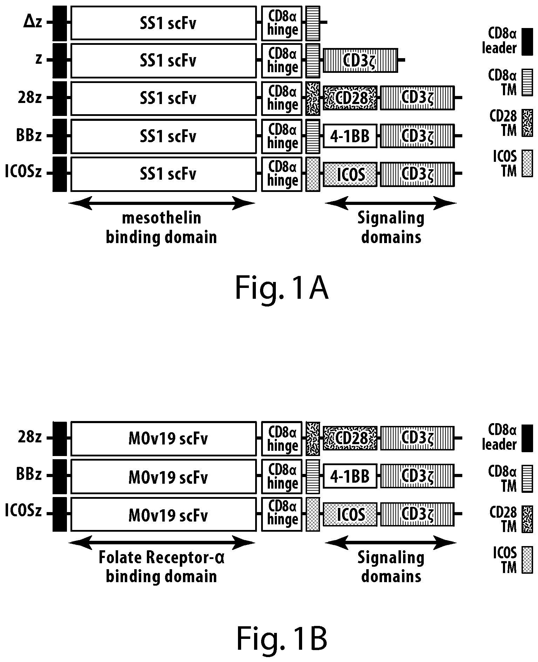

Figures 1A and 1B are schematic representations of ICOS-based CARs. Figure 1A shows a panel of chimeric receptors that contain the SS1 single chain fragment that binds to mesothelin and differ in the intracellular domain. Figure 1B shows a panel of chimeric receptors that contain the Mov19 single chain fragment that binds to Folate Receptor-α and differ in the intracellular domain.

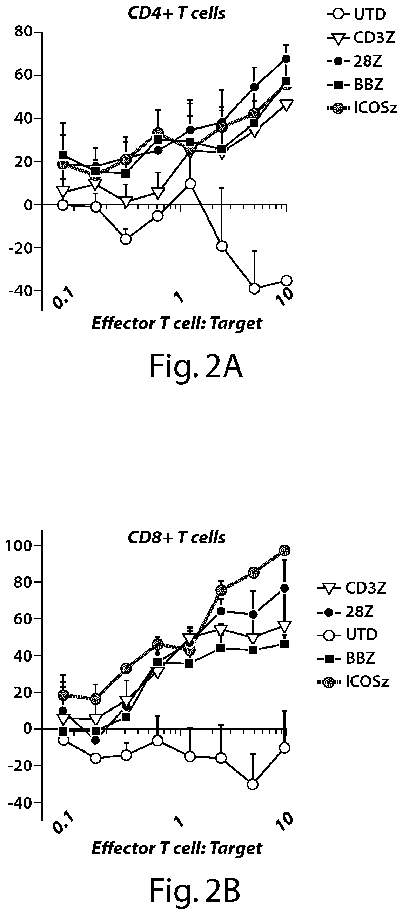

Figures 2A and 2B show tumor cell killing by CD4⁺ and CD8⁺ T cells redirected with SS1-CARs. CD4⁺ (Fig. 2A) and CD8⁺ T cells (Fig. 2B) were cocultured with firefly Luciferase (fLuc)-expressing L55 target cells for 18 hours at the indicated effector-target (E:T) ratios. Specific cytolysis was determined using a bioluminescence assay.

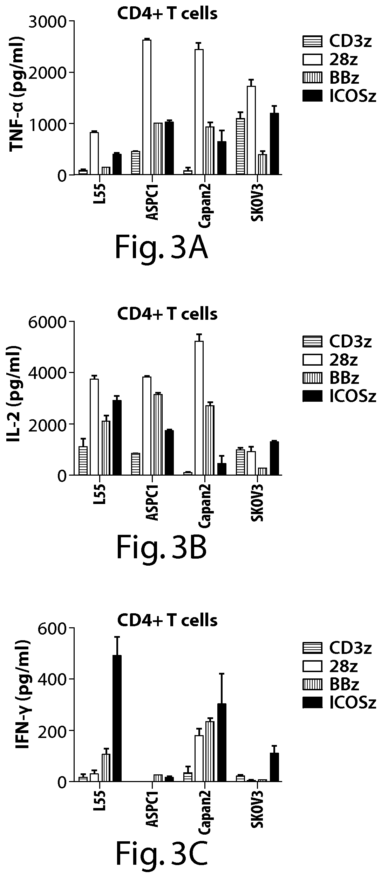

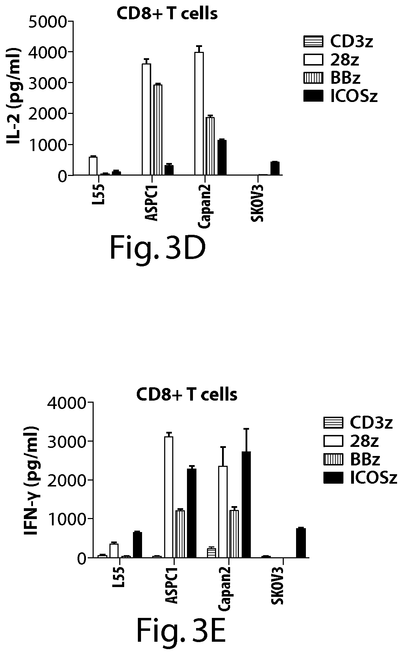

Figures 3A, 3B, 3C, 3D, and 3E show the cytokine release by redirected CD4⁺ and CD8⁺ T cells after antigen recognition in tumor cells. CD4⁺ T cells (Figs. 3A, 3B, and 3C) or CD8⁺ T cells (Fig. 3D and 3E) (4 x 10⁵, 60% chimeric receptor positive) were cocultured with 2 x 10⁵ tumor cells in culture media. Supernatants were obtained 24 hours after coculture, and TNF-α, IL-2 and IFN-γ were analyzed by ELISA. Error bars indicate standard deviation (SD) in duplicate samples.

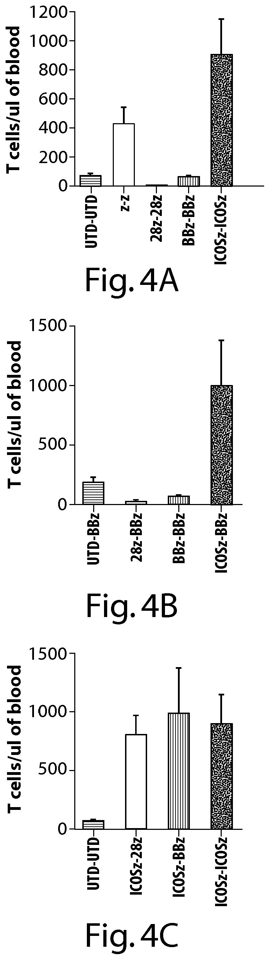

Figures 4A, 4B and 4C show that ICOS intracellular domain enhanced the *in vivo* persistence of CAR-expressing CD4⁺ T cells, an effect that is independent of the CAR intracellular domain used to redirect CD8⁺ T cells. NSG mice bearing subcutaneous non-small cell lung tumors (L55) were treated 30 days after tumor implantation with two doses CD4⁺ and CD8⁺ T cells redirected with SS1-CARs. The concentration of CD4⁺ T cells was determined in the blood of treated animals 22 days after T cell injection. Error bars represent SEM (n=7-10) Figure 4A shows that CD4⁺ and CD8⁺ T cells were redirected with the same construct (z, 28z, BBz or ICOSz). Figure 4B shows that CD8⁺ T cells were redirected with SS1-BBz and CD4⁺ T cells were left untransduced (UTD-BBz) or redirected with 28z (28z-BBz), BBz (BBz-BBz) or ICOSz (ICOSz-BBz). Figure 4C shows that CD4⁺ T cells were redirected with ICOSz and CD8⁺ T cells were redirected with 28z, BBz or ICOSz.

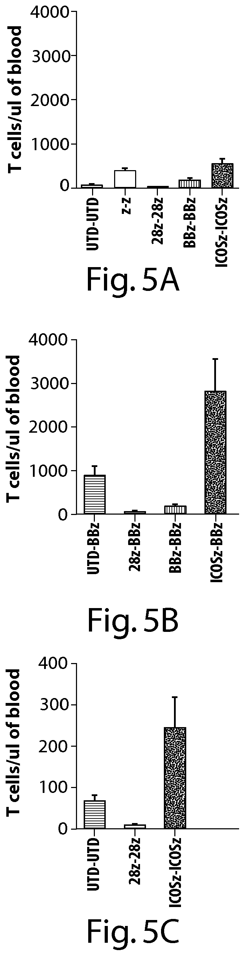

Figures 5A, 5B, and 5C show CD4⁺ T cells expressing an ICOS-based CAR significantly increased the persistence of CD8⁺ T cells expressing either CD28- or 4-1BB-based SS1-CARs. NSG mice bearing subcutaneous non-small cell lung tumors (L55) were treated 30 days after tumor implantation with two doses CD4⁺ and CD8⁺ T cells redirected with SS1-CARs. The concentration of CD8⁺ T cells was determined in the blood of treated animals 22 days after T cell injection. Error bars represent SEM (n=7-10). In Figure 5A, both CD4⁺ and CD8⁺ T cells were redirected with the same construct (z, 28z, BBz or ICOSz). In Figure 5B, CD8⁺ T cells were redirected with SS1-BBz and CD4⁺ T cells were left untransduced (UTD-BBz) or redirected with 28z (28z-BBz), BBz (BBz-BBz) or ICOSz (ICOSz-BBz). In Figure 5C, CD8⁺ T cells were redirected with 28z and CD4⁺ T cells were redirected with 28z or ICOSz.

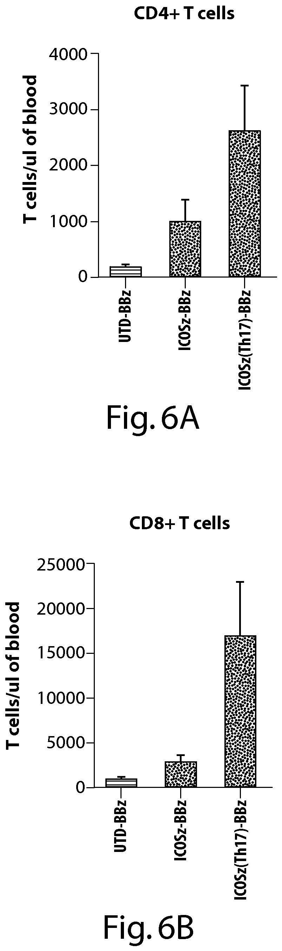

Figures 6A and 6B show T_{H}17-polarized CD4⁺ T cells expressing an ICOS-based CAR significantly increased the circulatory persistence of bulk CD8⁺ T cells expressing 4-1BB-based CARs. NSG mice bearing subcutaneous non-small cell lung tumors (L55) were treated 30 days after tumor implantation with two doses CD4⁺ and CD8⁺ T cells redirected with SS1-CARs. CD8⁺ T cells were redirected with BBz and CD4⁺ T cells were left untransduced or redirected with ICOSz. ICOSz redirected CD4⁺ T cells were cultured with or without T_{H}17 polarizing conditions. The concentration of CD4+ T cells (Fig. 6A) and CD8⁺ T cells (Fig. 6B) was determined in the blood of treated animals 22 days after T cell injection. Error bars represent SEM (n=7-10).

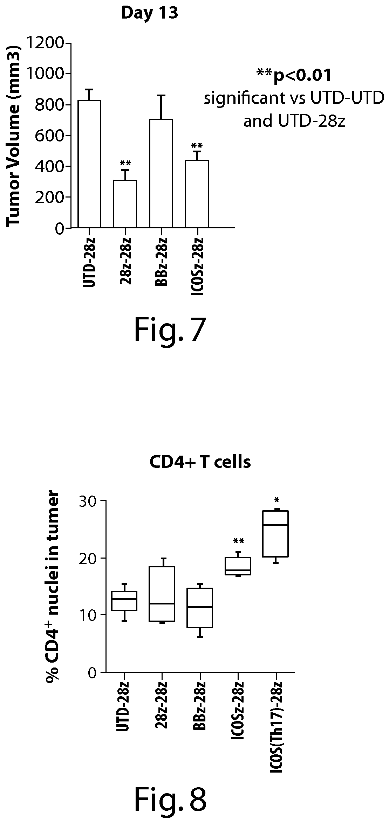

Figure 7 shows the antitumor effect and T cell persistence of CD4⁺ and CD8⁺ T cells redirected with SS1-CARs in mice with non-small cell lung tumors. NSG mice bearing subcutaneous ovarian tumors (L55) were treated 15 days after tumor implantation with two doses CD4⁺ and CD8⁺ T cells redirected with SS1-CARs. CD8⁺ T cells were redirected with 28z and CD4⁺ T cells were left untransduced or redirected with 28z, BBz or ICOSz. Figure 7 depicts tumor volume was analyzed 13 days following T cell injection. Results are expressed as a mean tumor volume (+/- SE) with n=7-8 mice per group.
Figure 8 shows CD4⁺ T cell infiltration in tumors treated with redirected T cells. NSG mice bearing subcutaneous non-small cell lung tumors (L55) were treated 15 days after tumor implantation with two doses CD4⁺ and CD8⁺ T cells redirected with SS1-CARs. CD8⁺ T cells were redirected with 28z and CD4⁺ T cells were left untransduced or redirected with 28z, BBz or ICOSz. ICOSz redirected CD4⁺ T cells were cultured with or without T_{H}17 polarizing conditions. The immune infiltrate was evaluated by immunohistochemistry for human CD4⁺ T cells at day 27 after treatment. The percentage of nuclei that stained positive for CD4⁺ T cells in intact areas of tumor was quantified with Aperio ImageScope software. Box plots show median (line) and 25th-75th percentile (box). The end of the whiskers represents the minimum and the maximum of all of the data. * p > 0.05 vs all groups, ** p > 0.05 vs UTD-28z and BBz-28z.

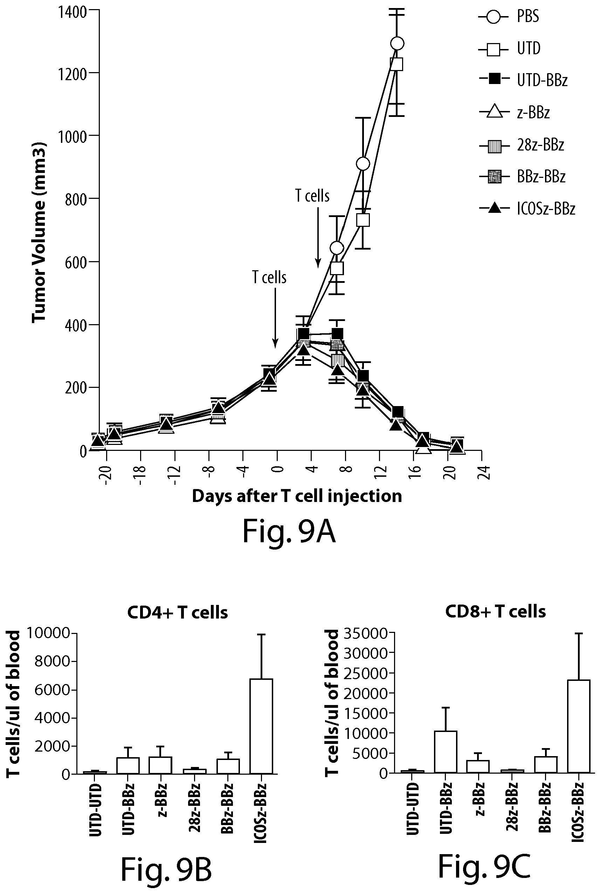

Figures 9A, 9B, and 9C show the antitumor effect and T cell persistence of CD4⁺ and CD8⁺ T cells redirected with Mov19-CARs in mice with ovarian tumors. NSG mice bearing subcutaneous ovarian tumors (SKOV3) were treated 30 days after tumor implantation with two doses CD4⁺ and CD8⁺ T cells redirected with Mov19-CARs. CD8⁺ T cells were redirected with BBz and CD4⁺ T cells were left untransduced or redirected with z, 28z, BBz or ICOSz. Figure 8A depicts tumor volume was analyzed at indicated time points. Results are expressed as a mean tumor volume (+/- SE) with n=6-8 mice per group. The concentration of CD4⁺ (Fig. 9B) and CD8⁺ T cells (Fig. 9C) were determined in the blood of treated animals 27 days after T cell injection. Error bars represent SEM (n=6-8).

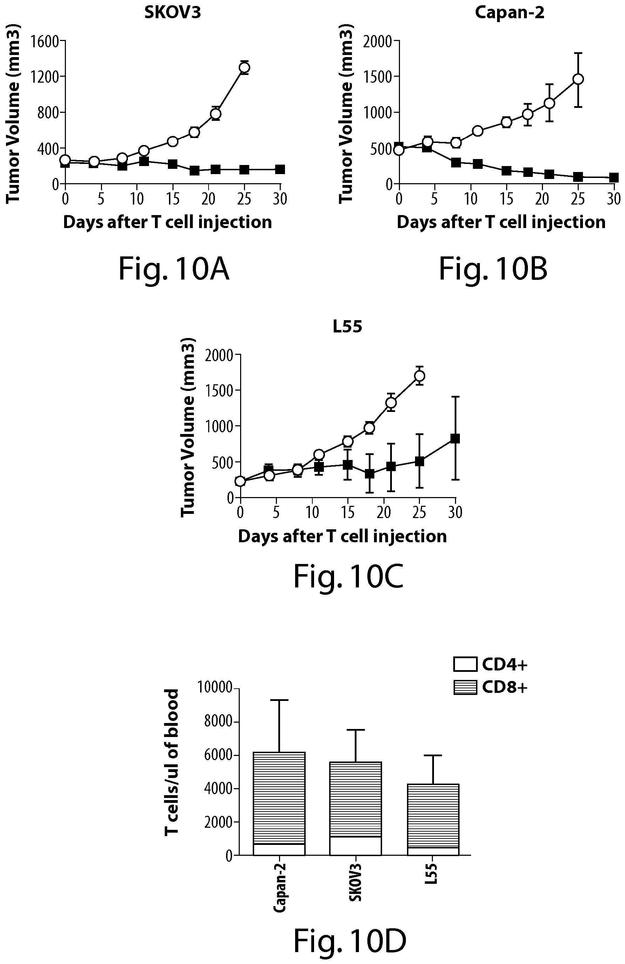

Figures 10A, 10B, 10C, and 10D show combination therapy using CD4⁺ T cells redirected with an ICOS-based CAR and CD8⁺ T cells redirected with 28z and BBz CARs. NSG mice bearing ovarian (SKOV3), pancreatic (Capan-2) or lung (L55) subcutaneous tumors were treated 15 days after tumor implantation with two doses CD4⁺ and CD8⁺ T cells redirected with SS1-CARs. CD4⁺ T cells were redirected with ICOSz and CD8⁺ T cells were redirected with a mix of 28z and BBz CARs. Tumor volume was analyzed at indicated time points for the ovarian tumor model (SKOV3) (Fig. 10A), pancreatic tumor model (Capan-2) (Fig. 10B), and lung tumor (L55) (Fig. 10C). Results are expressed as a mean tumor volume (+/- SE) with n=5 mice per group. White circles indicate the control population and black squares indicate the treated population. In Figure 10D, the concentrations of CD4⁺ (white bars) and CD8⁺ T cells (hatched bars) were determined in the blood of treated animals 33 days after T cell injection. Error bars represent SEM (n=5).

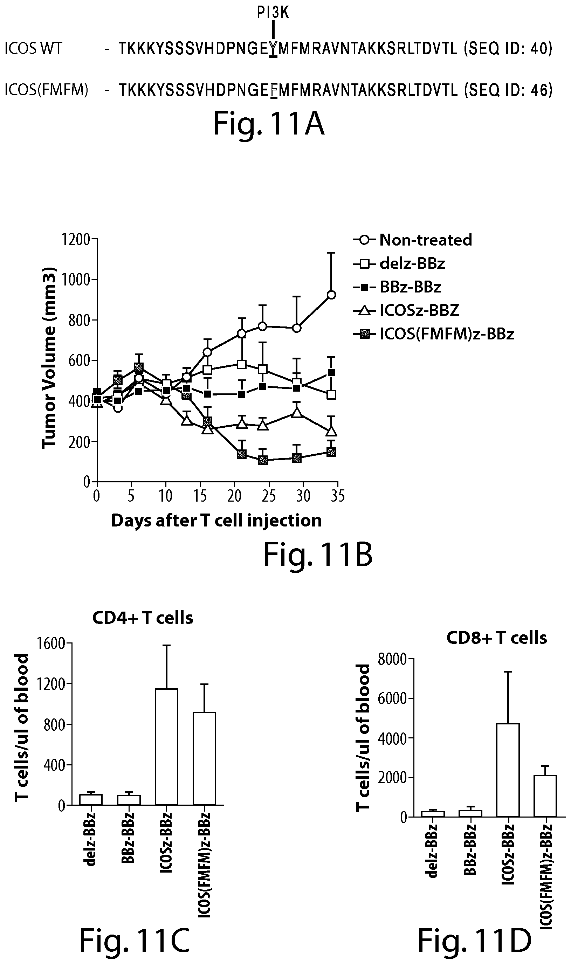

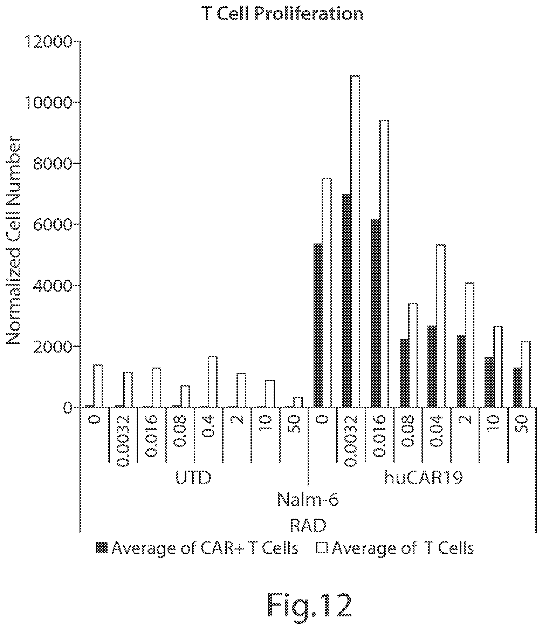

Figures 11A, 11B, 11C and 11D show the antitumor effect and T cell persistence of CD4⁺ T cells redirected with an ICOS-mutant-CAR in mice with pancreatic tumors. Figure 11A shows the amino acid sequences of the intracellular domain of ICOS used in chimeric antigen receptors. The FMFM (SEQ ID NO: 47) mutations are underlined and the signaling interaction with PI3K is indicated. NSG mice bearing subcutaneous pancreatic tumors (Capan-2) were treated 15 days after tumor implantation with two doses CD4⁺ and CD8⁺ T cells redirected with SS1-CARs. CD8⁺ T cells were redirected with BBz and CD4⁺ T cells were redirected with delz, BBz, ICOSz or ICOS(FMFM)z (Fig. 11B) ("FMFM" disclosed as SEQ ID NO: 47). Tumor volume was analyzed at indicated time points. Results are expressed as a mean tumor volume (+/- SE) with n=6-8 mice per group. The concentration of CD4⁺ (Fig. 11C) and CD8⁺ T cells (Fig. 12D) were determined in the blood of treated animals 21 days after T cell injection. Error bars represent SEM (n=6-8).
Figure 12 shows that the proliferation of CAR-expressing, transduced T cells is enhanced by low doses of RAD001 in a cell culture system. CARTs were co-cultured with NALM6 (Nalm-6) cells in the presence of different concentrations of RAD001 (nM). The number of CAR-positive CD3-positive T cells (black) and total T cells (white) was assessed after 4 days of co-culture.

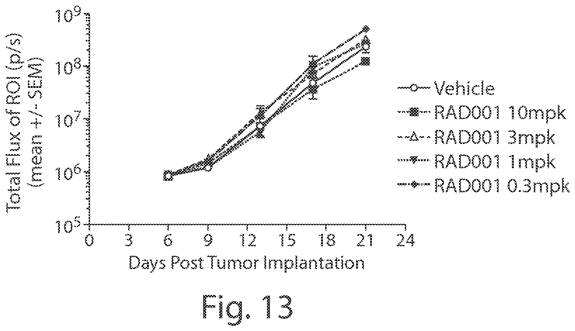

Figure 13 depicts tumor growth measurements of NALM6-luc cells with daily RAD001 dosing at 0.3, 1, 3, and 10 mg/kg (mpk) or vehicle dosing. Circles denote the vehicle; squares denote the 10 mg/kg dose of RAD001; triangles denote the 3 mg/kg dose of RAD001, inverted triangles denote the 1 mg/kg dose of RAD001; and diamonds denote the 0.3 mg/kg dose of RAD001.

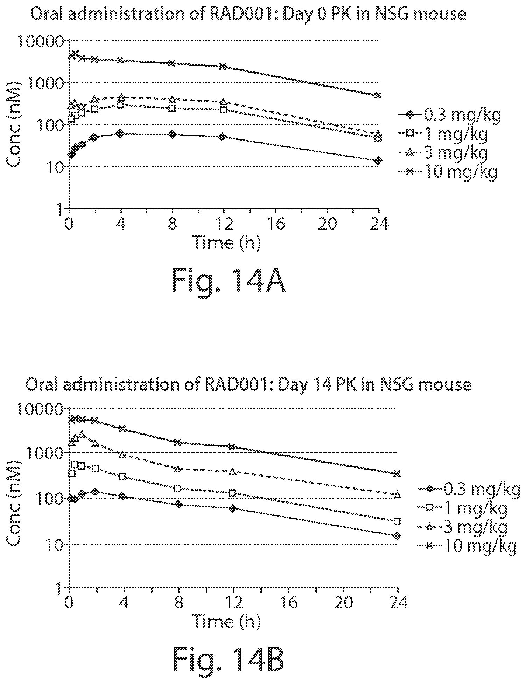

Figures 14A and 14B show pharmacokinetic curves showing the amount of RAD001 in the blood of NSG mice with NALM6 tumors. FIG. 14A shows day 0 PK following the first dose of RAD001. FIG. 14B shows Day 14 PK following the final RAD001 dose. Diamonds denote the 10 mg/kg dose of RAD001; squares denote the 1 mg/kg dose of RAD001; triangles denote the 3 mg/kg dose of RAD001; and x's denote the 10 mg/kg dose of RAD001.

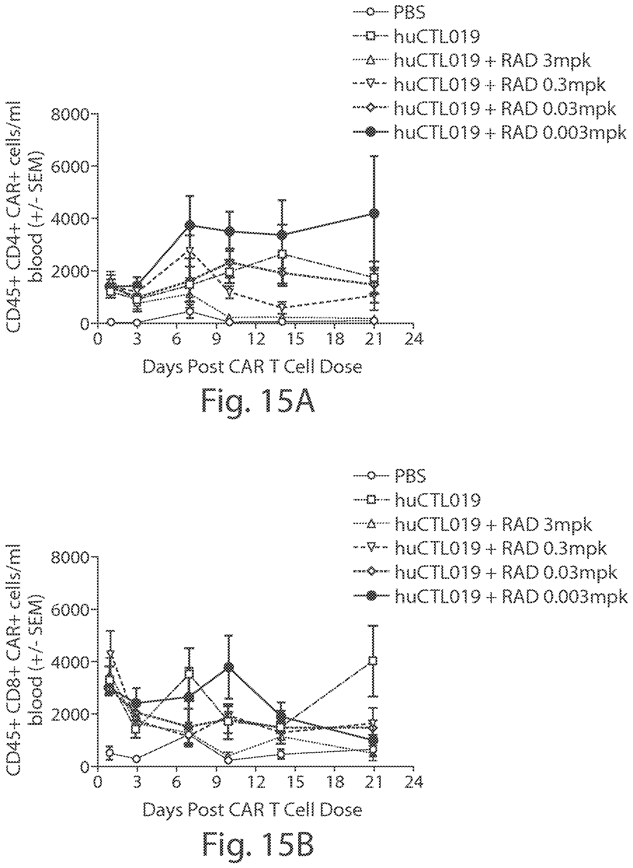

Figures 15A and 15B show in vivo proliferation of humanized CD19 CART cells with and without RAD001 dosing. Low doses of RAD001 (0.003 mg/kg) daily lead to an enhancement in CAR T cell proliferation, above the normal level of huCAR19 proliferation. Figures 15A shows CD4+ CAR T cells; FIG. 15B shows CD8+ CAR T cells. Circles denote PBS; squares denote huCTL019; triangles denote huCTL019 with 3 mg/kg RAD001; inverted triangles denote huCTL019 with 0.3 mg/kg RAD001; diamonds denote huCTL019 with 0.03 mg/kg RAD001; and circles denote huCTL019 with 0.003 mg/kg RAD001.

### DETAILED DESCRIPTION

### DEFINITIONS

Unless defined otherwise, all technical and scientific terms used herein have the same meaning as commonly understood by one of ordinary skill in the art to which the invention pertains.

"A" and "an" as the term is used herein, refers to one or to more than one (i.e., to at least one) of the grammatical object of the article. By way of example, "an element" means one element or more than one element.

"About" as the term is used herein, when referring to a measurable value such as an amount, a temporal duration, and the like, is meant to encompass variations of ±20% or in some embodiments ±10%, or in some embodiments ±5%, or in some embodiments ±1%, or in some embodiments ±0.1% from the specified value, as such variations are appropriate to perform the disclosed methods.

The phrase "about to receive", when used herein in the context of a patient receiving a first therapeutic who is about to receive a second therapeutic, refers to a situation where the patient is receiving or has received the first therapeutic for a disorder (e.g., a cancer), wherein the patient receives or will receive the second therapeutic in the course of treatment for that disorder.

An "antigen binding domain" as the term is used herein, refers to a molecule that has affinity for a target antigen, typically an antigen on a target cell, e.g., a cancer cell. An exemplary antigen binding domain comprises a polypeptide, e.g., an antibody molecule (which includes an antibody, and antigen binding fragments thereof, e.g., a immunoglobulin, single domain antibody (sdAb, e.g., a nanobody, and an scFv), or a non-antibody scaffold, e.g., a fibronectin, and the like. In embodiments, the antigen binding domain is a single polypeptide. In embodiments, the antigen binding domain comprises, one, two, or more, polypeptides. In embodiments the antigen binding domain comprises a fragment of an antibody that is sufficient to confer recognition and specific binding to the target antigen. Examples of an antibody fragment include, but are not limited to, an Fab, Fab', F(ab')₂, or Fv fragment, an scFv antibody fragment, a linear antibody, single domain antibody such as an sdAb, e.g., a nanobody, (either VL or VH), a camelid VHH domain, and multi-specific antibodies formed from antibody fragments. In an embodiment, the antigen binding domain is a "scFv," which can comprise a fusion protein comprising a VL chain and a VH chain of an antibody, where the VH and VL are linked via a short flexible polypeptide linker. The scFv is capable of being expressed as a single chain polypeptide and retains the specificity of the intact antibody from which it is derived. Moreover, the VL and VH variable chains can be linked in either order, e.g., with respect to the N-terminal and C-terminal ends of the polypeptide, the scFv may comprise VL-linker-VH or may comprise VH-linker-VL. In embodiments, the antigen binding domain comprises a non antibody scaffold, e.g., a fibronectin, ankyrin, domain antibody, e.g., a nanobody, lipocalin, small modular immuno-pharmaceutical, maxybody, Protein A, or affilin. The non antibody scaffold has the ability to bind to target antigen on a cell. In embodiments, the antigen binding domain is a polypeptide or fragment thereof of a naturally occurring protein expressed on a cell. In an embodiment, the antigen binding domain binds a growth factor or hormone receptor. While not wishing to be bound by theory, the antigen binding domain serves to provide specificity for target cells, and in embodiments, optimize and immune effector function by coupling antigen binding to generation of a signal by an intracellular signaling domain on an intracellular signaling member. Extracellular domains that bind a counter ligand, e.g., on target cells, are also within the definition of antigen binding domain.

As used herein, the term "antibody molecule" refers to a protein, e.g., an immunoglobulin chain or fragment thereof, comprising at least one immunoglobulin variable domain sequence. In an embodiment, an antibody molecule is a multispecific antibody molecule, e.g., it comprises a plurality of immunoglobulin variable domain sequences, wherein a first immunoglobulin variable domain sequence of the plurality has binding specificity for a first epitope and a second immunoglobulin variable domain sequence of the plurality has binding specificity for a second epitope. In an embodiment, a multispecific antibody molecule is a bispecific antibody molecule. A bispecific antibody has specificity for no more than two antigens. A bispecific antibody molecule is characterized by a first immunoglobulin variable domain sequence which has binding specificity for a first epitope and a second immunoglobulin variable domain sequence that has binding specificity for a second epitope.

The term "antibody," as used herein, refers to an immunoglobulin molecule which specifically binds with a target antigen. An antibody can be intact immunoglobulin derived from natural sources or from recombinant sources and can be an immunoreactive portion of intact immunoglobulin. Antibodies are typically tetramers of immunoglobulin molecules. The antibody molecule described herein may exist in a variety of forms where the antigen binding portion of the antibody is expressed as part of a contiguous polypeptide chain including, for example, a single domain antibody fragment (sdAb), a single chain antibody (scFv) and a humanized or human antibody, e.g., as described herein.

The term "antibody fragment" refers to a portion of an intact antibody and refers to the antigenic determining variable regions of an intact antibody. Examples of antibody fragments include, but are not limited to, a single chain domain antibody (sdAb), Fab, Fab', F(ab')2, Fv fragments, disulfide-linked Fvs (sdFv), a Fd fragment consisting of the VH and CH1 domains, linear antibodies, scFv antibodies, a linear antibody, single domain antibody such as an sdAb (either VL or VH), a camelid VHH domain, multispecific molecules formed from antibody fragments such as a bivalent fragment comprising two or more, e.g., two, Fab fragments linked by a disulfide bridge at the hinge region, or two or more, e.g., two isolated CDR or other epitope binding fragments of an antibody linked. An antibody fragment can also be incorporated into single domain antibodies, maxibodies, minibodies, nanobodies, intrabodies, diabodies, triabodies, tetrabodies, v-NAR and bis-scFv (see, e.g., Hollinger and Hudson, Nature Biotechnology 23:1126-1136, 2005). Antibody fragments can also be grafted into scaffolds based on polypeptides such as a fibronectin type III (Fn3)(see U.S. Patent No.: 6,703,199, which describes fibronectin polypeptide minibodies).

An "antibody heavy chain," as used herein, refers to the larger of the two types of polypeptide chains present in all antibody molecules in their naturally occurring conformations.

An "antibody light chain," as used herein, refers to the smaller of the two types of polypeptide chains present in all antibody molecules in their naturally occurring conformations. κ and λ light chains refer to the two major antibody light chain isotypes.

By the term "synthetic antibody" as used herein, is meant an antibody molecule which is generated using recombinant DNA technology, such as, for example, an antibody molecule expressed by a bacteriophage as described herein. The term should also be construed to mean an antibody molecule which has been generated by the synthesis of a DNA molecule encoding the antibody molecule and which DNA molecule expresses an antibody protein, or an amino acid sequence specifying the antibody, wherein the DNA or amino acid sequence has been obtained using synthetic DNA or amino acid sequence technology which is available and well known in the art.

The term "apheresis" as used herein refers to the art-recognized extracorporeal process by which the blood of a donor or patient is removed from the donor or patient and passed through an apparatus that separates out selected particular constituent(s) and returns the remainder to the circulation of the donor or patient, e.g., by retransfusion. Thus, "an apheresis sample" refers to a sample obtained using apheresis.

"Anti-tumor effect" as the term is used herein, refers to a biological effect which can be manifested by various means, including but not limited to, e.g., a decrease in tumor volume, a decrease in the number of tumor cells, a decrease in the number of metastases, an increase in life expectancy, decrease in tumor cell proliferation, decrease in tumor cell survival, or amelioration of various physiological symptoms associated with the cancerous condition. An "anti-tumor effect" can also be manifested by the ability of the peptides, polynucleotides, cells and antibodies of the disclosure in prevention of the occurrence of tumor in the first place.

"Autologous" as the term is used herein refers to any material derived from the same individual to whom it is later to be re-introduced.

"Allogeneic" as the term is used herein refers to any material derived from a different animal of the same species as the individual to whom the material is introduced. Two or more individuals are said to be allogeneic to one another when the genes at one or more loci are not identical. In some aspects, allogeneic material from individuals of the same species may be sufficiently unlike genetically to interact antigenically.

"Cancer" as the term is used herein, refers to a disease characterized by the rapid and uncontrolled growth of aberrant cells. Cancer cells can spread locally or through the bloodstream and lymphatic system to other parts of the body. Examples of various cancers include but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer and the like. In an embodiment, a cancer is characterized by expression of a PD-1 ligand, e.g., PD-L1 or PD-L2, on a cancer cell or in a tumor microenvironment. The term "cancer" includes all types of cancerous growths or oncogenic processes, metastatic tissues or malignantly transformed cells, tissues, or organs, irrespective of histopathologic type or stage of invasiveness. The terms "tumor" and "cancer" are used interchangeably herein, e.g., both terms encompass solid and liquid, e.g., diffuse or circulating, tumors. As used herein, the term "cancer" or "tumor" includes premalignant, as well as malignant cancers and tumors.

The terms "cancer associated antigen" or "tumor antigen" or "proliferative disorder antigen" or "antigen associated with a proliferative disorder" interchangeably refers to a molecule (typically protein, carbohydrate or lipid) that is preferentially expressed on the surface of a cancer cell, either entirely or as a fragment (e.g., MHC/peptide), in comparison to a normal cell, and which is useful for the preferential targeting of a pharmacological agent to the cancer cell. In some embodiments, the tumor antigen is an antigen that is common to a specific proliferative disorder. In some embodiments, a cancer-associated antigen is a cell surface molecule that is overexpressed in a cancer cell in comparison to a normal cell, for instance, 1-fold over expression, 2-fold overexpression, 3-fold overexpression or more in comparison to a normal cell. In some enbodiments, a cancer-associated antigen is a cell surface molecule that is inappropriately synthesized in the cancer cell, for instance, a molecule that contains deletions, additions or mutations in comparison to the molecule expressed on a normal cell. In some enbodiments, a cancer-associated antigen will be expressed exclusively on the cell surface of a cancer cell, entirely or as a fragment (e.g., MHC/peptide), and not synthesized or expressed on the surface of a normal cell. In some instances, the CARs of the present disclosure includes CARs comprising an antigen binding domain (e.g., antibody or antibody fragment) that binds to a MHC presented peptide. Normally, peptides derived from endogenous proteins fill the pockets of Major histocompatibility complex (MHC) class I molecules, and are recognized by T cell receptors (TCRs) on CD8 + T lymphocytes. The MHC class I complexes are constitutively expressed by all nucleated cells. In cancer, virus-specific and/or tumor-specific peptide/MHC complexes represent a unique class of cell surface targets for immunotherapy. TCR-like antibodies targeting peptides derived from viral or tumor antigens in the context of human leukocyte antigen (HLA)-A1 or HLA-A2 have been described (see, e.g., Sastry et al., J Virol. 2011 85(5):1935-1942; Sergeeva et al., Bood, 2011 117(16):4262-4272; Verma et al., J Immunol 2010 184(4):2156-2165; Willemsen et al., Gene Ther 2001 8(21) :1601-1608 ; Dao et al., Sci Transl Med 2013 5(176) :176ra33 ; Tassev et al., Cancer Gene Ther 2012 19(2):84-100). For example, TCR-like antibody can be identified from screening a library, such as a human scFv phage displayed library. Accordingly, the present disclosure provides CARs that comprising an antigen binding domain that binds to a MHC presented peptide of a molecule selected from the group of WT1, NY-ESO-1, LAGE-1a, MAGE-A1 and RAGE-1.

"Chimeric Antigen Receptor" or alternatively a "CAR" as the term is used herein, refers to a recombinant polypeptide construct comprising at least an extracellular antigen binding domain, a transmembrane domain and a cytoplasmic signaling domain (also referred to herein as "an intracellular signaling domain") comprising a functional signaling domain derived from a stimulatory molecule and/or costimulatory molecule as defined below. In some embodiments, the domains in the CAR polypeptide construct are in the same polypeptide chain, e.g., comprise a chimeric fusion protein. In other embodiments, the domains in the CAR polypeptide construct are not contiguous with each other, e.g., are in different polypeptide chains, e.g., as provided in an RCAR as described herein.

In one aspect, the stimulatory molecule of the CAR is the zeta chain associated with the T cell receptor complex (e.g., CD3 zeta). In one aspect, the cytoplasmic signaling domain comprises a primary signaling domain (e.g., a primary signaling domain of CD3-zeta). In one aspect, the cytoplasmic signaling domain further comprises one or more functional signaling domains derived from at least one costimulatory molecule as defined below. In one aspect, the costimulatory molecule is chosen from 4-1BB (i.e., CD137), CD27, CD28 and/or ICOS. In one aspect, the CAR comprises a chimeric fusion protein comprising an extracellular antigen binding domain, a transmembrane domain and an intracellular signaling domain comprising a functional signaling domain derived from a stimulatory molecule. In one aspect, the CAR comprises a chimeric fusion protein comprising an extracellular antigen binding domain, a transmembrane domain and an intracellular signaling domain comprising a functional signaling domain derived from a co-stimulatory molecule and a functional signaling domain derived from a stimulatory molecule. In one aspect, the CAR comprises a chimeric fusion protein comprising an extracellular antigen binding domain, a transmembrane domain and an intracellular signaling domain comprising two functional signaling domains derived from one or more co-stimulatory molecule(s) and a functional signaling domain derived from a stimulatory molecule. In one aspect, the CAR comprises a chimeric fusion protein comprising an extracellular antigen binding domain, a transmembrane domain and an intracellular signaling domain comprising at least two functional signaling domains derived from one or more co-stimulatory molecule(s) and a functional signaling domain derived from a stimulatory molecule. In one aspect the CAR comprises an optional leader sequence at the amino-terminus (N-ter) of the CAR fusion protein. In one aspect, the CAR further comprises a leader sequence at the N-terminus of the extracellular antigen binding domain, wherein the leader sequence is optionally cleaved from the antigen binding domain (e.g., a scFv) during cellular processing and localization of the CAR to the cellular membrane.

The portion of the CAR of the disclosure comprising an antibody or antibody fragment thereof may exist in a variety of forms, for example, where the antigen binding domain is expressed as part of a polypeptide chain including, for example, a single domain antibody fragment (sdAb), a single chain antibody (scFv), or e.g., a humanized antibody, or bispecific antibody (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc. Natl. Acad. Sci. USA 85:5879-5883; Bird et al., 1988, Science 242:423-426). In one aspect, the antigen binding domain of a CAR composition of the invention comprises an antibody fragment. In a further aspect, the CAR comprises an antibody fragment that comprises a scFv.

A CAR that comprises an antigen binding domain (e.g., a scFv, a single domain antibody, or TCR (e.g., a TCR alpha binding domain or TCR beta binding domain)) that targets a specific cancer associated antigen (or tumor marker) X, such as those described herein, is also referred to as XCAR. For example, a CAR that comprises an antigen binding domain that targets CD19 is referred to as CD19CAR.

"CARX cell," as that term is used herein, refers to a cell comprising CAR. Any cell that is engineered to express a CAR can be used as a CARX cell. Typically the CARX cell is a T cell comprising a CAR, and is referred to as a CART cell. In an instance, the CART is a CD4⁺ T cell comprising a CAR, and is referred to herein as a CD4⁺T cell comprising a CAR^{CD4+}. In an instance, the CART is a CD8+ T cell comprising a CAR, and is referred to herein as a CD8+ T cell comprising a CAR^{CD8+}. In an embodiment the CART cell is autologous to the patient. In an embodiment the CART is allogeneic to the patient. In an instance, a patient receives more than one kind of CART cell, e.g., the patient receives a CD4⁺ T cell comprising a CAR^{CD4+} and a CD8+ T cell comprising a CAR^{CD8+}.

"CAR^{CD4+}" and "CAR^{CD8+}" as those terms are used herein, refer to a CAR associated with, respectively, a CD4⁺ T cell and a CD8+ T cell. In an embodiment, the CAR^{CD4+} is provided in, designed to optimize the performance of, or optimizes the performance of, a CD4⁺ T cell. In an embodiment, the CAR^{CD8+} is provided in, designed to optimize the performance of, or optimizes the performance of, a CD8+ T cell. Typically, a CAR^{CD4+} optimizes the performance of a CD4⁺ T cell and, in instances, comprises an ICOS domain. Typically, a CAR^{CD8+} optimizes the performance of a CD8+ T cell and, in embodiments, comprises a CD28 or 4-1BB domain. Thus, CARs can be optimized for a selected subset of T cells, e.g., CD4⁺ T cells or CD8⁺ T cells.

A CD4⁺T cell comprising a CAR^{CD4+} and a CD8+ T cell comprising a CAR^{CD8+}, e.g., when provided in a kit, composition, or administered to a patient, differ from one another in structure. The CAR^{CD4+} and the CAR^{CD8+} can differ from one another, e.g., wherein the intracellular signaling domain of the CAR^{CD4+} differs from the CAR^{CD8+} intracellular signaling domain, wherein the the CAR^{CD4+} comprises a first costimulatory domain not present on the the CAR^{CD8+}, wherein the the CAR^{CD4+} comprises a first costimulatory domain and the CAR^{CD8+} comprises a second costimulatory domain, or wherein the the CAR^{CD4+} comprises a first costimulatory domain, e.g., an ICOS domain, not present on the CAR^{CD8+} and the CAR^{CD8+} comprises a second costimulatory domain, e.g., a CD28 or 4-1BB domain, not present on the CAR^{CD4+}.

A CD4⁺ T cell, as that term is used herein, refers to a T cell which expresses the surface protein CD4. In an embodiment, the T cell expresses the surface protein CD4 at a sufficient level to be detected, e.g., by flow cytometry.

A CD8⁺ T cell, as that term is used herein, refers to a T cell which expresses the surface protein CD8. In an embodiment, the T cell expresses the surface protein CD8 at a sufficient level to be detected, e.g., by flow cytometry.

The terms "complementarity determining region" or "CDR," as used herein, refer to the sequences of amino acids within antibody variable regions which confer antigen specificity and binding affinity. For example, in general, there are three CDRs in each heavy chain variable region (e.g., HCDR1, HCDR2, and HCDR3) and three CDRs in each light chain variable region (LCDR1, LCDR2, and LCDR3). The precise amino acid sequence boundaries of a given CDR can be determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme), or a combination thereof. Under the Kabat numbering scheme, in some embodiments, the CDR amino acid residues in the heavy chain variable domain (VH) are numbered 31-35 (HCDR1), 50-65 (HCDR2), and 95-102 (HCDR3); and the CDR amino acid residues in the light chain variable domain (VL) are numbered 24-34 (LCDR1), 50-56 (LCDR2), and 89-97 (LCDR3). Under the Chothia numbering scheme, in some embodiments, the CDR amino acids in the VH are numbered 26-32 (HCDR1), 52-56 (HCDR2), and 95-102 (HCDR3); and the CDR amino acid residues in the VL are numbered 26-32 (LCDR1), 50-52 (LCDR2), and 91-96 (LCDR3). In a combined Kabat and Chothia numbering scheme, in some embodiments, the CDRs correspond to the amino acid residues that are part of a Kabat CDR, a Chothia CDR, or both. For instance, in some embodiments, the CDRs correspond to amino acid residues 26-35 (HCDR1), 50-65 (HCDR2), and 95-102 (HCDR3) in a VH, e.g., a mammalian VH, e.g., a human VH; and amino acid residues 24-34 (LCDR1), 50-56 (LCDR2), and 89-97 (LCDR3) in a VL, e.g., a mammalian VL, e.g., a human VL.

"Costimulatory signaling domain," as that term is used herein, refers to an intracellular signaling domain of a molecule, e.g., an endogenous molecule, of the CART cell that, upon binding to its cognate counter ligand on a target cell, enhance, e.g., increases, an immune effector response. A costimulatory intracellular signaling domain can be the intracellular portion of a costimulatory molecule. Costimulatory molecules include, but are not limited to an MHC class I molecule, TNF receptor proteins, Immunoglobulin-like proteins, cytokine receptors, integrins, signaling lymphocytic activation molecules (SLAM proteins), activating NK cell receptors, BTLA, a Toll ligand receptor, OX40, CD2, CD7, CD27, CD28, CD30, CD40, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, and a ligand that specifically binds with CD83. "Costimulatory molecule" refers to a molecule comprising a "costimulatory signaling domain." A costimulatory intracellular signaling domain can be derived from the intracellular portion of a costimulatory molecule. The intracellular signaling domain can comprise the entire intracellular portion, or the entire native intracellular signaling domain, of the molecule from which it is derived, or a functional fragment thereof.

As used herein, the term "CD19" refers to the Cluster of Differentiation 19 protein, which is an antigenic determinant detectable on leukemia precursor cells. The human and murine amino acid and nucleic acid sequences can be found in a public database, such as GenBank, UniProt and Swiss-Prot. For example, the amino acid sequence of human CD19 can be found as UniProt/Swiss-Prot Accession No. P15391 and the nucleotide sequence encoding of the human CD19 can be found at Accession No. NM_001178098. As used herein, "CD19" includes proteins comprising mutations, e.g., point mutations, fragments, insertions, deletions and splice variants of full length wild-type CD19. CD19 is expressed on most B lineage cancers, including, e.g., acute lymphoblastic leukaemia, chronic lymphocyte leukaemia and non-Hodgkin lymphoma. Other cells with express CD19 are provided below in the definition of "disease associated with expression of CD19." It is also an early marker of B cell progenitors. See, e.g., Nicholson et al. Mol. Immun. 34 (16-17): 1157-1165 (1997). In one aspect the antigen-binding portion of the CART recognizes and binds an antigen within the extracellular domain of the CD19 protein. In one aspect, the CD19 protein is expressed on a cancer cell.

"Derived from" as that term is used herein, indicates a relationship between a first and a second molecule. It generally refers to structural similarity between the first molecule and a second molecule and does not conotate or include a process or source limitation on a first molecule that is derived from a second molecule. For example, in the case of an intracellular signaling domain that is derived from a CD3zeta molecule, the intracellular signaling domain retains sufficient CD3zeta structure such that is has the required function, namely, the ability to generate a signal under the appropriate conditions. It does not conotate or include a limitation to a particular process of producing the intracellular signaling domain, e.g., it does not mean that, to provide the intracellular signaling domain, one must start with a CD3zeta sequence and delete unwanted sequence, or impose mutations, to arrive at the intracellular signaling domain.

The phrase "disease associated with expression of a tumor marker as described herein" includes, but is not limited to, a disease associated with a cell that expresses a tumor marker as described herein or condition associated with a cell which expresses, or at any time expressed, a tumor marker as described herein including, e.g., proliferative diseases such as a cancer or malignancy or a precancerous condition such as a myelodysplasia, a myelodysplastic syndrome or a preleukemia; or a noncancer related indication associated with a cell which expresses a tumor marker as described herein. In one aspect, a cancer associated with expression of a tumor marker as described herein is a hematological cancer. In one aspect, a cancer associated with expression of a tumor marker as described herein is a solid cancer. Further diseases associated with expression of a tumor marker as described herein include, but not limited to, e.g., atypical and/or non-classical cancers, malignancies, precancerous conditions or proliferative diseases associated with expression of a tumor marker as described herein. Non-cancer related indications associated with expression of a tumor marker as described herein include, but are not limited to, e.g., autoimmune disease, (e.g., lupus), inflammatory disorders (allergy and asthma) and transplantation.

The phrase "disease associated with expression of CD19" includes, but is not limited to, a disease associated with a cell that expresses CD19 or condition associated with a cell which expresses, or at any time expressed, CD19 including, e.g., proliferative diseases such as a cancer or malignancy or a precancerous condition such as a myelodysplasia, a myelodysplastic syndrome or a preleukemia; or a noncancer related indication associated with a cell which expresses CD19. For the avoidance of doubt, a disease associated with expression of CD19 may include a condition associated with a cell which does not presently express CD19, e.g., because CD19 expression has been downregulated, e.g., due to treatment with a molecule targeting CD19, e.g., a CD19 CAR, but which at one time expressed CD19. In one aspect, a cancer associated with expression of CD19 is a hematological cancer. In one aspect, the hematolical cancer is a leukemia or a lymphoma. In one aspect, a cancer associated with expression of CD19 includes cancers and malignancies including, but not limited to, e.g., one or more acute leukemias including but not limited to, e.g., B-cell acute Lymphoid Leukemia (BALL), T-cell acute Lymphoid Leukemia (TALL), acute lymphoid leukemia (ALL); one or more chronic leukemias including but not limited to, e.g., chronic myelogenous leukemia (CML), Chronic Lymphoid Leukemia (CLL). Additional cancers or hematologic conditions associated with expression of CD19 comprise, but are not limited to, e.g., B cell prolymphocytic leukemia, blastic plasmacytoid dendritic cell neoplasm, Burkitt's lymphoma, diffuse large B cell lymphoma, Follicular lymphoma, Hairy cell leukemia, small cell- or a large cell-follicular lymphoma, malignant lymphoproliferative conditions, MALT lymphoma, mantle cell lymphoma (MCL), Marginal zone lymphoma, multiple myeloma, myelodysplasia and myelodysplastic syndrome, non-Hodgkin lymphoma, Hodgkin lymphoma, plasmablastic lymphoma, plasmacytoid dendritic cell neoplasm, Waldenstrom macroglobulinemia, and "preleukemia" which are a diverse collection of hematological conditions united by ineffective production (or dysplasia) of myeloid blood cells, and the like. Further diseases associated with expression of CD19 expression include, but not limited to, e.g., atypical and/or non-classical cancers, malignancies, precancerous conditions or proliferative diseases associated with expression of CD19. Non-cancer related indications associated with expression of CD19 include, but are not limited to, e.g., autoimmune disease, (e.g., lupus), inflammatory disorders (allergy and asthma) and transplantation. In some embodiments, the tumor antigen-expressing cell expresses, or at any time expressed, mRNA encoding the tumor antigen. In an embodiment, the tumor antigen -expressing cell produces the tumor antigen protein (e.g., wild-type or mutant), and the tumor antigen protein may be present at normal levels or reduced levels. In an embodiment, the tumor antigen -expressing cell produced detectable levels of a tumor antigen protein at one point, and subsequently produced substantially no detectable tumor antigen protein.

"dsRNA," as that term is used herein, refers to a nucleic acid molecule, having at least a region of duplexed structure, that is capable of mediating sequence specific inhibition of the expression of a target gene. dsRNAs comprise short interfering RNA (siRNA) and short hairpin RNA (shRNA). In instances, shRNA is similar in structure to an siRNA but includes a moiety, typically one or more RNA monomers, that connect a duplex region of sense and an antisense sequence. In an instance the shRNA, after intracellular processing (e.g., by Dicer), results in a 19-23 nucleotide duplex siRNA with 2 nucleotide 3' overhangs.

"Endogenous" as that term is used herein, refers to any material, e.g., a polypeptide, from or produced inside an organism, cell, tissue or system.

"Exogenous" as that term is used herein, refers to any material, e.g., a polypeptide, or dimerization molecule, introduced from or produced outside an organism, cell, tissue or system.

"ICOS domain" as that term is used herein, refers to a functional (in terms of intracellular signaling) fragment, or analog, of an ICOS molecule. It can comprise the entire intracellular region, or a fragment of the intracellular region which is sufficient for generation of an intracellular signal, e.g., when an antigen binding domain to which it is fused binds cognate antigen. In embodiments the ICOS domain has at least 70%, 75%, 80%, 85%, 90%, 95%, 98%, or 99 % sequence identity with, or differs by no more than 15, 10, 5, 4, 3, 2, or 1 amino acid residues from, the corresponding residues of the entire intracellular region, or a fragment of the intracellular region which is sufficient for generation of an intracellular signal, of a naturally occurring ICOS molecule, e.g., a human, or other mammalian, e.g., a nonhuman species, e.g., rodent, monkey, ape or murine intracellular costimulatory molecule. In embodiments the costimulatory domain has at least 70%, 75%, 80%, 85%, 90%, 95%, 98%, or 99 % sequence identity with, or differs by no more than 15, 10, 5, 4, 3, 2, or 1 amino acid residues from, SEQ ID NO: 40.

"Immune effector cell," as that term is used herein, refers to a cell that is involved in an immune response, e.g., in the promotion of an immune effector response. Examples of immune effector cells include T cells, e.g., alpha/beta T cells and gamma/delta T cells, B cells, natural killer (NK) cells, natural killer T (NKT) cells, mast cells, and myeloic-derived phagocytes.

"Immune effector function" or "immune effector response," as that term is used herein, refers to function or response, e.g., of an immune effector cell, that enhances or promotes an immune attack of a target cell. E.g., an immune effector function or response refers a property of a T or NK cell that promotes killing or the inhibition of growth or proliferation, of a target cell. In the case of a T cell, primary stimulation and costimulation are examples of immune effector function or response. An immune effector function or response can be promoted by the action of a CAR, and can, e.g., result in a CARX cell that is more effective at proliferation, cytokine production, cytotoxicity or upregulation of cell surface markers such as CD25, CD69, CD107a.

An "inhibitory extracellular domain," as that term is used herein, refers to polypeptide comprising an extracellular domain of an inhibitory molecule. Normally, binding to its counterligand has an inhibitory effect on the generation of an immune effector response. When linked, e.g., fused to an intracellular signaling domain, it redirects an interaction that normally inhibits the generation of an immune effector response into one that promotes an immune effector response.

"Inhibitory molecule," as that term is used herein, refers to a molecule, e.g., an endogenous molecule, of CARX cell, e.g., a CART cell that, upon binding to its cognate counter ligand on a target cell, minimizes, e.g., suppresses or inhibits, an immune effector response. Examples of inhibitory molecules include PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta.

The term "effector function" refers to a specialized function of a cell. Effector function of a T cell, for example, may be cytolytic activity or helper activity including the secretion of cytokines

"Intracellular signaling domain," as the term is used herein, refers to an intracellular portion of a molecule. In embodiments, the intracellular signal domain transduces the effector function signal and directs the cell to perform a specialized function. While the entire intracellular signaling domain can be employed, in many cases it is not necessary to use the entire chain. To the extent that a truncated portion of the intracellular signaling domain is used, such truncated portion may be used in place of the intact chain as long as it transduces the effector function signal. The term intracellular signaling domain is thus meant to include any truncated portion of the intracellular signaling domain sufficient to transduce the effector function signal.

In an embodiment, the intracellular signaling domain can comprise a primary intracellular signaling domain. Exemplary primary intracellular signaling domains include those derived from the molecules responsible for primary stimulation, or antigen dependent simulation. In an embodiment, the intracellular signaling domain can comprise a costimulatory intracellular domain. Exemplary costimulatory intracellular signaling domains include those derived from molecules responsible for costimulatory signals, or antigen independent stimulation. For example, in the case of a CART, a primary intracellular signaling domain can comprise cytoplasmic sequences of the T cell receptor, and a costimulatory intracellular signaling domain can comprise cytoplasmic sequence from co-receptor or costimulatory molecule.

Primary intracellular signaling domains can comprise signaling motifs which are known as immunoreceptor tyrosine-based activation motifs or ITAMs. Examples of ITAM containing primary cytoplasmic signaling sequences include those derived from CD3 zeta, FcR gamma, FcR beta, CD3 gamma, CD3 delta, CD3 epsilon, CD5, CD22, CD79a, CD79b, CD278 (also known as "ICOS"), FcεRI, and CD66d. Further examples of molecules containing a primary intracellular signaling domain that are of particular use in the invention include those of DAP10, DAP12, and CD32.

"Isolated" as that term is used herein refers to a nucleic acid or polypeptide means separated from at least one contaminating compound. With regard to a nucleic acid or polypeptide that exists in nature, it means free of a compound with which it occurs in nature, wherein in embodiments, the contaminating compound is a polynucleotide or polypeptide. With regard to a nucleic acid or polypeptide that is made synthetically, it means free of a sude reactant or compound used in its preparation, e.g., a solvent or starting reactant. For example, a nucleic acid or a polypeptide naturally present in a living animal is not "isolated," but the same nucleic acid or polypeptide partially or completely separated from the coexisting materials of its natural state is "isolated." An isolated nucleic acid or protein can exist in substantially purified form, or can exist in a non-native environment such as, for example, a host cell.

The term 'low, immune enhancing, dose" when used in conjuction with an mTOR inhibitor, e.g., an allosteric mTOR inhibitor, e.g., RAD001 or rapamycin, or a catalytic mTOR inhibitor, refers to a dose of mTOR inhibitor that partially, but not fully, inhibits mTOR activity, e.g., as measured by the inhibition of P70 S6 kinase activity. Methods for evaluating mTOR activity, e.g., by inhibition of P70 S6 kinase, are discussed herein. The dose is insufficient to result in complete immune suppression but is sufficient to enhance the immune response. In aninstance, the low, immune enhancing, dose of mTOR inhibitor results in a decrease in the number of PD-1 positive T cells and/or an increase in the number of PD-1 negative T cells; an increase in the ratio of PD-1 negative T cells/PD-1 positive T cells; an increase in the number of naive T cells; an increase in the number of memory T cell precursors (e.g., cells with any one or a combination of the following characteristics: increased CD62L^{high}, increased CD127^{high}, increased CD27+, decreased KLRG1, and increased BCL2), or an increase in the expression of one or more of the memory T cell precursor markers CD62L^{high}, CD127^{high}, CD27⁺, and BCL2; and/or a decrease in the expression of memory T cell precursor marker, KLRG1.

"Membrane anchor," as that term is used herein, refers to a polypeptide sufficient to anchor an extracellular domain to the plasma membrane.

"Membrane tethering domain", as that term is used herein, refers to a polypeptide or moiety, e.g., a myristoyl group, sufficient to anchor an extracellular or intracellular domain to the plasma membrane.

"Nucleic acid-based inhibitor," as that term is used herein, refers to a nucleic acid molecule that can inhibit expression of a target gene, e.g., an inhibitory molecule. It comprises double stranded RNA (dsRNA), including short hairpin RNA (shRNA) and short interfering RNA (siRNA), antisense RNA, and microRNA (miRNA). In an instance, the nucleic-acid based inhibitor binds to the target mRNA and inhibits the production of protein therefrom, e.g., by cleavage of the target mRNA.

"Refractory" as used herein refers to a disease, e.g., cancer, that does not respond to a treatment. In embodiments, a refractory cancer can be resistant to a treatment before or at the beginning of the treatment. In other embodiments, the refractory cancer can become resistant during a treatment. A refractory cancer is also called a resistant cancer.

"Relapsed" or a "relapse" as used herein refers to the reappearance of a disease (e.g., cancer) or the signs and symptoms of a disease such as cancer after a period of improvement or responsiveness, e.g., after prior treatment of a therapy, e.g., cancer therapy. For example, the period of responsiveness may involve the level of cancer cells falling below a certain threshold, e.g., below 20%, 1%, 10%, 5%, 4%, 3%, 2%, or 1%. The reappearance may involve the level of cancer cells rising above a certain threshold, e.g., above 20%, 1%, 10%, 5%, 4%, 3%, 2%, or 1%.

"Subject", as that term is used herein, refers to living organisms in which an immune response can be elicited (e.g., mammals). Examples of subjects include humans, monkeys, chimpanzees, dogs, cats, mice, rats, and transgenic species thereof. In an embodiment the subject is a human.

"Transmembrane domain," as that term is used herein, refers to a polypeptide that spans the plasma membrane. In an instance, it links an extracellular sequence, e.g., a switch domain, an extracellular recognition element, e.g., an antigen binding domain, an inhibitory counter ligand binding domain, or costimulatory ECD domain, to an intracellular sequence, e.g., to a switch domain or an intracellular signaling domain. A transmembrane domain of particular use in this invention may include at least the transmembrane region(s) of e.g., the alpha, beta or zeta chain of the T-cell receptor, CD28, CD3 epsilon, CD45, CD4, CD5, CD8 (e.g., CD8 alpha, CD8 beta), CD9, CD16, CD22, CD33, CD37, CD64, CD80, CD86, CD134, CD137, CD154. In some embodiments, a transmembrane domain may include at least the transmembrane region(s) of, e.g., KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp.

"Unit dosage form" as the term is used herein refers to a dosage for suitable one administration. By way of example a unit dosage form can be a tablet, a capsule, or an amount of therapeutic disposed in a delivery device, e.g., a syringe or intravenous drip bag. In an embodiment a unit dosage form is administered in a single administration. In an embodiment more than one unit dosage form, e.g., two tablets, can be administered simultaneously.

"Xenogeneic" as the term is used herein refers to a graft derived from an animal of a different species.

"Regulatable chimeric antigen receptor (RCAR),"as that term is used herein, refers to a set of polypeptides, typically two in the simplest embodiments, which when in a RCARX cell, provides the RCARX cell with specificity for a target cell, typically a cancer cell, and with regulatable intracellular signal generation or proliferation, which can optimize an immune effector property of the RCARX cell. An RCARX cell relies at least in part, on an antigen binding domain to provide specificity to a target cell that comprises the antigen bound by the antigen binding domain. In an embodiment, an RCAR includes a dimerization switch that, upon the presence of a dimerization molecule, can couple an intracellular signaling domain to the antigen binding domain.

"Switch domain," as that term is used herein, e.g., when referring to an RCAR, refers to an entity, typically a polypeptide-based entity, that, in the presence of a dimerization molecule, associates with another switch domain. The association results in a functional coupling of a first entity linked to, e.g., fused to, a first switch domain, and a second entity linked to, e.g., fused to, a second switch domain. A first and second switch domain are collectively referred to as a dimerization switch. In embodiments, the first and second switch domains are the same as one another, e.g., they are polypeptides having the same primary amino acid sequence, and are referred to collectively as a homodimerization switch. In embodiments, the first and second switch domains are different from one another, e.g., they are polypeptides having different primary amino acid sequences, and are referred to collectively as a heterodimerization switch. In embodiments, the switch is intracellular. In embodiments, the switch is extracellular. In embodiments, the switch domain is a polypeptide-based entity, e.g., FKBP or FRB-based, and the dimerization molecule is small molecule, e.g., a rapalogue. In embodiments, the switch domain is a polypeptide-based entity, e.g., an scFv that binds a myc peptide, and the dimerization molecule is a polypeptide, a fragment thereof, or a multimer of a polypeptide, e.g., a myc ligand or multimers of a myc ligand that bind to one or more myc scFvs. In embodiments, the switch domain is a polypeptide-based entity, e.g., myc receptor, and the dimerization molecule is an antibody or fragments thereof, e.g., myc antibody.

"Dimerization molecule," as that term is used herein, e.g., when referring to an RCAR, refers to a molecule that promotes the association of a first switch domain with a second switch domain. In embodiments, the dimerization molecule does not naturally occur in the subject, or does not occur in concentrations that would result in significant dimerization. In embodiments, the dimerization molecule is a small molecule, e.g., rapamycin or a rapalogue, e.g, RAD001.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. As another example, a range such as 95-99% identity, includes something with 95%, 96%, 97%, 98% or 99% identity, and includes subranges such as 96-99%, 96-98%, 96-97%, 97-99%, 97-98% and 98-99% identity. This applies regardless of the breadth of the range.

### DESCRIPTION

### Chimeric Antigen Receptor (CAR)

The present disclosure encompasses a recombinant DNA construct comprising sequences encoding a CAR, wherein the CAR comprises an antigen binding domain (e.g., antibody, antibody molecule, or antibody fragment, TCR or TCR fragment) that binds specifically to a tumor antigen, e.g., a tumor antigen described herein, wherein the sequence of the antigen binding domain is contiguous with and in the same reading frame as a nucleic acid sequence encoding an intracellular signaling domain. The intracellular signaling domain can comprise a costimulatory signaling domain and/or a primary signaling domain, e.g., a zeta chain. The costimulatory signaling domain refers to a portion of the CAR comprising at least a portion of the intracellular domain of a costimulatory molecule.

In specific aspects, a CAR construct of the disclosure comprises a scFv domain, wherein the scFv may be preceded by an optional leader sequence such as provided in SEQ ID NO: 2, and followed by an optional hinge sequence such as provided in SEQ ID NO:4 or SEQ ID NO:6 or SEQ ID NO:8 or SEQ ID NO:10, a transmembrane region such as provided in SEQ ID NO:12 or SEQ ID NO: 42, an intracellular signaling domain, e.g., a costimulatory signaling domain, that includes SEQ ID NO: 14,SEQ ID NO:16, SEQ ID NO: 40 or SEQ ID NO: 44 and a CD3 zeta sequence that includes SEQ ID NO:18 or SEQ ID NO:20, e.g., wherein the domains are contiguous with and in the same reading frame to form a single fusion protein.

In one aspect, an exemplary CAR constructs comprise an optional leader sequence (e.g., a leader sequence described herein), an extracellular antigen binding domain (e.g., an antigen binding domain described herein), a hinge (e.g., a hinge region described herein), a transmembrane domain (e.g., a transmembrane domain described herein), and an intracellular stimulatory domain (e.g., an intracellular stimulatory domain described herein). In one aspect, an exemplary CAR construct comprises an optional leader sequence (e.g., a leader sequence described herein), an extracellular antigen binding domain (e.g., an antigen binding domain described herein), a hinge (e.g., a hinge region described herein), a transmembrane domain (e.g., a transmembrane domain described herein), an intracellular costimulatory signaling domain (e.g., a costimulatory signaling domain described herein) and/or an intracellular primary signaling domain (e.g., a primary signaling domain described herein).

An exemplary leader sequence is provided as SEQ ID NO: 2. An exemplary hinge/spacer sequence is provided as SEQ ID NO: 4 or SEQ ID NO:6 or SEQ ID NO:8 or SEQ ID NO:10. An exemplary transmembrane domain sequence is provided as SEQ ID NO:12 or SEQ ID NO: 42. An exemplary sequence of the intracellular signaling domain of the 4-1BB protein is provided as SEQ ID NO: 14. An exemplary sequence of the intracellular signaling domain of CD27 is provided as SEQ ID NO:16. An exemplary CD3zeta domain sequence is provided as SEQ ID NO: 18 or SEQ ID NO:20. An exemplary intracellular signaling domain of CD28 is provided as SEQ ID NO: 44. An exemplary intracellular signaling domain of ICOS is provided as SEQ ID NO: 40.

Sequences of some examples of various components of CARs of the instant disclosure, and nucleic acids that encode them are listed in Table 1, where aa stands for amino acids, and na stands for nucleic acids that encode the corresponding peptide.

**Table 1. Sequences of various components of CAR (aa - amino acids, na - nucleic acids that encodes the corresponding protein)**

| SEQ ID NO | Description | Sequence |
|---|---|---|
| 1 | EF-1 promoter (na) | |
| 2 | Leader (aa) | MALPVTALLLPLALLLHAARP |
| 3 | Leader (na) | |
| 4 | CD 8 hinge (aa) | TTTPAPRPPTPAPTIASQPLSLRPEACRPAAGGAVHTRGLDFACD |
| 5 | CD8 hinge (na) | |
| 6 | Ig4 hinge (aa) | |
| 7 | Ig4 hinge (na) | |
| | | |
| 8 | IgD hinge (aa) | |
| 9 | IgD hinge (na) | |
| 10 | GS hinge/linker (aa) | GGGGSGGGGS |
| 11 | GS hinge/linker (na) | GGTGGCGGAGGTTCTGGAGGTGGAGGTTCC |
| 12 | CD8TM (aa) | IYIWAPLAGTCGVLLLSLVITLYC |
| 13 | CD8 TM (na) | |
| 14 | 4-1BB intracellular domain (aa) | KRGRKKLLYIFKQPFMRPVQTTQEEDGCSCRFPEEEEGGCEL |
| 15 | 4-1BB intracellular domain (na) | |
| 16 | CD27 (aa) | QRRKYRSNKGESPVEPAEPCRYSCPREEEGSTIPIQEDYRKPEPACSP |
| 17 | CD27 (na) | |
| | | |
| 18 | CD3-zeta (aa) | |
| 19 | CD3-zeta (na) | |
| 20 | CD3-zeta (aa) | |
| 21 | CD3-zeta (na) | |
| 22 | linker | GGGGS |
| 23 | linker | GGTGGCGGAGGTTCTGGAGGTGGAGGTTCC |
| 24 | PD-1 extracellular domain (aa) | |
| 25 | PD-1 extracellular domain (na) | |
| 26 | PD-1 CAR (aa) with signal | |
| 27 | PD-1 CAR (na) | |
| | | |
| 28 | linker | (Gly-Gly-Gly-Ser)ₙ, where n = 1-10 |
| 29 | linker | (Gly4 Ser)4 |
| 30 | linker | (Gly4 Ser)3 |
| 31 | linker | (Gly3Ser) |
| 32 | polyA (2000 A's) | a[a]₁₉₉₉ |
| 33 | polyA (150 A's) | a[a]₁₄₉ |
| 34 | polyA (5000 A's) | a[a]₄₉₉₉ |
| 35 | polyA (100 T's) | t[t]₉₉ |
| 36 | polyA (500 T's) | t[t]₄₉₉ |
| 37 | polyA (64 A's) | a[a]63 |
| 38 | polyA (400 A's) | a[a]₃₉₉ |
| 39 | PD1 CAR (aa) | |
| 40 | ICOS ICD domain (aa) | |
| 41 | ICOS ICD domain (na) | |
| 42 | ICOS TM domain (aa) | |
| 43 | ICOS TM domain (na) | |
| 44 | CD28 domain (aa) | RSKRSRLLHSDYMNMTPRRPGPTRKHYQPYAPPRDFAAYRS |
| 45 | CD28 domain (na) | |
| | | |
| 46 | ICOS (FMFM) ICD domain (aa) ("FMFM" disclosed as SEQ ID NO: 47) | TKKKYSSSVHDPNGEFMFMRAVNTAKKSRLTDVTL |

Ininstances, CAR scFv fragments are cloned into lentiviral vectors to create a full length CAR construct in a single coding frame, and using a promoter, e.g., EF1 alpha promoter, for expression (SEQ ID NO: 1).

In one aspect, the present disclosure encompasses a recombinant nucleic acid construct comprising a nucleic acid molecule encoding a CAR, wherein the nucleic acid molecule comprises the nucleic acid sequence encoding an antigen binding domain, e.g., described herein, that is contiguous with and in the same reading frame as a nucleic acid sequence encoding an intracellular signaling domain.

In one aspect, the present disclosure encompasses a recombinant nucleic acid construct comprising a nucleic acid molecule encoding a CAR, wherein the nucleic acid molecule comprises a nucleic acid sequence encoding an antigen binding domain, wherein the sequence is contiguous with and in the same reading frame as the nucleic acid sequence encoding an intracellular signaling domain. An exemplary intracellular signaling domain that can be used in the CAR includes, but is not limited to, one or more intracellular signaling domains of, e.g., CD3-zeta, CD28, CD27, 4-1BB, ICOS, and the like. In some instances, the CAR can comprise any combination of CD3-zeta, CD28, 4-1BB, ICOS and the like.

In an instance, the CAR comprises an ICOS domain and is provided in a CD4 + T cell. In an instance, the CAR comprises a CD28 or 4-1BB domain, and is provided in a CD8 + T cell.

The nucleic acid sequences coding for the desired molecules can be obtained using recombinant methods known in the art, such as, for example by screening libraries from cells expressing the nucleic acid molecule, by deriving the nucleic acid molecule from a vector known to include the same, or by isolating directly from cells and tissues containing the same, using standard techniques. Alternatively, the nucleic acid of interest can be produced synthetically, rather than cloned.

The present disclosure includes retroviral and lentiviral vector constructs expressing a CAR that can be directly transduced into a cell.

The present disclosure also includes an RNA construct that can be directly transfected into a cell. A method for generating mRNA for use in transfection involves *in vitro* transcription (IVT) of a template with specially designed primers, followed by polyA addition, to produce a construct containing 3' and 5' untranslated sequence ("UTR") (e.g., a 3' and/or 5' UTR described herein), a 5' cap (e.g., a 5' cap described herein) and/or Internal Ribosome Entry Site (IRES) (e.g., an IRES described herein), the nucleic acid to be expressed, and a polyA tail, typically 50-2000 bases in length (SEQ ID NO:32). RNA so produced can efficiently transfect different kinds of cells. In one instance, the template includes sequences for the CAR. In an instance, an RNA CAR vector is transduced into a cell, e.g., a T cell by electroporation.

### ANTIGEN BINDING DOMAIN

The CARs described herein can include an antigen binding domain in the extracellular region.

In one instance, the antigen binding domain is a murine antibody or antibody fragment described herein. In one instance, the antigen binding domain is a humanized antibody or antibody fragment.

The choice of an antigen binding domain can depend upon the type and number of ligands or receptors that define the surface of a target cell. For example, the antigen binding domain may be chosen to recognize an antigen that acts as a cell surface marker on target cells associated with a particular disease state. Examples of cell surface markers that may act as ligands or receptors include a cell surface marker associated with a particular disease state, e.g., cell surface makers for viral diseases, bacterial diseases parasitic infections, autoimmune diseases and disorders associated with unwanted cell proliferation, e.g., a cancer, e.g., a cancer described herein.

In certain aspects, the proliferative disorder antigens of the present disclosure are derived from, cancers including but not limited to primary or metastatic melanoma, thymoma, lymphoma, sarcoma, lung cancer (e.g., NSCLC or SCLC), liver cancer, non-Hodgkin's lymphoma, Hodgkin's lymphoma, leukemias, multiple myeloma, glioblastoma, neuroblastoma, uterine cancer, cervical cancer, renal cancer, thyroid cancer, bladder cancer, kidney cancer and adenocarcinomas such as breast cancer, prostate cancer, ovarian cancer, pancreatic cancer, colon cancer and the like. In some embodiments, the cancer is B-cell acute lymphoid leukemia ("BALL"), T-cell acute lymphoid leukemia ("TALL"), acute lymphoid leukemia (ALL), acute myelogenous leukemia (AML); one or more chronic leukemias including but not limited to chronic myelogenous leukemia (CML), chronic lymphocytic leukemia (CLL); additional hematologic cancers or hematologic conditions including, but not limited to B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin's lymphoma, plasmablastic lymphoma, plasmacytoid dendritic cell neoplasm, Waldenstrom macroglobulinemia.

In one embodiment, the tumor antigen comprises one or more antigenic cancer epitopes immunologically recognized by tumor infiltrating lymphocytes (TIL) derived from a cancer tumor of a mammal.

Tumor antigens are proteins that are produced by tumor cells that elicit an immune response, particularly T-cell mediated immune responses. The selection of the antigen binding domain of the disclosure will depend on the particular type of cancer to be treated. Tumor antigens are well known in the art and include, for example, a glioma-associated antigen, carcinoembryonic antigen (CEA), EGFRvIII, IL-11Ra, IL-13Ra, EGFR, FAP, B7H3, Kit, CA-IX, CS-1, MUC1, BCMA, bcr-abl, HER2, β-human chorionic gonadotropin, alphafetoprotein (AFP), ALK, CD19, CD123, cyclin B1, lectin-reactive AFP, Fos-related antigen 1, ADRB3, thyroglobulin, EphA2, RAGE-1, RU1, RU2, SSX2, AKAP-4, LCK, OY-TES1, PAX5, SART3, CLL-1, fucosyl GM1, GloboH, MN-CA IX, EPCAM, EVT6-AML, TGS5, human telomerase reverse transcriptase, plysialic acid, PLAC1, RU1, RU2 (AS), intestinal carboxyl esterase, lewisY, sLe, LY6K, mut hsp70-2, M-CSF, MYCN, RhoC, TRP-2, CYP1B1, BORIS, prostase, prostate-specific antigen (PSA), PAX3, PAP, NY-ESO-1, LAGE-1a, LMP2, NCAM, p53, p53 mutant, Ras mutant, gp100, prostein, OR51E2, PANX3, PSMA, PSCA, Her2/neu, hTERT, HMWMAA, HAVCR1, VEGFR2, PDGFR-beta, survivin and telomerase, legumain, HPV E6,E7, sperm protein 17, SSEA-4, tyrosinase, TARP, WT1, prostate-carcinoma tumor antigen-1 (PCTA-1), ML-IAP, MAGE, MAGE-A1,MAD-CT-1, MAD-CT-2, MelanA/MART1, XAGE1, ELF2M, ERG (TMPRSS2 ETS fusion gene), NA17, neutrophil elastase, sarcoma translocation breakpoints, NY-BR-1, ephrinB2, CD20, CD22, CD24, CD30, CD33, CD38, CD44v6, CD97, CD171, CD179a, androgen receptor, FAP, insulin growth factor (IGF)-I, IGF-II, IGF-I receptor, GD2, o-acetyl-GD2, GD3, GM3, GPRC5D, GPR20, CXORF61, folate receptor (FRa), folate receptor beta, ROR1, Flt3, TAG72, TN Ag, Tie 2, TEM1, TEM7R, CLDN6, TSHR, UPK2, and mesothelin. In a preferred embodiment, the tumor antigen is selected from the group consisting of folate receptor (FRa), mesothelin, EGFRvIII, IL-13Ra, CD123, CD19, CD33, BCMA, GD2, CLL-1, CA-IX, MUC1, HER2, and any combination thereof.

In one embodiment, the tumor antigen comprises one or more antigenic cancer epitopes associated with a malignant tumor. Malignant tumors express a number of proteins that can serve as target antigens for an immune attack. These molecules include but are not limited to tissue-specific antigens such as MART-1, tyrosinase and GP 100 in melanoma and prostatic acid phosphatase (PAP) and prostate-specific antigen (PSA) in prostate cancer. Other target antigens include transformation-related molecules such as the oncogene HER-2/Neu/ErbB-2. Yet another group of target antigens are onco-fetal antigens such as carcinoembryonic antigen (CEA). In B-cell lymphoma the tumor-specific idiotype immunoglobulin constitutes a truly tumor-specific immunoglobulin antigen that is unique to the individual tumor. B-cell differentiation antigens such as CD19, CD20 and CD37 are other candidates for target antigens in B-cell lymphoma.

Non-limiting examples of tumor antigens include the following: Differentiation antigens such as MART-1/MelanA (MART-I), gp100 (Pmel 17), tyrosinase, TRP-1, TRP-2 and tumor-specific multilineage antigens such as MAGE-1, MAGE-3, BAGE, GAGE-1, GAGE-2, p15; overexpressed embryonic antigens such as CEA; overexpressed oncogenes and mutated tumor-suppressor genes such as p53, Ras, HER-2/neu; unique tumor antigens resulting from chromosomal translocations; such as BCR-ABL, E2A-PRL, H4-RET, IGH-IGK, MYL-RAR; and viral antigens, such as the Epstein Barr virus antigens EBVA and the human papillomavirus (HPV) antigens E6 and E7. Other large, protein-based antigens include TSP-180, MAGE-4, MAGE-5, MAGE-6, RAGE, NY-ESO, p185erbB2, p180erbB-3, c-met, nm-23H1, PSA, TAG-72, CA 19-9, CA 72-4, CAM 17.1, NuMa, K-ras, beta-Catenin, CDK4, Mum-1, p 15, p 16, 43-9F, 5T4, 791Tgp72, alpha-fetoprotein, beta-HCG, BCA225, BTAA, CA 125, CA 15-3\CA 27.29\BCAA, CA 195, CA 242, CA-50, CAM43, CD68\P1, CO-029, FGF-5, G250, Ga733\EpCAM, HTgp-175, M344, MA-50, MG7-Ag, MOV18, NB/70K, NY-CO-1, RCAS1, SDCCAG16, TA-90\Mac-2 binding protein\cyclophilin C-associated protein, TAAL6, TAG72, TLP, and TPS.

In some embodiments, the tumor antigen is a tumor antigen described in International Application WO2015/142675. In some embodiments, the tumor antigen is chosen from one or more of: CD19; CD123; CD22; CD30; CD171; CS-1 (also referred to as CD2 subset 1, CRACC, SLAMF7, CD319, and 19A24); C-type lectin-like molecule-1 (CLL-1 or CLECL1); CD33; epidermal growth factor receptor variant III (EGFRvIII); ganglioside G2 (GD2); ganglioside GD3 (aNeu5Ac(2-8)aNeu5Ac(2-3)bDGalp(1-4)bDGlcp(1-1)Cer); TNF receptor family member B cell maturation (BCMA); Tn antigen ((Tn Ag) or (GalNAcα-Ser/Thr)); prostate-specific membrane antigen (PSMA); Receptor tyrosine kinase-like orphan receptor 1 (ROR1); Fms-Like Tyrosine Kinase 3 (FLT3); Tumor-associated glycoprotein 72 (TAG72); CD38; CD44v6; Carcinoembryonic antigen (CEA); Epithelial cell adhesion molecule (EPCAM); B7H3 (CD276); KIT (CD117); Interleukin-13 receptor subunit alpha-2 (IL-13Ra2 or CD213A2); Mesothelin; Interleukin 11 receptor alpha (IL-11Ra); prostate stem cell antigen (PSCA); Protease Serine 21 (Testisin or PRSS21); vascular endothelial growth factor receptor 2 (VEGFR2); Lewis(Y) antigen; CD24; Platelet-derived growth factor receptor beta (PDGFR-beta); Stage-specific embryonic antigen-4 (SSEA-4); CD20; Folate receptor alpha; Receptor tyrosine-protein kinase ERBB2 (Her2/neu); Mucin 1, cell surface associated (MUC1); epidermal growth factor receptor (EGFR); neural cell adhesion molecule (NCAM); Prostase; prostatic acid phosphatase (PAP); elongation factor 2 mutated (ELF2M); Ephrin B2; fibroblast activation protein alpha (FAP); insulin-like growth factor 1 receptor (IGF-I receptor), carbonic anhydrase IX (CAIX); Proteasome (Prosome, Macropain) Subunit, Beta Type, 9 (LMP2); glycoprotein 100 (gp100); oncogene fusion protein consisting of breakpoint cluster region (BCR) and Abelson murine leukemia viral oncogene homolog 1 (Abl) (bcr-abl); tyrosinase; ephrin type-A receptor 2 (EphA2); Fucosyl GM1; sialyl Lewis adhesion molecule (sLe); ganglioside GM3 (aNeu5Ac(2-3)bDGalp(1-4)bDGlcp(1-1)Cer); transglutaminase 5 (TGS5); high molecular weight-melanoma-associated antigen (HMWMAA); o-acetyl-GD2 ganglioside (OAcGD2); Folate receptor beta; tumor endothelial marker 1 (TEM1/CD248); tumor endothelial marker 7-related (TEM7R); claudin 6 (CLDN6); thyroid stimulating hormone receptor (TSHR); G protein-coupled receptor class C group 5, member D (GPRC5D); chromosome X open reading frame 61 (CXORF61); CD97; CD179a; anaplastic lymphoma kinase (ALK); Polysialic acid; placenta-specific 1 (PLAC1); hexasaccharide portion of globoH glycoceramide (GloboH); mammary gland differentiation antigen (NY-BR-1); uroplakin 2 (UPK2); Hepatitis A virus cellular receptor 1 (HAVCR1); adrenoceptor beta 3 (ADRB3); pannexin 3 (PANX3); G protein-coupled receptor 20 (GPR20); lymphocyte antigen 6 complex, locus K 9 (LY6K); Olfactory receptor 51E2 (OR51E2); TCR Gamma Alternate Reading Frame Protein (TARP); Wilms tumor protein (WT1); Cancer/testis antigen 1 (NY-ESO-1); Cancer/testis antigen 2 (LAGE-1a); Melanoma-associated antigen 1 (MAGE-A1); ETS translocation-variant gene 6, located on chromosome 12p (ETV6-AML); sperm protein 17 (SPA17); X Antigen Family, Member 1A (XAGE1); angiopoietin-binding cell surface receptor 2 (Tie 2); melanoma cancer testis antigen-1 (MAD-CT-1); melanoma cancer testis antigen-2 (MAD-CT-2); Fos-related antigen 1; tumor protein p53 (p53); p53 mutant; prostein; surviving; telomerase; prostate carcinoma tumor antigen-1 (PCTA-1 or Galectin 8), melanoma antigen recognized by T cells 1 (MelanA or MARTI); Rat sarcoma (Ras) mutant; human Telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N-Acetyl glucosaminyl-transferase V (NA17); paired box protein Pax-3 (PAX3); Androgen receptor; Cyclin B1; v-myc avian myelocytomatosis viral oncogene neuroblastoma derived homolog (MYCN); Ras Homolog Family Member C (RhoC); Tyrosinase-related protein 2 (TRP-2); Cytochrome P450 1B1 (CYP1B1); CCCTC-Binding Factor (Zinc Finger Protein)-Like (BORIS or Brother of the Regulator of Imprinted Sites), Squamous Cell Carcinoma Antigen Recognized By T Cells 3 (SART3); Paired box protein Pax-5 (PAX5); proacrosin binding protein sp32 (OY-TES1); lymphocyte-specific protein tyrosine kinase (LCK); A kinase anchor protein 4 (AKAP-4); synovial sarcoma, X breakpoint 2 (SSX2); Receptor for Advanced Glycation Endproducts (RAGE-1); renal ubiquitous 1 (RU1); renal ubiquitous 2 (RU2); legumain; human papilloma virus E6 (HPV E6); human papilloma virus E7 (HPV E7); intestinal carboxyl esterase; heat shock protein 70-2 mutated (mut hsp70-2); CD79a; CD79b; CD72; Leukocyte-associated immunoglobulin-like receptor 1 (LAIR1); Fc fragment of IgA receptor (FCAR or CD89); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module-containing mucin-like hormone receptor-like 2 (EMR2); lymphocyte antigen 75 (LY75); Glypican-3 (GPC3); Fc receptor-like 5 (FCRL5); and immunoglobulin lambda-like polypeptide 1 (IGLL1). In some embodiments, the tumor antigen is GFRa4 (see Spinasanta, "The Endocrine Society's 97th Annual Meeting & Expo: Targeted Therapies in Medullary Thyroid Cancer" March 13, 2015).

In some embodiments, tumor antigen bound by the CAR molecule is chosen from one or more of: TSHR, CD171, CS-1, CLL-1, GD3, Tn Ag, FLT3, CD38, CD44v6, B7H3, KIT, IL-13Ra2, IL-11Ra, PSCA, PRSS21, VEGFR2, LewisY, CD24, PDGFR-beta, SSEA-4, MUC1, EGFR, NCAM, CAIX, LMP2, EphA2, Fucosyl GM1, sLe, GM3, TGS5, HMWMAA, o-acetyl-GD2, Folate receptor beta, TEM1/CD248, TEM7R, CLDN6, GPRC5D, CXORF61, CD97, CD179a, ALK, Polysialic acid, PLAC1, GloboH, NY-BR-1, UPK2, HAVCR1, ADRB3, PANX3, GPR20, LY6K, OR51E2, TARP, WT1, ETV6-AML, sperm protein 17, XAGE1, Tie 2, MAD-CT-1, MAD-CT-2, Fos-related antigen 1, p53 mutant, hTERT, sarcoma translocation breakpoints, ML-IAP, ERG (TMPRSS2 ETS fusion gene), NA17, PAX3, Androgen receptor, Cyclin B1, MYCN, RhoC, CYP1B1, BORIS, SART3, PAX5, OY-TES1, LCK, AKAP-4, SSX2, CD79a, CD79b, CD72, LAIR1, FCAR, LILRA2, CD300LF, CLEC12A, BST2, EMR2, LY75, GPC3, FCRL5, and IGLL1.

In certain embodiments, the tumor antigen bound by the CAR molecule is chosen from one or more of: TSHR, CLDN6, GPRC5D, CXORF61, CD97, CD179a, ALK, Polysialic acid, PLAC1, GloboH, NY-BR-1, UPK2, HAVCR1, ADRB3, PANX3, GPR20, LY6K, and OR51E2.

Depending on the desired antigen to be targeted, the CAR of the disclosure can be engineered to include the appropriate antigen bind domain that is specific to the desired antigen target.

A CAR as described herein, can include an antigen binding domain (e.g., antibody or antibody fragment) that binds to a MHC presented-peptide. Normally, peptides derived from endogenous proteins fill the pockets of Major histocompatibility complex (MHC) class I molecules, and are recognized by T cell receptors (TCRs) on CD8 + T lymphocytes. The MHC class I complexes are constitutively expressed by all nucleated cells. In cancer, virus-specific and/or tumor-specific peptide/MHC complexes represent a unique class of cell surface targets for immunotherapy. TCR-like antibodies targeting peptides derived from viral or tumor antigens in the context of human leukocyte antigen (HLA)-A1 or HLA-A2 have been described (see, e.g., Sastry et al., J Virol. 2011 85(5):1935-1942; Sergeeva et al., Bood, 2011 117(16):4262-4272; Verma et al., J Immunol 2010 184(4):2156-2165; Willemsen et al., Gene Ther 2001 8(21) :1601-1608 ; Dao et al., Sci Transl Med 2013 5(176) :176ra33 ; Tassev et al., Cancer Gene Ther 2012 19(2):84-100). For example, TCR-like antibody can be identified from screening a library, such as a human scFv phage displayed library. Accordingly, the present disclosure provides a CAR described herein, that comprises an antigen binding domain that binds to a MHC presented peptide of a molecule selected from any tumor antigen described above that is expressed intracellularly, e.g., p53, BCR-Abl, Ras, K-ras, and c-met.

In one aspect, the CARX cell described herein can further comprise a second CAR, e.g., a second CAR that includes a different antigen binding domain, e.g., to the same target (a cancer associated antigen as described herein) or a different target (e.g., CD19, CD123, CD22, CD30, CD34, CD171, CS-1, CLL-1, CD33, EGFRvIII, GD2, GD3, BCMA, Tn Ag, PSMA, ROR1, FLT3, FAP, TAG72, CD38, CD44v6, CEA, EPCAM, B7H3, KIT, IL-13Ra2, Mesothelin, IL-11Ra, PSCA, VEGFR2, LewisY, CD24, PDGFR-beta, SSEA-4, CD20, Folate receptor alpha, ERBB2 (Her2/neu), MUC1, EGFR, NCAM, Prostase, PAP, ELF2M, Ephrin B2, IGF-I receptor, CAIX, LMP2, gp100, bcr-abl, tyrosinase, EphA2, Fucosyl GM1, sLe, GM3, TGS5, HMWMAA, o-acetyl-GD2, Folate receptor beta, TEM1/CD248, TEM7R, CLDN6, TSHR, GPRC5D, CXORF61, CD97, CD179a, ALK, Plysialic acid, PLAC1, GloboH, NY-BR-1, UPK2, HAVCR1, ADRB3, PANX3, GPR20, LY6K, OR51E2, TARP, WT1, NY-ESO-1, LAGE-1a, legumain, HPV E6,E7, MAGE-A1, MAGE A1, ETV6-AML, sperm protein 17, XAGE1, Tie 2, MAD-CT-1, MAD-CT-2, Fos-related antigen 1, p53, p53 mutant, prostein, survivin and telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, intestinal carboxyl esterase, mut hsp70-2, CD79a, CD79b, CD72, LAIR1, FCAR, LILRA2, CD300LF, CLEC12A, BST2, EMR2, LY75, GPC3, FCRL5, or IGLL1). In one instance, when the CARX cell comprises two or more different CARs, the antigen binding domains of the different CARs can be such that the antigen binding domains do not interact with one another. For example, a cell expressing a first and second CAR can have an antigen binding domain of the first CAR, e.g., as a fragment, e.g., an scFv, that does not form an association with the antigen binding domain of the second CAR, e.g., the antigen binding domain of the second CAR is a VHH.

### Antigen binding domains derived from an Antibody Molecule

The antigen binding domain can be derived from an antibody molecule, e.g., one or more of monoclonal antibodies, polyclonal antibodies, recombinant antibodies, human antibodies, humanized antibodies, single-domain antibodies e.g., a heavy chain variable domain (VH), a light chain variable domain (VL) and a variable domain (VHH) from, e.g., human or camelid origin. In some instances, it is beneficial for the antigen binding domain to be derived from the same species in which the CAR will ultimately be used in, e.g., for use in humans, it may be beneficial for the antigen binding domain of the CAR, described herein, to comprise a human or a humanized antigen binding domain. Antibodies can be obtained using known techniques known in the art.

In embodiments, the antigen binding domain comprises a fragment of an antibody that is sufficient to confer recognition and specific binding to the target antigen. Examples of an antibody fragment include, but are not limited to, an Fab, Fab', F(ab')₂, or Fv fragment, an scFv antibody fragment, a linear antibody, single domain antibody such as an sdAb (either VL or VH), a camelid VHH domain, and multi-specific antibodies formed from antibody fragments.

In an embodiment, the antigen binding domain is a "scFv," which can comprise a fusion protein comprising a VL chain and a VH chain of an antibody, where the VH and VL are, e.g., linked via a short flexible polypeptide linker, e.g., a linker described herein. The scFv is capable of being expressed as a single chain polypeptide and retains the specificity of the intact antibody from which it is derived. Moreover, the VL and VH variable chains can be linked in either order, e.g., with respect to the N-terminal and C-terminal ends of the polypeptide, the scFv may comprise VL-linker-VH or may comprise VH-linker-VL. An scFv that can be prepared according to method known in the art (see, for example, Bird et al., (1988) Science 242:423-426 and Huston et al., (1988) Proc. Natl. Acad. Sci. USA 85:5879-5883).

As described above and elsewhere, scFv molecules can be produced by linking VH and VL chians together using flexible polypeptide linkers. In some embodiments, the scFv molecules comprise flexible polypeptide linker with an optimized length and/or amino acid composition. The flexible polypeptide linker length can greatly affect how the variable regions of a scFv fold and interact. In fact, if a short polypeptide linker is employed (e.g., between 5-10 amino acids, intrachain folding is prevented. For examples of linker orientation and size see, e.g., Hollinger et al. 1993 Proc Natl Acad. Sci. U.S.A. 90:6444-6448, U.S. Patent Application Publication Nos. 2005/0100543, 2005/0175606, 2007/0014794, and PCT publication Nos. WO2006/020258 and WO2007/024715. In one embodiment, the peptide linker of the scFv consists of amino acids such as glycine and/or serine residues used alone or in combination, to link variable heavy and variable light chain regions together. In one embodiment, the flexible polypeptide linker is a Gly/Ser linker and, e.g., comprises the amino acid sequence (Gly-Gly-Gly-Ser)n (SEQ ID NO: 28), where n is a positive integer equal to or greater than 1. For example, n=1, n=2, n=3. n=4, n=5 and n=6, n=7, n=8, n=9 and n=10. In one embodiment, the flexible polypeptide linkers include, but are not limited to, (Gly4 Ser)4 (SEQ ID NO: 29) or (Gly4 Ser)3 (SEQ ID NO: 30). In another embodiment, the linkers include multiple repeats of (Gly2Ser), (GlySer) or (Gly3Ser) (SEQ ID NO: 31).

In some embodiments, the antigen binding domain is a single domain antigen binding (SDAB) molecules. A SDAB molecule includes molecules whose complementary determining regions are part of a single domain polypeptide. Examples include, but are not limited to, heavy chain variable domains, binding molecules naturally devoid of light chains, single domains derived from conventional 4-chain antibodies, engineered domains and single domain scaffolds other than those derived from antibodies (e.g., described in more detail below). SDAB molecules may be any of the art, or any future single domain molecules. SDAB molecules may be derived from any species including, but not limited to mouse, human, camel, llama, fish, shark, goat, rabbit, and bovine. This term also includes naturally occurring single domain antibody molecules from species other than *Camelidae* and sharks.

In one aspect, an SDAB molecule can be derived from a variable region of the immunoglobulin found in fish, such as, for example, that which is derived from the immunoglobulin isotype known as Novel Antigen Receptor (NAR) found in the serum of shark. Methods of producing single domain molecules derived from a variable region of NAR ("IgNARs") are described in WO 03/014161 and Streltsov (2005) Protein Sci. 14:2901-2909.

According to another aspect, an SDAB molecule is a naturally occurring single domain antigen binding molecule known as a heavy chain devoid of light chains. Such single domain molecules are disclosed in WO 9404678 and Hamers-Casterman, C. et al. (1993) Nature 363:446-448, for example. For clarity reasons, this variable domain derived from a heavy chain molecule naturally devoid of light chain is known herein as a VHH or nanobody to distinguish it from the conventional VH of four chain immunoglobulins. Such a VHH molecule can be derived from *Camelidae* species, for example in camel, llama, dromedary, alpaca and guanaco. Other species besides *Camelidae* may produce heavy chain molecules naturally devoid of light chain; such VHHs are within the scope of the invention.

Antibody proteins obtained from members of the camel and dromedary (Camelus bactrianus and Calelus dromaderius) family including new world members such as llama species (Lama paccos, Lama glama and Lama vicugna) have been characterized with respect to size, structural complexity and antigenicity for human subjects. Certain IgG antibodies from this family of mammals as found in nature lack light chains, and are thus structurally distinct from the typical four chain quaternary structure having two heavy and two light chains, for antibodies from other animals. See PCT/EP93/02214 (WO 94/04678 published 3 March 1994).

A region of the camelid antibody which is the small single variable domain identified as VHH can be obtained by genetic engineering to yield a small protein having high affinity for a target, resulting in a low molecular weight antibody-derived protein known as a "camelid nanobody". See U.S. patent number 5,759,808 issued June 2, 1998; see also Stijlemans et al., (2004) J Biol Chem 279:1256-1261; Dumoulin et al., (2003) Nature 424:783-788; Pleschberger et al., (2003) Bioconjugate Chem 14:440-448; Cortez-Retamozo et al., (2002) Int J Cancer 89:456-62; and Lauwereys et al., (1998) EMBO J 17:3512-3520. Engineered libraries of camelid antibodies and antibody fragments are commercially available, for example, from Ablynx, Ghent, Belgium (e.g., US20060115470; Domantis (US20070065440, US20090148434). As with other antibodies of non-human origin, an amino acid sequence of a camelid antibody can be altered recombinantly to obtain a sequence that more closely resembles a human sequence, *i*.*e*., the nanobody can be "humanized". Thus the natural low antigenicity of camelid antibodies to humans can be further reduced.

The camelid nanobody has a molecular weight approximately one-tenth that of a human IgG molecule, and the protein has a physical diameter of only a few nanometers. One consequence of the small size is the ability of camelid nanobodies to bind to antigenic sites that are functionally invisible to larger antibody proteins, *i*.*e*., camelid nanobodies are useful as reagents detect antigens that are otherwise cryptic using classical immunological techniques, and as possible therapeutic agents. Thus yet another consequence of small size is that a camelid nanobody can inhibit as a result of binding to a specific site in a groove or narrow cleft of a target protein, and hence can serve in a capacity that more closely resembles the function of a classical low molecular weight drug than that of a classical antibody.

The low molecular weight and compact size further result in camelid nanobodies being extremely thermostable, stable to extreme pH and to proteolytic digestion, and poorly antigenic. Another consequence is that camelid nanobodies readily move from the circulatory system into tissues, and even cross the blood-brain barrier and can treat disorders that affect nervous tissue. Nanobodies can further facilitated drug transport across the blood brain barrier. See U.S. patent application 20040161738 published August 19, 2004. These features combined with the low antigenicity to humans indicate great therapeutic potential. Further, these molecules can be fully expressed in prokaryotic cells such as E. coli and are expressed as fusion proteins with bacteriophage and are functional.

An antigen binding domain can comprise a camelid antibody or nanobody, or an antigen binding fragment thereof. Such antibodies can have high affinity for its cognate antigen. In certain embodiments herein, the camelid antibody or nanobody is naturally produced in the camelid animal, *i*.*e*., is produced by the camelid following immunization with antigen or a peptide fragment thereof. Alternatively, the camelid nanobody is engineered, *i*.*e*., produced by selection for example from a library of phage displaying appropriately mutagenized camelid nanobody proteins using panning procedures with the target antigen. Engineered nanobodies can further be customized by genetic engineering to have a half life in a recipient subject of from 45 minutes to two weeks. In a specific instance, the camelid antibody or nanobody is obtained by grafting the CDRs sequences of the heavy or light chain of the human antibodies of the disclosure into nanobody or single domain antibody framework sequences, as described for example in WO94/04678.

An antigen binding domain can comprise a single domain antibody, e.g., which relies only on a heavy chain variable region for binding, e.g., a nanobody. Nanobodies suitable for use herein can be made by the methods described in US2010/0028341, WO2009/030285, and WO2010/007376.

In certain embodiments, the SDAB molecule is a single chain fusion polypeptide comprising one or more single domain molecules (e.g., nanobodies), devoid of a complementary variable domain or an immunoglobulin constant, *e.g*., Fc, region, that binds to one or more target antigens.

The SDAB molecules can be recombinant, CDR-grafted, humanized, camelized, de-immunized and/or in vitro generated (e.g., selected by phage display).

In one embodiment, the antigen biding domain portion comprises a human antibody or a fragment thereof.

In some embodiments, a non-human antibody is humanized, where specific sequences or regions of the antibody are modified to increase similarity to an antibody naturally produced in a human. In an embodiment, the antigen binding domain is humanized.

Non human antibodies can be humanized using a variety of techniques known in the art, e.g., CDR-grafting (see, e.g., European Patent No. EP 239,400; International Publication No. WO 91/09967; and U.S. Pat. Nos. 5,225,539, 5,530,101, and 5,585,089), veneering or resurfacing (see, e.g., European Patent Nos. EP 592,106 and EP 519,596; Padlan, 1991, Molecular Immunology, 28(4/5):489-498; Studnicka et al., 1994, Protein Engineering, 7(6):805-814; and Roguska et al., 1994, PNAS, 91:969-973), chain shuffling (see, e.g., U.S. Pat. No. 5,565,332), and techniques disclosed in, e.g., U.S. Patent Application Publication No. US2005/0042664, U.S. Patent Application Publication No. US2005/0048617, U.S. Pat. No. 6,407,213, U.S. Pat. No. 5,766,886, International Publication No. WO 9317105, Tan et al., 2002, J. Immunol., 169:1119-25; Caldas et al., 2000, Protein Eng., 13(5):353-60; Morea et al., 2000, Methods, 20:267-79; Baca et al., 1997, J. Biol. Chem., 272:10678-84; Roguska et al., 1996, Protein Eng., 9(10):895-904; Couto et al., 1995, Cancer Res., 55 :5973s-5977; Couto et al., 1995, Cancer Res., 55(8):1717-22; Sandhu 1994 Gene, 150(2):409-10; and Pedersen et al., 1994, J. Mol. Biol., 235(3):959-73. Often, framework residues in the framework regions will be substituted with the corresponding residue from the CDR donor antibody to alter, for example improve, antigen binding. These framework substitutions are identified by methods well-known in the art, e.g., by modeling of the interactions of the CDR and framework residues to identify framework residues important for antigen binding and sequence comparison to identify unusual framework residues at particular positions. (See, e.g., Queen et al., U.S. Pat. No. 5,585,089; and Riechmann et al., 1988, Nature, 332:323). In preferred embodiments, the humanized antibody molecule comprises a sequence described herein, e.g., a variable light chain and/or a variable heavy chain described herein, e.g., a humanized variable light chain and/or variable heavy chain described in Table X.

A humanized antibody has one or more amino acid residues introduced into it from a source which is nonhuman. These nonhuman amino acid residues are often referred to as "import" residues, which are typically taken from an "import" variable domain. Thus, humanized antibodies comprise one or more CDRs from nonhuman immunoglobulin molecules and framework regions from human. Humanization of antibodies is well-known in the art and can essentially be performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody, i.e., CDR-grafting (EP 239,400; PCT Publication No. WO 91/09967; and U.S. Pat. Nos. 4,816,567; 6,331,415; 5,225,539; 5,530,101; 5,585,089; 6,548,640). In such humanized chimeric antibodies, substantially less than an intact human variable domain has been substituted by the corresponding sequence from a nonhuman species. In practice, humanized antibodies are typically human antibodies in which some CDR residues and possibly some framework (FR) residues are substituted by residues from analogous sites in rodent antibodies. Humanization of antibodies can also be achieved by veneering or resurfacing (EP 592,106; EP 519,596; Padlan, 1991, Molecular Immunology, 28(4/5):489-498; Studnicka et al., Protein Engineering, 7(6):805-814 (1994); and Roguska et al., PNAS, 91:969-973 (1994)) or chain shuffling (U.S. Pat. No. 5,565,332).

In some instances, the antibody of the disclosure is further prepared using an antibody having one or more of the VH and/or VL sequences disclosed herein can be used as starting material to engineer a modified antibody, which modified antibody may have altered properties as compared to the starting antibody. In various embodiments, the antibody is engineered by modifying one or more amino acids within one or both variable regions (i.e., VH and/or VL), for example within one or more CDR regions and/or within one or more framework regions.

In another aspect, the antigen binding domain is a T cell receptor ("TCR"), or a fragment thereof, for example, a single chain TCR (scTCR). Methods to make such TCRs are known in the art. See, e.g., Willemsen RA et al, Gene Therapy 7: 1369-1377 (2000); Zhang T et al, Cancer Gene Ther 11: 487-496 (2004); Aggen et al, Gene Ther. 19(4):365-74 (2012). For example, scTCR can be engineered that contains the Vα and Vβ genes from a T cell clone linked by a linker (e.g., a flexible peptide). This approach is very useful to cancer associated target that itself is intracellular, however, a fragment of such antigen (peptide) is presented on the surface of the cancer cells by MHC.

An antigen binding domain can comprise a sequence from Table 2.

**Table 2: Exemplary Sequences for Antigen Binding Domains**

| **Target Antigen** | **Name** | **Amino Acid Sequence** | **SEQ ID NO:** |
|---|---|---|---|
| CD19 | huscFv1 | | 48 |
| CD19 | huscFv2 | | 49 |
| CD19 | huscFv3 | | 50 |
| CD19 | huscFv4 | | 51 |
| CD19 | huscFv5 | | 52 |
| CD19 | huscFv6 | | 53 |
| CD19 | huscFv7 | | 54 |
| CD19 | huscFv8 | | 55 |
| CD19 | huscFv9 | | 56 |
| CD19 | Hu scfv10 | | 57 |
| | | | |
| CD19 | Hu scFv11 | | 58 |
| CD19 | Hu scFv12 | | 59 |
| CD19 | muCTL 019 | | 60 |
| CD123 | Mu1172 | | 61 |
| CD123 | Mu1176 | | 62 |
| CD123 | huscFv1 | | 63 |
| CD123 | huscFv2 | | 64 |
| CD123 | huscFv3 | | 65 |
| CD123 | huscFv4 | | 66 |
| CD123 | huscFv5 | | 67 |
| CD123 | huscFv6 | | 68 |
| CD123 | huscFv7 | | 69 |
| CD123 | huscFv8 | | 70 |
| EGFR vIII | huscFv1 | | 71 |
| EGFR vIII | huscFv2 | | 72 |
| EGFR vIII | huscFv3 | | 73 |
| EGFR vIII | huscFv4 | | 74 |
| EGFR vIII | huscFv5 | | 75 |
| EGFR vIII | huscFv6 | | 76 |
| EGFR vIII | huscFv7 | | 77 |
| EGFR vIII | huscFv8 | | 78 |
| EGFR vIII | Mu310 C | | 79 |
| mesothelin | ss1 | | 80 |
| Folate Receptor α | MOv19 | | 81 |

In an embodiment, the antigen binding domain comprises any antibody, or a fragment thereof, e.g., an scFv, known in the art that targets or specifically binds to any one of the following: BCMA (also known as TNFRSF17, Tumor Necrosis Factor Receptor Superfamily, Member 17, or B Cell Maturation Antigen), CD33, CLL-1 (also known as C-type Lectin-Like domain family 1, or CLECL1), claudin-6 (CLDN6) or WT-1 (Wilms tumor 1). The antibody, or fragment thereof, can be a murine, humanized, or fully human antibody or fragment thereof.

In some embodiments, the antigen binding domain comprises a HC CDR1, a HC CDR2, and a HC CDR3 of any heavy chain binding domain amino acid sequences listed in Table 2. In embodiments, the antigen binding domain further comprises a LC CDR1, a LC CDR2, and a LC CDR3. In embodiments, the antigen binding domain comprises a LC CDR1, a LC CDR2, and a LC CDR3 of any light chain binding domain amino acid sequences listed in Table 2.

In some embodiments, the antigen binding domain comprises one, two or all of LC CDR1, LC CDR2, and LC CDR3 of any light chain binding domain amino acid sequences listed in Table 2, and one, two or all of HC CDR1, HC CDR2, and HC CDR3 of any heavy chain binding domain amino acid sequences listed in Table 2.

In some embodiments, the CDRs are defined according to the Kabat numbering scheme, the Chothia numbering scheme, or a combination thereof.

In embodiments, the order in which the VL and VH domains appear in the scFv is varied (i.e., VL-VH, or VH-VL orientation), and where either three or four copies of the "G4S" (SEQ ID NO:31) subunit, in which each subunit comprises the sequence GGGGS (SEQ ID NO:31) (e.g., (G4S)3 (SEQ ID NO:30) or (G4S)4(SEQ ID NO:29)), connect the variable domains to create the entirety of the scFv domain. Alternatively, the CAR construct can include, for example, a linker including the sequence GSTSGSGKPGSGEGSTKG (SEQ ID NO: 86)

Exemplary sequences of various scFv fragments and other CAR components are provided herein. It is noted that these CAR components (e.g., of SEQ ID NOs. 26, 89) without a leader sequence (e.g., without the amino acid sequence of SEQ ID NO: 2 or the nucleotide sequence of SEQ ID NO:3), are also provided herein.

In instances, the CAR sequences described herein contain a Q/K residue change in the signal domain of the co-stimulatory domain derived from CD3zeta chain.

In one embodiment, the portion of the CAR comprising the antigen binding domain comprises an antigen binding domain that binds specifically to CD19. In one aspect, the antigen binding domain targets human CD19. In one aspect, the antigen binding domain of the CAR has the same or a similar binding specificity as the FMC63 scFv fragment described in Nicholson et al. Mol. Immun. 34 (16-17): 1157-1165 (1997). In one embodiment, the antigen binding domain of the CAR includes the scFv fragment described in Nicholson et al. Mol. Immun. 34 (16-17): 1157-1165 (1997). A CD19 antibody molecule can be, e.g., an antibody molecule (e.g., a humanized anti-CD19 antibody molecule) described in WO2014/153270. WO2014/153270 also describes methods of assaying the binding and efficacy of various CART constructs.

In one aspect, the parental murine scFv sequence is the CAR19 construct provided in PCT publication WO2012/079000 and provided herein as SEQ ID NO:60. In one embodiment, the anti-CD19 binding domain is a scFv described in WO2012/079000 and provided in SEQ ID NO:60.

In some aspects, the antibodies of the disclosure are incorporated into a chimeric antigen receptor (CAR). In one aspect, the CAR comprises the polypeptide sequence provided as SEQ ID NO: 12 in PCT publication WO2012/079000, and provided herein as SEQ ID NO: 89, wherein the scFv domain is substituted by one or more sequences selected from SEQ ID NOS: 48-59. In one aspect, the scFv domains of SEQ ID NOS:48-59 are humanized variants of the scFv domain of SEQ ID NO:60 which is an scFv fragment of murine origin that specifically binds to human CD19. Humanization of this mouse scFv may be desired for the clinical setting, where the mouse-specific residues may induce a human-anti-mouse antigen (HAMA) response in patients who receive CART19 treatment, e.g., treatment with T cells transduced with the CAR19 construct.

The CD19 CAR provided as SEQ ID NO: 12 in PCT publication WO2012/079000 is:

In an embodiment, the antigen binding domain comprises an anti-CD 19 antibody, or fragment thereof, e.g., an scFv. For example, the antigen binding domain comprises a variable heavy chain and a variable light chain listed in Table 3. The linker sequence joining the variable heavy and variable light chains can be any of the linker sequences described herein, or alternatively, can be GSTSGSGKPGSGEGSTKG (SEQ ID NO:86).

**Table 3: Anti-CD 19 antibody binding domains**

| Antibody | VH Sequence | VL Sequence |
|---|---|---|
| SJ25-C1 | | |

Any known CD19 CAR, e.g., the CD19 antigen binding domain of any known CD19 CAR, in the art can be used in accordance with the instant invention. For example, LG-740; CD19 CAR described in the US Pat. No. 8,399,645; US Pat. No. 7,446,190; Xu et al., Leuk Lymphoma. 2013 54(2):255-260(2012); Cruz et al., Blood 122(17):2965-2973 (2013); Brentjens et al., Blood, 118(18):4817-4828 (2011); Kochenderfer et al., Blood 116(20):4099-102 (2010); Kochenderfer et al., Blood 122 (25):4129-39(2013); and 16th Annu Meet Am Soc Gen Cell Ther (ASGCT) (May 15-18, Salt Lake City) 2013, Abst 10.

Exemplary target antigens that can be targeted using the CAR-expressing cells, include, but are not limited to, CD19, CD123, EGFRvIII, mesothelin, among others, as described in, for example, WO 2014/130635, WO 2014/130657, and WO 2015/090230.

In one embodiment, the CAR T cell that specifically binds to CD19 has the USAN designation TISAGENLECLEUCEL-T. CTL019 is made by a gene modification of T cells is mediated by stable insertion via transduction with a self-inactivating, replication deficient Lentiviral (LV) vector containing the CTL019 transgene under the control of the EF-1 alpha promoter. CTL019 can be a mixture of transgene positive and negative T cells that are delivered to the subject on the basis of percent transgene positive T cells.

In other embodiments, the CAR-expressing cells can specifically bind to human CD19, *e.g.*, can include a CAR molecule, or an antigen binding domain (e.g., a humanized antigen binding domain) according to Table 3 of WO2014/153270.

In other embodiments, the CAR-expressing cells can specifically bind to CD123, *e.g.*, can include a CAR molecule (e.g., any of the CAR1-CAR8), or an antigen binding domain according to Tables 1-2 of WO 2014/130635.

In other embodiments, the CAR-expressing cells can specifically bind to EGFRvIII, *e.g.*, can include a CAR molecule, or an antigen binding domain according to Table 2 or SEQ ID NO:11 of WO 2014/130657.

In other embodiments, the CAR-expressing cells can specifically bind to mesothelin, *e.g.*, can include a CAR molecule, or an antigen binding domain according to Tables 2-3 of WO 2015/090230.

In one embodiment, the antigen binding domain comprises one, two three (e.g., all three) heavy chain CDRs, HC CDR1, HC CDR2 and HC CDR3, from an antibody listed above, and/or one, two, three (e.g., all three) light chain CDRs, LC CDR1, LC CDR2 and LC CDR3, from an antibody listed above. In one embodiment, the antigen binding domain comprises a heavy chain variable region and/or a variable light chain region of an antibody listed or described above.

### Bispecific CARs

In an embodiment a multispecific antibody molecule is a bispecific antibody molecule. A bispecific antibody has specificity for no more than two antigens. A bispecific antibody molecule is characterized by a first immunoglobulin variable domain sequence which has binding specificity for a first epitope and a second immunoglobulin variable domain sequence that has binding specificity for a second epitope. In an embodiment the first and second epitopes are on the same antigen, e.g., the same protein (or subunit of a multimeric protein). In an embodiment the first and second epitopes overlap. In an embodiment the first and second epitopes do not overlap. In an embodiment the first and second epitopes are on different antigens, e.g., different proteins (or different subunits of a multimeric protein). In an embodiment a bispecific antibody molecule comprises a heavy chain variable domain sequence and a light chain variable domain sequence which have binding specificity for a first epitope and a heavy chain variable domain sequence and a light chain variable domain sequence which have binding specificity for a second epitope. In an embodiment a bispecific antibody molecule comprises a half antibody having binding specificity for a first epitope and a half antibody having binding specificity for a second epitope. In an embodiment a bispecific antibody molecule comprises a half antibody, or fragment thereof, having binding specificity for a first epitope and a half antibody, or fragment thereof, having binding specificity for a second epitope. In an embodiment a bispecific antibody molecule comprises a scFv, or fragment thereof, have binding specificity for a first epitope and a scFv, or fragment thereof, have binding specificity for a second epitope.

In certain embodiments, the antibody molecule is a multi-specific (e.g., a bispecific or a trispecific) antibody molecule. Protocols for generating bispecific or heterodimeric antibody molecules are known in the art; including but not limited to, for example, the "knob in a hole" approach described in, e.g., US 5731168; the electrostatic steering Fc pairing as described in, e.g., WO 09/089004, WO 06/106905 and WO 2010/129304; Strand Exchange Engineered Domains (SEED) heterodimer formation as described in, e.g., WO 07/110205; Fab arm exchange as described in, e.g., WO 08/119353, WO 2011/131746, and WO 2013/060867; double antibody conjugate, e.g., by antibody cross-linking to generate a bi-specific structure using a heterobifunctional reagent having an amine-reactive group and a sulfhydryl reactive group as described in, e.g., US 4433059; bispecific antibody determinants generated by recombining half antibodies (heavy-light chain pairs or Fabs) from different antibodies through cycle of reduction and oxidation of disulfide bonds between the two heavy chains, as described in, e.g., US 4444878; trifunctional antibodies, e.g., three Fab' fragments cross-linked through sulfhdryl reactive groups, as described in, e.g., US5273743; biosynthetic binding proteins, e.g., pair of scFvs cross-linked through C-terminal tails preferably through disulfide or amine-reactive chemical cross-linking, as described in, e.g., US5534254; bifunctional antibodies, e.g., Fab fragments with different binding specificities dimerized through leucine zippers (e.g., c-fos and c-jun) that have replaced the constant domain, as described in, e.g., US5582996; bispecific and oligospecific mono-and oligovalent receptors, e.g., VH-CH1 regions of two antibodies (two Fab fragments) linked through a polypeptide spacer between the CH1 region of one antibody and the VH region of the other antibody typically with associated light chains, as described in, e.g., US5591828; bispecific DNA-antibody conjugates, e.g., crosslinking of antibodies or Fab fragments through a double stranded piece of DNA, as described in, e.g., US5635602; bispecific fusion proteins, e.g., an expression construct containing two scFvs with a hydrophilic helical peptide linker between them and a full constant region, as described in, e.g., US5637481; multivalent and multispecific binding proteins, e.g., dimer of polypeptides having first domain with binding region of Ig heavy chain variable region, and second domain with binding region of Ig light chain variable region, generally termed diabodies (higher order structures are also encompassed creating for bispecifc, trispecific, or tetraspecific molecules, as described in, e.g., US5837242; minibody constructs with linked VL and VH chains further connected with peptide spacers to an antibody hinge region and CH3 region, which can be dimerized to form bispecific/multivalent molecules, as described in, e.g., US5837821; VH and VL domains linked with a short peptide linker (e.g., 5 or 10 amino acids) or no linker at all in either orientation, which can form dimers to form bispecific diabodies; trimers and tetramers, as described in, e.g., US5844094; String of VH domains (or VL domains in family members) connected by peptide linkages with crosslinkable groups at the C-terminus futher associated with VL domains to form a series of FVs (or scFvs), as described in, e.g., US5864019; and single chain binding polypeptides with both a VH and a VL domain linked through a peptide linker are combined into multivalent structures through non-covalent or chemical crosslinking to form, e.g., homobivalent, heterobivalent, trivalent, and tetravalent structures using both scFV or diabody type format, as described in, e.g., US5869620. Additional exemplary multispecific and bispecific molecules and methods of making the same are found, for example, in US5910573, US5932448, US5959083, US5989830, US6005079, US6239259, US6294353, US6333396, US6476198, US6511663, US6670453, US6743896, US6809185, US6833441, US7129330, US7183076, US7521056, US7527787, US7534866, US7612181, US2002004587A1, US2002076406A1, US2002103345A1, US2003207346A1, US2003211078A1, US2004219643A1, US2004220388A1, US2004242847A1, US2005003403A1, US2005004352A1, US2005069552A1, US2005079170A1, US2005100543A1, US2005136049A1, US2005136051A1, US2005163782A1, US2005266425A1, US2006083747A1, US2006120960A1, US2006204493A1, US2006263367A1, US2007004909A1, US2007087381A1, US2007128150A1, US2007141049A1, US2007154901A1, US2007274985A1, US2008050370A1, US2008069820A1, US2008152645A1, US2008171855A1, US2008241884A1, US2008254512A1, US2008260738A1, US2009130106A1, US2009148905A1, US2009155275A1, US2009162359A1, US2009162360A1, US2009175851A1, US2009175867A1, US2009232811A1, US2009234105A1, US2009263392A1, US2009274649A1, EP346087A2, WO0006605A2, WO02072635A2, WO04081051A1, WO06020258A2, WO2007044887A2, WO2007095338A2, WO2007137760A2, WO2008119353A1, WO2009021754A2, WO2009068630A1, WO9103493A1, WO9323537A1, WO9409131A1, WO9412625A2, WO9509917A1, WO9637621A2, WO9964460A1.

Within each antibody or antibody fragment (e.g., scFv) of a bispecific antibody molecule, the VH can be upstream or downstream of the VL. In some embodiments, the upstream antibody or antibody fragment (e.g., scFv) is arranged with its VH (VH1) upstream of its VL (VL1) and the downstream antibody or antibody fragment (e.g., scFv) is arranged with its VL (VL2) upstream of its VH (VH2), such that the overall bispecific antibody molecule has the arrangement VH1-VL1-VL2-VH2. In other embodiments, the upstream antibody or antibody fragment (e.g., scFv) is arranged with its VL (VL1) upstream of its VH (VH1) and the downstream antibody or antibody fragment (e.g., scFv) is arranged with its VH (VH2) upstream of its VL (VL2), such that the overall bispecific antibody molecule has the arrangement VL1-VH1-VH2-VL2. Optionally, a linker is disposed between the two antibodies or antibody fragments (e.g., scFvs), e.g., between VL1 and VL2 if the construct is arranged as VH1-VL1-VL2-VH2, or between VH1 and VH2 if the construct is arranged as VL1-VH1-VH2-VL2. The linker may be a linker as described herein, e.g., a (Gly4-Ser)n linker, wherein n is 1, 2, 3, 4, 5, or 6, preferably 4 (SEQ ID NO: 29). In general, the linker between the two scFvs should be long enough to avoid mispairing between the domains of the two scFvs. Optionally, a linker is disposed between the VL and VH of the first scFv. Optionally, a linker is disposed between the VL and VH of the second scFv. In constructs that have multiple linkers, any two or more of the linkers can be the same or different. Accordingly, in some embodiments, a bispecific CAR comprises VLs, VHs, and optionally one or more linkers in an arrangement as described herein.

In one aspect, the bispecific antibody molecule is characterized by a first immunoglobulin variable domain sequence, e.g., a scFv, which has binding specificity for a first cancer-associated antigen, e.g., comprises a scFv as described herein, e.g., as described in Table 2, or comprises the light chain CDRs and/or heavy chain CDRs from a scFv described herein, and a second immunoglobulin variable domain sequence that has binding specificity for a second epitope on a different antigen. In some aspects the second immunoglobulin variable domain sequence has binding specificity for an antigen expressed on AML cells. For example, the second immunoglobulin variable domain sequence has binding specificity for CD123. As another example, the second immunoglobulin variable domain sequence has binding specificity for CD33. As another example, the second immunoglobulin variable domain sequence has binding specificity for CLL-1. As another example, the second immunoglobulin variable domain sequence has binding specificity for CD34. As another example, the second immunoglobulin variable domain sequence has binding specificity for FLT3. For example, the second immunoglobulin variable domain sequence has binding specificity for folate receptor beta. In some aspects, the second immunoglobulin variable domain sequence has binding specificity for an antigen expressed on B-cells, for example, CD19, CD20, CD22 or ROR1.

### Chimeric TCR

In one aspect, the antibodies and antibody fragments disclosed herein (for example, those disclosed in Table 2) can be grafted to one or more constant domain of a T cell receptor ("TCR") chain, for example, a TCR alpha or TCR beta chain, to create an chimeric TCR that binds specifically to a cancer associated antigen. Without being bound by theory, it is believed that chimeric TCRs will signal through the TCR complex upon antigen binding. For example, an scFv as disclosed herein, can be grafted to the constant domain, e.g., at least a portion of the extracellular constant domain, the transmembrane domain and the cytoplasmic domain, of a TCR chain, for example, the TCR alpha chain and/or the TCR beta chain. As another example, an antibody fragment, for example a VL domain as described herein, can be grafted to the constant domain of a TCR alpha chain, and an antibody fragment, for example a VH domain as described herein, can be grafted to the constant domain of a TCR beta chain (or alternatively, a VL domain may be grafted to the constant domain of the TCR beta chain and a VH domain may be grafted to a TCR alpha chain). As another example, the CDRs of an antibody or antibody fragment, e.g., the CDRs of an antibody or antibody fragment as described in Table 3 may be grafted into a TCR alpha and/or beta chain to create a chimeric TCR that binds specifically to a cancer associated antigen. For example, the LC CDRs disclosed herein may be grafted into the variable domain of a TCR alpha chain and the HC CDRs disclosed herein may be grafted to the variable domain of a TCR beta chain, or vice versa. Such chimeric TCRs may be produced by any appropriate method (For example, Willemsen RA et al, Gene Therapy 2000; 7: 1369-1377; Zhang T et al, Cancer Gene Ther 2004; 11: 487-496; Aggen et al, Gene Ther. 2012 Apr;19(4):365-74).

### Non-Antibody Scaffolds

In embodiments, the antigen binding domain comprises a non antibody scaffold, e.g., a fibronectin, ankyrin, domain antibody, lipocalin, small modular immuno-pharmaceutical, maxybody, Protein A, or affilin. The non antibody scaffold has the ability to bind to target antigen on a cell. In embodiments, the antigen binding domain is a polypeptide or fragment thereof of a naturally occurring protein expressed on a cell. In some embodiments, the antigen binding domain comprises a non-antibody scaffold. A wide variety of non-antibody scaffolds can be employed so long as the resulting polypeptide includes at least one binding region which specifically binds to the target antigen on a target cell.

Non-antibody scaffolds include: fibronectin (Novartis, MA), ankyrin (Molecular Partners AG, Zurich, Switzerland), domain antibodies (Domantis, Ltd., Cambridge, MA, and Ablynx nv, Zwijnaarde, Belgium), lipocalin (Pieris Proteolab AG, Freising, Germany), small modular immuno-pharmaceuticals (Trubion Pharmaceuticals Inc., Seattle, WA), maxybodies (Avidia, Inc., Mountain View, CA), Protein A (Affibody AG, Sweden), and affilin (gamma-crystallin or ubiquitin) (Scil Proteins GmbH, Halle, Germany).

Fibronectin scaffolds can be based on fibronectin type III domain (e.g., the tenth module of the fibronectin type III (¹⁰ Fn3 domain)). The fibronectin type III domain has 7 or 8 beta strands which are distributed between two beta sheets, which themselves pack against each other to form the core of the protein, and further containing loops (analogous to CDRs) which connect the beta strands to each other and are solvent exposed. There are at least three such loops at each edge of the beta sheet sandwich, where the edge is the boundary of the protein perpendicular to the direction of the beta strands (see US 6,818,418). Because of this structure, this non-antibody scaffold mimics antigen binding properties that are similar in nature and affinity to those of antibodies. These scaffolds can be used in a loop randomization and shuffling strategy in vitro that is similar to the process of affinity maturation of antibodies in vivo.

The ankyrin technology is based on using proteins with ankyrin derived repeat modules as scaffolds for bearing variable regions which can be used for binding to different targets. The ankyrin repeat module is a 33 amino acid polypeptide consisting of two anti-parallel α-helices and a β-turn. Binding of the variable regions is mostly optimized by using ribosome display.

Avimers are derived from natural A-domain containing protein such as HER3. These domains are used by nature for protein-protein interactions and in human over 250 proteins are structurally based on A-domains. Avimers consist of a number of different "A-domain" monomers (2-10) linked via amino acid linkers. Avimers can be created that can bind to the target antigen using the methodology described in, for example, U.S. Patent Application Publication Nos. 20040175756; 20050053973; 20050048512; and 20060008844.

Affibody affinity ligands are small, simple proteins composed of a three-helix bundle based on the scaffold of one of the IgG-binding domains of Protein A. Protein A is a surface protein from the bacterium Staphylococcus aureus. This scaffold domain consists of 58 amino acids, 13 of which are randomized to generate affibody libraries with a large number of ligand variants (See e.g., US 5,831,012). Affibody molecules mimic antibodies, they have a molecular weight of 6 kDa, compared to the molecular weight of antibodies, which is 150 kDa. In spite of its small size, the binding site of affibody molecules is similar to that of an antibody.

Protein epitope mimetics (PEM) are medium-sized, cyclic, peptide-like molecules (MW 1-2kDa) mimicking beta-hairpin secondary structures of proteins, the major secondary structure involved in protein-protein interactions. Antigen binding domains, e.g., those comprising scFv, single domain antibodies, or camelid antibodies, can be directed to any target receptor/ligand described herein, e.g., the the PD1 receptors, PD-L1 or PD-L2.

In an embodiment the antigen binding domain comprises the extracellular domain, or a counter-ligand binding fragment thereof, of molecule that binds a counterligand on the surface of a target cell.

An antigen binding domain can comprise the extracellular domain of an inhibitory receptors. Engagement with a counterligand of the coinhibitory molecule is redirected into an optimization of immune effector response.

An antigen binding domain can comprise the extracellular domain of a costimulatory molecule, referred to as a Costimulatory ECD domain, Engagement with a counter ligand of the costimulatory molecule results in optimization of immune effector response.

### TRANSMEMBRANE DOMAIN

In instances, a CAR described herein comprises a transmembrane domain that is fused to an extracellular sequence, e.g., an extracellular recognition element, which can comprise an antigen binding domain, an inhibitory counter ligand binding domain, or a costimulatory ECD domain. In an instance, the transmembrane domain is one that naturally is associated with one of the domains in the CAR. In an instance, the transmembrane domain is one that is not naturally associated with one of the domains in the CAR.

A transmembrane domain can include one or more additional amino acids adjacent to the transmembrane region, e.g., one or more amino acid associated with the extracellular region of the protein from which the transmembrane was derived (e.g., 1, 2, 3, 4, 5, 6, 7, 8, 9, 10 up to 15 amino acids of the extracellular region) and/or one or more additional amino acids associated with the intracellular region of the protein from which the transmembrane protein is derived (e.g., 1, 2, 3, 4, 5, 6, 7, 8, 9, 10 up to 15 amino acids of the intracellular region). In one aspect, the transmembrane domain is one that is associated with one of the other domains of the CAR e.g., in one embodiment, the transmembrane domain may be from the same protein that the signaling domain, costimulatory domain or the hinge domain is derived from. In another aspect, the transmembrane domain is not derived from the same protein that any other domain of the CAR is derived from.

In embodiments, the transmembrane domain is one which minimizes interactions with other elements, e.g., other transmembrane domains. In some instances, the transmembrane domain minimizes binding of such domains to the transmembrane domains of the same or different surface membrane proteins, e.g., to minimize interactions with other members of the receptor complex. Suitable examples can be derived by selection or modification of amino acid substitution of a known transmembrane domain. In an embodiment, the transmembrane domain is capable of promoting homodimerization with another CAR on the cell surface. In a different aspect the amino acid sequence of the transmembrane domain may be modified or substituted so as to minimize interactions with the binding domains of the native binding partner present in the same CAR-expressing cell.

The transmembrane domain may comprise a naturally occurring, or a non-naturally occurring synthetic sequence. Where naturally occurring, the transmembrane domain may be derived from any membrane-bound or transmembrane protein.

Transmembrane regions suitable for use in molecules described herein may be derived from any one or more of e.g., the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137, CD154. In some embodiments, a transmembrane domain may include at least the transmembrane region(s) of, e.g., KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), NKp44, NKp30, NKp46, CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp , NKG2D, and NKG2C. In an embodiment the transmembrane domain is derived from CD8. In an embodiment the transmembrane domain is derived from CD28. In one aspect, the transmembrane domain is a transmembrane domain from the sequence provided as SEQ ID NO: 12 or SEQ ID NO: 42.

In an embodiment, a sequence, e.g., a hinge or spacer sequence, can be disposed between a transmembrane domain and another sequence or domain to which it is fused. In embodiments, a variety of human hinges (aka "spacers") can be employed as well, e.g., including but not limited to the human Ig (immunoglobulin) hinge. In one embodiment, the hinge can be a human Ig (immunoglobulin) hinge (e.g., an IgG4 hinge an IgD hinge), a GS linker (e.g., a GS linker described herein), a KIR2DS2 hinge or a CD8a hinge. Optionally, a short oligo- or polypeptide linker, between 2 and 10 amino acids in length may form the linkage between the transmembrane domain and another domain, e.g., an intracellular signaling domain or costimulatory domain, of a CAR. A glycine-serine doublet provides a particularly suitable linker. In one aspect, the hinge or spacer is the amino acid sequence provided as SEQ ID NO: 4, SEQ ID NO: 6, or SEQ ID NO: 8. In one aspect, the hinge or spacer comprises a KIR2DS2 hinge.

In an embodiment, the transmembrane domain may be a non-naturally occurring sequence, in which case can comprise predominantly hydrophobic residues such as leucine and valine. In an embodiment, a triplet of phenylalanine, tryptophan and valine will be found at each end of a transmembrane domain.

Optionally, a short oligo- or polypeptide linker, between 2 and 10 amino acids in length may form the linkage between the transmembrane domain and the cytoplasmic region of the CAR. A glycine-serine doublet provides a particularly suitable linker. For example, in one aspect, the linker comprises the amino acid sequence of GGGGSGGGGS (SEQ ID NO:10). In some embodiments, the linker is encoded by a nucleotide sequence of GGTGGCGGAGGTTCTGGAGGTGGAGGTTCC (SEQ ID NO:11).

### INTRACELLULAR SIGNALING DOMAIN

In instances, an intracellular signaling domain produces an intracellular signal when an extracellular domain, e.g., an antigen binding domain, to which it is fused, binds a counter ligand. Intracellular signaling domains can include primary intracellular signaling domains and costimulatory signaling domains. In an instance, a CAR molecule can be constructed for expression in an immune cell, e.g., a T cell, such that the CAR molecule comprises a domain, e.g., a primary intracellular signaling domains, costimulatory signaling domain, inhibitory domains, etc., that is derived from a polypeptide that is typically associated with the immune cell. For example, a CAR for expression in a T cell can comprise a 41BB domain and a CD3 zeta domain. In this instance, both the 41BB and CD3 zeta domains are derived from polypeptides associated with the T cell. In another embodiment, a CAR for expression in a T cell can comprise a CD28 domain and a CD3 zeta domain. In another embodiment, a CAR for expression in a T cell can comprise an ICOS domain and a CD3 zeta domain. In another embodiment, a CAR for expression in a T cell can comprise a CD27 deomain and a CD3 zeta domain. In another instance, a CAR molecule can be constructed for expression in an immune cell e.g., a T cell, such that the CAR molecule comprises a domain that is derived from a polypeptide that is not typically associated with the immune cell.

### PRIMARY INTRACELLULAR SIGNALING DOMAIN

In an instance, a primary intracellular signaling domain produces an intracellular signal when an extracellular domain, e.g., an antigen binding domain, to which it is fused binds cognate antigen. The primary intracellular signaling domain is derived from a primary stimulatory molecule, e.g., it comprises intracellular sequence of a primary stimulatory molecule. The primary intracellular signaling domain comprises sufficient primary stimulatory molecule sequence to produce an intracellular signal, e.g., when an antigen binding domain to which it is fused binds cognate antigen.

A primary stimulatory molecule, is a molecule, that upon binding cognate ligand, mediates an immune effector response, e.g., in the cell in which it is expressed. Typically, it generates an intracellular signal that is dependent on binding to a cognate ligand that comprises antigen. The TCR/CD3 complex is an exemplary primary stimulatory molecule; it generates an intracellular signal upon binding to cognate ligand, e.g., an MHC molecule loaded with a peptide. Typically, e.g., in the case of the TCR/CD3 primary stimulatory molecule, the generation of an intracellular signal by a primary intracellular signaling domain is dependent on binding of the primary stimulatory molecule to antigen.

Primary stimulation can mediate altered expression of certain molecules, such as downregulation of TGF-β, and/or reorganization of cytoskeletal structures, and the like. Stimulation, can, e.g., in the presence of costimulation, result in an optimization, e.g., an increase, in an immune effector function of the CARX cell, e.g., CART cell. Stimulation, e.g., in the context of a CART cell, can mediate a T cell response, e.g., proliferation, activation, differentiation, and the like.

In an embodiment, the primary intracellular signaling domain comprises a signaling motif, e.g., an immunoreceptor tyrosine-based activation motif or ITAMs. A primary intracellular signaling domain can comprise ITAM containing cytoplasmic signaling sequences from (for example) TCR zeta (CD3 zeta), common FcR gamma, (FCER1G), Fc gamma RIIa, FcR beta (Fc Epsilon R1b), CD3 gamma, CD3 delta, CD3 epsilon, CD5, CD22, CD79a, CD79b, CD278 (also known as "ICOS"), FcεRI, DAP10, DAP12, and CD66d.

Exemplary primary intracellular signaling domains are provided in Table 4.

| Table 4. Primary Intracellular Signaling Domains In embodiments the domain comprises an ITAM |
|---|
| |
| TCR zeta |
| FcR gamma |
| FcR beta |
| CD3 gamma |
| CD3 delta |
| CD3 epsilon |
| CD3 zeta |
| CD5 |
| CD22 |
| CD79a |
| CD79b |
| CD66d |
| DAP10 |
| DAP12 |
| CD32 |

A primary intracellular signaling domain comprises a functional fragment, or analog, of a primary stimulatory molecule (e.g., CD3 zeta - GenBank Acc. No. BAG36664.1). The primary intracellular signalin domain can comprise the entire intracellular region or a fragment of the intracellular region which is sufficient for generation of an intracellular signal when an antigen binding domain to which it is fused binds cognate antigen. In embodiments the primary intracellular signaling domain has at least 70, 75, 80, 85, 90, 95, 98, or 99 % sequence identity with the entire intracellular region, or a fragment of the intracellular region which is sufficient for generation of an intracellular signal, of a naturally occurring primary stimulatory molecule, e.g., a human (GenBank Acc No. BAG36664.1), or other mammalian, e.g., a nonhuman species, e.g., rodent, monkey, ape or murine intracellular primary stimulatory molecule. In embodiments the primary intracellular signaling domain has at least 70, 75, 80, 85, 90, 95, 98, or 99 % sequence identity with SEQ ID NO: 18 or SEQ ID NO: 20.

In embodiments the primary intracellular signaling domain, has at least 70, 75, 80, 85, 90, 95, 96, 97, 98, or 99% identity with, or differs by no more than 30, 25, 20, 15, 10, 5, 4, 3, 2, or 1 amino acid residues from the corresponding residues of the entire intracellular region, or a fragment of the intracellular region which is sufficient for generation of an intracellular signal, of a naturally occurring human primary stimulatory molecule, e.g., a naturally occurring human primary stimulatory molecule disclosed herein.

### COSTIMULATORY SIGNALING DOMAIN

In an instance, a costimulatory signaling domain produces an intracellular signal when an extracellular domain, e.g., an antigen binding domain to which it is fused binds cognate ligand. The costimulatory signaling domain is derived from a costimulatory molecule. The costimulatory signaling domain comprises sufficient primary costimulatory molecule sequence to produce an intracellular signal, e.g., when an extracellular domain, e.g., an antigen binding domain, to which it is fused binds cognate ligand.

The costimulatory domain can be one which optimizes the performance, e.g., the persistence, or immune effector function, of a T cell that comprises a CAR which comprises the costimulatory domain. E.g., a CAR (a CAR^{CD4+}) for use in CD4⁺ T cells can comprise an ICOS domain. E.g., a CAR (a CAR^{CD8+}) for use in CD8⁺ T cells can comprise a CD28 or a 4-1BB domain.

Costimulatory molecules are cell surface molecules, other than antigen receptors or their counter ligands that promote an immune effector response. In some cases they are required for an efficient or enhanced immune response. Typically, a costimulatory molecule generates an intracellular signal that is dependent on binding to a cognate ligand that is, in instances, other than an antigen, e.g., the antigen recognized by an antigen binding domain of a CARX cell, e.g., CART cell. Typically, signaling from a primary stimulatory molecule and a costimulatory molecule contribute to an immune effector response, and in some cases both are required for efficient or enhanced generation of an immune effector response.

A costimulatory domain comprises a functional fragment, or analog, of a costimulatory molecule (e.g., ICOS, CD28, or 4-1BB). It can comprise the entire intracellular region or a fragment of the intracellular region which is sufficient for generation of an intracellular signal, e.g., when an antigen binding domain to which it is fused binds cognate antigen. In embodiments the costimulatory domain has at least 70%, 75%, 80%, 85%, 90%, 95%, 98%, or 99 % sequence identity with the entire intracellular region, or a fragment of the intracellular region which is sufficient for generation of an intracellular signal, of a naturally occurring costimulatory molecule, e.g., a human, or other mammalian, e.g., a nonhuman species, e.g., rodent, monkey, ape or murine intracellular costimulatory molecule. In embodiments the costimulatory domain has at least 70%, 75%, 80%, 85%, 90%, 95%, 98%, or 99 % sequence identity with SEQ ID NO: 14, SEQ ID NO: 16, SEQ ID NO: 40, or SEQ ID NO: 44.

Exemplary costimulatory signaling domains (intracellular signaling domains) are provided in Table 5.

| Table 5: Costimulatory Signaling Domains for CARX (identified by the Costimulatory Molecules from which they are derived) |
|---|
| CD27 |
| CD28 |
| 4-1BB (CD137) |
| OX40 |
| CD30 |
| CD40 |
| ICOS (CD278) |
| ICAM-1 |
| LFA-1 (CD11a/CD18) |
| CD2 |
| CD7 |
| LIGHT |
| NKG2C |
| B7-H3 |
| a ligand that specifically binds with CD83 |
| CDS |
| GITR |
| BAFFR |
| HVEM (LIGHTR) |
| SLAMf7 |
| NKP80 (KLRF1) |
| CD160 (BY55) |
| CD19 |
| CD4 |
| CD8 alpha |
| CD8 beta |
| IL2R beta |
| IL2R gamma |
| IL7R alpha |
| ITGA4 |
| VLA1 |
| CD49a |
| ITGA4 |
| IA4 |
| CD49D |
| ITGA6 |
| VLA-6 |
| C49f |
| ITGAD |
| CD11d |
| ITGAE |
| CD103 |
| ITGAL |
| CD11a |
| LFA-1 |
| ITGAM |
| CD11b |
| ITGAX |
| CD11c |
| ITGB1 |
| CD29 |
| ITGB2 |
| CD18 |
| ITGB7 |
| TNFR2 |
| TRANCE/RANKL |
| DNAM1 (CD226) |
| SLAMF4 (C244, 2B4) |
| CD84 |
| CD96 (Tactile) |
| CEACAM1 |
| CRTAM |
| Ly9 (CD229) |
| PSGL1 |
| C100 (SEMA4D) |
| CD69 |
| SLAMF6 (NTB-A, Ly108) |
| SLAM (SLAMF1, CD150, IPO-3) |
| BLAME (SLAMF8) |
| SELPLG (CD162) |
| LTBR |
| LAT |
| GADS |
| PAG/Cbp |

In embodiments the costimulatory signaling domain, has at least 70, 75, 80, 85, 90, 95, 96, 97, 98, or 99% identity with, or differs by no more than 30, 25, 20, 15, 10, 5, 4, 3, 2, or 1 amino acid residues from the corresponding residues of the entire intracellular region, or a fragment of the intracellular region which is sufficient for generation of an intracellular signal, of, a naturally occurring human costimulatory molecule, e.g., a naturally occurring human costimulatory molecule disclosed herein.

### INHIBITORY MOLECULES: INHIBITION

Inhibitory molecules, e.g., PD1, can, in some instances, decrease the ability of a CARX cell to mount an immune effector response. Inhibition of an inhibitory molecule, e.g., by inhibition at the DNA, RNA or protein level, can optimize CARX cell performance. In instances an inhibitory nucleic acid, e.g., an inhibitory nucleic acid, e.g., a dsRNA, e.g., an siRNA or shRNA, can be used to inhibit expression of an inhibitory molecule in the CARX cell. In an instance the inhibitor is a shRNA. In an instance, the inhibitory molecule is inhibited within a CARX cell. In these instances, a dsRNA molecule that inhibits expression of the inhibitory molecule is linked to the nucleic acid that encodes a component, e.g., all of the components, of the CAR.

Exemplary inhibitory molecules, useful e.g., as shRNA targets, are provided in Table 6.

| Table 6: Inhibitory molecules |
|---|
| CD160 |
| 2B4 |
| PD1 |
| TIM3 |
| LAG3 |
| TIGIT |
| CTLA-4 |
| BTLA |
| LAIR1 |
| PD-L1 |
| VISTA |

In another aspect, the CAR-expressing cell described herein can further express another agent, e.g., an agent which enhances the activity of a CAR-expressing cell. For example, in one instance, the agent can be an agent which inhibits an inhibitory molecule. Inhibitory molecules, e.g., PD1, can, in some instances, decrease the ability of a CAR-expressing cell to mount an immune effector response. Examples of inhibitory molecules include PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta. In one instance, the agent which inhibits an inhibitory molecule, e.g., is a molecule described herein, e.g., an agent that comprises a first polypeptide, e.g., an inhibitory molecule, associated with a second polypeptide that provides a positive signal to the cell, e.g., an intracellular signaling domain described herein. In one instance, the agent comprises a first polypeptide, e.g., of an inhibitory molecule such as PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta, or a fragment of any of these (e.g., at least a portion of an extracellular domain of any of these), and a second polypeptide which is an intracellular signaling domain described herein (e.g., comprising a costimulatory domain (e.g., 4-1BB, ICOS, CD27 or CD28, e.g., as described herein) and/or a primary signaling domain (e.g., a CD3 zeta signaling domain described herein). In one instance, the agent comprises a first polypeptide of PD1 or a fragment thereof (e.g., at least a portion of an extracellular domain of PD1), and a second polypeptide of an intracellular signaling domain described herein (e.g., a CD28 signaling domain described herein and/or a CD3 zeta signaling domain described herein). In instances, the CAR-expressing cell described herein comprises a switch costimulatory receptor, e.g., as described in WO 2013/019615. PD1 is an inhibitory member of the CD28 family of receptors that also includes CD28, CTLA-4, ICOS, and BTLA. PD-1 is expressed on activated B cells, T cells and myeloid cells (Agata et al. 1996 Int. Immunol 8:765-75). Two ligands for PD1, PD-L1 and PD-L2 have been shown to downregulate T cell activation upon binding to PD1 (Freeman et a. 2000 J Exp Med 192:1027-34; Latchman et al. 2001 Nat Immunol 2:261-8; Carter et al. 2002 Eur J Immunol 32:634-43). PD-L1 is abundant in human cancers (Dong et al. 2003 J Mol Med 81:281-7; Blank et al. 2005 Cancer Immunol. Immunother 54:307-314; Konishi et al. 2004 Clin Cancer Res 10:5094). Immune suppression can be reversed by inhibiting the local interaction of PD1 with PD-L1.

### REDIRECTED SWITCHABLE INHIBITORY RECEPTORS: INHIBITORY EXTRACELLULAR DOMAINS

Extracellular domains of inhibitory receptors can be coupled to intracellular signaling domains that promote an immune effector response. Thus, engagement with a counterligand of the coinhibitory molecule is redirected into an optimization of immune effector response.

In an instance the CAR comprising an extracellular domain (ECD) of an inhibitory molecule is a CAR^{CD4+}, e.g., comprising an ICOS doamain, and is disposed in a CD4⁺ T cell. In an embodiment the CAR comprising an extracellular domain (ECD) of an inhibitory molecule is a CAR^{CDI+} , e.g., comprising a CD28 or 4-1BB domain, and is disposed in a CD8+ T cell.

In one instance, the extracellular domain (ECD) of an inhibitory molecule, e.g., an inhibitory molecule described herein such as, e.g., Programmed Death 1 (PD1), can be fused to a transmembrane domain and intracellular signaling domain described herein, e.g., an intracellular signaling domain comprising a costimulatory signaling domain such as, e.g., 41BB OX40, Cd28, CD27, ICOS, and/or a primary signaling domain, e.g., of CD3 zeta. In one instance, the inhibitory molecule CAR, e.g., PD1 CAR, can be used alone. In one instance, the inhibitory molecule CAR, e.g., inhibitory molecule CAR, e.g., PD1 CAR, can be used in combination with another CAR, e.g., CD19CAR (e.g., a CD19CAR). In one instance, the PD1 CAR improves the persistence of the T cell. Examples of inhibitory molecules include PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta. In one instance, the inhibitory molecule CAR comprises a first polypeptide, e.g., of an inhibitory molecule such as PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta, or a fragment of any of these (e.g., at least a portion of an extracellular domain of any of these), and a second polypeptide which is an intracellular signaling domain described herein (e.g., comprising a costimulatory domain (e.g., 41BB, ICOS, CD27 or CD28, e.g., as described herein) and/or a primary signaling domain (e.g., a CD3 zeta signaling domain described herein).

In one instance, the inhibitory molecule CAR comprises the extracellular domain (ECD) of PD1 fused to a transmembrane domain and intracellular signaling domains such as 41BB and CD3 zeta (also referred to herein as a PD1 CAR). In one instance, the PD1 CAR improves the persistence of the cell CAR-expressing cell. In one instance, the PD1 CAR comprises the extracellular domain of PD1; the amino acid sequence of the extracellular domain of PD1 is provided as SEQ ID NO: 24.

In one instance, the PD1 CAR comprises the amino acid sequence of SEQ ID NO: 26 or 39.

In one instance, the PD1 CAR, e.g., the PD1 CAR described herein, is encoded by a nucleic acid sequence of SEQ ID NO: 27, or at least comprises the nucleic acid sequence of SEQ ID NO: 25 encoding the extracellular domain of PD-1.

Exemplary inhibitory extracellular domains are provided in Table 7.

| Table 7: Extracellular counter ligand binding domains from coinhibitory molecules (identified by the Coinibitory Molecules from which they are derived) |
|---|
| B7-H1 |
| B7-1 |
| CD160 |
| P1H |
| 2B4 |
| PD1 |
| TIM3 |
| LAG3 |
| TIGIT |
| CTLA-4 |
| BTLA |
| LAIR1 |
| TGF-beta receptor |

In instances the inhibitory extracellular domain, has at least 70, 75, 80, 85, 90, 95, 96, 97, 98, or 99% identity with, or differs by no more than 30, 25, 20, 15, 10, 5, 4, 3, 2, or 1 amino acid residues from the corresponding residues of the entire extracellular region, or a fragment of the extracellular region which is sufficient for engagement with the counter ligand, of a naturally occurring human inhibitory molecule, e.g., a naturally occurring human primary stimulatory molecule disclosed herein.

### COSTIMULATORY MOLECULE LIGAND BINDING DOMAINS

Extracellular ligand binding domains of costimulatory molecules, referred to as a costimulatory ECD domain, can be coupled to intracellular signaling domains that promote an immune effector response. Thus, engagement with a counter ligand of the costimulatory molecule results in optimization of immune effector response.

In an instance the CAR comprising a costimulatory ECD domain is a CAR^{CD4+}, comprising, e.g., an ICOS domain, and is disposed in a CD4⁺ T cell. In an embodiment the CAR comprising a costimulatory ECD domain is a CAR^{CD8+}, comprising, e.g., a CD28 or 4-1BB domain, and is disposed in a CD8+ T cell.

Exemplary Costimulatory ECD domains are provided in the Table 8

| Table 8: Costimulatory ECD domains from costimulatory molecules (identified by the Costimulatory Molecules from which they are derived) |
|---|
| |
| ICOS |
| CD28 |
| CD27 |
| HVEM |
| LIGHT |
| CD40L |
| 4-1BB |
| OX40 |
| DR3 |
| GITR |
| CD30 |
| TIM1 |
| SLAM |
| CD2 |
| CD226 |

In embodiments the Costimulatory ECD domain, has at least 70, 75, 80, 85, 90, 95, 96, 97, 98, or 99% identity with, or differs by no more than 30, 25, 20, 15, 10, 5, 4, 3, 2, or 1 amino acid residues from the corresponding residues of the entire extracellular region, or a fragment of the extracellular region which is sufficient for engagement with the counter ligand, of a naturally occurring human inhibitory molecule, e.g., a naturally occurring human costimulatory molecule disclosed herein.

### ICARs

CARs disclosed herein can include an inhibitory CAR (iCAR) member. An iCAR member comprises: an antigen binding domain (or other extracelluar domain) that recognizes an antigen on a non-target, e.g., a noncancer, cell; a transmembrane domain; and, a domain from an inhibitory molecule, e.g., an intracellular domain from an inhibitory molecule, e.g., from PD-1, CTLA4, or from a protein listed in Table 9. In an instance, the iCAR member comprises a second inhibitory intracellular signaling domain, e.g., from PD-1, CTLA4, or from a protein listed in Table 9.

Upon engagement of the antigen binding domain (or other extracelluar domain) of the iCAR member with its target antigen (or counter-ligand), the iCAR contributes to inhibiting, e.g., reversibly inhibiting, or minimizing, activation of the cell comprising the iCAR. As such, inclusion of an iCAR member in a CAR, e.g., and CART, cell, can limit damage to non-target, e.g., bystander, cells. While not wishing to be bound by theory, it is believed that an iCAR member, upon engagement with its antigen (or counter-ligand), limits one or more of cytokine secretion, cytotoxicity, and proliferation. In instances the effect is temporary, and upon subsequent engagement with a target cell the CAR, e.g., CART, cell is activated and attacks the target cell.

A target antigen for an iCAR member can be an antigen that has an expression profile on target cells and non-target cells such that an acceptably high level of attack on target cells and an acceptably low level of attack on non-target cells is achieved. Not only choice of antigen, but iCAR affinity for its antigen (or counter-ligand), CAR affinity for its antigen, level of expression of the iCAR, or levels of expression of the CAR can be used to optimize the ratio of on-target/off-target response.

In an instance, the antigen is absent, or down-regulated on tumor cells. In an instance the antigen comprises an HLA molecule. In an instance the antigen comprises a cell suface tumor suppressor antigen. In an instance the antigen comprises PCML (or another antigen that is down-regulated in lymphomas, breast or prostate cancer), HYAL2, DCC, or SMAR1.

In an instance, the antigen comprises a protein, carbohydrate, lipid, or a post-translational modification of a cell surface moiety, e.g., a mucin-type O-glycan (a core 3 O-glycan).

In an instance, the antigen comprises a moiety that is down-regulated by tumor cells undergoing an epithelial to mesenchymal transition.

In an instance, the antigen comprises E-cadherin.

In an instance a domain from an inhibitory molecule,, e.g., an intracellular signaling domain from PD-1 or CTLA4, produces an intracellular signal when an extracellular domain, e.g., an antigen binding domain, to which it is fused binds cognate antigen (or counter ligand). The inhibitory intracellular signaling domain is derived from an inhibitory molecule, e.g., it comprises intracellular sequence of an inhibitory molecule. It comprises sufficient inhibitory molecule sequence to produce an intracellular signal, e.g., when an antigen binding domain to which it is fused binds its cognate antigen.

In an instance, the primary intracellular signaling domain comprises a signaling motif, e.g., an immunoreceptor tyrosine-based activation motif or ITIM.

A domain from an inhibitory molecule, comprises a functional fragment, or analog, of an inhibitory molecule intracellular domain. It can comprise the entire intracellular region or a fragment of the intracellular region which is sufficient for generation of an intracellular signal when an antigen binding domain to which it is fused, binds cognate antigen. In instances the inhibitory intracellular signaling domain has at least 70, 75, 80, 85, 90, 95, 98, or 99 % sequence identity with, or differs by no more than 30, 25, 20, 15, 10, 5, 4, 3, 2, or 1 amino acid residues from, the corresponding residues oa naturally occurring inhibitory molecule, e.g., a a molecule from Table 9.

Exemplary inhibitory molecules which can provide intracellular signaling domains are provided in Table 9.

| Table 9: Inhibitory molecules |
|---|
| B7-H1 |
| B7-1 |
| CD160 |
| P1H |
| 2B4 |
| PD1 |
| TIM3 |
| LAG3 |
| TIGIT |
| CTLA-4 |
| BTLA |
| LAIR1 |
| TGF-beta receptor |

Thus, in one, aspect, disclosed herein is, an CAR comprising an iCAR member. The iCAR member comprises:
an antigen binding domain (or other extracelluar domain) that recognizes an antigen on a non-target, e.g., a noncancer cell;
a transmembrane domain; and
a domain from an inhibitory molecule,, e.g., from PD-1, CTLA4, or from a protein listed in Table 4.

In an instance, the iCAR member comprises a second inhibitory intracellular signaling domain, e.g., from PD-1, CTLA4, or from a protein listed in Table 9.

### NATURAL KILLER CELL RECEPTOR (NKR) CARS

In an instance, a CAR molecule described herein comprises one or more components of a natural killer cell receptor (NKR), thereby forming an NKR-CAR. The NKR component can be a transmembrane domain, a hinge domain, or a cytoplasmic domain from any of the following natural killer cell receptors: killer cell immunoglobulin-like receptor (KIR), e.g., KIR2DL1, KIR2DL2/L3, KIR2DL4, KIR2DL5A, KIR2DL5B, KIR2DS1, KIR2DS2, KIR2DS3, KIR2DS4, DIR2DS5, KIR3DL1/S1, KIR3DL2, KIR3DL3, KIR2DP1, and KIR3DP1; natural cyotoxicity receptor (NCR), e.g., NKp30, NKp44, NKp46; signaling lymphocyte activation molecule (SLAM) family of immune cell receptors, e.g., CD48, CD229, 2B4, CD84, NTB-A, CRACC, BLAME, and CD2F-10; Fc receptor (FcR), e.g., CD16, and CD64; and Ly49 receptors, e.g., LY49A, LY49C. The NKR-CAR molecules described herein may interact with an adaptor molecule or intracellular signaling domain, e.g., DAP12. Exemplary configurations and sequences of CAR molecules comprising NKR components are described in International Publication No. WO2014/145252.

### STRATEGIES FOR REGULATING CHIMERIC ANTIGEN RECEPTORS

There are many ways CAR activities can be regulated. In some embodiments, a regulatable CAR (RCAR) where the CAR activity can be controlled is desirable to optimize the safety and efficacy of a CAR therapy. For example, inducing apoptosis using, e.g., a caspase fused to a dimerization domain (see, e.g., Di et al., N Engl. J. Med. 2011 Nov. 3; 365(18):1673-1683), can be used as a safety switch in the CAR therapy of the instant invention. In another example, CAR-expressing cells can also express an inducible Caspase-9 (iCaspase-9) molecule that, upon administration of a dimerizer drug (e.g., rimiducid (also called AP1903 (Bellicum Pharmaceuticals) or AP20187 (Ariad)) leads to activation of the Caspase-9 and apoptosis of the cells. The iCaspase-9 molecule contains a chemical inducer of dimerization (CID) binding domain that mediates dimerization in the presence of a CID. This results in inducible and selective depletion of CAR-expressing cells. In some cases, the iCaspase-9 molecule is encoded by a nucleic acid molecule separate from the CAR-encoding vector(s). In some cases, the iCaspase-9 molecule is encoded by the same nucleic acid molecule as the CAR-encoding vector. The iCaspase-9 can provide a safety switch to avoid any toxicity of CAR-expressing cells. See, e.g., Song et al. Cancer Gene Ther. 2008; 15(10):667-75; Clinical Trial Id. No. NCT02107963; and Di Stasi et al. N. Engl. J. Med. 2011; 365:1673-83.

Alternative strategies for regulating the CAR therapy of the instant invention include utilizing small molecules or antibodies that deactivate or turn off CAR activity, e.g., by deleting CAR-expressing cells, e.g., by inducing antibody dependent cell-mediated cytotoxicity (ADCC). For example, CAR-expressing cells described herein may also express an antigen that is recognized by molecules capable of inducing cell death, e.g., ADCC or compliment-induced cell death. For example, CAR expressing cells described herein may also express a receptor capable of being targeted by an antibody or antibody fragment. Examples of such receptors include EpCAM, VEGFR, integrins (e.g., integrins αvβ3, α4, αI3/4β3, α4β7, α5β1, αvβ3, αv), members of the TNF receptor superfamily (e.g., TRAIL-R1, TRAIL-R2), PDGF Receptor, interferon receptor, folate receptor, GPNMB, ICAM-1 , HLA-DR, CEA, CA-125, MUC1 , TAG-72, IL-6 receptor, 5T4, GD2, GD3, CD2, CD3, CD4, CD5, CD11, CD11 a/LFA-1, CD15, CD18/ITGB2, CD19, CD20, CD22, CD23/lgE Receptor, CD25, CD28, CD30, CD33, CD38, CD40, CD41 , CD44, CD51 , CD52, CD62L, CD74, CD80, CD125, CD147/basigin, CD152/CTLA-4, CD154/CD40L, CD195/CCR5, CD319/SLAMF7, and EGFR, and truncated versions thereof (e.g., versions preserving one or more extracellular epitopes but lacking one or more regions within the cytoplasmic domain). For example, CAR-expressing cells described herein may also express a truncated epidermal growth factor receptor (EGFR) which lacks signaling capacity but retains the epitope that is recognized by molecules capable of inducing ADCC, e.g., cetuximab (ERBITUX®), such that administration of cetuximab induces ADCC and subsequent depletion of the CAR-expressing cells (see, e.g., WO2011/056894, and Jonnalagadda et al., Gene Ther. 2013; 20(8)853-860). Another strategy includes expressing a highly compact marker/suicide gene that combines target epitopes from both CD32 and CD20 antigens in the CAR-expressing cells described herein, which binds rituximab, resulting in selective depletion of the CAR-expressing cells, e.g., by ADCC (see, e.g., Philip et al., Blood. 2014; 124(8)1277-1287). Other methods for depleting CAR-expressing cells described herein include administration of CAMPATH®, a monoclonal anti-CD52 antibody that selectively binds and targets mature lymphocytes, e.g., CAR-expressing cells, for destruction, e.g., by inducing ADCC. In other instances, the CAR-expressing cell can be selectively targeted using a CAR ligand, e.g., an anti-idiotypic antibody. In some instances, the anti-idiotypic antibody can cause effector cell activity, e.g, ADCC or ADC activities, thereby reducing the number of CAR-expressing cells. In other instances, the CAR ligand, e.g., the anti-idiotypic antibody can be coupled to an agent that induces cell killing, e.g., a toxin, thereby reducing the number of CAR-expressing cells. In other instances, CAR-expressing cells can be selectively targeted using a CAR ligand, e.g., an anti-idiotypic antibody. In some instances, the anti-idiotypic antibody can cause effector cell activity, e.g, ADCC or ADC activities, thereby reducing the number of CAR-expressing cells. In other instances, the CAR ligand, e.g., the anti-idiotypic antibody, can be coupled to an agent that induces cell killing, e.g., a toxin, thereby reducing the number of CAR-expressing cells. Alternatively, the CAR molecules themselves can be configured such that the activity can be regulated, e.g., turned on and off, as described below.

In an aspect, a RCAR comprises a set of polypeptides, typically two in the simplest instances, in which the components of a standard CAR described herein, e.g., an antigen binding domain and an intracellular signaling domain, are partitioned on separate polypeptides or members. In some instances, the set of polypeptides include a dimerization switch that, upon the presence of a dimerization molecule, can couple the polypeptides to one another, e.g., can couple an antigen binding domain to an intracellular signaling domain. In one instance, the CARs of the present disclosure utilizes a dimerization switch as those described in, e.g., WO2014127261. Additional description and exemplary configurations of such regulatable CARs are provided herein and in International Publiciation No. WO 2015/090229.

In an aspect, an RCAR comprises two polypeptides or members: 1) an intracellular signaling member comprising an intracellular signaling domain, e.g., a primary intracellular signaling domain described herein, and a first switch domain; 2) an antigen binding member comprising an antigen binding domain, e.g., that binds specifically to CD19, as described herein and a second switch domain. Optionally, the RCAR comprises a transmembrane domain described herein. In an embodiment, a transmembrane domain can be disposed on the intracellular signaling member, on the antigen binding member, or on both. (Unless otherwise indicated, when members or elements of an RCAR are described herein, the order can be as provided, but other orders are included as well. In other words, in an embodiment, the order is as set out in the text, but in other embodiments, the order can be different. E.g., the order of elements on one side of a transmembrane region can be different from the example, e.g., the placement of a switch domain relative to a intracellular signaling domain can be different, e.g., reversed).

In an embodiment, the first and second switch domains can form an intracellular or an extracellular dimerization switch. In an embodiment, the dimerization switch can be a homodimerization switch, e.g., where the first and second switch domain are the same, or a heterodimerization switch, e.g., where the first and second switch domain are different from one another.

In embodiments, an RCAR can comprise a "multi switch." A multi switch can comprise heterodimerization switch domains or homodimerization switch domains. A multi switch comprises a plurality of, e.g., 2, 3, 4, 5, 6, 7, 8, 9, or 10, switch domains, independently, on a first member, e.g., an antigen binding member, and a second member, e.g., an intracellular signaling member. In an embodiment, the first member can comprise a plurality of first switch domains, e.g., FKBP-based switch domains, and the second member can comprise a plurality of second switch domains, e.g., FRB-based switch domains. In an embodiment, the first member can comprise a first and a second switch domain, e.g., a FKBP-based switch domain and a FRB-based switch domain, and the second member can comprise a first and a second switch domain, e.g., a FKBP-based switch domain and a FRB-based switch domain.

In an embodiment, the intracellular signaling member comprises one or more intracellular signaling domains, e.g., a primary intracellular signaling domain and one or more costimulatory signaling domains.

In an embodiment, the antigen binding member may comprise one or more intracellular signaling domains, e.g., one or more costimulatory signaling domains. In an embodiment, the antigen binding member comprises a plurality, e.g., 2 or 3 costimulatory signaling domains described herein, e.g., selected from 4-1BB, CD28, CD27, ICOS, and OX40, and in embodiments, no primary intracellular signaling domain. In an embodiment, the antigen binding member comprises the following costimulatory signaling domains, from the extracellular to intracellular direction: 4-1BB - CD27; 4-1BB - CD27; CD27 - 4-1BB; 4-1BB - CD28; CD28 - 4-1BB; OX40 - CD28; CD28 - OX40; CD28 - 4-1BB; or 4-1BB - CD28. In such embodiments, the intracellular binding member comprises a CD3zeta domain. In one such embodiment the RCAR comprises (1) an antigen binding member comprising, an antigen binding domain, a transmembrane domain, and two costimulatory domains and a first switch domain; and (2) an intracellular signaling domain comprising a transmembrane domain or membrane tethering domain and at least one primary intracellular signaling domain, and a second switch domain.

An instance provides RCARs wherein the antigen binding member is not tethered to the surface of the CAR cell. This allows a cell having an intracellular signaling member to be conveniently paired with one or more antigen binding domains, without transforming the cell with a sequence that encodes the antigen binding member. In such instances, the RCAR comprises: 1) an intracellular signaling member comprising: a first switch domain, a transmembrane domain, an intracellular signaling domain, e.g., a primary intracellular signaling domain, and a first switch domain; and 2) an antigen binding member comprising: an antigen binding domain, and a second switch domain, wherein the antigen binding member does not comprise a transmembrane domain or membrane tethering domain, and, optionally, does not comprise an intracellular signaling domain. In some instances, the RCAR may further comprise 3) a second antigen binding member comprising: a second antigen binding domain, e.g., a second antigen binding domain that binds a different antigen than is bound by the antigen binding domain; and a second switch domain.

Also provided herein are RCARs wherein the antigen binding member comprises bispecific activation and targeting capacity. In this instance, the antigen binding member can comprise a plurality, e.g., 2, 3, 4, or 5 antigen binding domains, e.g., scFvs, wherein each antigen binding domain binds to a target antigen, e.g. different antigens or the same antigen, e.g., the same or different epitopes on the same antigen. In an instance, the plurality of antigen binding domains are in tandem, and optionally, a linker or hinge region is disposed between each of the antigen binding domains. Suitable linkers and hinge regions are described herein.

An instance provides RCARs having a configuration that allows switching of proliferation. In this instance, the RCAR comprises: 1) an intracellular signaling member comprising: optionally, a transmembrane domain or membrane tethering domain; one or more co-stimulatory signaling domain, e.g., selected from 4-1BB, CD28, CD27, ICOS, and OX40, and a switch domain; and 2) an antigen binding member comprising: an antigen binding domain, a transmembrane domain, and a primary intracellular signaling domain, e.g., a CD3zeta domain, wherein the antigen binding member does not comprise a switch domain, or does not comprise a switch domain that dimerizes with a switch domain on the intracellular signaling member. In an instance, the antigen binding member does not comprise a co-stimulatory signaling domain. In an instance, the intracellular signaling member comprises a switch domain from a homodimerization switch. In an instance, the intracellular signaling member comprises a first switch domain of a heterodimerization switch and the RCAR comprises a second intracellular signaling member which comprises a second switch domain of the heterodimerization switch. In such instances, the second intracellular signaling member comprises the same intracellular signaling domains as the intracellular signaling member. In an einstance, the dimerization switch is intracellular. In an instance, the dimerization switch is extracellular.

In any of the RCAR configurations described here, the first and second switch domains comprise a FKBP-FRB based switch as described herein.

Also provided herein are cells comprising an RCAR described herein. Any cell that is engineered to express a RCAR can be used as a RCARX cell. In an instance the RCARX cell is a T cell, and is referred to as a RCART cell. In an instance the RCARX cell is an NK cell, and is referred to as a RCARN cell.

Also provided herein are nucleic acids and vectors comprising RCAR encoding sequences. Sequence encoding various elements of an RCAR can be disposed on the same nucleic acid molecule, e.g., the same plasmid or vector, e.g., viral vector, e.g., lentiviral vector. In aninstance, (i) sequence encoding an antigen binding member and (ii) sequence encoding an intracellular signaling member, can be present on the same nucleic acid, e.g., vector. Production of the corresponding proteins can be achieved, e.g., by the use of separate promoters, or by the use of a bicistronic transcription product (which can result in the production of two proteins by cleavage of a single translation product or by the translation of two separate protein products). In an instance, a sequence encoding a cleavable peptide, e.g., a P2A or F2A sequence, is disposed between (i) and (ii). In an instance, a sequence encoding an IRES, e.g., an EMCV or EV71 IRES, is disposed between (i) and (ii). In these instances, (i) and (ii) are transcribed as a single RNA. In an instance, a first promoter is operably linked to (i) and a second promoter is operably linked to (ii), such that (i) and (ii) are transcribed as separate mRNAs.

Alternatively, the sequence encoding various elements of an RCAR can be disposed on the different nucleic acid molecules, e.g., different plasmids or vectors, e.g., viral vector, e.g., lentiviral vector. E.g., the (i) sequence encoding an antigen binding member can be present on a first nucleic acid, e.g., a first vector, and the (ii) sequence encoding an intracellular signaling member can be present on the second nucleic acid, e.g., the second vector.

### Dimerization switches

Dimerization switches can be non-covalent or covalent. In a non-covalent dimerization switch, the dimerization molecule promotes a non-covalent interaction between the switch domains. In a covalent dimerization switch, the dimerization molecule promotes a covalent interaction between the switch domains.

In an embodiment, the RCAR comprises a FKBP/FRAP, or FKBP/FRB,-based dimerization switch. FKBP12 (FKBP, or FK506 binding protein) is an abundant cytoplasmic protein that serves as the initial intracellular target for the natural product immunosuppressive drug, rapamycin. Rapamycin binds to FKBP and to the large PI3K homolog FRAP (RAFT, mTOR). FRB is a 93 amino acid portion of FRAP, that is sufficient for binding the FKBP-rapamycin complex (Chen, J., Zheng, X. F., Brown, E. J. & Schreiber, S. L. (1995) Identification of an 11-kDa FKBP12-rapamycin-binding domain within the 289-kDa FKBP12-rapamycin-associated protein and characterization of a critical serine residue. Proc Natl Acad Sci U S A 92: 4947-51.)

In embodiments, an FKBP/FRAP, e.g., an FKBP/FRB, based switch can use a dimerization molecule, e.g., rapamycin or a rapamycin analog.

The amino acid sequence of FKBP is as follows:

In embodiments, an FKBP switch domain can comprise a fragment of FKBP having the ability to bind with FRB, or a fragment or analog thereof, in the presence of rapamycin or a rapalog, e.g., the underlined portion of SEQ ID NO: 90, which is:

The amino acid sequence of FRB is as follows:

"FKBP/FRAP, e.g., an FKBP/FRB, based switch" as that term is used herein, refers to a dimerization switch comprising: a first switch domain, which comprises an FKBP fragment or analog thereof having the ability to bind with FRB, or a fragment or analog thereof, in the presence of rapamycin or a rapalog, e.g., RAD001, and has at least 70, 75, 80, 85, 90, 95, 96, 97, 98, or 99% identity with, or differs by no more than 30, 25, 20, 15, 10, 5, 4, 3, 2, or 1 amino acid residues from, the FKBP sequence of SEQ ID NO: 90 or 91; and a second switch domain, which comprises an FRB fragment or analog thereof having the ability to bind with FRB, or a fragment or analog thereof, in the presence of rapamycin or a rapalog, and has at least 70, 75, 80, 85, 90, 95, 96, 97, 98, or 99% identity with, or differs by no more than 30, 25, 20, 15, 10, 5, 4, 3, 2, or 1 amino acid residues from, the FRB sequence of SEQ ID NO: 92. In an instance, a RCAR described herein comprises one switch domain comprises amino acid residues disclosed in SEQ ID NO: 90 (or SEQ ID NO: 91), and one switch domain comprises amino acid residues disclosed in SEQ ID NO: 92.

In embodiments, the FKBP/FRB dimerization switch comprises a modified FRB switch domain that exhibits altered, e.g., enhanced, complex formation between an FRB-based switch domain, e.g., the modified FRB switch domain, a FKBP-based switch domain, and the dimerization molecule, e.g., rapamycin or a rapalogue, e.g., RAD001. In an embodiment, the modified FRB switch domain comprises one or more mutations, e.g., 2, 3, 4, 5, 6, 7, 8, 9, 10 or more, selected from mutations at amino acid position(s) L2031, E2032, S2035, R2036, F2039, G2040, T2098, W2101, D2102, Y2105, and F2108, where the wild-type amino acid is mutated to any other naturally-occurring amino acid. In an embodiment, a mutant FRB comprises a mutation at E2032, where E2032 is mutated to phenylalanine (E2032F), methionine (E2032M), arginine (E2032R), valine (E2032V), tyrosine (E2032Y), isoleucine (E2032I), e.g., SEQ ID NO: 93, or leucine (E2032L), e.g., SEQ ID NO: 94. In an embodiment, a mutant FRB comprises a mutation at T2098, where T2098 is mutated to phenylalanine (T2098F) or leucine (T2098L), e.g., SEQ ID NO: 95. In an embodiment, a mutant FRB comprises a mutation at E2032 and at T2098, where E2032 is mutated to any amino acid, and where T2098 is mutated to any amino acid, e.g., SEQ ID NO: 96. In an embodiment, a mutant FRB comprises an E2032I and a T2098L mutation, e.g., SEQ ID NO: 97. In an embodiment, a mutant FRB comprises an E2032L and a T2098L mutation, e.g., SEQ ID NO: 98.

**Table 10. Exemplary mutant FRB having increased affinity for a dimerization molecule.**

| **FRB mutant** | **Amino Acid Sequence** | **SEQ ID NO:** |
|---|---|---|
| E2032I mutant | | 93 |
| E2032L mutant | | 94 |
| T2098L mutant | | 95 |
| E2032, T2098 mutant | | 96 |
| E2032I, T2098L mutant | | 97 |
| E2032L, T2098L mutant | | 98 |

Other suitable dimerization switches include a GyrB-GyrB based dimerization switch, a Gibberellin-based dimerization switch, a tag/binder dimerization switch, and a halo-tag/snap-tag dimerization switch. Following the guidance provided herein, such switches and relevant dimerization molecules will be apparent to one of ordinary skill.

### Dimerization molecule

Association between the switch domains is promoted by the dimerization molecule. In the presence of dimerization molecule interaction or association between switch domains allows for signal transduction between a polypeptide associated with, e.g., fused to, a first switch domain, and a polypeptide associated with, e.g., fused to, a second switch domain. In the presence of non-limiting levels of dimerization molecule signal transduction is increased by 1.1, 1.2, 1.3, 1.4, 1.5, 1.6, 1.7, 1.8, 1.9, 2, 5, 10, 50, 100 fold, e.g., as measured in a system described herein.

Rapamycin and rapamycin analogs (sometimes referred to as rapalogues), e.g., RAD001, can be used as dimerization molecules in a FKBP/FRB-based dimerization switch described herein. In an instance the dimerization molecule can be selected from rapamycin (sirolimus), RAD001 (everolimus), zotarolimus, temsirolimus, AP-23573 (ridaforolimus), biolimus and AP21967. Additional rapamycin analogs suitable for use with FKBP/FRB-based dimerization switches are further described in the section entitled "Combination Therapies", or in the subsection entitled "mTOR inhibitors".

### SPLIT CAR

In some embodiments, the CAR-expressing cell uses a split CAR. The split CAR approach is described in more detail in publications WO2014/055442 and WO2014/055657. Briefly, a split CAR system comprises a cell expressing a first CAR having a first antigen binding domain and a costimulatory domain (e.g., 4-1BB), and the cell also expresses a second CAR having a second antigen binding domain and an intracellular signaling domain (e.g., CD3 zeta). When the cell encounters the first antigen, the costimulatory domain is activated, and the cell proliferates. When the cell encounters the second antigen, the intracellular signaling domain is activated and cell-killing activity begins. Thus, the CAR-expressing cell is only fully activated in the presence of both antigens. In embodiments, the first antigen binding domain recognizes a cancer associated antigen described herein (e.g., CD19, CD123, CD22, CD30, CD34, CD171, CS-1, CLL-1, CD33, EGFRvIII, GD2, GD3, BCMA, Tn Ag, PSMA, ROR1, FLT3, FAP, TAG72, CD38, CD44v6, CEA, EPCAM, B7H3, KIT, IL-13Ra2, Mesothelin, IL-11Ra, PSCA, VEGFR2, LewisY, CD24, PDGFR-beta, SSEA-4, CD20, Folate receptor alpha, ERBB2 (Her2/neu), MUC1, EGFR, NCAM, Prostase, PAP, ELF2M, Ephrin B2, IGF-I receptor, CAIX, LMP2, gp100, bcr-abl, tyrosinase, EphA2, Fucosyl GM1, sLe, GM3, TGS5, HMWMAA, o-acetyl-GD2, Folate receptor beta, TEM1/CD248, TEM7R, CLDN6, TSHR, GPRC5D, CXORF61, CD97, CD179a, ALK, Plysialic acid, PLAC1, GloboH, NY-BR-1, UPK2, HAVCR1, ADRB3, PANX3, GPR20, LY6K, OR51E2, TARP, WT1, NY-ESO-1, LAGE-1a, legumain, HPV E6,E7, MAGE-A1, MAGE A1, ETV6-AML, sperm protein 17, XAGE1, Tie 2, MAD-CT-1, MAD-CT-2, Fos-related antigen 1, p53, p53 mutant, prostein, survivin and telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, intestinal carboxyl esterase, mut hsp70-2, CD79a, CD79b, CD72, LAIR1, FCAR, LILRA2, CD300LF, CLEC12A, BST2, EMR2, LY75, GPC3, FCRL5, or IGLL1).

### NUCLEIC ACID CONSTRUCTS ENCODING A CAR

The present disclosure also provides nucleic acid molecules encoding one or more CAR constructs described herein. In one aspect, the nucleic acid molecule is provided as a messenger RNA transcript. In one aspect, the nucleic acid molecule is provided as a DNA construct.

Accordingly, in one aspect, the present disclosure pertains to a nucleic acid molecule encoding a chimeric antigen receptor (CAR), wherein the CAR comprises an antigen binding domain that binds to a tumor antigen described herein, a transmembrane domain (e.g., a transmembrane domain described herein), and an intracellular signaling domain (e.g., an intracellular signaling domain described herein) comprising a stimulatory domain, e.g., a costimulatory signaling domain (e.g., a costimulatory signaling domain described herein) and/or a primary signaling domain (e.g., a primary signaling domain described herein, e.g., a zeta chain described herein). In one instance, the transmembrane domain is transmembrane domain of a protein selected from the group consisting of the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137 and CD154. In some instances, a transmembrane domain may include at least the transmembrane region(s) of, e.g., KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp.

In one instance, the transmembrane domain is a transmembrane domain of a protein selected from the group consisting of the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137 and CD154. In some instances, a transmembrane domain may include at least the transmembrane region(s) of, e.g., KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), NKp44, NKp30, NKp46, CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp. In one instance, the transmembrane domain comprises a sequence that encodes SEQ ID NO: 12 or SEQ ID NO: 42, or a sequence with 95-99% identity thereof.

In one instance, the antigen binding domain is connected to the transmembrane domain by a hinge region, e.g., a hinge described herein. In one instance, the hinge region comprises a sequence that encodes SEQ ID NO:4 or SEQ ID NO:6 or SEQ ID NO:8 or SEQ ID NO:10, or a sequence with 95-99% identity thereof.

In one instance, the isolated nucleic acid molecule further comprises a sequence encoding a costimulatory domain. In one instance, the costimulatory domain is a functional signaling domain of a protein selected from the group consisting of MHC class I molecule, TNF receptor proteins, Immunoglobulin-like proteins, cytokine receptors, integrins, signaling lymphocytic activation molecules (SLAM proteins), activating NK cell receptors, BTLA, a Toll ligand receptor, OX40, CD2, CD7, CD27, CD28, CD30, CD40, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, and a ligand that specifically binds with CD83. In instances, the costimulatory domain comprises 4-1BB, CD27, CD28, or ICOS.

In one instance, the costimulatory domain comprises a sequence that encodes SEQ ID NO:16, or a sequence with 95-99% identity thereof. In one instance, the intracellular signaling domain comprises a functional signaling domain of 4-1BB and a functional signaling domain of CD3 zeta. In one instance, the intracellular signaling domain comprises a sequence that encodes SEQ ID NO: 14, SEQ ID NO: 16, SEQ ID NO: 40 or SEQ ID NO: 44, or a sequence with 95-99% identity thereof, and a sequence that encodes SEQ ID NO: 18 or SEQ ID NO:20, or a sequence with 95-99% identity thereof, wherein the sequences comprising the intracellular signaling domain are expressed in the same frame and as a single polypeptide chain.

In another aspect, the present disclosure pertains to an isolated nucleic acid molecule encoding a CAR construct comprising a leader sequence of SEQ ID NO: 3, a scFv domain as described herein, a hinge region of SEQ ID NO:5 or SEQ ID NO:7 or SEQ ID NO:9 or SEQ ID NO: 11 (or a sequence with 95-99% identity thereof), a transmembrane domain having a sequence of SEQ ID NO: 13 or SEQ ID NO: 43 (or a sequence with 95-99% identity thereof), a 4-1BB costimulatory domain having a sequence of SEQ ID NO:15 or a CD27 costimulatory domain having a sequence of SEQ ID NO:17 or a ICOS costimulatory domain having a sequence of SEQ ID NO: 41 or 45 (or a sequence with 95-99% identity thereof), and a CD3 zeta stimulatory domain having a sequence of SEQ ID NO: 19 (mutant CD3 zeta) or SEQ ID NO: 21 (wild-type human CD3 zeta) or a sequence with 95-99% identity thereof.

In another aspect, the present disclosure pertains to a nucleic acid molecule encoding a chimeric antigen receptor (CAR) molecule that comprises an antigen binding domain, a transmembrane domain, and an intracellular signaling domain comprising a stimulatory domain, and wherein said antigen binding domain binds to a tumor antigen selected from a group consisting of: CD19, CD123, CD22, CD30, CD171, CS-1, CLL-1, CD33, EGFRvIII, GD2, GD3, BCMA, Tn Ag, PSMA, ROR1, FLT3, FAP, TAG72, CD38, CD44v6, CEA, EPCAM, B7H3, KIT, IL-13Ra2, Mesothelin, IL-11Ra, PSCA, VEGFR2, LewisY, CD24, PDGFR-beta, SSEA-4, CD20, Folate receptor alpha, ERBB2 (Her2/neu), MUC1, EGFR, NCAM, Prostase, PRSS21, PAP, ELF2M, Ephrin B2, IGF-I receptor, CAIX, LMP2, gp100, bcr-abl, tyrosinase, EphA2, Fucosyl GM1, sLe, GM3, TGS5, HMWMAA, o-acetyl-GD2, Folate receptor beta, TEM1/CD248, TEM7R, CLDN6, TSHR, GPRC5D, CXORF61, CD97, CD179a, ALK, Plysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6,E7, MAGE A1, ETV6-AML, sperm protein 17, XAGE1, Tie 2, MAD-CT-1, MAD-CT-2, Fos-related antigen 1, p53, p53 mutant, prostein, survivin and telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, intestinal carboxyl esterase, mut hsp70-2, CD79a, CD79b, CD72, LAIR1, FCAR, LILRA2, CD300LF, CLEC12A, BST2, EMR2, LY75, GPC3, FCRL5, and IGLL1.

The nucleic acid sequences coding for the desired molecules can be obtained using recombinant methods known in the art, such as, for example by screening libraries from cells expressing the gene, by deriving the gene from a vector known to include the same, or by isolating directly from cells and tissues containing the same, using standard techniques. Alternatively, the gene of interest can be produced synthetically, rather than cloned.

### VECTORS ENCODING A CAR

The present disclosure also provides vectors in which a DNA of the present disclosure is inserted. Vectors derived from retroviruses such as the lentivirus are suitable tools to achieve long-term gene transfer since they allow long-term, stable integration of a transgene and its propagation in daughter cells. Lentiviral vectors have the added advantage over vectors derived from onco-retroviruses such as murine leukemia viruses in that they can transduce non-proliferating cells, such as hepatocytes. They also have the added advantage of low immunogenicity. A retroviral vector may also be, e.g., a gammaretroviral vector. A gammaretroviral vector may include, e.g., a promoter, a packaging signal (ψ), a primer binding site (PBS), one or more (e.g., two) long terminal repeats (LTR), and a transgene of interest, e.g., a gene encoding a CAR. A gammaretroviral vector may lack viral structural gens such as gag, pol, and env. Exemplary gammaretroviral vectors include Murine Leukemia Virus (MLV), Spleen-Focus Forming Virus (SFFV), and Myeloproliferative Sarcoma Virus (MPSV), and vectors derived therefrom. Other gammaretroviral vectors are described, e.g., in Tobias Maetzig et al., "Gammaretroviral Vectors: Biology, Technology and Application" Viruses. 2011 Jun; 3(6): 677-713.

In another instance, the vector comprising the nucleic acid encoding the desired CAR of the disclosure is an adenoviral vector (A5/35). In another instance, the expression of nucleic acids encoding CARs can be accomplished using of transposons such as sleeping beauty, crisper, CAS9, and zinc finger nucleases. See below June et al. 2009 Nature Reviews Immunology 9.10: 704-716.

In brief summary, the expression of natural or synthetic nucleic acids encoding CARs is typically achieved by operably linking a nucleic acid encoding the CAR polypeptide or portions thereof to a promoter, and incorporating the construct into an expression vector. The vectors can be suitable for replication and integration eukaryotes. Typical cloning vectors contain transcription and translation terminators, initiation sequences, and promoters useful for regulation of the expression of the desired nucleic acid sequence.

The expression constructs of the present disclosure may also be used for nucleic acid immunization and gene therapy, using standard gene delivery protocols. Methods for gene delivery are known in the art. See, e.g., U.S. Pat. Nos. 5,399,346, 5,580,859, 5,589,466. In another instance, the present disclosure provides a gene therapy vector.

The nucleic acid can be cloned into a number of types of vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art and is described, for example, in Sambrook et al., 2012, MOLECULAR CLONING: A LABORATORY MANUAL, volumes 1 -4, Cold Spring Harbor Press, NY), and in other virology and molecular biology manuals. Viruses, which are useful as vectors include, but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers, (e.g., WO 01/96584; WO 01/29058; and U.S. Pat. No. 6,326,193).

A number of viral based systems have been developed for gene transfer into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either in vivo or ex vivo. A number of retroviral systems are known in the art. In some embodiments, adenovirus vectors are used. A number of adenovirus vectors are known in the art. In one embodiment, lentivirus vectors are used. Exemplary promoters include the CMV IE gene, EF-1α, ubiquitin C, or phosphoglycerokinase (PGK) promoters. In an embodiment, the promoter is a PGK promoter, e.g., a truncated PGK promoter as described herein.

Additional promoter elements, e.g., enhancers, regulate the frequency of transcriptional initiation. Typically, these are located in the region 30-110 bp upstream of the start site, although a number of promoters have been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline. Depending on the promoter, it appears that individual elements can function either cooperatively or independently to activate transcription.

An example of a promoter that is capable of expressing a CAR encoding nucleic acid molecule in a mammalian T cell is the EF1a promoter. The native EF1a promoter drives expression of the alpha subunit of the elongation factor-1 complex, which is responsible for the enzymatic delivery of aminoacyl tRNAs to the ribosome. The EF1a promoter has been extensively used in mammalian expression plasmids and has been shown to be effective in driving CAR expression from nucleic acid molecules cloned into a lentiviral vector. See, e.g., Milone et al., Mol. Ther. 17(8): 1453-1464 (2009). In one aspect, the EF1a promoter comprises the sequence provided as SEQ ID NO:1.

Another example of a promoter is the immediate early cytomegalovirus (CMV) promoter sequence. This promoter sequence is a strong constitutive promoter sequence capable of driving high levels of expression of any polynucleotide sequence operatively linked thereto. However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the elongation factor-1α, promoter, the hemoglobin promoter, and the creatine kinase promoter. Further, the invention should not be limited to the use of constitutive promoters. Inducible promoters are also contemplated as part of the invention. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter.

Another example of a promoter is the phosphoglycerate kinase (PGK) promoter. In embodiments, a truncated PGK promoter (e.g., a PGK promoter with one or more, e.g., 1, 2, 5, 10, 100, 200, 300, or 400, nucleotide deletions when compared to the wild-type PGK promoter sequence) may be desired. The nucleotide sequences of exemplary PGK promoters are provided below.

WT PGK Promoter:

Exemplary truncated PGK Promoters:
PGK100:
PGK200:
PGK300:
PGK400:

A vector may also include, e.g., a signal sequence to facilitate secretion, a polyadenylation signal and transcription terminator (e.g., from Bovine Growth Hormone (BGH) gene), an element allowing episomal replication and replication in prokaryotes (e.g. SV40 origin and ColE1 or others known in the art) and/or elements to allow selection (e.g., ampicillin resistance gene and/or zeocin marker).

In order to assess the expression of a CAR polypeptide or portions thereof, the expression vector to be introduced into a cell can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co- transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected or transduced cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene (e.g., Ui-Tei et al., 2000 FEBS Letters 479: 79-82). Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter-driven transcription.

In instances, the vector may comprise two or more nucleic acid sequences encoding a CAR, e.g., a first CAR described herein and a second CAR, e.g., an inhibitory CAR or a CAR that specifically binds to a second antigen, e.g., another cancer associated antigen described herein (e.g., CD19, CD123, CD22, CD30, CD34, CD171, CS-1, CLL-1, CD33, EGFRvIII, GD2, GD3, BCMA, Tn Ag, PSMA, ROR1, FLT3, FAP, TAG72, CD38, CD44v6, CEA, EPCAM, B7H3, KIT, IL-13Ra2, Mesothelin, IL-11Ra, PSCA, VEGFR2, LewisY, CD24, PDGFR-beta, SSEA-4, CD20, Folate receptor alpha, ERBB2 (Her2/neu), MUC1, EGFR, NCAM, Prostase, PAP, ELF2M, Ephrin B2, IGF-I receptor, CAIX, LMP2, gp100, bcr-abl, tyrosinase, EphA2, Fucosyl GM1, sLe, GM3, TGS5, HMWMAA, o-acetyl-GD2, Folate receptor beta, TEM1/CD248, TEM7R, CLDN6, TSHR, GPRC5D, CXORF61, CD97, CD179a, ALK, Plysialic acid, PLAC1, GloboH, NY-BR-1, UPK2, HAVCR1, ADRB3, PANX3, GPR20, LY6K, OR51E2, TARP, WT1, NY-ESO-1, LAGE-1a, legumain, HPV E6,E7, MAGE-A1, MAGE A1, ETV6-AML, sperm protein 17, XAGE1, Tie 2, MAD-CT-1, MAD-CT-2, Fos-related antigen 1, p53, p53 mutant, prostein, survivin and telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, intestinal carboxyl esterase, mut hsp70-2, CD79a, CD79b, CD72, LAIR1, FCAR, LILRA2, CD300LF, CLEC12A, BST2, EMR2, LY75, GPC3, FCRL5, or IGLL1). In such instances, the two or more nucleic acid sequences encoding the CAR are encoded by a single nucleic molecule in the same frame and as a single polypeptide chain. In this aspect, the two or more CARs, can, e.g., be separated by one or more peptide cleavage sites. (e.g., an auto-cleavage site or a substrate for an intracellular protease). Examples of peptide cleavage sites include the following, wherein the GSG residues are optional:

| | |
|---|---|
| T2A: | (GSG)EGRGSLLTCGDVEENPGP (SEQ ID NO: 104) |
| P2A: | (GSG)ATNFSLLKQAGDVEENPGP (SEQ ID NO: 105) |
| E2A: | (GSG)QCTNYALLKLAGDVESNPGP (SEQ ID NO: 106) |
| F2A: | (GSG)VKQTLNFDLLKLAGDVESNPGP (SEQ ID NO: 107) |

Methods of introducing and expressing genes into a cell are known in the art. In the context of an expression vector, the vector can be readily introduced into a host cell, e.g., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al., 2012, MOLECULAR CLONING: A LABORATORY MANUAL, volumes 1 -4, Cold Spring Harbor Press, NY). A preferred method for the introduction of a polynucleotide into a host cell is calcium phosphate transfection

Biological methods for introducing a polynucleotide of interest into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like. See, for example, U.S. Pat. Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle in vitro and in vivo is a liposome (e.g. , an artificial membrane vesicle). Other methods of state-of-the-art targeted delivery of nucleic acids are available, such as delivery of polynucleotides with targeted nanoparticles or other suitable sub-micron sized delivery system.

In the case where a non-viral delivery system is utilized, an exemplary delivery vehicle is a liposome. The use of lipid formulations is contemplated for the introduction of the nucleic acids into a host cell (in vitro, ex vivo or in vivo). In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

Lipids suitable for use can be obtained from commercial sources. For example, dimyristyl phosphatidylcholine ("DMPC") can be obtained from Sigma, St. Louis, MO; dicetyl phosphate ("DCP") can be obtained from K & K Laboratories (Plainview, NY); cholesterol ("Choi") can be obtained from Calbiochem-Behring; dimyristyl phosphatidylglycerol ("DMPG") and other lipids may be obtained from Avanti Polar Lipids, Inc. (Birmingham, AL.). Stock solutions of lipids in chloroform or chloroform/methanol can be stored at about - 20°C. Chloroform is used as the only solvent since it is more readily evaporated than methanol. "Liposome" is a generic term encompassing a variety of single and multilamellar lipid vehicles formed by the generation of enclosed lipid bilayers or aggregates. Liposomes can be characterized as having vesicular structures with a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh et al., 1991 Glycobiology 5: 505-10). However, compositions that have different structures in solution than the normal vesicular structure are also encompassed. For example, the lipids may assume a micellar structure or merely exist as nonuniform aggregates of lipid molecules. Also contemplated are lipofectamine-nucleic acid complexes.

Regardless of the method used to introduce exogenous nucleic acids into a host cell or otherwise expose a cell to the inhibitor of the present disclosure, in order to confirm the presence of the recombinant DNA or RNA sequence in the host cell, a variety of assays may be performed. Such assays include, for example, "molecular biological" assays well known to those of skill in the art, such as Southern and Northern blotting, RT-PCR and PCR; "biochemical" assays, such as detecting the presence or absence of a particular peptide, e.g., by immunological means (ELISAs and Western blots) or by assays described herein to identify agents falling within the scope of the disclosure.

The present disclosure further provides a vector comprising a CAR encoding nucleic acid molecule. In one aspect, a CAR vector can be directly transduced into a cell, *e*.*g*., a T cell. In one aspect, the vector is a cloning or expression vector, *e*.*g*., a vector including, but not limited to, one or more plasmids (*e*.*g*., expression plasmids, cloning vectors, minicircles, minivectors, double minute chromosomes), retroviral and lentiviral vector constructs. In one aspect, the vector is capable of expressing the CAR construct in T cells, e.g., CD4⁺ T cells or CD8⁺ T cells. In one aspect, the mammalian T cell is a human T cell.

### RNAs ENCODING CARS; RNA TRANSFECTION

Also disclosed herein are methods for producing an in vitro transcribed RNA CAR. The present disclosure includes a CAR encoding RNA construct that can be directly transfected into a cell. A method for generating mRNA for use in transfection can involve in vitro transcription (IVT) of a template with specially designed primers, followed by polyA addition, to produce a construct containing 3' and 5' untranslated sequence ("UTR"), a 5' cap and/or Internal Ribosome Entry Site (IRES), the nucleic acid to be expressed, and a polyA tail, typically 50-2000 bases in length (SEQ ID NO:32). RNA so produced can efficiently transfect different kinds of cells. In one aspect, the template includes sequences for the CAR.

In one aspect, a CAR of the present diclosure is encoded by a messenger RNA (mRNA). In one aspect, the mRNA encoding a CAR described herein is introduced into a T cell for production of a CART cell.

In one instance, the *in vitro* transcribed RNA CAR can be introduced to a cell as a form of transient transfection. The RNA is produced by *in vitro* transcription using a polymerase chain reaction (PCR)-generated template. DNA of interest from any source can be directly converted by PCR into a template for in vitro mRNA synthesis using appropriate primers and RNA polymerase. The source of the DNA can be, for example, genomic DNA, plasmid DNA, phage DNA, cDNA, synthetic DNA sequence or any other appropriate source of DNA. The desired temple for *in vitro* transcription is a CAR described herein. For example, the template for the RNA CAR comprises an extracellular region comprising a single chain variable domain of an antibody to a tumor associated antigen described herein; a hinge region (e.g., a hinge region described herein), a transmembrane domain (e.g., a transmembrane domain described herein such as a transmembrane domain of CD8a); and a cytoplasmic region that includes an intracellular signaling domain, e.g., an intracellular signaling domain described herein, e.g., comprising the signaling domain of CD3-zeta and the signaling domain of 4-1BB.

In one instance, the DNA to be used for PCR contains an open reading frame. The DNA can be from a naturally occurring DNA sequence from the genome of an organism. In one instance, the nucleic acid can include some or all of the 5' and/or 3' untranslated regions (UTRs). The nucleic acid can include exons and introns. In one instance, the DNA to be used for PCR is a human nucleic acid sequence. In another instance, the DNA to be used for PCR is a human nucleic acid sequence including the 5' and 3' UTRs. The DNA can alternatively be an artificial DNA sequence that is not normally expressed in a naturally occurring organism. An exemplary artificial DNA sequence is one that contains portions of genes that are ligated together to form an open reading frame that encodes a fusion protein. The portions of DNA that are ligated together can be from a single organism or from more than one organism.

PCR is used to generate a template for in vitro transcription of mRNA which is used for transfection. Methods for performing PCR are well known in the art. Primers for use in PCR are designed to have regions that are substantially complementary to regions of the DNA to be used as a template for the PCR. "Substantially complementary," as used herein, refers to sequences of nucleotides where a majority or all of the bases in the primer sequence are complementary, or one or more bases are non-complementary, or mismatched. Substantially complementary sequences are able to anneal or hybridize with the intended DNA target under annealing conditions used for PCR. The primers can be designed to be substantially complementary to any portion of the DNA template. For example, the primers can be designed to amplify the portion of a nucleic acid that is normally transcribed in cells (the open reading frame), including 5' and 3' UTRs. The primers can also be designed to amplify a portion of a nucleic acid that encodes a particular domain of interest. In one instance, the primers are designed to amplify the coding region of a human cDNA, including all or portions of the 5' and 3' UTRs. Primers useful for PCR can be generated by synthetic methods that are well known in the art. "Forward primers" are primers that contain a region of nucleotides that are substantially complementary to nucleotides on the DNA template that are upstream of the DNA sequence that is to be amplified. "Upstream" is used herein to refer to a location 5, to the DNA sequence to be amplified relative to the coding strand. "Reverse primers" are primers that contain a region of nucleotides that are substantially complementary to a double-stranded DNA template that are downstream of the DNA sequence that is to be amplified. "Downstream" is used herein to refer to a location 3' to the DNA sequence to be amplified relative to the coding strand.

Any DNA polymerase useful for PCR can be used in the methods disclosed herein. The reagents and polymerase are commercially available from a number of sources.

Chemical structures with the ability to promote stability and/or translation efficiency may also be used. The RNA preferably has 5' and 3' UTRs. In one embodiment, the 5' UTR is between one and 3000 nucleotides in length. The length of 5' and 3' UTR sequences to be added to the coding region can be altered by different methods, including, but not limited to, designing primers for PCR that anneal to different regions of the UTRs. Using this approach, one of ordinary skill in the art can modify the 5' and 3' UTR lengths required to achieve optimal translation efficiency following transfection of the transcribed RNA.

The 5' and 3' UTRs can be the naturally occurring, endogenous 5' and 3' UTRs for the nucleic acid of interest. Alternatively, UTR sequences that are not endogenous to the nucleic acid of interest can be added by incorporating the UTR sequences into the forward and reverse primers or by any other modifications of the template. The use of UTR sequences that are not endogenous to the nucleic acid of interest can be useful for modifying the stability and/or translation efficiency of the RNA. For example, it is known that AU-rich elements in 3' UTR sequences can decrease the stability of mRNA. Therefore, 3' UTRs can be selected or designed to increase the stability of the transcribed RNA based on properties of UTRs that are well known in the art.

In one embodiment, the 5' UTR can contain the Kozak sequence of the endogenous nucleic acid. Alternatively, when a 5' UTR that is not endogenous to the nucleic acid of interest is being added by PCR as described above, a consensus Kozak sequence can be redesigned by adding the 5' UTR sequence. Kozak sequences can increase the efficiency of translation of some RNA transcripts, but does not appear to be required for all RNAs to enable efficient translation. The requirement for Kozak sequences for many mRNAs is known in the art. In other embodiments the 5' UTR can be 5'UTR of an RNA virus whose RNA genome is stable in cells. In other embodiments various nucleotide analogues can be used in the 3' or 5' UTR to impede exonuclease degradation of the mRNA.

To enable synthesis of RNA from a DNA template without the need for gene cloning, a promoter of transcription should be attached to the DNA template upstream of the sequence to be transcribed. When a sequence that functions as a promoter for an RNA polymerase is added to the 5' end of the forward primer, the RNA polymerase promoter becomes incorporated into the PCR product upstream of the open reading frame that is to be transcribed. In one preferred embodiment, the promoter is a T7 polymerase promoter, as described elsewhere herein. Other useful promoters include, but are not limited to, T3 and SP6 RNA polymerase promoters. Consensus nucleotide sequences for T7, T3 and SP6 promoters are known in the art.

In a preferred embodiment, the mRNA has both a cap on the 5' end and a 3' poly(A) tail which determine ribosome binding, initiation of translation and stability mRNA in the cell. On a circular DNA template, for instance, plasmid DNA, RNA polymerase produces a long concatameric product which is not suitable for expression in eukaryotic cells. The transcription of plasmid DNA linearized at the end of the 3' UTR results in normal sized mRNA which is not effective in eukaryotic transfection even if it is polyadenylated after transcription.

On a linear DNA template, phage T7 RNA polymerase can extend the 3' end of the transcript beyond the last base of the template (Schenborn and Mierendorf, Nuc Acids Res., 13:6223-36 (1985); Nacheva and Berzal-Herranz, Eur. J. Biochem., 270:1485-65 (2003).

The conventional method of integration of polyA/T stretches into a DNA template is molecular cloning. However polyA/T sequence integrated into plasmid DNA can cause plasmid instability, which is why plasmid DNA templates obtained from bacterial cells are often highly contaminated with deletions and other aberrations. This makes cloning procedures not only laborious and time consuming but often not reliable. That is why a method which allows construction of DNA templates with polyA/T 3' stretch without cloning highly desirable.

The polyA/T segment of the transcriptional DNA template can be produced during PCR by using a reverse primer containing a polyT tail, such as 100T tail (SEQ ID NO: 35) or after PCR by any other method, including, but not limited to, DNA ligation or in vitro recombination. In one embodiment, the poly(T) tail is from 50 to 5000 nucleotides in length (SEQ ID NO: 87). Poly(A) tails also provide stability to RNAs and reduce their degradation. Generally, the length of a poly(A) tail positively correlates with the stability of the transcribed RNA. In one embodiment, the poly(A) tail is between 100 and 5000 nucleotides in length (SEQ ID NO: 88).

Poly(A) tails of RNAs can be further extended following in vitro transcription with the use of a poly(A) polymerase, such as E. coli polyA polymerase (E-PAP). In one embodiment, increasing the length of a poly(A) tail from 100 nucleotides to between 300 and 400 nucleotides (SEQ ID NO: 38) results in about a two-fold increase in the translation efficiency of the RNA. Additionally, the attachment of different chemical groups to the 3' end can increase mRNA stability. Such attachment can contain modified/artificial nucleotides, aptamers and other compounds. For example, ATP analogs can be incorporated into the poly(A) tail using poly(A) polymerase. ATP analogs can further increase the stability of the RNA.

5' caps on also provide stability to RNA molecules. In a preferred embodiment, RNAs produced by the methods disclosed herein include a 5' cap. The 5' cap is provided using techniques known in the art and described herein (Cougot, et al., Trends in Biochem. Sci., 29:436-444 (2001); Stepinski, et al., RNA, 7:1468-95 (2001); Elango, et al., Biochim. Biophys. Res. Commun., 330:958-966 (2005)).

The RNAs produced by the methods disclosed herein can also contain an internal ribosome entry site (IRES) sequence. The IRES sequence may be any viral, chromosomal or artificially designed sequence which initiates cap-independent ribosome binding to mRNA and facilitates the initiation of translation. Any solutes suitable for cell electroporation, which can contain factors facilitating cellular permeability and viability such as sugars, peptides, lipids, proteins, antioxidants, and surfactants can be included.

There are several advantages of RNA transfection methods of the present disclosure. For example, RNA transfection is essentially transient and a vector-free: An RNA transgene can be delivered to a lymphocyte and expressed therein following a brief in vitro cell activation, as a minimal expressing cassette without the need for any additional viral sequences. Under these conditions, integration of the transgene into the host cell genome is unlikely. Cloning of cells is not necessary because of the efficiency of transfection of the RNA and its ability to uniformly modify the entire lymphocyte population. Furthermore, gene expression from an RNA source does not require transcription and the protein product is produced rapidly after the transfection. Since the RNA has to only gain access to the cytoplasm, rather than the nucleus, typical transfection methods result in an extremely high rate of transfection.

RNA can be introduced into target cells using any of a number of different methods, for instance, commercially available methods which include, but are not limited to, electroporation (Amaxa Nucleofector-II (Amaxa Biosystems, Cologne, Germany)), (ECM 830 (BTX) (Harvard Instruments, Boston, Mass.) or the Gene Pulser II (BioRad, Denver, Colo.), Multiporator (Eppendort, Hamburg Germany), cationic liposome mediated transfection using lipofection, polymer encapsulation, peptide mediated transfection, or biolistic particle delivery systems such as "gene guns" (see, for example, Nishikawa, et al. Hum Gene Ther., 12(8):861-70 (2001).

### NON-VIRAL DELIVERY METHODS

In some aspects, non-viral methods can be used to deliver a nucleic acid encoding a CAR described herein into a cell or tissue or a subject.

In some instances, the non-viral method includes the use of a transposon (also called a transposable element). In some instances, a transposon is a piece of DNA that can insert itself at a location in a genome, for example, a piece of DNA that is capable of self-replicating and inserting its copy into a genome, or a piece of DNA that can be spliced out of a longer nucleic acid and inserted into another place in a genome. For example, a transposon comprises a DNA sequence made up of inverted repeats flanking genes for transposition.

Exemplary methods of nucleic acid delivery using a transposon include a Sleeping Beauty transposon system (SBTS) and a piggyBac (PB) transposon system. See, e.g., Aronovich et al. Hum. Mol. Genet. 20.R1(2011):R14-20; Singh et al. Cancer Res. 15(2008):2961-2971; Huang et al. Mol. Ther. 16(2008):580-589; Grabundzija et al. Mol. Ther. 18(2010):1200-1209; Kebriaei et al. Blood. 122.21(2013):166; Williams. Molecular Therapy 16.9(2008):1515-16; Bell et al. Nat. Protoc. 2.12(2007):3153-65; and Ding et al. Cell. 122.3(2005):473-83.

The SBTS includes two components: 1) a transposon containing a transgene and 2) a source of transposase enzyme. The transposase can transpose the transposon from a carrier plasmid (or other donor DNA) to a target DNA, such as a host cell chromosome/genome. For example, the transposase binds to the carrier plasmid/donor DNA, cuts the transposon (including transgene(s)) out of the plasmid, and inserts it into the genome of the host cell. See, e.g., Aronovich et al. supra.

Exemplary transposons include a pT2-based transposon. See, e.g., Grabundzija et al. Nucleic Acids Res. 41.3(2013):1829-47; and Singh et al. Cancer Res. 68.8(2008): 2961-2971. Exemplary transposases include a Tc1/mariner-type transposase, e.g., the SB10 transposase or the SB11 transposase (a hyperactive transposase which can be expressed, e.g., from a cytomegalovirus promoter). See, e.g., Aronovich et al.; Kebriaei et al.; and Grabundzija et al..

Use of the SBTS permits efficient integration and expression of a transgene, e.g., a nucleic acid encoding a CAR described herein. Provided herein are methods of generating a cell, e.g., T cell or NK cell, that stably expresses a CAR described herein, e.g., using a transposon system such as SBTS.

In accordance with methods described herein, in some instances, one or more nucleic acids, e.g., plasmids, containing the SBTS components are delivered to a cell (e.g., T or NK cell). For example, the nucleic acid(s) are delivered by standard methods of nucleic acid (e.g., plasmid DNA) delivery, e.g., methods described herein, e.g., electroporation, transfection, or lipofection. In some instances, the nucleic acid contains a transposon comprising a transgene, e.g., a nucleic acid encoding a CAR described herein. In some instances, the nucleic acid contains a transposon comprising a transgene (e.g., a nucleic acid encoding a CAR described herein) as well as a nucleic acid sequence encoding a transposase enzyme. In other instances, a system with two nucleic acids is provided, e.g., a dual-plasmid system, e.g., where a first plasmid contains a transposon comprising a transgene, and a second plasmid contains a nucleic acid sequence encoding a transposase enzyme. For example, the first and the second nucleic acids are co-delivered into a host cell.

In some instances, cells, e.g., T or NK cells, are generated that express a CAR described herein by using a combination of gene insertion using the SBTS and genetic editing using a nuclease (e.g., Zinc finger nucleases (ZFNs), Transcription Activator-Like Effector Nucleases (TALENs), the CRISPR/Cas system, or engineered meganuclease re-engineered homing endonucleases).

In some instances, use of a non-viral method of delivery permits reprogramming of cells, e.g., T or NK cells, and direct infusion of the cells into a subject. Advantages of non-viral vectors include but are not limited to the ease and relatively low cost of producing sufficient amounts required to meet a patient population, stability during storage, and lack of immunogenicity.

### IMMUNE EFFECTOR CELLS, E.G., T CELLS

Methods described herein provide CD4⁺ cells having a CAR^{CD4+} and CD8⁺ T cells having a CAR^{CD8+}, wherein the CAR^{CD4+} and CAR^{CD8+} differ from one another. T cells can be obtained by a variety of methods from a variety of sources, e.g., as described in the section herein entitled **"SOURCES OF CELLS."** It can be desirable to have separate populations of CD4⁺ and CD8⁺ T cells for methods described herein. Preparations of CD4⁺ and preparations of CD8⁺ T cells can be obtained by a variety of methods e.g., as described in the section herein entitled **"SEPARATION** OF **T CELLS."** The T cells are transformed with CARs and expanded. CD4⁺ cells having a CAR^{CD4+} and CD8⁺ cells having a CAR^{CD8+} can be expanded, by a variety of methods, e.g., methods described in the section herein entitled "**ACTIVATION AND EXPANSION OF T CELLS**." CD4⁺ T cells can be Th17 polarized. Th17 polarized CD4⁺ cells having a CAR^{CD4+} can be polarized, and expanded, by a variety of methods, e.g., methods described in the section herein entitled "**ACTIVATION AND EXPANSION OF TH17 CELLS**." CD4⁺ T cells, CD8⁺ T cells, and Th17 cells can be transformed by a variety of methods, e.g., methods described in the section herein entitled **"NUCLEIC ACID**

### CONSTRUCTS ENCODING A CAR."

### SOURCES OF CELLS

In instances, prior to expansion and genetic modification or other modification, a source of cells, e.g., T cells or natural killer (NK) cells, can be obtained from a subject. Examples of subjects include humans, monkeys, chimpanzees, dogs, cats, mice, rats, and transgenic species thereof. T cells can be obtained from a number of sources, including peripheral blood mononuclear cells, bone marrow, lymph node tissue, cord blood, thymus tissue, tissue from a site of infection, ascites, pleural effusion, spleen tissue, and tumors.

In instances, immune effector cells, e.g., T cells can be obtained from a unit of blood collected from a subject using any number of techniques known to the skilled artisan, such as Ficoll™ separation. In an embodiment, cells from the circulating blood of an individual are obtained by apheresis. The apheresis product typically contains lymphocytes, including T cells, monocytes, granulocytes, B cells, other nucleated white blood cells, red blood cells, and platelets. In an instance, the cells collected by apheresis may be washed to remove the plasma fraction and optionally to place the cells in an appropriate buffer or media for subsequent processing steps. In an instance, the cells are washed with phosphate buffered saline (PBS). In an alternative instance, the wash solution lacks calcium and may lack magnesium or may lack many if not all divalent cations. Surprisingly, the initial activation steps in the absence of calcium lead to magnified signal activation. A washing step may be accomplished by methods known to those in the art, such as by using a semi-automated "flow-through" centrifuge (for example, the Cobe 2991 cell processor, the Baxter CytoMate, or the Haemonetics Cell Saver 5) according to the manufacturer's instructions. After washing, the cells may be resuspended in a variety of biocompatible buffers, such as, for example, Ca-free, Mg-free PBS, PlasmaLyte A, or other saline solution with or without buffer. Alternatively, the undesirable components of the apheresis sample may be removed and the cells directly resuspended in culture media.

It is recognized that the methods of the application can utilize culture media conditions comprising 5% or less, for example 2%, human AB serum, and employ known culture media conditions and compositions, for example those described in Smith et al., "Ex vivo expansion of human T cells for adoptive immunotherapy using the novel Xeno-free CTS Immune Cell Serum Replacement" Clinical & Translational Immunology (2015) 4, e31; doi:10.1038/cti.2014.31.

In an instance, T cells are isolated from peripheral blood lymphocytes by lysing the red blood cells and depleting the monocytes, for example, by centrifugation through a PERCOLL™ gradient or by counterflow centrifugal elutriation. A specific subpopulation of T cells, such as CD3+, CD28+, CD4⁺, CD8+, CD45RA+, and CD45RO+T cells, can be further isolated by positive or negative selection techniques.

T cell lines available in the art, may be used.

### SEPARATION OF T CELLS

Methods described herein provide CD4⁺ cells having a CAR^{CD4+} and CD8⁺ cells having a CAR^{CD8+} , wherein the a CAR^{CD4+} and a CAR^{CD8+} differ from one another. It can be desirable to have separate populations of CD4⁺ and CD8⁺ T cells for these methods.

In an instance, a CD4⁺ T cell or a population of CD4⁺ T cells is isolated by positive selection. For example, the T cells isolated from the blood of a subject can be incubated with an antibody that specifically recognizes CD4 under condition suitable for antibody labelling of the CD4⁺ T cells. Antibodies that specifically recognize CD4 are known in the art, e.g., anti-CD4 antibody clone M-T466 (Miltenyi Biotech), anti-CD4 antibody clone OKT4 (Affymetrix). In an instance, the anti-CD4 antibody is conjugated to a fluorescent molecule, e.g., FITC, and the T cells are sorted using flow cytometry to separate the CD4-expressing T cells from T cells that do not express CD4, e.g., CD8⁺ T cells. In some instances, the anti-CD4 antibody is conjugated to a surface or a solid support, e.g., a magnetic bead, and the CD4⁺ cells can be isolated using chromatography methods known in the art.

A CD4⁺ T cell or population of CD4⁺ T cells can also be enriched for by negative selection. In an instance, an antibody cocktail is utilized that includes antibodies against markers that are present on cells that do not express CD4, e.g., CD8, CD16, CD19, CD36, CD56, CD66b, TCRγ/δ, and glycophorin A. The antibody cocktail is added to the blood cell sample, mixed, and incubated, e.g., for 20 minutes at room temperature. A density medium is layered over the sample, and the mixture is centrifuged. The enriched cells expressing CD4⁺ will be present at the interface of the plasma and the density medium, and can be isolated. The unwanted cells, e.g., cells that do not express CD4, are found at the bottom of the tube, e.g., below the density medium. In another instance, the antibody cocktail can be conjugated to a surface or a bead, and the CD4⁺ cells can be collected from flow through over the surface or bead, while unwanted cells that do not express CD4 are immobilized on the surface or bead. In another instance, the monoclonal antibody cocktail includes antibodies to CD14, CD20, CD11b, CD16, HLA-DR, and CD8.

In an instance, a CD8⁺ T cell or a population of CD8⁺ T cells is isolated by positive selection. For example, the T cells isolated from the blood of a subject can be incubated with an antibody that specifically recognizes CD8 under condition suitable for antibody labelling of the CD8⁺ T cells. Antibodies that specifically recognize CD8 are known in the art, e.g., anti-CD8 antibody clone OKT8 (Affymetrix), anti-CD8 antibody clone C8/144B (Dako). In an instance, the anti-CD8 antibody is conjugated to a fluorescent molecule, e.g., FITC, and the T cells are sorted using flow cytometry to separate the CD8-expressing T cells from T cells that do not express CD8, e.g., CD4⁺ T cells. In some instances, the anti-CD8 antibody is conjugated to a surface or a solid support, e.g., a magnetic bead, and the CD8⁺ cells can be isolated using chromatography methods known in the art.

A CD8⁺ T cell or population of CD8⁺ T cells can also be enriched for by negative selection. In an instance, an antibody cocktail is utilized that includes antibodies against markers present on cells that do not express CD8, e.g., antibodies against CD4, CD16, CD19, CD36, CD56, CD66b, CD123, and TCRγ/δ. The antibody cocktail is added to the blood cell sample, mixed, and incubated, e.g., for 20 minutes at room temperature. A density medium is layered over the sample, and the mixture is centrifuged. The enriched cells expressing CD8⁺ will be present at the interface of the plasma and the density medium, and can be isolated. The unwanted cells, e.g., cells that do not express CD8, are found at the bottom of the tube, e.g., below the density medium. In another instance, the antibody cocktail can be conjugated to a surface or a bead, and the CD8⁺ cells can be collected from flow through over the surface or bead, while unwanted cells that do not express CD8 are immobilized on the surface or bead.

The methods described herein can include, e.g., selection of a specific subpopulation of immune effector cells, e.g., T cells, e.g., CD4⁺ and/or CD8⁺ T cells, that are a T regulatory cell-depleted population, CD25+ depleted cells, using, e.g., a negative selection technique, e.g., described herein. Preferably, the population of T regulatory depleted cells contains less than 30%, 25%, 20%, 15%, 10%, 5%, 4%, 3%, 2%, 1% of CD25+ cells.

In one instance, T regulatory cells, e.g., CD25+ T cells, are removed from the population using an anti-CD25 antibody, or fragment thereof, or a CD25-binding ligand, IL-2. In one instance, the anti-CD25 antibody, or fragment thereof, or CD25-binding ligand is conjugated to a substrate, e.g., a bead, or is otherwise coated on a substrate, e.g., a bead. In one instance, the anti-CD25 antibody, or fragment thereof, is conjugated to a substrate as described herein.

In one instance, the T regulatory cells, e.g., CD25+ T cells, are removed from the population using CD25 depletion reagent from Miltenyi™. In one instance, the ratio of cells to CD25 depletion reagent is 1e7 cells to 20 uL, or 1e7 cells to 15 uL, or 1e7 cells to 10 uL, or 1e7 cells to 5 uL, or 1e7 cells to 2.5 uL, or 1e7 cells to 1.25 uL. In one instance, e.g., for T regulatory cells, e.g., CD25+ depletion, greater than 500 million cells/ml is used. In a further aspect, a concentration of cells of 600, 700, 800, or 900 million cells/ml is used.

In one instance, the population of immune effector cells to be depleted includes about 6 x 10⁹ CD25+ T cells. In other aspects, the population of immune effector cells to be depleted include about 1 x 10⁹ to 1x 10¹⁰ CD25+ T cell, and any integer value in between. In one instance, the resulting population T regulatory depleted cells has 2 x 10⁹ T regulatory cells, e.g., CD25+ cells, or less (e.g., 1 x 10⁹, 5 x 10⁸, 1 x 10⁸, 5 x 10⁷, 1 x 10⁷, or less CD25+ cells).

In one instance, the T regulatory cells, e.g., CD25+ cells, are removed from the population using the CliniMAC system with a depletion tubing set, such as, e.g., tubing 162-01. In one instance, the CliniMAC system is run on a depletion setting such as, e.g., DEPLETION2.1.

Without wishing to be bound by a particular theory, decreasing the level of negative regulators of immune cells (e.g., decreasing the number of unwanted immune cells, e.g., T_{REG} cells), in a subject prior to apheresis or during manufacturing of a CAR-expressing cell product can reduce the risk of subject relapse. For example, methods of depleting T_{REG} cells are known in the art. Methods of decreasing T_{REG} cells include, but are not limited to, cyclophosphamide, anti-GITR antibody (an anti-GITR antibody described herein), CD25-depletion, and combinations thereof.

In some instances, the manufacturing methods comprise reducing the number of (e.g., depleting) T_{REG} cells prior to manufacturing of the CAR-expressing cell. For example, manufacturing methods comprise contacting the sample, e.g., the apheresis sample, with an anti-GITR antibody and/or an anti-CD25 antibody (or fragment thereof, or a CD25-binding ligand), e.g., to deplete T_{REG} cells prior to manufacturing of the CAR-expressing cell (e.g., T cell, NK cell) product.

In some instances, the manufacturing methods comprise reducing the number of (e.g., depleting) T_{REG} cells prior to manufacturing of the CAR-expressing cell. For example, manufacturing methods comprise contacting the sample, e.g., the apheresis sample, with an anti-GITR antibody and/or an anti-CD25 antibody (or fragment thereof, or a CD25-binding ligand), e.g., to deplete T_{REG} cells prior to manufacturing of the CAR-expressing cell (e.g., T cell, NK cell) product.

In an instance, a subject is pre-treated with one or more therapies that reduce T_{REG} cells prior to collection of cells for CAR-expressing cell product manufacturing, thereby reducing the risk of subject relapse to CAR-expressing cell treatment. In an instance, methods of decreasing T_{REG} cells include, but are not limited to, administration to the subject of one or more of cyclophosphamide, anti-GITR antibody, CD25-depletion, or a combination thereof. Administration of one or more of cyclophosphamide, anti-GITR antibody, CD25-depletion, or a combination thereof, can occur before, during or after an infusion of the CAR-expressing cell product.

In an instance, a subject is pre-treated with cyclophosphamide prior to collection of cells for CAR-expressing cell product manufacturing, thereby reducing the risk of subject relapse to CAR-expressing cell treatment. In an instance, a subject is pre-treated with an anti-GITR antibody prior to collection of cells for CAR-expressing cell product manufacturing, thereby reducing the risk of subject relapse to CAR-expressing cell treatment.

In one instance, the population of cells to be removed are neither the regulatory T cells or tumor cells, but cells that otherwise negatively affect the expansion and/or function of CART cells, e.g. cells expressing CD14, CD11b, CD33, CD15, or other markers expressed by potentially immune suppressive cells. In one instance, such cells are envisioned to be removed concurrently with regulatory T cells and/or tumor cells, or following said depletion, or in another order.

The methods described herein can include more than one selection step, e.g., more than one depletion step. Enrichment of a T cell population by negative selection can be accomplished, e.g., with a combination of antibodies directed to surface markers unique to the negatively selected cells. One method is cell sorting and/or selection via negative magnetic immunoadherence or flow cytometry that uses a cocktail of monoclonal antibodies directed to cell surface markers present on the cells negatively selected. For example, to enrich for CD4+ cells by negative selection, a monoclonal antibody cocktail can include antibodies to CD14, CD20, CD11b, CD16, HLA-DR, and CD8.

The methods described herein can further include removing cells from the population which express a tumor antigen, e.g., a tumor antigen that does not comprise CD25, e.g., CD19, CD30, CD38, CD123, CD20, CD14 or CD11b, to thereby provide a population of T regulatory depleted, e.g., CD25+ depleted, and tumor antigen depleted cells that are suitable for expression of a CAR, e.g., a CAR described herein. In one instance, tumor antigen expressing cells are removed simultaneously with the T regulatory, e.g., CD25+ cells. For example, an anti-CD25 antibody, or fragment thereof, and an anti-tumor antigen antibody, or fragment thereof, can be attached to the same substrate, e.g., bead, which can be used to remove the cells or an anti-CD25 antibody, or fragment thereof, or the anti-tumor antigen antibody, or fragment thereof, can be attached to separate beads, a mixture of which can be used to remove the cells. In other instances, the removal of T regulatory cells, e.g., CD25+ cells, and the removal of the tumor antigen expressing cells is sequential, and can occur, e.g., in either order.

Also provided are methods that include removing cells from the population which express a checkpoint inhibitor, e.g., a checkpoint inhibitor described herein, e.g., one or more of PD1+ cells, LAG3+ cells, and TIM3+ cells, to thereby provide a population of T regulatory depleted, e.g., CD25+ depleted cells, and check point inhibitor depleted cells, e.g., PD1+, LAG3+ and/or TIM3+ depleted cells. Exemplary checkpoint inhibitors include PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta. In one instance, check point inhibitor expressing cells are removed simultaneously with the T regulatory, e.g., CD25+ cells. For example, an anti-CD25 antibody, or fragment thereof, and an anti-check point inhibitor antibody, or fragment thereof, can be attached to the same bead which can be used to remove the cells, or an anti-CD25 antibody, or fragment thereof, and the anti-check point inhibitor antibody, or fragment there, can be attached to separate beads, a mixture of which can be used to remove the cells. In other instances, the removal of T regulatory cells, e.g., CD25+ cells, and the removal of the checkpoint inhibitor expressing cells is sequential, and can occur, e.g., in either order.

Methods described herein can include a positive selection step. For example, T cells (e.g CD4+ T cells or CD8+ T cells) can be isolated by incubation with anti-CD3/anti-CD28 (e.g., 3x28)-conjugated beads, such as DYNABEADS® M-450 CD3/CD28 T, for a time period sufficient for positive selection of the desired T cells. In one instance, the time period is about 30 minutes. In a further instance, the time period ranges from 30 minutes to 36 hours or longer and all integer values there between. In a further instance, the time period is at least 1, 2, 3, 4, 5, or 6 hours. In yet another instance, the time period is 10 to 24 hours, e.g., 24 hours. Longer incubation times may be used to isolate T cells in any situation where there are few T cells as compared to other cell types, such in isolating tumor infiltrating lymphocytes (TIL) from tumor tissue or from immunocompromised individuals. Further, use of longer incubation times can increase the efficiency of capture of CD8+ T cells. Thus, by simply shortening or lengthening the time T cells are allowed to bind to the CD3/CD28 beads and/or by increasing or decreasing the ratio of beads to T cells (as described further herein), subpopulations of T cells can be preferentially selected for or against at culture initiation or at other time points during the process. Additionally, by increasing or decreasing the ratio of anti-CD3 and/or anti-CD28 antibodies on the beads or other surface, subpopulations of T cells can be preferentially selected for or against at culture initiation or at other desired time points.

In one instance, a T cell population (e.g., T cells such as CD4+ T cells or CD8+ T cells) can be selected that expresses one or more of IFN-^{γ}, TNFα, IL-17A, IL-2, IL-3, IL-4, GM-CSF, IL-10, IL-13, granzyme B, and perforin, or other appropriate molecules, e.g., other cytokines. Methods for screening for cell expression can be determined, e.g., by the methods described in PCT Publication No.: WO 2013/126712.

For isolation of a desired population of cells by positive or negative selection, the concentration of cells and surface (e.g., particles such as beads) can be varied. In certain instances, it may be desirable to significantly decrease the volume in which beads and cells are mixed together (i.e., increase the concentration of cells), to ensure maximum contact of cells and beads. In an instance, a concentration of 10 billion cells/ml, 9 billion/ml, 8 billion/ml, 7 billion/ml, 6 billion/ml, or 5 billion/ml is used. In an instance, a concentration of 1 billion cells/ml is used. In a further instance, greater than 100 million cells/ml is used. In a further instance, a concentration of cells of 10, 15, 20, 25, 30, 35, 40, 45, or 50 million cells/ml is used. In yet another instance, a concentration of cells from 75, 80, 85, 90, 95, or 100 million cells/ml is used. In further instances, concentrations of 125 or 150 million cells/ml can be used. Using high concentrations can result in increased cell yield, cell activation, and cell expansion. Further, use of high cell concentrations allows more efficient capture of cells that may weakly express target antigens of interest, such as CD28-negative T cells, or from samples where there are many tumor cells present (i.e., leukemic blood, tumor tissue, etc.). Such populations of cells may have therapeutic value and would be desirable to obtain. For example, using high concentration of cells allows more efficient selection of CD8⁺ T cells that normally have weaker CD28 expression.

In a related instance it may be desirable to use lower concentrations of cells. By significantly diluting the mixture of T cells and surface (e.g., particles such as beads), interactions between the particles and cells is minimized. This selects for cells that express high amounts of desired antigens to be bound to the particles. For example, CD4⁺ T cells express higher levels of CD28 and are more efficiently captured than CD8⁺ T cells in dilute concentrations. In an instance, the concentration of cells used is 5 X 10⁶ /ml. In other instances, the concentration used can be from about 1 X 10⁵/ml to 1 X 10⁶/ml, and any integer value in between. In other instances, the cells may be incubated on a rotator for varying lengths of time at varying speeds at either 2-10°C or at room temperature.

T cells for stimulation can also be frozen after a washing step. Wishing not to be bound by theory, the freeze and subsequent thaw step provides a more uniform product by removing granulocytes and to some extent monocytes in the cell population. After the washing step that removes plasma and platelets, the cells may be suspended in a freezing solution. While many freezing solutions and parameters are known in the art and will be useful in this context, one method involves using PBS containing 20% DMSO and 8% human serum albumin, or culture media containing 10% Dextran 40 and 5% Dextrose, 20% Human Serum Albumin and 7.5% DMSO, or 31.25% Plasmalyte-A, 31.25% Dextrose 5%, 0.45% NaCl, 10% Dextran 40 and 5% Dextrose, 20% Human Serum Albumin, and 7.5% DMSO or other suitable cell freezing media containing for example, Hespan and PlasmaLyte A, the cells then are frozen to -80°C at a rate of 1° per minute and stored in the vapor phase of a liquid nitrogen storage tank. Other methods of controlled freezing may be used as well as uncontrolled freezing immediately at -20° C or in liquid nitrogen.

In certain instances, cryopreserved cells are thawed and washed as described herein and allowed to rest for one hour at room temperature prior to activation using the methods described herein.

In an instance the collection of blood samples or apheresis product from a subject is made at a time period prior to when the expanded cells might be needed. As such, the source of the cells to be expanded can be collected at any time point necessary, and desired cells, such as T cells, isolated and frozen for later use in, e.g., T cell therapy for any number of diseases or conditions that would benefit from such T cell therapy. In an instance a blood sample or an apheresis is taken from a generally healthy subject. In certain instances, a blood sample or an apheresis is taken from a generally healthy subject who is at risk of developing a disease, but who has not yet developed a disease, and the cells of interest are isolated and frozen for later use. In certain instances, the T cells may be expanded, frozen, and used at a later time. In certain instances, samples are collected from a patient shortly after diagnosis of a particular disease but prior to any treatments. In a further instance, the cells are isolated from a blood sample or an apheresis from a subject prior to any number of relevant treatment modalities, including but not limited to treatment with agents such as natalizumab, efalizumab, antiviral agents, chemotherapy, radiation, immunosuppressive agents, such as cyclosporin, azathioprine, methotrexate, mycophenolate, and FK506, antibodies, or other immunoablative agents such as CAMPATH, anti-CD3 antibodies, cytoxan, fludarabine, cyclosporin, FK506, rapamycin, mycophenolic acid, steroids, FR901228, and irradiation. These drugs inhibit either the calcium dependent phosphatase calcineurin (cyclosporine and FK506) or inhibit the p70S6 kinase that is important for growth factor induced signaling (rapamycin). (Liu et al., Cell 66:807-815, 1991; Henderson et al., Immun. 73:316-321, 1991; Bierer et al., Curr. Opin. Immun. 5:763-773, 1993). In a further instance, the cells are isolated for a patient and frozen for later use in conjunction with (e.g., before, simultaneously or following) bone marrow or stem cell transplantation, T cell ablative therapy using either chemotherapy agents such as, fludarabine, external-beam radiation therapy (XRT), cyclophosphamide, or antibodies such as OKT3 or CAMPATH. In an instance, the cells are isolated prior to and can be frozen for later use for treatment following B-cell ablative therapy such as agents that react with CD20, e.g., Rituxan.

In a further instance, T cells are obtained from a patient directly following treatment that leaves the subject with functional T cells. In this regard, it has been observed that following certain cancer treatments, in particular treatments with drugs that damage the immune system, shortly after treatment during the period when patients would normally be recovering from the treatment, the quality of T cells obtained may be optimal or improved for their ability to expand ex vivo. Likewise, following ex vivo manipulation using the methods described herein, these cells may be in a preferred state for enhanced engraftment and in vivo expansion. Thus, it is contemplated within the context of the present disclosure to collect blood cells, including T cells, dendritic cells, or other cells of the hematopoietic lineage, during this recovery phase. Further, in certain instances, mobilization (for example, mobilization with GM-CSF) and conditioning regimens can be used to create a condition in a subject wherein repopulation, recirculation, regeneration, and/or expansion of particular cell types is favored, especially during a defined window of time following therapy. Illustrative cell types include T cells, B cells, dendritic cells, and other cells of the immune system.

In one instance, the immune effector cells expressing a CAR molecule, e.g., a CAR molecule described herein, are obtained from a subject that has received a low, immune enhancing dose of an mTOR inhibitor. In an instance, the population of immune effector cells, e.g., T cells, to be engineered to express a CAR, are harvested after a sufficient time, or after sufficient dosing of the low, immune enhancing, dose of an mTOR inhibitor, such that the level of PD1 negative immune effector cells, e.g., T cells, or the ratio of PD1 negative immune effector cells, e.g., T cells/ PD1 positive immune effector cells, e.g., T cells, in the subject or harvested from the subject has been, at least transiently, increased.

In other instance, a population of immune effector cells, e.g., T cells, which have, or will be engineered to express a CAR, can be treated ex vivo by contact with an amount of an mTOR inhibitor that increases the number of PD1 negative immune effector cells, e.g., T cells or increases the ratio of PD1 negative immune effector cells, e.g., T cells/ PD1 positive immune effector cells, e.g., T cells.

In one instance, a T cell population is diaglycerol kinase (DGK)-deficient. DGK-deficient cells include cells that do not express DGK RNA or protein, or have reduced or inhibited DGK activity. DGK-deficient cells can be generated by genetic approaches, e.g., administering RNA-interfering agents, e.g., siRNA, shRNA, miRNA, to reduce or prevent DGK expression. Alternatively, DGK-deficient cells can be generated by treatment with DGK inhibitors described herein.

In one instance, a T cell population is Ikaros-deficient. Ikaros-deficient cells include cells that do not express Ikaros RNA or protein, or have reduced or inhibited Ikaros activity, Ikaros-deficient cells can be generated by genetic approaches, e.g., administering RNA-interfering agents, e.g., siRNA, shRNA, miRNA, to reduce or prevent Ikaros expression. Alternatively, Ikaros-deficient cells can be generated by treatment with Ikaros inhibitors, e.g., lenalidomide.

In instances, a T cell population is DGK-deficient and Ikaros-deficient, e.g., does not express DGK and Ikaros, or has reduced or inhibited DGK and Ikaros activity. Such DGK and Ikaros-deficient cells can be generated by any of the methods described herein.

In an instance, the NK cells are obtained from the subject. In another instance, the NK cells are an NK cell line, e.g., NK-92 cell line (Conkwest).

### ALLOGENEIC CAR

In instances described herein, the immune effector cell (e.g., a T cell such as a CD4+ T cell or a CD8+ T cell) can be an allogeneic immune effector cell, e.g., T cell or NK cell. For example, the cell can be an allogeneic T cell, e.g., an allogeneic T cell lacking expression of a functional T cell receptor (TCR) and/or human leukocyte antigen (HLA), e.g., HLA class I and/or HLA class II.

A T cell lacking a functional TCR can be, e.g., engineered such that it does not express any functional TCR on its surface, engineered such that it does not express one or more subunits that comprise a functional TCR (e.g., engineered such that it does not express (or exhibits reduced expression) of TCR alpha, TCR beta, TCR gamma, TCR delta, TCR epsilon, and/or TCR zeta) or engineered such that it produces very little functional TCR on its surface. Alternatively, the T cell can express a substantially impaired TCR, e.g., by expression of mutated or truncated forms of one or more of the subunits of the TCR. The term "substantially impaired TCR" means that this TCR will not elicit an adverse immune reaction in a host.

A T cell described herein can be, e.g., engineered such that it does not express a functional HLA on its surface. For example, a T cell described herein, can be engineered such that cell surface expression HLA, e.g., HLA class 1 and/or HLA class II, is downregulated. In some instances, downregulation of HLA may be accomplished by reducing or eliminating expression of beta-2 microglobulin (B2M).

In some instances, the T cell can lack a functional TCR and a functional HLA, e.g., HLA class I and/or HLA class II.

Modified T cells that lack expression of a functional TCR and/or HLA can be obtained by any suitable means, including a knock out or knock down of one or more subunit of TCR or HLA. For example, the T cell can include a knock down of TCR and/or HLA using siRNA, shRNA, clustered regularly interspaced short palindromic repeats (CRISPR) transcription-activator like effector nuclease (TALEN), or zinc finger endonuclease (ZFN).

In some embodiments, the allogeneic cell can be a cell which does not express or expresses at low levels an inhibitory molecule, e.g. a cell engineered by any method described herein. For example, the cell can be a cell that does not express or expresses at low levels an inhibitory molecule, e.g., that can decrease the ability of a CAR-expressing cell to mount an immune effector response. Examples of inhibitory molecules include PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine,and TGFR beta. Inhibition of an inhibitory molecule, e.g., by inhibition at the DNA, RNA or protein level, can optimize a CAR-expressing cell performance. In instances, an inhibitory nucleic acid, e.g., an inhibitory nucleic acid, e.g., a dsRNA, e.g., an siRNA or shRNA, a clustered regularly interspaced short palindromic repeats (CRISPR), a transcription-activator like effector nuclease (TALEN), or a zinc finger endonuclease (ZFN), e.g., as described herein, can be used.

### siRNA and shRNA to inhibit TCR or HLA

In some instances, TCR expression and/or HLA expression can be inhibited using siRNA or shRNA that targets a nucleic acid encoding a TCR and/or HLA, and/or an inhibitory molecule described herein (e.g., PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta), in a T cell.

Expression of siRNA and shRNAs in T cells can be achieved using any conventional expression system, e.g., such as a lentiviral expression system.

Exemplary shRNAs that downregulate expression of components of the TCR are described, e.g., in US Publication No.: 2012/0321667. Exemplary siRNA and shRNA that downregulate expression of HLA class I and/or HLA class II genes are described, e.g., in U.S. publication No.: US 2007/0036773.

### CRISPR to inhibit TCR or HLA

"CRISPR" or "CRISPR to TCR and/or HLA" or "CRISPR to inhibit TCR and/or HLA" as used herein refers to a set of clustered regularly interspaced short palindromic repeats, or a system comprising such a set of repeats. "Cas", as used herein, refers to a CRISPR-associated protein. A "CRISPR/Cas" system refers to a system derived from CRISPR and Cas which can be used to silence or mutate a TCR and/or HLA gene, and/or an inhibitory molecule described herein (e.g., PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta).

Naturally-occurring CRISPR/Cas systems are found in approximately 40% of sequenced eubacteria genomes and 90% of sequenced archaea. Grissa et al. (2007) BMC Bioinformatics 8: 172. This system is a type of prokaryotic immune system that confers resistance to foreign genetic elements such as plasmids and phages and provides a form of acquired immunity. Barrangou et al. (2007) Science 315: 1709-1712; Marragini et al. (2008) Science 322: 1843-1845.

The CRISPR/Cas system has been modified for use in gene editing (silencing, enhancing or changing specific genes) in eukaryotes such as mice or primates. Wiedenheft et al. (2012) Nature 482: 331-8. This is accomplished by introducing into the eukaryotic cell a plasmid containing a specifically designed CRISPR and one or more appropriate Cas.

The CRISPR sequence, sometimes called a CRISPR locus, comprises alternating repeats and spacers. In a naturally-occurring CRISPR, the spacers usually comprise sequences foreign to the bacterium such as a plasmid or phage sequence; in the TCR and/or HLA CRISPR/Cas system, the spacers are derived from the TCR or HLA gene sequence.

RNA from the CRISPR locus is constitutively expressed and processed by Cas proteins into small RNAs. These comprise a spacer flanked by a repeat sequence. The RNAs guide other Cas proteins to silence exogenous genetic elements at the RNA or DNA level. Horvath et al. (2010) Science 327: 167-170; Makarova et al. (2006) Biology Direct 1: 7. The spacers thus serve as templates for RNA molecules, analogously to siRNAs. Pennisi (2013) Science 341: 833-836.

As these naturally occur in many different types of bacteria, the exact arrangements of the CRISPR and structure, function and number of Cas genes and their product differ somewhat from species to species. Haft et al. (2005) PLoS Comput. Biol. 1: e60; Kunin et al. (2007) Genome Biol. 8: R61; Mojica et al. (2005) J. Mol. Evol. 60: 174-182; Bolotin et al. (2005) Microbiol. 151: 2551-2561; Pourcel et al. (2005) Microbiol. 151: 653-663; and Stern et al. (2010) Trends. Genet. 28: 335-340. For example, the Cse (Cas subtype, E. coli) proteins (e.g., CasA) form a functional complex, Cascade, that processes CRISPR RNA transcripts into spacer-repeat units that Cascade retains. Brouns et al. (2008) Science 321: 960-964. In other prokaryotes, Cas6 processes the CRISPR transcript. The CRISPR-based phage inactivation in E. coli requires Cascade and Cas3, but not Cas1 or Cas2. The Cmr (Cas RAMP module) proteins in Pyrococcus furiosus and other prokaryotes form a functional complex with small CRISPR RNAs that recognizes and cleaves complementary target RNAs. A simpler CRISPR system relies on the protein Cas9, which is a nuclease with two active cutting sites, one for each strand of the double helix. Combining Cas9 and modified CRISPR locus RNA can be used in a system for gene editing. Pennisi (2013) Science 341: 833-836.

The CRISPR/Cas system can thus be used to edit a TCR and/or HLA gene (adding or deleting a basepair), or introducing a premature stop which thus decreases expression of a TCR and/or HLA. The CRISPR/Cas system can alternatively be used like RNA interference, turning off TCR and/or HLA gene in a reversible fashion. In a mammalian cell, for example, the RNA can guide the Cas protein to a TCR and/or HLA promoter, sterically blocking RNA polymerases.

Artificial CRISPR/Cas systems can be generated which inhibit TCR and/or HLA, using technology known in the art, e.g., that described in U.S. Publication No.20140068797, and Cong (2013) Science 339: 819-823. Other artificial CRISPR/Cas systems that are known in the art may also be generated which inhibit TCR and/or HLA, e.g., that described in Tsai (2014) Nature Biotechnol., 32:6 569-576, U.S. Patent No.: 8,871,445; 8,865,406; 8,795,965; 8,771,945; and 8,697,359.

### TALEN to inhibit TCR and/or HLA

"TALEN" or "TALEN to HLA and/or TCR" or "TALEN to inhibit HLA and/or TCR" refers to a transcription activator-like effector nuclease, an artificial nuclease which can be used to edit the HLA and/or TCR gene, and/or an inhibitory molecule described herein (e.g., PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta).

TALENs are produced artificially by fusing a TAL effector DNA binding domain to a DNA cleavage domain. Transcription activator-like effects (TALEs) can be engineered to bind any desired DNA sequence, including a portion of the HLA or TCR gene. By combining an engineered TALE with a DNA cleavage domain, a restriction enzyme can be produced which is specific to any desired DNA sequence, including a HLA or TCR sequence. These can then be introduced into a cell, wherein they can be used for genome editing. Boch (2011) Nature Biotech. 29: 135-6; and Boch et al. (2009) Science 326: 1509-12; Moscou et al. (2009) Science 326: 3501.

TALEs are proteins secreted by Xanthomonas bacteria. The DNA binding domain contains a repeated, highly conserved 33-34 amino acid sequence, with the exception of the 12th and 13th amino acids. These two positions are highly variable, showing a strong correlation with specific nucleotide recognition. They can thus be engineered to bind to a desired DNA sequence.

To produce a TALEN, a TALE protein is fused to a nuclease (N), which is a wild-type or mutated FokI endonuclease. Several mutations to FokI have been made for its use in TALENs; these, for example, improve cleavage specificity or activity. Cermak et al. (2011) Nucl. Acids Res. 39: e82; Miller et al. (2011) Nature Biotech. 29: 143-8; Hockemeyer et al. (2011) Nature Biotech. 29: 731-734; Wood et al. (2011) Science 333: 307; Doyon et al. (2010) Nature Methods 8: 74-79; Szczepek et al. (2007) Nature Biotech. 25: 786-793; and Guo et al. (2010) J. Mol. Biol. 200: 96.

The FokI domain functions as a dimer, requiring two constructs with unique DNA binding domains for sites in the target genome with proper orientation and spacing. Both the number of amino acid residues between the TALE DNA binding domain and the FokI cleavage domain and the number of bases between the two individual TALEN binding sites appear to be important parameters for achieving high levels of activity. Miller et al. (2011) Nature Biotech. 29: 143-8.

A HLA or TCR TALEN can be used inside a cell to produce a double-stranded break (DSB). A mutation can be introduced at the break site if the repair mechanisms improperly repair the break via non-homologous end joining. For example, improper repair may introduce a frame shift mutation. Alternatively, foreign DNA can be introduced into the cell along with the TALEN; depending on the sequences of the foreign DNA and chromosomal sequence, this process can be used to correct a defect in the HLA or TCR gene or introduce such a defect into a wt HLA or TCR gene, thus decreasing expression of HLA or TCR.

TALENs specific to sequences in HLA or TCR can be constructed using any method known in the art, including various schemes using modular components. Zhang et al. (2011) Nature Biotech. 29: 149-53; Geibler et al. (2011) PLoS ONE 6: e19509.

### Zinc finger nuclease to inhibit HLA and/or TCR

"ZFN" or "Zinc Finger Nuclease" or "ZFN to HLA and/or TCR" or "ZFN to inhibit HLA and/or TCR" refer to a zinc finger nuclease, an artificial nuclease which can be used to edit the HLA and/or TCR gene, and/or an inhibitory molecule described herein (e.g., PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta).

Like a TALEN, a ZFN comprises a FokI nuclease domain (or derivative thereof) fused to a DNA-binding domain. In the case of a ZFN, the DNA-binding domain comprises one or more zinc fingers. Carroll et al. (2011) Genetics Society of America 188: 773-782; and Kim et al. (1996) Proc. Natl. Acad. Sci. USA 93: 1156-1160.

A zinc finger is a small protein structural motif stabilized by one or more zinc ions. A zinc finger can comprise, for example, Cys2His2, and can recognize an approximately 3-bp sequence. Various zinc fingers of known specificity can be combined to produce multi-finger polypeptides which recognize about 6, 9, 12, 15 or 18-bp sequences. Various selection and modular assembly techniques are available to generate zinc fingers (and combinations thereof) recognizing specific sequences, including phage display, yeast one-hybrid systems, bacterial one-hybrid and two-hybrid systems, and mammalian cells.

Like a TALEN, a ZFN must dimerize to cleave DNA. Thus, a pair of ZFNs are required to target non-palindromic DNA sites. The two individual ZFNs must bind opposite strands of the DNA with their nucleases properly spaced apart. Bitinaite et al. (1998) Proc. Natl. Acad. Sci. USA 95: 10570-5.

Also like a TALEN, a ZFN can create a double-stranded break in the DNA, which can create a frame-shift mutation if improperly repaired, leading to a decrease in the expression and amount of HLA and/or TCR in a cell. ZFNs can also be used with homologous recombination to mutate in the HLA or TCR gene.

ZFNs specific to sequences in HLA AND/OR TCR can be constructed using any method known in the art. See, e.g., Provasi (2011) Nature Med. 18: 807-815; Torikai (2013) Blood 122: 1341-1349; Cathomen et al. (2008) Mol. Ther. 16: 1200-7; and Guo et al. (2010) J. Mol. Biol. 400: 96; U.S. Patent Publication 2011/0158957; and U.S. Patent Publication 2012/0060230.

### TELOMERASE EXPRESSION

While not wishing to be bound by any particular theory, in some instances, a therapeutic T cell (e.g., a CD4+ T cell or a CD8+ T cell) has short term persistence in a patient, due to shortened telomeres in the T cell; accordingly, transfection with a telomerase gene can lengthen the telomeres of the T cell and improve persistence of the T cell in the patient. See Carl June, "Adoptive T cell therapy for cancer in the clinic", Journal of Clinical Investigation, 117:1466-1476 (2007). Thus, in an instance, an immune effector cell, e.g., a T cell, ectopically expresses a telomerase subunit, e.g., the catalytic subunit of telomerase, e.g., TERT, e.g., hTERT. In some aspects, this disclosure provides a method of producing a CAR-expressing cell, comprising contacting a cell with a nucleic acid encoding a telomerase subunit, e.g., the catalytic subunit of telomerase, e.g., TERT, e.g., hTERT. The cell may be contacted with the nucleic acid before, simultaneous with, or after being contacted with a construct encoding a CAR.

In one aspect, the disclosure features a method of making a population of immune effector cells (e.g., T cells or NK cells). In an instance, the method comprises: providing a population of immune effector cells (e.g., T cells or NK cells), contacting the population of immune effector cells with a nucleic acid encoding a CAR; and contacting the population of immune effector cells with a nucleic acid encoding a telomerase subunit, e.g., hTERT, under conditions that allow for CAR and telomerase expression.

In an instance, the nucleic acid encoding the telomerase subunit is DNA. In an instance, the nucleic acid encoding the telomerase subunit comprises a promoter capable of driving expression of the telomerase subunit.

In an instance, hTERT has the amino acid sequence of GenBank Protein ID AAC51724.1 (Meyerson et al., "hEST2, the Putative Human Telomerase Catalytic Subunit Gene, Is Up-Regulated in Tumor Cells and during Immortalization" Cell Volume 90, Issue 4, 22 August 1997, Pages 785-795) as follows:

In an instance, the hTERT has a sequence at least 80%, 85%, 90%, 95%, 96^, 97%, 98%, or 99% identical to the sequence of SEQ ID NO: 108. In an instance, the hTERT has a sequence of SEQ ID NO: 108. In an instance, the hTERT comprises a deletion (e.g., of no more than 5, 10, 15, 20, or 30 amino acids) at the N-terminus, the C-terminus, or both. In an instance, the hTERT comprises a transgenic amino acid sequence (e.g., of no more than 5, 10, 15, 20, or 30 amino acids) at the N-terminus, the C-terminus, or both.

In an instance, the hTERT is encoded by the nucleic acid sequence of GenBank Accession No. AF018167 (Meyerson et al., "hEST2, the Putative Human Telomerase Catalytic Subunit Gene, Is Up-Regulated in Tumor Cells and during Immortalization" Cell Volume 90, Issue 4, 22 August 1997, Pages 785-795):

In an instance, the hTERT is encoded by a nucleic acid having a sequence at least 80%, 85%, 90%, 95%, 96, 97%, 98%, or 99% identical to the sequence of SEQ ID NO: 109. In an instance, the hTERT is encoded by a nucleic acid of SEQ ID NO: 109.

### ACTIVATION AND EXPANSION OF IMMUNE EFFECTOR CELLS, E.G., T CELLS

Immune effector cells such as T cells may be activated and expanded generally using methods as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and U.S. Patent Application Publication No. 20060121005.

The procedure for *ex vivo* expansion of hematopoietic stem and progenitor cells is described in U.S. Pat. No. 5,199,942 can be applied to the cells of the present disclosure. Other suitable methods are known in the art, therefore the present invention is not limited to any particular method of ex vivo expansion of the cells. Briefly, ex vivo culture and expansion of T cells can comprise: (1) collecting CD34+ hematopoietic stem and progenitor cells from a mammal from peripheral blood harvest or bone marrow explants; and (2) expanding such cells ex vivo. In addition to the cellular growth factors described in U.S. Pat. No. 5,199,942, other factors such as flt3-L, IL-1, IL-3 and c-kit ligand, can be used for culturing and expansion of the cells.

Generally, population of immune effector cells, e.g., T regulatory cell depleted cells, may be expanded by contact with a surface having attached thereto an agent that stimulates a CD3/TCR complex associated signal and a ligand that stimulates a costimulatory molecule on the surface of the T cells. In a T cell a costimulatory molecule is a binding partner on a T cell that binds to a costimulatory ligand, mediating a costimulatory response in the T cell, i.e., an MHC class I molecule, e.g., CD28. For stimulation of an accessory molecule (e.g., CD3) on the surface of the T cells, a ligand that binds the accessory molecule is used. A population of T cells can be expanded with an anti-CD3 antibody and an anti-CD28 antibody under conditions appropriate for stimulating proliferation of the T cells. To stimulate proliferation of either CD4⁺ T cells or CD8⁺ T cells, an anti-CD3 antibody and an anti-CD28 antibody would be used. Examples of an anti-CD28 antibody include 9.3, B-T3, XR-CD28 (Diaclone, Besançon, France; (Berg et al., Transplant Proc. 30(8):3975-3977, 1998; Haanen et al., J. Exp. Med. 190(9):13191328, 1999; Garland et al., J. Immunol Meth. 227(1-2):53-63, 1999).

In certain instances, the primary activation signal and the costimulatory signal for the T cell may be provided by different protocols. For example, the agents providing each signal may be in solution or coupled to a surface. When coupled to a surface, the agents may be coupled to the same surface (i.e., in "cis" formation) or to separate surfaces (i.e., in "trans" formation). Alternatively, one agent may be coupled to a surface and the other agent in solution. In an instances, the agent providing the costimulatory signal is bound to a cell surface and the agent providing the primary activation signal is in solution or coupled to a surface. In certain instances, both agents can be in solution. In an instance, the agents may be in soluble form, and then cross-linked to a surface, such as a cell expressing Fc receptors or an antibody or other binding agent which will bind to the agents. In this regard, see for example, U.S. Patent Application Publication Nos. 20040101519 and 20060034810 for artificial antigen presenting cells (aAPCs) that are contemplated for use in activating and expanding T cells.

In an instance, the two agents are immobilized on beads, either on the same bead, i.e., "cis," or to separate beads, i.e., "trans." By way of example, the agent providing the primary activation signal is an anti-CD3 antibody or an antigen-binding fragment thereof and the agent providing the costimulatory signal is an anti-CD28 antibody or antigen-binding fragment thereof; and both agents are co-immobilized to the same bead in equivalent molecular amounts. In an instance, a 1:1 ratio of each antibody bound to the beads for CD4⁺ T cell and/or CD8+ T cell expansion and T cell growth is used. In certain instances, a ratio of anti CD3:CD28 antibodies bound to the beads is used such that an increase in T cell expansion is observed as compared to the expansion observed using a ratio of 1:1. In an instance an increase of from about 1 to about 3 fold is observed as compared to the expansion observed using a ratio of 1:1. In an instance, the ratio of CD3:CD28 antibody bound to the beads ranges from 100:1 to 1:100 and all integer values there between. In an instance, more anti-CD28 antibody is bound to the particles than anti-CD3 antibody, i.e., the ratio of CD3:CD28 is less than one. In certain instances, the ratio of anti CD28 antibody to anti CD3 antibody bound to the beads is greater than 2:1. In an instance, a 1:100 CD3:CD28 ratio of antibody bound to beads is used. In an instance, a 1:75 CD3:CD28 ratio of antibody bound to beads is used. In a further instance, a 1:50 CD3:CD28 ratio of antibody bound to beads is used. In an instance, a 1:30 CD3:CD28 ratio of antibody bound to beads is used. In an instance, a 1:10 CD3:CD28 ratio of antibody bound to beads is used. In another instance, a 1:3 CD3:CD28 ratio of antibody bound to the beads is used. In yet another instance, a 3:1 CD3:CD28 ratio of antibody bound to the beads is used.

Ratios of particles to cells from 1:500 to 500:1 and any integer values in between may be used to stimulate T cells or other target cells. As those of ordinary skill in the art can readily appreciate, the ratio of particles to cells may depend on particle size relative to the target cell. For example, small sized beads could only bind a few cells, while larger beads could bind many. In certain instances the ratio of cells to particles ranges from 1:100 to 100:1 and any integer values in-between and in further instances the ratio comprises 1:9 to 9:1 and any integer values in between, can also be used to stimulate T cells. The ratio of anti-CD3- and anti-CD28-coupled particles to T cells that result in T cell stimulation can vary as noted above, however certain preferred values include 1:100, 1:50, 1:40, 1:30, 1:20, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, and 15:1 with one preferred ratio being at least 1:1 particles per T cell. In an instance, a ratio of particles to cells of 1:1 or less is used. In one particular instance, a preferred particle: cell ratio is 1:5. In further instances, the ratio of particles to cells can be varied depending on the day of stimulation. For example, in an instance, the ratio of particles to cells is from 1:1 to 10:1 on the first day and additional particles are added to the cells every day or every other day thereafter for up to 10 days, at final ratios of from 1:1 to 1:10 (based on cell counts on the day of addition). In one particular instance, the ratio of particles to cells is 1:1 on the first day of stimulation and adjusted to 1:5 on the third and fifth days of stimulation. In an instance, particles are added on a daily or every other day basis to a final ratio of 1:1 on the first day, and 1:5 on the third and fifth days of stimulation. In an instance, the ratio of particles to cells is 2:1 on the first day of stimulation and adjusted to 1:10 on the third and fifth days of stimulation. In an instance, particles are added on a daily or every other day basis to a final ratio of 1:1 on the first day, and 1:10 on the third and fifth days of stimulation. One of skill in the art will appreciate that a variety of other ratios may be suitable for use. In particular, ratios will vary depending on particle size and on cell size and type. In an instance the most typical ratios for use are in the neighborhood of 1:1, 2:1 and 3:1 on the first day.

In further instances, the cells, e.g., T cells, are combined with agent-coated beads, the beads and the cells are subsequently separated, and then the cells are cultured. In an alternative instance, prior to culture, the agent-coated beads and cells are not separated but are cultured together. In a further instance, the beads and cells are first concentrated by application of a force, such as a magnetic force, resulting in increased ligation of cell surface markers, thereby inducing cell stimulation.

By way of example, cell surface proteins may be ligated by allowing paramagnetic beads to which anti-CD3 and anti-CD28 are attached (3x28 beads) to contact the T cells. In an instance the cells (e.g., 10⁴ to 10⁹ T cells) and beads (e.g., DYNABEADS® M-450 CD3/CD28 T paramagnetic beads at a ratio of 1:1) are combined in a buffer, for example PBS (without divalent cations such as, calcium and magnesium). Again, those of ordinary skill in the art can readily appreciate any cell concentration may be used. For example, the target cell may be very rare in the sample and comprise only 0.01% of the sample or the entire sample (i.e., 100%) may comprise the target cell of interest. In certain instances, it may be desirable to significantly decrease the volume in which particles and cells are mixed together (i.e., increase the concentration of cells), to ensure maximum contact of cells and particles. For example, in an instance, a concentration of about 10 billion cells/ml, 9 billion/ml, 8 billion/ml, 7 billion/ml, 6 billion/ml, 5 billion/ml, or 2 billion cells/ml is used. In an instance, greater than 100 million cells/ml is used. In a further instance, a concentration of cells of 10, 15, 20, 25, 30, 35, 40, 45, or 50 million cells/ml is used. In yet an instance, a concentration of cells from 75, 80, 85, 90, 95, or 100 million cells/ml is used. In further instances, concentrations of 125 or 150 million cells/ml can be used. Using high concentrations can result in increased cell yield, cell activation, and cell expansion. Further, use of high cell concentrations allows more efficient capture of cells that may weakly express target antigens of interest, such as CD28-negative T cells. Such populations of cells may have therapeutic value and would be desirable to obtain in certain instances. For example, using high concentration of cells allows more efficient selection of CD8⁺ T cells that normally have weaker CD28 expression.

In one instance, cells (e.g., T cells such as CD4+ T cells or CD8+ T cells) transduced with a nucleic acid encoding a CAR, e.g., a CAR described herein, are expanded, e.g., by a method described herein. In one instance, the cells are expanded in culture for a period of several hours (e.g., about 2, 3, 4, 5, 6, 7, 8, 9, 10, 15, 18, 21 hours) to about 14 days (e.g., 1, 2, 3, 4, 5, 6, 7, 8, 9, 10, 11, 12, 13 or 14 days). In one instance, the cells are expanded for a period of 4 to 9 days. In one instance, the cells are expanded for a period of 8 days or less, e.g., 7, 6 or 5 days. In one instance, the cells, e.g., a CD19 CAR cell described herein, are expanded in culture for 5 days, and the resulting cells are more potent than the same cells expanded in culture for 9 days under the same culture conditions. Potency can be defined, e.g., by various T cell functions, e.g. proliferation, target cell killing, cytokine production, activation, migration, or combinations thereof. In one instance, the cells, e.g., a CD19 CAR cell described herein, expanded for 5 days show at least a one, two, three or four fold increase in cells doublings upon antigen stimulation as compared to the same cells expanded in culture for 9 days under the same culture conditions. In one instance, the cells, e.g., the cells expressing a CD19 CAR described herein, are expanded in culture for 5 days, and the resulting cells exhibit higher proinflammatory cytokine production, e.g., IFN-γ and/or GM-CSF levels, as compared to the same cells expanded in culture for 9 days under the same culture conditions. In one instance, the cells, e.g., a CD19 CAR cell described herein, expanded for 5 days show at least a one, two, three, four, five, ten fold or more increase in pg/ml of proinflammatory cytokine production, e.g., IFN-γ and/or GM-CSF levels, as compared to the same cells expanded in culture for 9 days under the same culture conditions.

In an instance, the mixture (of T cells and stimulation agent) may be cultured for several hours (about 3 hours) to about 14 days or any hourly integer value in between. In an instance, the mixture may be cultured for 21 days. In an instance the beads and the T cells are cultured together for about eight days. In an instance, the beads and T cells are cultured together for 2-3 days.

Several cycles of stimulation may also be desired such that culture time of T cells can be 60 days or more. Conditions appropriate for T cell culture include an appropriate media (e.g., Minimal Essential Media or RPMI Media 1640 or, X-vivo 15, (Lonza)) that may contain factors necessary for proliferation and viability, including serum (e.g., fetal bovine or human serum), interleukin-2 (IL-2), insulin, IFN-γ, IL-4, IL-7, GM-CSF, IL-10, IL-12, IL-15, TGFβ, and TNF-α or any other additives for the growth of cells known to the skilled artisan. Other additives for the growth of cells include, but are not limited to, surfactant, plasmanate, and reducing agents such as N-acetyl-cysteine and 2-mercaptoethanol. Media can include RPMI 1640, AIM-V, DMEM, MEM, α-MEM, F-12, X-Vivo 15, and X-Vivo 20, Optimizer, with added amino acids, sodium pyruvate, and vitamins, either serum-free or supplemented with an appropriate amount of serum (or plasma) or a defined set of hormones, and/or an amount of cytokine(s) sufficient for the growth and expansion of T cells. Antibiotics, e.g., penicillin and streptomycin, are included only in experimental cultures, not in cultures of cells that are to be infused into a subject. The target cells are maintained under conditions necessary to support growth for example, an appropriate temperature (e.g., 37°C) and atmosphere (e.g., air plus 5% CO₂).

In one instance, the cells (e.g., T cells such as CD4+ T cells or CD8+ T cells) are expanded in an appropriate media (e.g., media described herein) that includes one or more interleukin that result in at least a 200-fold (e.g., 200-fold, 250-fold, 300-fold, 350-fold) increase in cells over a 14 day expansion period, e.g., as measured by a method described herein such as flow cytometry. In one instance, the cells are expanded in the presence of IL-15 and/or IL-7 (e.g., IL-15 and IL-7).

In instances, methods described herein, e.g., CAR-expressing cell manufacturing methods, comprise removing T regulatory cells, e.g., CD25+ T cells, from a cell population, (e.g., T cells such as CD4+ T cells or CD8+ T cells) e.g., using an anti-CD25 antibody, or fragment thereof, or a CD25-binding ligand, IL-2. Methods of removing T regulatory cells, e.g., CD25+ T cells, from a cell population are described herein. In instances, the methods, e.g., manufacturing methods, further comprise contacting a cell population (e.g., a cell population in which T regulatory cells, such as CD25+ T cells, have been depleted; or a cell population that has previously contacted an anti-CD25 antibody, fragment thereof, or CD25-binding ligand) with IL-15 and/or IL-7. For example, the cell population (e.g., that has previously contacted an anti-CD25 antibody, fragment thereof, or CD25-binding ligand) is expanded in the presence of IL-15 and/or IL-7.

In some instances a CAR-expressing cell described herein (e.g., a T cell such as a CD4+ T cell or a CD8+ T cell) is contacted with a composition comprising a interleukin-15 (IL-15) polypeptide, a interleukin-15 receptor alpha (IL-15Ra) polypeptide, or a combination of both a IL-15 polypeptide and a IL-15Ra polypeptide e.g., hetIL-15, during the manufacturing of the CAR-expressing cell, e.g., ex vivo. In instances, a CAR-expressing cell described herein is contacted with a composition comprising a IL-15 polypeptide during the manufacturing of the CAR-expressing cell, e.g., ex vivo. In instances, a CAR-expressing cell described herein is contacted with a composition comprising a combination of both a IL-15 polypeptide and a IL-15 Ra polypeptide during the manufacturing of the CAR-expressing cell, e.g., ex vivo. In instances, a CAR-expressing cell described herein is contacted with a composition comprising hetIL-15 during the manufacturing of the CAR-expressing cell, e.g., ex vivo.

In one instance the CAR-expressing cell (e.g., a T cell such as a CD4+ T cell or a CD8+ T cell) described herein is contacted with a composition comprising hetIL-15 during ex vivo expansion. In an instance, the CAR-expressing cell described herein is contacted with a composition comprising an IL-15 polypeptide during ex vivo expansion. In an instance, the CAR-expressing cell described herein is contacted with a composition comprising both an IL-15 polypeptide and an IL-15Ra polypeptide during ex vivo expansion. In one instance the contacting results in the survival and proliferation of a lymphocyte subpopulation, e.g., CD8+ T cells.

In an instance, the method of making discosed herein further comprises contacting the population of immune effector cells (e.g., T cells such as CD4+ T cells or CD8+ T cells) with a nucleic acid encoding a telomerase subunit, e.g., hTERT. The the nucleic acid encoding the telomerase subunit can be DNA.

Various assays can be used to evaluate the activity of the CAR molecule, such as but not limited to, the ability to expand T cells following antigen stimulation, sustain T cell expansion in the absence of re-stimulation, and anti-cancer activities in appropriate animal models. Assays to evaluate the effects of the CAR, e.g., an EGFRvIII CAR, are described in further detail below.

Western blot analysis of CAR expression in primary T cells can be used to detect their presence using published methods for CARs. See, e.g., Milone et al., Molecular Therapy 17(8): 1453-1464 (2009). Very briefly, T cells (1:1 mixture of CD4⁺ and CD8⁺ T cells) expressing the RCARs are expanded in vitro for more than 10 days followed by lysis and SDS-PAGE under reducing conditions. CARs containing the full length TCR-ζ cytoplasmic domain and the endogenous TCR-ζ chain are detected by western blotting using an antibody to the TCR-ζ chain. The same T cell subsets are used for SDS-PAGE analysis under non-reducing conditions to permit evaluation of covalent dimer formation.

In vitro expansion of CAR⁺ T cells (i.e., CART cells) following antigen stimulation can be measured by flow cytometry. For example, CD4⁺ and CD8⁺ T cells, or a mixture thereof, are stimulated with αCD3/αCD28 aAPCs or beads followed by transduction with lentiviral vectors expressing GFP under the control of the promoters to be analyzed. Exemplary promoters include the CMV IE gene, EF-1α, ubiquitin C, or phosphoglycerokinase (PGK) promoters. GFP fluorescence is evaluated on day 6 of culture in the CD4⁺ and/or CD8⁺ T cell subsets by flow cytometry. See, e.g., Milone et al., Molecular Therapy 17(8): 1453-1464 (2009). Alternatively, CD4⁺ and CD8⁺ T cells, or a mixture thereof, are stimulated with α CD3/αCD28 coated magnetic beads on day 0, and transduced with CAR on day 1 using a bicistronic lentiviral vector expressing CAR along with eGFP using a 2A ribosomal skipping sequence. Cultures are re-stimulated with CAR constructs in the presence of antiCD3 and anti-CD28 antibody (K562-BBL-3/28) following washing. Exogenous IL-2 is added to the cultures every other day at 100 IU/ml. GFP⁺ T cells are enumerated by flow cytometry using bead-based counting. See, e.g., Milone et al., Molecular Therapy 17(8): 1453-1464 (2009).

Sustained CAR⁺ T cell expansion in the absence of re-stimulation can also be measured. See, e.g., Milone et al., Molecular Therapy 17(8): 1453-1464 (2009). Briefly, mean T cell volume (fl) is measured on day 8 of culture using a Coulter Multisizer III particle counter, a Nexcelom Cellometer Vision or Millipore Scepter, following stimulation with αCD3/αCD28 coated magnetic beads on day 0, and transduction with the indicated CAR on day 1.

Assessment of cell proliferation and cytokine production has been previously described, e.g., at Milone et al., Molecular Therapy 17(8): 1453-1464 (2009). Briefly, assessment of CAR-mediated proliferation is performed in microtiter plates by mixing washed T cells with target cells, such as U87MG, BHK or CHO cells expressing EGFRvIII or EGFR wildtype (wt) or CD32 and CD137 (KT32-BBL) for a final T-cell:target cell ratio of 1:1. Anti-CD3 (clone OKT3) and anti-CD28 (clone 9.3) monoclonal antibodies are added to cultures with KT32-BBL cells to serve as a positive control for stimulating T-cell proliferation since these signals support long-term CD8⁺ T cell expansion ex vivo. T cells are enumerated in cultures using CountBright™ fluorescent beads (Invitrogen, Carlsbad, CA) and flow cytometry as described by the manufacturer. RCAR⁺ T cells are identified by GFP expression using T cells that are engineered with eGFP-2A linked CAR-expressing lentiviral vectors. For CAR+ T cells not expressing GFP, the RCAR+ T cells are detected with biotinylated recombinant protein, e.g., EGFRvIII and a secondary avidin-PE conjugate. CD4⁺ and CD8⁺ expression on T cells are also simultaneously detected with specific monoclonal antibodies (BD Biosciences). Cytokine measurements are performed on supernatants collected 24 hours following re-stimulation using the human TH1/TH2 cytokine cytometric bead array kit (BD Biosciences, San Diego, CA) according the manufacturer's instructions. Fluorescence is assessed using a FACScalibur flow cytometer, and data is analyzed according to the manufacturer's instructions.

Cytotoxicity can be assessed by a standard ⁵¹Cr-release assay. See, e.g., Milone et al., Molecular Therapy 17(8): 1453-1464 (2009). Briefly, target cells (e.g., U87MG, BHK or CHO cells expressing CAR, e.g., EGFRvIII or EGFR wildtype (wt) are loaded with ⁵¹Cr (as NaCrO₄, New England Nuclear, Boston, MA) at 37°C for 2 hours with frequent agitation, washed twice in complete RPMI and plated into microtiter plates. Effector T cells are mixed with target cells in the wells in complete RPMI at varying ratios of effector cell:target cell (E:T). Additional wells containing media only (spontaneous release, SR) or a 1% solution of triton-X 100 detergent (total release, TR) are also prepared. After 4 hours of incubation at 37°C, supernatant from each well is harvested. Released ⁵¹Cr is then measured using a gamma particle counter (Packard Instrument Co., Waltham, MA). Each condition is performed in at least triplicate, and the percentage of lysis is calculated using the formula: % Lysis = (ER- SR) / (TR - SR), where ER represents the average ⁵¹Cr released for each experimental condition. Alternative cytotoxicity assays may also be used, such as flow based cytotoxicity assays. Other assays, including those described in the Example section herein as well as those that are known in the art can also be used to evaluate the CAR constructs.

Alternatively, or in combination to the methods disclosed herein, methods and compositions for one or more of detection and/or quantification of CAR-expressing cells (e.g., in vitro or in vivo (e.g., clinical monitoring)), immune cell expansion and/or activation, and/or CAR-specific selection, that involve the use of a CAR ligand, are disclosed. In one exemplary instance, the CAR ligand is an antibody that binds to the CAR molecule, e.g., binds to the extracellular antigen binding domain of CAR (e.g., an antibody that binds to the antigen binding domain, e.g., an anti-idiotypic antibody; or an antibody that binds to a constant region of the extracellular binding domain). In other instances, the CAR ligand is a CAR antigen molecule (e.g., a CAR antigen molecule as described herein).

In one aspect, a method for detecting and/or quantifying CAR-expressing cells is disclosed. For example, the CAR ligand can be used to detect and/or quantify CAR-expressing cells in vitro or in vivo (e.g., clinical monitoring of CAR-expressing cells in a patient, or dosing a patient). The method includes:
providing the CAR ligand (optionally, a labelled CAR ligand, e.g., a CAR ligand that includes a tag, a bead, a radioactive or fluorescent label);
acquiring the CAR-expressing cell (e.g., acquiring a sample containing CAR-expressing cells, such as a manufacturing sample or a clinical sample);
contacting the CAR-expressing cell with the CAR ligand under conditions where binding occurs, thereby detecting the level (e.g., amount) of the CAR-expressing cells present. Binding of the CAR-expressing cell with the CAR ligand can be detected using standard techniques such as FACS, ELISA and the like.

In another aspect, a method of expanding and/or activating cells (e.g., immune effector cells) is disclosed. The method includes:
providing a CAR-expressing cell (e.g., a first CAR-expressing cell or a transiently expressing CAR cell);
contacting said CAR-expressing cell with a CAR ligand, e.g., a CAR ligand as described herein), under conditions where immune cell expansion and/or proliferation occurs, thereby producing the activated and/or expanded cell population.

In certain instances, the CAR ligand is present on (e.g., is immobilized or attached to a substrate, e.g., a non-naturally occurring substrate). In some instances, the substrate is a non-cellular substrate. The non-cellular substrate can be a solid support chosen from, e.g., a plate (e.g., a microtiter plate), a membrane (e.g., a nitrocellulose membrane), a matrix, a chip or a bead. In instances, the CAR ligand is present in the substrate (e.g., on the substrate surface). The CAR ligand can be immobilized, attached, or associated covalently or non-covalently (e.g., cross-linked) to the substrate. In one instance, the CAR ligand is attached (e.g., covalently attached) to a bead. In the aforesaid instances, the immune cell population can be expanded in vitro or ex vivo. The method can further include culturing the population of immune cells in the presence of the the ligand of the CAR molecule, e.g., using any of the methods described herein.

In other instances, the method of expanding and/or activating the cells further comprises addition of a second stimulatory molecule, e.g., CD28. For example, the CAR ligand and the second stimulatory molecule can be immobilized to a substrate, e.g., one or more beads, thereby providing increased cell expansion and/or activation.

In other instances, a method for selecting or enriching for a CAR expressing cell is provided. The method includes contacting the CAR expressing cell with a CAR ligand as described herein; and selecting the cell on the basis of binding of the CAR ligand.

In yet other instances, a method for depleting (e.g., reducing and/or killing) a CAR expressing cell is provided. The method includes contacting the CAR expressing cell with a CAR ligand as described herein; and targeting the cell on the basis of binding of the CAR ligand thereby reducing the number, and/or killing, the CAR-expressing cell. In one instance, the CAR ligand is coupled to a toxic agent (e.g., a toxin or a cell ablative drug). In another instance, the anti-idiotypic antibody can cause effector cell activity, e.g., ADCC or ADC activities.

Exemplary anti-CAR antibodies that can be used in the methods disclosed herein are described, e.g., in WO 2014/190273 and by Jena et al., "Chimeric Antigen Receptor (CAR)-Specific Monoclonal Antibody to Detect CD19-Specific T cells in Clinical Trials", PLOS March 2013 8:3 e57838. In one instance, the anti-idiotypic antibody molecule recognizes an anti-CD 19 antibody molecule, e.g., an anti-CD 19 scFv. For instance, the anti-idiotypic antibody molecule can compete for binding with the CD19-specific CAR mAb clone no. 136.20.1 described in Jena et al., PLOS March 2013 8:3 e57838; may have the same CDRs (e.g., one or more of, e.g., all of, VH CDR1, VH CDR2, CH CDR3, VL CDR1, VL CDR2, and VL CDR3, using the Kabat definition, the Chothia definition, or a combination of tthe Kabat and Chothia definitions) as the CD19-specific CAR mAb clone no. 136.20.1; may have one or more (e.g., 2) variable regions as the CD19-specific CAR mAb clone no. 136.20.1, or may comprise the CD19-specific CAR mAb clone no. 136.20.1. In some instances, the anti-idiotypic antibody was made according to a method described in Jena et al. In another instance, the anti-idiotypic antibody molecule is an anti-idiotypic antibody molecule described in WO 2014/190273. In some instances, the anti-idiotypic antibody molecule has the same CDRs (e.g., one or more of, e.g., all of, VH CDR1, VH CDR2, CH CDR3, VL CDR1, VL CDR2, and VL CDR3) as an antibody molecule of WO 2014/190273 such as 136.20.1; may have one or more (e.g., 2) variable regions of an antibody molecule of WO 2014/190273, or may comprise an antibody molecule of WO 2014/190273 such as 136.20.1. In other instances, the anti-CAR antibody binds to a constant region of the extracellular binding domain of the CAR molecule, e.g., as described in WO 2014/190273. In some instances, the anti-CAR antibody binds to a constant region of the extracellular binding domain of the CAR molecule, e.g., a heavy chain constant region (e.g., a CH2-CH3 hinge region) or light chain constant region. For instance, in some instances the anti-CAR antibody competes for binding with the 2D3 monoclonal antibody described in WO 2014/190273, has the same CDRs (e.g., one or more of, e.g., all of, VH CDR1, VH CDR2, CH CDR3, VL CDR1, VL CDR2, and VL CDR3) as 2D3, or has one or more (e.g., 2) variable regions of 2D3, or comprises 2D3 as described in WO 2014/190273.

### ACTIVATION AND EXPANSION OF TH17 CELLS

In an embodiment, it may be desirable to obtain a T-helper 17 (Th17) cell, or a population of Th17 cells. Th17 cells are a subgroup of T helper cells. Thl7 cells (inflammatory T-helper or inflammatory Th) promote inflammation responses trough secretion of pro-inflammatory cytokines, such as IL-1, IL-6, TNF-a, IL-17, IL21, IL23, and/or through activation and/or inhibition of other T cells including other Th cells (for example Thl cell suppresses Th2 and Thl7, Th2 suppresses Thl and Thl7).

Thl 7 cells or otherwise cells exhibiting Thl 7 cell phenotype may have a variety of specific phenotypic properties, depending on the conditions employed. Such phenotypic properties include production of IL-17A and IFNγ. Moreover, expanded Th17 cells continue to produce both IL-17A and IFNγ event after their primary expansion. In some instances, Thl 7 cells coexpressed both RORγt and T-bet, transcription factors that regulate Thl7 and Thl cell development, respectively. In some embodiments, expanded Th17 cells express IL-23R and/or CD 161 on their cell surface, phenotypic markers associated with umbilical cord Thl7 cells. In some instances, Thl7 cells express RORγt.

Methods for obtaining a Th17 cell or population of Th17 cells include polarizing or differentiating a CD4⁺ T cell, or a population of CD4⁺ T cells. The CD4⁺ T cells, e.g., a population isolated using the methods described above, are stimulated with agents that activate CD3 and ICOS, e.g., antibodies against CD3 and ICOS, and are then cultured in the presence of cytokines and molecules that allow differentiation to Th17 and Th17 function, as described in further detail below.

To stimulate proliferation of Thl7 cells, cells can be contacted with an anti-CD3 antibody and an anti-ICOS antibody, under conditions appropriate for stimulating proliferation of the Th17 cells. Thl7 cells can also be stimulated with ICOS ligand (ICOSL)-expressing artificial antigen presenting cells (aAPCs). Stimulation can be performed in the presence of Thl7-polarizing cytokines. An example of Thl7-polarizing cytokines include but is not limited to IL-6, IL-I β and IL-23 cytokines and neutralizing IFNy and IL-4 antibodies.

A CD4⁺ T cell may be stimulated to produce a Th17 cell by contacting an agent with a cell surface moiety on the T cell. In one aspect of the present disclosure, antibodies to CD3 and ICOS are loaded onto an aAPC. Further, stimulation may include any ligand that binds the TCR/CD3 complex and initiates a primary stimulation signal. This ligand may be utilized as a primary activation agent loaded onto or expressed by the aAPC. Any ligand that binds ICOS and initiates the ICOS signal transduction pathway, thus causing co-stimulation of the cell with a CD3 ligand and enhancing activation of a population of T cells, is an ICOS ligand and accordingly, is a co-stimulatory agent.

T cells can be exposed to a bead comprising a first agent that binds the TCR/CD3 complex and initiates a primary stimulation signal and a second agent that binds ICOS and initiates the ICOS signal transduction pathway, thus causing co-stimulation of the cell with a CD3 ligand and enhancing activation of a population of T cells.

Stimulated cells are activated as shown by the induction of signal transduction, expression of cell surface markers and/or proliferation. Markers appropriate for Thl7 cells include but are not limited to their capacity to secrete heightened levels of IL-17A, IL-17F and CCL20. Moreover, cells generated and expanded according to the ICOS costimulation method not only exhibit elevated production of Thl7-associated cytokines but also exhibit elevated secretion of IFNγ, TNFa and IL-21 compared to CD28 costimulated cells.

In the context of generating Thl7 cells by way of stimulating ICOS on T cells, an aAPC can be engineered to comprise a first agent that binds to TCR/CD3 complex of the T cell and a second agent that binds ICOS, the aAPC can further be engineered to comprise a cytokine that promotes Thl7 differentiation. Exemplary Thl7 differentiating cytokines include but are not limited to IL-2, IL-6, IL-23, and IL-1.

Accordingly, T cell stimulation may include an aAPC that has been genetically modified to express stimulatory agents, co-stimulatory agents, and/or cytokines as well as other polypeptides. The aAPC can be engineered to express and secrete any desirable cytokine that promotes Thl7 differentiation using the methods disclosed herein or known methods in the art for genetically modifying a cell. The cytokine can be a full-length, fragment, homologue, variant or mutant of the cytokine. A cytokine includes a protein that is capable of affecting the biological function of another cell. A biological function affected by a cytokine can include, but is not limited to, cell growth, cell differentiation or cell death. In stimulating the stimulation of Thl7 cells, the cytokine can bind to a specific receptor on the surface of cell, thereby promoting Thl7 differentiation. A preferred cytokine includes, among others, a hematopoietic growth factor, an interleukin, an interferon, an immunoglobulin superfamily molecule, a tumor necrosis factor family molecule and/or a chemokine. A cytokine includes but is not limited to granulocyte macrophage colony stimulating factor (GM-CSF), tumor necrosis factor alpha (TNFa), tumor necrosis factor beta (TNFP), macrophage colony stimulating factor (M-CSF), interleukin- 1 (IL-1), interleukin-2 (IL-2), interleukin-4 (IL-4), interleukin-5 (IL-5), interleukin-6 (IL-6), interleukin- 10 (IL-10), interleukin- 12 (IL-12), interleukin- 15 (IL-15), interleukin-21 (IL-21), interleukin-23 (IL-23), interferon alpha (IFNa), interferon beta (ΓPNβ), interferon gamma (IFNy), and IGIF, among many others. A more preferred cytokine includes a cytokine that promotes Thl7 differentiation including but not limited to IL-2, IL-6, IL-1 (e.g., IL-Iβ). One skilled in the art would appreciate, once armed with the teachings provided herein, that the disclosure encompasses any Thl7 differentiation promoting cytokine, such as those known in the art, as well as any discovered in the future.

In addition to engineering an aAPC to comprise a Thl7 differentiation promoting cytokine, the aAPC can be engineered to comprise an inhibitory molecule that can block a cytokine that interferes with the Thl7 differentiation process. For example, the aAPC can be engineered to secrete a neutralizing antibody than can inhibit a cytokine that interferes with Thl7 differentiation. A cytokine that interferes with Thl7 differentiation process includes but is not limited to IFNy and IL-4.

When the aAPC has been engineered to express a desired cytokine that promotes Thl7 differentiation and/or inhibitor of a cytokine that interferes with Thl7 differentiation, a method for activating and/or stimulating a population of T cells to promote Thl7 differentiation in the absence of exogenously added cytokines is provided. Further, such Thl7 differentiation may occur in vivo.

In certain instances, the primary stimulatory signal and the co-stimulatory signal for the T cell may be provided by different protocols, as described previously.

In one instance, the two agents are immobilized on beads, either on the same bead, i.e., "cis," or to separate beads, i.e., "trans." By way of example, the agent providing the primary activation signal is an anti-CD3 antibody or an antigen-binding fragment thereof and the agent providing the co-stimulatory signal is an anti-ICOS antibody or antigen-binding fragment thereof; and both agents are co-immobilized to the same bead in equivalent molecular amounts. In one instance, a 1:1 ratio of each antibody bound to the beads for Thl7 growth is used. In certain aspects of the present disclosure, a ratio of anti CD3:ICOS antibodies bound to the beads is used such that an increase in Thl7 cell expansion is observed as compared to the expansion observed using a ratio of 1 : 1. In one instance, the ratio of CD3 :ICOS antibody bound to the beads ranges from 100: 1 to 1 : 100 and all integer values there between. In one aspect of the present disclosure, more anti-ICOS antibody is bound to the particles than anti-CD3 antibody, i.e., the ratio of CD3 :ICOS is less than one. In certain instance of the disclosure, the ratio of anti ICOS antibody to anti CD3 antibody bound to the beads is greater than 2: 1. In one particular instance, a 1:100 CD3:ICOS ratio of antibody bound to beads is used. In another instance, a 1:75 CD3:ICOS ratio of antibody bound to beads is used. In a further instance, a 1:50 CD3:ICOS ratio of antibody bound to beads is used. In another instance, a 1:30 CD3:ICOS ratio of antibody bound to beads is used. In one instance, a 1:10 CD3:ICOS ratio of antibody bound to beads is used. In another instance, a 3:1 CD3:ICOS ratio of antibody bound to the beads is used. In a preferred instance, a 1:3 CD3:ICOS ratio of antibody bound to the beads is used.

Ratios of particles to cells from 1:500 to 500:1 and any integer values in between may be used to stimulate T cells or other target cells. As those of ordinary skill in the art can readily appreciate, the ratio of particles to cells may depend on particle size relative to the target cell, as described perviously. For example, small sized beads could only bind a few cells, while larger beads could bind many. In certain instances the ratio of cells to particles ranges from 1:100 to 100:1 and any integer values in-between and in further instances the ratio comprises 1:9 to 9:1 and any integer values in between, can also be used to stimulate T cells. The ratio of anti-CD3- and anti-ICOS-coupled particles to T cells that result in T cell stimulation can vary as noted above, however certain preferred values include 1:100, 1:50, 1:40, 1:30, 1:20, 1:10, 1:9, 1:8, 1:7, 1:6, 1:5, 1:4, 1:3, 1:2, 1:1,2:1,3:1,4:1,5:1,6:1,7:1,8:1,9:1, 10:1, and 15:1. One of skill in the art will appreciate that a variety of other ratios may be suitable for use in the present disclosure. In particular, ratios will vary depending on particle size and on cell size and type.

In further instances of the present disclosure, the cells, such as T cells, are combined with agent-coated beads, the beads and the cells are subsequently separated, and then the cells are cultured. In an alternative instance, prior to culture, the agent-coated beads and cells are not separated but are cultured together. In a further instance, the beads and cells are first concentrated by application of a force, such as a magnetic force, resulting in increased ligation of cell surface markers, thereby inducing cell stimulation.

By way of example, cell surface proteins may be ligated by allowing paramagnetic beads to which anti-CD3 and anti-ICOS are attached to contact the T cells. In one instance the cells (for example, 10⁴ to 10⁹ T cells) and beads (for example, paramagnetic beads at a ratio of 1 : 1) are combined in a buffer, preferably PBS (without divalent cations such as, calcium and magnesium). Again, those of ordinary skill in the art can readily appreciate any cell concentration may be used. In one instance of the present disclosure, the mixture may be cultured for several hours (about 3 hours) to about 14 days or any hourly integer value in between. In another instance, the mixture may be cultured for 21 days. In one instance of the disclosure the beads and the T cells are cultured together for about eight days. In another instance, the beads and T cells are cultured together for 2-3 days. Several cycles of stimulation may also be desired such that culture time of T cells can be 60 days or more.

In a preferred instance, the T cells that are polarized to Th17 cells are cultured in the presence of cytokines and other molecules that result in the polarization to Th17 cells and maintenance of Th17 phenotype and/or function. Examples of such cytokines include: IL-1β (e.g., 10ng/ml), IL-4 (e.g, 10ng/ml), IL-23 (e.g., 20ng/ml). In some instances, IL-2 is added 1, 2, 3, 4, 5, or 6 days after activation to a final concentration of 50 IU/ml. Examples of neutralizing antibodies (e.g., 10µg/ml) for polarizing Th17 cells include: anti-IL-4 antibodies and anti-IFNy antibodies.

Conditions appropriate for T17h cell culture include an appropriate media (e.g., Minimal Essential Media or RPMI Media 1640 or, X-vivo 15, (Lonza)) that may contain factors necessary for proliferation and viability to maintain Th17 phenotype, including serum (e.g., fetal bovine or human serum), interleukin-2 (IL-2), insulin, IFN-γ, IL-4, IL-7, GM-CSF, IL-10, IL-12, IL-15, TGF , and TNF-a or any other additives for the growth of cells known to the skilled artisan. Other additives for the growth of cells include, but are not limited to, surfactant, plasmanate, and reducing agents such as N-acetyl-cysteine and 2-mercaptoethanol. Media can include RPMI 1640, AIM-V, DMEM, MEM, α-MEM, F-12, X-Vivo 15, and X-Vivo 20, Optimizer, with added amino acids, sodium pyruvate, and vitamins, either serum-free or supplemented with an appropriate amount of serum (or plasma) or a defined set of hormones, and/or an amount of cytokine(s) sufficient for the growth and expansion of T cells. Antibiotics, e.g., penicillin and streptomycin, are included only in experimental cultures, not in cultures of cells that are to be infused into a subject. The target cells are maintained under conditions necessary to support growth, for example, an appropriate temperature (e.g., 37° C) and atmosphere (e.g., air plus 5% C0₂).

Those of ordinary skill in the art will readily appreciate that the cell stimulation methodologies described herein may be carried out in a variety of environments (i.e., containers). For example, such containers may be culture flasks, culture bags, or any container capable of holding cells, preferably in a sterile environment. In one instance of the present disclosure a bioreactor is also useful. For example, several manufacturers currently make devices that can be used to grow cells and be used in combination with the methods of the present disclosure. See for example, patents covering bioreactors such as U.S. Pat. Nos. 6,096,532; 5,985,653; 5,888,807; 5,190,878.

### PHARMACEUTICAL COMPOSITIONS AND TREATMENTS

Pharmaceutical compositions of the present disclosure comprises one or both of a CAR^{CD4+}-expressing CD4⁺T cell, e.g., a plurality of CAR^{CD4+}-expressing CD4⁺T cell, as described herein, and a CAR^{CD8+}-expressing CD8⁺T cell, e.g., a plurality of CAR^{CD8+}-expressing CD8⁺T cell, as described herein, wherein the CAR^{CD4+} and the CAR^{CD8+} are different, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are in one aspect formulated for intravenous administration.

In an embodiment, the composition further comprises a second CD8+ T cell comprising a second CAR^{CD8+}, wherein the second CAR^{CD8+} is different from the first CAR^{CD8+} and the CAR^{CD4+}. The CAR^{CD4+}-expressing CD4⁺T cell, the CAR^{CD8+}-expressing CD8⁺T cell, and the second CAR^{CD8+}-expressing CD8⁺T cell can all be in the same composition, or are each in separate compositions. Any combination of the CAR^{CD4+}-expressing CD4⁺T cell, the CAR^{CD8+}-expressing CD8⁺T cell, and the second CAR^{CD8+}-expressing CD8⁺T cell can be in the same composition or in different compositions.

Pharmaceutical compositions of the present invention may be administered in a manner appropriate to the disease to be treated (or prevented). The quantity and frequency of administration will be determined by such factors as the condition of the patient, and the type and severity of the patient's disease, although appropriate dosages may be determined by clinical trials.

In one embodiment, the pharmaceutical composition is substantially free of, e.g., there are no detectable levels of a contaminant, e.g., selected from the group consisting of endotoxin, mycoplasma, replication competent lentivirus (RCL), p24, VSV-G nucleic acid, HIV gag, residual anti-CD3/anti-CD28 coated beads, mouse antibodies, pooled human serum, bovine serum albumin, bovine serum, culture media components, vector packaging cell or plasmid components, a bacterium and a fungus. In one embodiment, the bacterium is at least one selected from the group consisting of Alcaligenes faecalis, Candida albicans, Escherichia coli, Haemophilus influenza, Neisseria meningitides, Pseudomonas aeruginosa, Staphylococcus aureus, Streptococcus pneumonia, and Streptococcus pyogenes group A.

When "an immunologically effective amount," "an anti-tumor effective amount," "a tumor-inhibiting effective amount," or "therapeutic amount" is indicated, the precise amount of the compositions of the present invention to be administered can be determined by a physician with consideration of individual differences in age, weight, tumor size, extent of infection or metastasis, and condition of the patient (subject). It can generally be stated that a pharmaceutical composition comprising the T cells described herein may be administered at a dosage of 10⁴ to 10⁹ cells/kg body weight, in some instances 10⁵ to 10⁶ cells/kg body weight, including all integer values within those ranges. T cell compositions may also be administered multiple times at these dosages. The cells can be administered by using infusion techniques that are commonly known in immunotherapy (see, e.g., Rosenberg et al., New Eng. J. of Med. 319:1676, 1988).

In certain aspects, it may be desired to administer activated T cells to a subject and then subsequently redraw blood (or have an apheresis performed), activate T cells therefrom according to the present disclosure, and reinfuse the patient with these activated and expanded T cells. This process can be carried out multiple times every few weeks. In certain aspects, T cells can be activated from blood draws of from 10cc to 400cc. In certain aspects, T cells are activated from blood draws of 20cc, 30cc, 40cc, 50cc, 60cc, 70cc, 80cc, 90cc, or 100cc.

The administration of the subject compositions may be carried out in any convenient manner, including by aerosol inhalation, injection, ingestion, transfusion, implantation or transplantation. The compositions described herein may be administered to a patient trans arterially, subcutaneously, intradermally, intratumorally, intranodally, intramedullary, intramuscularly, by intravenous (i.v.) injection, or intraperitoneally. In one aspect, the T cell compositions of the present invention are administered to a patient by intradermal or subcutaneous injection. In one aspect, the T cell compositions of the present invention are administered by i.v. injection. The compositions of T cells may be injected directly into a tumor, lymph node, or site of infection.

In a particular exemplary aspect, subjects may undergo leukapheresis, wherein leukocytes are collected, enriched, or depleted ex vivo to select and/or isolate the cells of interest, e.g., T cells. These T cell isolates may be expanded by methods known in the art and treated such that one or more CAR constructs of the present diclosure may be introduced, thereby creating a CAR T cell of the disclosure. Subjects in need thereof may subsequently undergo standard treatment with high dose chemotherapy followed by peripheral blood stem cell transplantation. In certain aspects, following or concurrent with the transplant, subjects receive an infusion of the expanded CAR T cells of the present disclosure. In an additional aspect, expanded cells are administered before or following surgery.

In instances, lymphodepletion is performed on a subject, e.g., prior to administering one or more cells that express a CAR described herein, e.g., a CD20-binding CAR described herein. In instances, the lymphodepletion comprises administering one or more of melphalan, cytoxan, cyclophosphamide, and fludarabine.

The dosage of the above treatments to be administered to a patient will vary with the precise nature of the condition being treated and the recipient of the treatment. The scaling of dosages for human administration can be performed according to art-accepted practices. The dose for CAMPATH, for example, will generally be in the range 1 to about 100 mg for an adult patient, usually administered daily for a period between 1 and 30 days. The preferred daily dose is 1 to 10 mg per day although in some instances larger doses of up to 40 mg per day may be used (described in U.S. Patent No. 6,120,766).

In one instance, the CAR^{CD4+} is introduced into a CD4⁺ T cell, and the CAR^{CD8+} is introduced into a CD8⁺ T cells, wherein the CAR^{CD4+} and the CAR^{CD8+} are different, , e.g., using lentiviral transduction. The subject (e.g., human) receives an initial administration of the CD4⁺ T cell comprising the CAR^{CD4+} and the CD8⁺ T cell comprising the CAR^{CD8+}, and one or more subsequent administrations CD4⁺ and the CD8⁺T cells of the disclosure, wherein the one or more subsequent administrations are administered less than 15 days, e.g., 14, 13, 12, 11, 10, 9, 8, 7, 6, 5, 4, 3, or 2 days after the previous administration. In one instance, more than one administration of the CD4⁺ and the CD8⁺T cells of the disclosure are administered to the subject (e.g., human) per week, e.g., 2, 3, or 4 administrations of the CD4⁺ and the CD8⁺T cells of the disclosure are administered per week. In one embodiment, the subject (e.g., human subject) receives more than one administration of the CD4⁺ and the CD8⁺T cells per week (e.g., 2, 3 or 4 administrations per week) (also referred to herein as a cycle), followed by a week of no CD4⁺ and the CD8⁺T cells administrations, and then one or more additional administration of the CD4⁺ and the CD8⁺T cells (e.g., more than one administration of the CD4⁺ and the CD8⁺T cells per week) is administered to the subject. In another embodiment, the subject (e.g., human subject) receives more than one cycle of CD4⁺ and the CD8⁺T cells, and the time between each cycle is less than 10, 9, 8, 7, 6, 5, 4, or 3 days. In one embodiment, the CD4⁺ and the CD8⁺T cells are administered every other day for 3 administrations per week. In one instance, the CD4⁺ and the CD8⁺T cells of the disclosure are administered for at least two, three, four, five, six, seven, eight or more weeks.

In one aspect, CARTs of the present invention are generated using lentiviral viral vectors, such as lentivirus. CARTs generated that way will have stable CAR expression.

In one aspect, CARTs are generated using a viral vector such as a gammaretroviral vector, e.g., a gammaretroviral vector described herein. CARTs generated using these vectors can have stable CAR expression.

In one aspect, CARTs transiently express CAR vectors for 4, 5, 6, 7, 8, 9, 10, 11, 12, 13, 14, 15 days after transduction. Transient expression of CARs can be effected by RNA CAR vector delivery. In one aspect, the CAR RNA is introduced into the T cell by electroporation.

A potential issue that can arise in patients being treated using transiently expressing CAR-expressing CD4⁺ T cells and CD8⁺ T cells (particularly with murine scFv bearing CARs) is anaphylaxis after multiple treatments.

Without being bound by this theory, it is believed that such an anaphylactic response might be caused by a patient developing humoral anti-CAR response, i.e., anti-CAR antibodies having an anti-IgE isotype. It is thought that a patient's antibody producing cells undergo a class switch from IgG isotype (that does not cause anaphylaxis) to IgE isotype when there is a ten to fourteen day break in exposure to antigen.

If a patient is at high risk of generating an anti-CAR antibody response during the course of transient CAR therapy (such as those generated by RNA transductions), CART infusion breaks should not last more than ten to fourteen days.

Also provided herein are kits comprising one or more, or all of the following components: a nucleic acid sequence that encodes a CAR^{CD4+}, a nucleic acid sequence that encodes a CAR^{CD8+}, and a nucleic acid sequence that encodes a second CAR^{CD8+}, wherein the CAR^{CD4+}, the CAR^{CD8+} and the second CAR^{CD8+} are different. Each component may be disposed in a separate container. The kit further comprises additional agents that may be required for introduction into a T cell, such as a transfection reagent, a transduction reagent, or a buffer.

Also provided herein are kits comprising one or more, or all of the following components: a CD4⁺ T cell, or preparation thereof, comprising a CAR^{CD4+}, a CD8+ T cell, or preparation thereof, comprising a CAR^{CD8+}, and a second CD8+ T cell, or preration thereof, comprising a second CAR^{CD8+}, wherein the CAR^{CD4+}, the CAR^{CD8+} and the second CAR^{CD8+} are different. Each of the components may be disposed in a separate container. Methods of making such kits are also described herein.

### THERAPEUTIC APPLICATIONS

In one aspect, the present disclosure provides methods for treating a disease associated with expression of a cancer associated antigen described herein.

In one aspect, the present disclosure provides methods of treating cancer by providing to the subject in need thereof CD4⁺ T cells and CD8⁺ T cells that are engineered to express an XCAR, wherein X represents a tumor antigen (or cancer associated antigen) as described herein, and wherein the cancer cells express said X tumor antigen (or cancer associated antigen).

In one aspect, the present disclosure provides methods of treating cancer by providing to the subject in need thereof CD4⁺ T cells and CD8⁺ T cells that are engineered to express a XCAR described herein, wherein the cancer cells express X. In one instance, X is expressed on both normal cells and cancers cells, but is expressed at lower levels on normal cells. In one instance, the method further comprises selecting a CAR that binds X with an affinity that allows the XCAR to bind and kill the cacner cells expressing X but less than 30%, 25%, 20%, 15%, 10%, 5% or less of the normal cells expressing X are killed, e.g., as determined by an assay described herein. For example, the assay described in Figure 14 can be used or a killing assay such as flow cytometry based on Cr51 CTL. In one instance, the selected CAR has an antigen binding domain that has a binding affinity KD of 10⁻⁴ M to 10⁻⁸ M, e.g., 10⁻⁵ M to 10⁻⁷ M, e.g., 10⁻⁶ M or 10⁻⁷ M, for the target antigen. In one instance, the selected antigen binding domain has a binding affinity that is at least five-fold, 10-fold, 20-fold, 30-fold, 50-fold, 100-fold or 1,000-fold less than a reference antibody, e.g., an antibody described herein.

In another aspect, a method of treating a subject, e.g., reducing or ameliorating, a hyperproliferative condition or disorder (e.g., a cancer), e.g., solid tumor, a soft tissue tumor, or a metastatic lesion, in a subject is provided. As used herein, the term "cancer" is meant to include all types of cancerous growths or oncogenic processes, metastatic tissues or malignantly transformed cells, tissues, or organs, irrespective of histopathologic type or stage of invasiveness. Examples of solid tumors include malignancies, e.g., sarcomas, adenocarcinomas, and carcinomas, of the various organ systems, such as those affecting liver, lung, breast, lymphoid, gastrointestinal (e.g., colon), genitourinary tract (e.g., renal, urothelial cells), prostate and pharynx. Adenocarcinomas include malignancies such as most colon cancers, rectal cancer, renal-cell carcinoma, liver cancer, non-small cell carcinoma of the lung, cancer of the small intestine and cancer of the esophagus. In one instance, the cancer is a melanoma, e.g., an advanced stage melanoma. Metastatic lesions of the aforementioned cancers can also be treated or prevented using the methods and compositions of the disclosure. Examples of other cancers that can be treated include bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemias including acute myeloid leukemia, chronic myeloid leukemia, acute lymphoblastic leukemia, chronic lymphocytic leukemia, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers including those induced by asbestos, and combinations of said cancers. Treatment of metastatic cancers, e.g., metastatic cancers that express PD-L1 (Iwai et al. (2005) Int. Immunol. 17:133-144) can be effected using the antibody molecules described herein.

Exemplary cancers whose growth can be inhibited include cancers typically responsive to immunotherapy. Non-limiting examples of cancers for treatment include melanoma (e.g., metastatic malignant melanoma), renal cancer (e.g. clear cell carcinoma), prostate cancer (e.g. hormone refractory prostate adenocarcinoma), breast cancer, colon cancer and lung cancer (e.g. non-small cell lung cancer). Additionally, refractory or recurrent malignancies can be treated using the molecules described herein.

In one aspect, the present disclosure pertains to a vector comprising a CAR operably linked to promoter for expression in mammalian immune effector cells (e.g., T cells, NK cells). In one aspect, the disclosure provides a recombinant T cell expressing the CARs of the present disclosure for use in treating cancer expressing a cancer associate antigen as described herein. In one aspect, CARTs of the disclosure is capable of contacting a tumor cell with at least one cancer associated antigen expressed on its surface such that the CART targets the tumor cell and growth of the tumor is inhibited.

In one aspect, the present disclosure pertains to a method of inhibiting growth of a cancer, comprising contacting the cancer cell with a CART of the present disclosure such that the CART is activated in response to the antigen and targets the cancer cell, wherein the growth of the tumor is inhibited.

The present disclosure includes a type of cellular therapy where T cells are genetically modified to express a chimeric antigen receptor (CAR) and the CAR T cell is infused to a recipient in need thereof. The infused cell is able to kill tumor cells in the recipient. Unlike antibody therapies, CAR-modified T cells, are able to replicate in vivo resulting in long-term persistence that can lead to sustained tumor control. In various aspects, T cells administered to the patient, or their progeny, persist in the patient for at least four months, five months, six months, seven months, eight months, nine months, ten months, eleven months, twelve months, thirteen months, fourteen month, fifteen months, sixteen months, seventeen months, eighteen months, nineteen months, twenty months, twenty-one months, twenty-two months, twenty-three months, two years, three years, four years, or five years after administration of the T cell to the patient.

The present disclosure also includes a type of cellular therapy where T cells are modified, e.g., by in vitro transcribed RNA, to transiently express a chimeric antigen receptor (CAR) and the CAR T cell is infused to a recipient in need thereof. The infused cell is able to kill tumor cells in the recipient. Thus, in various aspects, the T cellsadministered to the patient, is present for less than one month, e.g., three weeks, two weeks, one week, after administration of the T cell to the patient.

Without wishing to be bound by any particular theory, the anti-tumor immunity response elicited by the CAR-modified T cells may be an active or a passive immune response, or alternatively may be due to a direct vs indirect immune response. In one aspect, the CAR transduced T cells) exhibit specific proinflammatory cytokine secretion and potent cytolytic activity in response to human cancer cells expressing the a cancer associate antigen as described herein, resist soluble a cancer associate antigen as described herein inhibition, mediate bystander killing and mediate regression of an established human tumor. For example, antigen-less tumor cells within a heterogeneous field of a cancer associate antigen as described herein-expressing tumor may be susceptible to indirect destruction by a cancer associate antigen as described herein-redirected CD4⁺ T cells and CD8⁺ T cells that has previously reacted against adjacent antigen-positive cancer cells.

In one aspect, the fully-human CAR-modified CD4⁺ T cells and CD8⁺ T cells of the present disclosure may be a type of vaccine for ex vivo immunization and/or in vivo therapy in a mammal. In one aspect, the mammal is a human.

With respect to ex vivo immunization, at least one of the following occurs in vitro prior to administering the cell into a mammal: i) expansion of the cells, ii) introducing a nucleic acid encoding a CAR to the cells or iii) cryopreservation of the cells.

Ex vivo procedures are well known in the art and are discussed more fully below. Briefly, cells are isolated from a mammal (e.g., a human) and genetically modified (i.e., transduced or transfected in vitro) with a vector expressing a CAR disclosed herein. The CAR-modified CD4⁺ T cells and CD8⁺ T cells can be administered to a mammalian recipient to provide a therapeutic benefit. The mammalian recipient may be a human and the CAR-modified CD4⁺ T cells and CD8⁺ T cells can be autologous with respect to the recipient. Alternatively, the cells can be allogeneic, syngeneic or xenogeneic with respect to the recipient.

Generally, the cells activated and expanded as described herein may be utilized in the treatment and prevention of diseases that arise in individuals who are immunocompromised. In particular, the CAR-modified CD4⁺ T cells and CD8⁺ T cells of the present disclosure are used in the treatment of diseases, disorders and conditions associated with expression of a cancer associate antigen as described herein. In certain aspects, the cells of the present disclosure are used in the treatment of patients at risk for developing diseases, disorders and conditions associated with expression of a cancer associate antigen as described herein. Thus, the present disclosure provides methods for the treatment or prevention of diseases, disorders and conditions associated with expression of a cancer associate antigen as described herein comprising administering to a subject in need thereof, a therapeutically effective amount of the CAR-modified CD4⁺ T cells and CD8⁺ T cells of the present disclosure.

The CAR-modified CD4⁺ T cells and CD8⁺ T cells of the present disclosure may be administered either alone, or as a pharmaceutical composition in combination with diluents and/or with other components such as IL-2 or other cytokines or cell populations.

### Hematologic Cancer

Hematological cancer conditions are the types of cancer such as leukemia and malignant lymphoproliferative conditions that affect blood, bone marrow and the lymphatic system.

Leukemia can be classified as acute leukemia and chronic leukemia. Acute leukemia can be further classified as acute myelogenous leukemia (AML) and acute lymphoid leukemia (ALL). Chronic leukemia includes chronic myelogenous leukemia (CML) and chronic lymphoid leukemia (CLL). Other related conditions include myelodysplastic syndromes (MDS, formerly known as "preleukemia") which are a diverse collection of hematological conditions united by ineffective production (or dysplasia) of myeloid blood cells and risk of transformation to AML.

The present disclosure provides for compositions and methods for treating cancer. In one aspect, the cancer is a hematologic cancer including but is not limited to hematological cancer is a leukemia or a lymphoma. In one aspect, the CART cells of the present disclosure may be used to treat cancers and malignancies such as, but not limited to, e.g., acute leukemias including but not limited to, e.g., B-cell acute lymphoid leukemia ("BALL"), T-cell acute lymphoid leukemia ("TALL"), acute lymphoid leukemia (ALL); one or more chronic leukemias including but not limited to, e.g., chronic myelogenous leukemia (CML), chronic lymphocytic leukemia (CLL); additional hematologic cancers or hematologic conditions including, but not limited to, e.g., B cell prolymphocytic leukemia, blastic plasmacytoid dendritic cell neoplasm, Burkitt's lymphoma, diffuse large B cell lymphoma, Follicular lymphoma, Hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin's lymphoma, plasmablastic lymphoma, plasmacytoid dendritic cell neoplasm, Waldenstrom macroglobulinemia, and "preleukemia" which are a diverse collection of hematological conditions united by ineffective production (or dysplasia) of myeloid blood cells, and the like. Further a disease associated with a cancer associate antigen as described herein expression includes, but not limited to, e.g., atypical and/or non-classical cancers, malignancies, precancerous conditions or proliferative diseases expressing a cancer associate antigen as described herein.

The present disclosure also provides methods for inhibiting the proliferation or reducing a cancer associated antigen as described herein-expressing cell population, the methods comprising contacting a population of cells comprising a cancer associated antigen as described herein-expressing cell with the CAR-expressing CD4⁺ T cells and CD8⁺ T cells of the present disclosure that binds to the cancer associated antigen as described herein-expressing cell. In a specific aspect, the present disclosure provides methods for inhibiting the proliferation or reducing the population of cancer cells expressing a cancer associated antigen as described herein, the methods comprising contacting a cancer associated antigen as described herein-expressing cancer cell population with the CAR-expressing CD4⁺ T cells and CD8⁺ T cells of the present disclosure that binds to the cancer associated antigen as described herein-expressing cell. In one aspect, the present disclosure provides methods for inhibiting the proliferation or reducing the population of cancer cells expressing a cancer associate antigen as described herein, the methods comprising contacting a cancer associated antigen as described herein-expressing cancer cell population with the CAR-expressing CD4⁺ T cells and CD8⁺ T cells of the present disclosure that binds to the a cancer associated antigen as described herein-expressing cell. In certain aspects, the CAR-expressing CD4⁺ T cells and CD8⁺ T cells of the present disclosure reduces the quantity, number, amount or percentage of cells and/or cancer cells by at least 25%, at least 30%, at least 40%, at least 50%, at least 65%, at least 75%, at least 85%, at least 95%, or at least 99% in a subject with or animal model for myeloid leukemia or another cancer associated with a cancer associated antigen as described herein-expressing cells relative to a negative control. In one aspect, the subject is a human.

The present disclosure also provides methods for preventing, treating and/or managing a disease associated with a cancer associated antigen as described herein-expressing cells (e.g., a hematologic cancer or atypical cancer expessing a cancer associate antigen as described herein), the methods comprising administering to a subject in need the CAR-expressing CD4⁺ T cells and CD8⁺ T cells that binds to a cancer associate antigen as described herein-expressing cell. In one aspect, the subject is a human. Non-limiting examples of disorders associated with a cancer associated antigen as described herein-expressing cells include autoimmune disorders (such as lupus), inflammatory disorders (such as allergies and asthma) and cancers (such as hematological cancers or atypical cancers expessing a cancer associate antigen as described herein).

In some instances, a cancer that can be treated with CARTs of the present disclosure is multiple myeloma. Multiple myeloma is a cancer of the blood, characterized by accumulation of a plasma cell clone in the bone marrow. Current therapies for multiple myeloma include, but are not limited to, treatment with lenalidomide, which is an analog of thalidomide. Lenalidomide has activities which include anti-tumor activity, angiogenesis inhibition, and immunomodulation. Generally, myeloma cells are thought to be negative for a cancer associate antigen as described herein expression by flow cytometry. Thus, in some instances, a CD19 CAR, e.g., as described herein, may be used to target myeloma cells. In some instances, CARs of the present disclosure therapy can be used in combination with one or more additional therapies, e.g., lenalidomide treatment.

The present disclosure also provides methods for preventing, treating and/or managing a disease associated with a cancer associated antigen as described herein-expressing cells, the methods comprising administering to a subject in need the CAR-expressing CD4⁺ T cells and CD8⁺ T cells of the present disclosure that binds to the cancer associate antigen as described herein-expressing cell. In one aspect, the subject is a human.

The present disclosure provides methods for preventing relapse of cancer associated with a cancer associated antigen as described herein-expressing cells, the methods comprising administering to a subject in need thereof the CAR-expressing CD4⁺ T cells and CD8⁺ T cells of the disclosure that binds to the cancer associated antigen as described herein-expressing cell. In one aspect, the methods comprise administering to the subject in need thereof an effective amount of the CAR-expressing CD4⁺ T cells and CD8⁺ T cells described herein that binds to the cancer associate antigen as described herein-expressing cell in combination with an effective amount of another therapy.

### COMBINATION THERAPIES

The CAR-expressing CD4⁺ T cells and CD8⁺ T cells described herein may be used in combination with other known agents and therapies. Administered "in combination", as used herein, means that two (or more) different treatments are delivered to the subject during the course of the subject's affliction with the disorder, e.g., the two or more treatments are delivered after the subject has been diagnosed with the disorder and before the disorder has been cured or eliminated or treatment has ceased for other reasons. In some embodiments, the delivery of one treatment is still occurring when the delivery of the second begins, so that there is overlap in terms of administration. This is sometimes referred to herein as "simultaneous" or "concurrent delivery". In other embodiments, the delivery of one treatment ends before the delivery of the other treatment begins. In some embodiments of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some embodiments, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered.

The CAR-expressing CD4⁺ T cells and CD8⁺ T cells described herein and the at least one additional therapeutic agent can be administered simultaneously, in the same or in separate compositions, or sequentially. For sequential administration, the CAR-expressing cell described herein can be administered first, and the additional agent can be administered second, or the order of administration can be reversed.

The CAR therapy and/or other therapeutic agents, procedures or modalities can be administered during periods of active disorder, or during a period of remission or less active disease. The CAR therapy can be administered before another treatment, concurrently with the treatment, post-treatment, or during remission of the disorder.

When administered in combination, the CAR therapy and the additional agent (e.g., second or third agent), or all, can be administered in an amount or dose that is higher, lower or the same than the amount or dosage of each agent used individually, e.g., as a monotherapy. In certain embodiments, the administered amount or dosage of the CAR therapy, the additional agent (e.g., second or third agent), or all, is lower (e.g., at least 20%, at least 30%, at least 40%, or at least 50%) than the amount or dosage of each agent used individually, e.g., as a monotherapy. In other embodiments, the amount or dosage of the CAR therapy, the additional agent (e.g., second or third agent), or all, that results in a desired effect (e.g., treatment of cancer) is lower (e.g., at least 20%, at least 30%, at least 40%, or at least 50% lower) than the amount or dosage of each agent used individually, e.g., as a monotherapy, required to achieve the same therapeutic effect.

In further aspects, the CAR-expressing CD4⁺ T cells and CD8⁺ T cells described herein may be used in a treatment regimen in combination with surgery, chemotherapy, radiation, an mTOR pathway inhibitor, immunosuppressive agents, such as cyclosporin, azathioprine, methotrexate, mycophenolate, and FK506, antibodies, or other immunoablative agents such as CAMPATH, anti-CD3 antibodies or other antibody therapies, cytoxin, fludarabine, cyclosporin, FK506, rapamycin, mycophenolic acid, steroids, FR901228, cytokines, and irradiation, peptide vaccine, such as that described in Izumoto et al. 2008 J Neurosurg 108:963-971.

In one instance, the CAR-expressing CD4⁺ T cells and CD8⁺ T cells described herein can be used in combination with a chemotherapeutic agent. Exemplary chemotherapeutic agents include an anthracycline (e.g., doxorubicin (e.g., liposomal doxorubicin)). a vinca alkaloid (e.g., vinblastine, vincristine, vindesine, vinorelbine), an alkylating agent (e.g., cyclophosphamide, decarbazine, melphalan, ifosfamide, temozolomide), an immune cell antibody (e.g., alemtuzamab, gemtuzumab, rituximab, tositumomab), an antimetabolite (including, e.g., folic acid antagonists, pyrimidine analogs, purine analogs and adenosine deaminase inhibitors (e.g., fludarabine)), an mTOR inhibitor, a TNFR glucocorticoid induced TNFR related protein (GITR) agonist, a proteasome inhibitor (e.g., aclacinomycin A, gliotoxin or bortezomib), an immunomodulator such as thalidomide or a thalidomide derivative (e.g., lenalidomide).

General Chemotherapeutic agents considered for use in combination therapies include anastrozole (Arimidex®), bicalutamide (Casodex®), bleomycin sulfate (Blenoxane®), busulfan (Myleran®), busulfan injection (Busulfex®), capecitabine (Xeloda®), N4-pentoxycarbonyl-5-deoxy-5-fluorocytidine, carboplatin (Paraplatin®), carmustine (BiCNU®), chlorambucil (Leukeran®), cisplatin (Platinol®), cladribine (Leustatin®), cyclophosphamide (Cytoxan® or Neosar®), cytarabine, cytosine arabinoside (Cytosar-U®), cytarabine liposome injection (DepoCyt®), dacarbazine (DTIC-Dome®), dactinomycin (Actinomycin D, Cosmegan), daunorubicin hydrochloride (Cerubidine®), daunorubicin citrate liposome injection (DaunoXome®), dexamethasone, docetaxel (Taxotere®), doxorubicin hydrochloride (Adriamycin®, Rubex®), etoposide (Vepesid®), fludarabine phosphate (Fludara®), 5-fluorouracil (Adrucil®, Efudex®), flutamide (Eulexin®), tezacitibine, Gemcitabine (difluorodeoxycitidine), hydroxyurea (Hydrea®), Idarubicin (Idamycin®), ifosfamide (IFEX®), irinotecan (Camptosar®), L-asparaginase (ELSPAR®), leucovorin calcium, melphalan (Alkeran®), 6-mercaptopurine (Purinethol®), methotrexate (Folex®), mitoxantrone (Novantrone®), mylotarg, paclitaxel (Taxol®), phoenix (Yttrium90/MX-DTPA), pentostatin, polifeprosan 20 with carmustine implant (Gliadel®), tamoxifen citrate (Nolvadex®), teniposide (Vumon®), 6-thioguanine, thiotepa, tirapazamine (Tirazone®), topotecan hydrochloride for injection (Hycamptin®), vinblastine (Velban®), vincristine (Oncovin®), and vinorelbine (Navelbine®).

Exemplary alkylating agents include, without limitation, nitrogen mustards, ethylenimine derivatives, alkyl sulfonates, nitrosoureas and triazenes): uracil mustard (Aminouracil Mustard®, Chlorethaminacil®, Demethyldopan®, Desmethyldopan®, Haemanthamine®, Nordopan®, Uracil nitrogen mustard®, Uracillost®, Uracilmostaza®, Uramustin®, Uramustine®), chlormethine (Mustargen®), cyclophosphamide (Cytoxan®, Neosar®, Clafen®, Endoxan®, Procytox®, Revimmune™), ifosfamide (Mitoxana®), melphalan (Alkeran®), Chlorambucil (Leukeran®), pipobroman (Amedel®, Vercyte®), triethylenemelamine (Hemel®, Hexalen®, Hexastat®), triethylenethiophosphoramine, Temozolomide (Temodar®), thiotepa (Thioplex®), busulfan (Busilvex®, Myleran®), carmustine (BiCNU®), lomustine (CeeNU®), streptozocin (Zanosar®), and Dacarbazine (DTIC-Dome®). Additional exemplary alkylating agents include, without limitation, Oxaliplatin (Eloxatin®); Temozolomide (Temodar® and Temodal®); Dactinomycin (also known as actinomycin-D, Cosmegen®); Melphalan (also known as L-PAM, L-sarcolysin, and phenylalanine mustard, Alkeran®); Altretamine (also known as hexamethylmelamine (HMM), Hexalen®); Carmustine (BiCNU®); Bendamustine (Treanda®); Busulfan (Busulfex® and Myleran®); Carboplatin (Paraplatin®); Lomustine (also known as CCNU, CeeNU®); Cisplatin (also known as CDDP, Platinol® and Platinol®-AQ); Chlorambucil (Leukeran®); Cyclophosphamide (Cytoxan® and Neosar®); Dacarbazine (also known as DTIC, DIC and imidazole carboxamide, DTIC-Dome®); Altretamine (also known as hexamethylmelamine (HMM), Hexalen®); Ifosfamide (Ifex®); Prednumustine; Procarbazine (Matulane®); Mechlorethamine (also known as nitrogen mustard, mustine and mechloroethamine hydrochloride, Mustargen®); Streptozocin (Zanosar®); Thiotepa (also known as thiophosphoamide, TESPA and TSPA, Thioplex®); Cyclophosphamide (Endoxan®, Cytoxan®, Neosar®, Procytox®, Revimmune®); and Bendamustine HCl (Treanda®).

Exemplary mTOR inhibitors include, e.g., temsirolimus; ridaforolimus (formally known as deferolimus, (1*R*,2*R*,4*S*)-4-[(2*R*)-2[(1*R*,9*S*,12*S*,15*R*,16*E*,18*R*,19*R*,21*R*, 23*S*,24*E*,26*E*,28*Z*,30*S*,32*S*,35*R*)-1,18-dihydroxy-19,30-dimethoxy-15,17,21,23,29,35-hexamethyl-2,3,10,14,20-pentaoxo-11,36-dioxa-4-azatricyclo[30.3.1.0^{4,9}] hexatriaconta-16,24,26,28-tetraen-12-yl]propyl]-2-methoxycyclohexyl dimethylphosphinate, also known as AP23573 and MK8669, and described in PCT Publication No. WO 03/064383); everolimus (Afinitor® or RAD001); rapamycin (AY22989, Sirolimus®); simapimod (CAS 164301-51-3); emsirolimus, (5-{2,4-Bis[(3*S*)-3-methylmorpholin-4-yl]pyrido[2,3-*d*]pyrimidin-7-yl}-2-methoxyphenyl)methanol (AZD8055); 2-Amino-8-[*trans*-4-(2-hydroxyethoxy)cyclohexyl]-6-(6-methoxy-3-pyridinyl)-4-methyl-pyrido[2,3-*d*]pyrimidin-7(8*H*)-one (PF04691502, CAS 1013101-36-4); and *N*²-[1,4-dioxo-4-[[4-(4-oxo-8-phenyl-4*H*-1-benzopyran-2-yl)morpholinium-4-yl]methoxy]butyl]-L-arginylglycyl-L-α-aspartylL-serine- (SEQ ID NO: 84), inner salt (SF1126, CAS 936487-67-1), and XL765.

Exemplary immunomodulators include, e.g., afutuzumab (available from Roche®); pegfilgrastim (Neulasta®); lenalidomide (CC-5013, Revlimid®); thalidomide (Thalomid®), actimid (CC4047); and IRX-2 (mixture of human cytokines including interleukin 1, interleukin 2, and interferon γ, CAS 951209-71-5, available from IRX Therapeutics).

Exemplary anthracyclines include, e.g., doxorubicin (Adriamycin® and Rubex®); bleomycin (lenoxane®); daunorubicin (dauorubicin hydrochloride, daunomycin, and rubidomycin hydrochloride, Cerubidine®); daunorubicin liposomal (daunorubicin citrate liposome, DaunoXome®); mitoxantrone (DHAD, Novantrone®); epirubicin (Ellence™); idarubicin (Idamycin®, Idamycin PFS®); mitomycin C (Mutamycin®); geldanamycin; herbimycin; ravidomycin; and desacetylravidomycin.

Exemplary vinca alkaloids include, e.g., vinorelbine tartrate (Navelbine®), Vincristine (Oncovin®), and Vindesine (Eldisine®)); vinblastine (also known as vinblastine sulfate, vincaleukoblastine and VLB, Alkaban-AQ® and Velban®); and vinorelbine (Navelbine®).

Exemplary proteosome inhibitors include bortezomib (Velcade®); carfilzomib (PX-171-007, (*S*)-4-Methyl-*N*-((*S*)-1-(((*S*)-4-methyl-1-((*R*)-2-methyloxiran-2-yl)-1-oxopentan-2-yl)amino)-1-oxo-3-phenylpropan-2-yl)-2-((*S*)-2-(2-morpholinoacetamido)-4-phenylbutanamido)-pentanamide); marizomib (NPI-0052); ixazomib citrate (MLN-9708); delanzomib (CEP-18770); and *O*-Methyl-*N*-[(2-methyl-5-thiazolyl)carbonyl]-L-seryl-*O-*methyl-*N*-[(1*S*)-2-[(2*R*)-2-methyl-2-oxiranyl]-2-oxo-1-(phenylmethyl)ethyl]-L-serinamide (ONX-0912).

In one instance, cells expressing a CAR described herein are administered to a subject in combination with a molecule targeting GITR and/or modulating GITR functions, such as a GITR agonist and/or a GITR antibody that depletes regulatory T cells (Tregs). In one instance, the GITR binding molecules and/or molecules modulating GITR functions (e.g., GITR agonist and/or Treg depleting GITR antibodies) are administered prior to the CAR-expressing cell. For example, in one instance, the GITR agonist can be administered prior to apheresis of the cells. In one instance, the subject has CLL.

Exemplary GITR agonists include, e.g., GITR fusion proteins and anti-GITR antibodies (e.g., bivalent anti-GITR antibodies) such as, e.g., a GITR fusion protein described in U.S. Patent No.: 6,111,090, European Patent No.: 090505B1, U.S Patent No.: 8,586,023, PCT Publication Nos.: WO 2010/003118 and 2011/090754, or an anti-GITR antibody described, e.g., in U.S. Patent No.: 7,025,962, European Patent No.: 1947183B1, U.S. Patent No.: 7,812,135, U.S. Patent No.: 8,388,967, U.S. Patent No.: 8,591,886, European Patent No.: EP 1866339, PCT Publication No.: WO 2011/028683, PCT Publication No.:WO 2013/039954, PCT Publication No.: WO2005/007190, PCT Publication No.: WO 2007/133822, PCT Publication No.: WO2005/055808, PCT Publication No.: WO 99/40196, PCT Publication No.: WO 2001/03720, PCT Publication No.: WO99/20758, PCT Publication No.: WO2006/083289, PCT Publication No.: WO 2005/115451, U.S. Patent No.: 7,618,632, and PCT Publication No.: WO 2011/051726.

In an instance, CARX described herein are administered to a subject in combination with a molecule that decreases the Treg cell population. Methods that decrease the number of (e.g., deplete) Treg cells are known in the art and include, e.g., CD25 depletion, cyclophosphamide administration, and modulating GITR function. Without wishing to be bound by theory, it is believed that reducing the number of Treg cells in a subject prior to apheresis or prior to administration of a CAR-expressing cell described herein reduces the number of unwanted immune cells (e.g., Tregs) in the tumor microenvironment and reduces the subject's risk of relapse. In one instance, CARX cells described herein are administered to a subject in combination with a molecule targeting GITR and/or modulating GITR functions, such as a GITR agonist and/or a GITR antibody that depletes regulatory T cells (Tregs). In one instance, CARX cells described herein are administered to a subject in combination with cyclophosphamide. In one instance, the GITR binding molecule and/or molecule modulating GITR function (e.g., GITR agonist and/or Treg depleting GITR antibodies) is administered prior to the CARX cells. For example, in one instance, the GITR agonist can be administered prior to apheresis of the cells. In instances, cyclophosphamide is administered to the subject prior to administration (e.g., infusion or re-infusion) of the CAR-expressing cell or prior to aphersis of the cells. In instances, cyclophosphamide and an anti-GITR antibody are administered to the subject prior to administration (e.g., infusion or re-infusion) of the CAR-expressing cell or prior to apheresis of the cells. In oneinstance, the subject has cancer (e.g., a solid cancer or a hematological cancer such as ALL or CLL). In one instance, the subject has CLL. In instances, the subject has a solid cancer, e.g., a solid cancer described herein.

In one instance, a CAR expressing cell described herein is administered to a subject in combination with an mTOR inhibitor, e.g., an mTOR inhibitor described herein, e.g., a rapalog such as everolimus. In one instance, the mTOR inhibitor is administered prior to the CAR-expressing cell. For example, in one instance, the mTOR inhibitor can be administered prior to apheresis of the cells. In one instance, the subject has CLL.

In one instance, a CAR expressing cell described herein is administered to a subject in combination with a GITR agonist, e.g., a GITR agonist described herein. In one instance, the GITR agonist is administered prior to the CAR-expressing cell. For example, in one instance, the GITR agonist can be administered prior to apheresis of the cells. In one instance, the subject has CLL.

In one instance, a CAR-expressing cell described herein can be used in combination with a kinase inhibitor. In one instance, the kinase inhibitor is a CDK4 inhibitor, e.g., a CDK4 inhibitor described herein, e.g., a CDK4/6 inhibitor, such as, e.g., 7-cyclopentyl-*N,N*-dimethyl-2-((5-(piperazin-1-yl)pyridine-2-yl)amino)-7*H*-pyrrolo[2,3-d]pyrimidine-6-carboxamide (also referred to as LEE011) or 6-Acetyl-8-cyclopentyl-5-methyl-2-(5-piperazin-1-yl-pyridin-2-ylamino)-8*H*-pyrido[2,3-*d*]pyrimidin-7-one, hydrochloride (also referred to as palbociclib or PD0332991). In one instance, the kinase inhibitor is a BTK inhibitor, e.g., a BTK inhibitor described herein, such as, e.g., ibrutinib. In one instance, the kinase inhibitor is an mTOR inhibitor, e.g., an mTOR inhibitor described herein, such as, e.g., rapamycin, a rapamycin analog, OSI-027. The mTOR inhibitor can be, e.g., an mTORC1 inhibitor and/or an mTORC2 inhibitor, e.g., an mTORC1 inhibitor and/or mTORC2 inhibitor described herein. In one instance, the kinase inhibitor is a MNK inhibitor, e.g., a MNK inhibitor described herein, such as, e.g., 4-amino-5-(4-fluoroanilino)-pyrazolo[3,4-*d*]pyrimidine. The MNK inhibitor can be, e.g., a MNK1a, MNK1b, MNK2a and/or MNK2b inhibitor.

In one instance, the kinase inhibitor is a CDK4 inhibitor selected from 7-cyclopentyl-*N*,*N*-dimethyl-2-((5-(piperazin-1-yl)pyridine-2-yl)amino)-7*H*-pyrrolo[2,3-d]pyrimidine-6-carboxamide (also referred to as LEE011); aloisine A; flavopiridol or HMR-1275, 2-(2-chlorophenyl)-5,7-dihydroxy-8-[(3S,4R)-3-hydroxy-1-methyl-4-piperidinyl]-4-chromenone; crizotinib (PF-02341066; 2-(2-Chlorophenyl)-5,7-dihydroxy-8-[(2*R*,3*S*)-2-(hydroxymethyl)-1-methyl-3-pyrrolidinyl]-4*H*-1-benzopyran-4-one, hydrochloride (P276-00); 1-methyl-5-[[2-[5-(trifluoromethyl)-1*H*-imidazol-2-yl]-4-pyridinyl]oxy]-*N*-[4-(trifluoromethyl)phenyl]-1*H-*benzimidazol-2-amine (RAF265); indisulam (E7070); roscovitine (CYC202); palbociclib (PD0332991); dinaciclib (SCH727965); N-[5-[[(5-tert-butyloxazol-2-yl)methyl]thio]thiazol-2-yl]piperidine-4-carboxamide (BMS 387032); 4-[[9-chloro-7-(2,6-difluorophenyl)-5*H-*pyrimido[5,4-*d*][2]benzazepin-2-yl]amino]-benzoic acid (MLN8054); 5-[3-(4,6-difluoro-1H-benzimidazol-2-yl)-1H-indazol-5-yl]-N-ethyl-4-methyl-3-pyridinemethanamine (AG-024322); 4-(2,6-dichlorobenzoylamino)-1H-pyrazole-3-carboxylic acid N-(piperidin-4-yl)amide (AT7519); 4-[2-methyl-1-(1-methylethyl)-1*H*-imidazol-5-yl]-*N*-[4-(methylsulfonyl)phenyl]-2-pyrimidinamine (AZD5438); and XL281 (BMS908662).

In one instance, the kinase inhibitor is a CDK4 inhibitor, e.g., palbociclib (PD0332991), and the palbociclib is administered at a dose of about 50 mg, 60 mg, 70 mg, 75 mg, 80 mg, 90 mg, 100 mg, 105 mg, 110 mg, 115 mg, 120 mg, 125 mg, 130 mg, 135 mg (e.g., 75 mg, 100 mg or 125 mg) daily for a period of time, e.g., daily for 14-21 days of a 28 day cycle, or daily for 7-12 days of a 21 day cycle. In one instance, 1, 2, 3, 4, 5, 6, 7, 8, 9, 10, 11, 12 or more cycles of palbociclib are administered.

In one instance, the kinase inhibitor is a BTK inhibitor selected from ibrutinib (PCI-32765); GDC-0834; RN-486; CGI-560; CGI-1764; HM-71224; CC-292; ONO-4059; CNX-774; and LFM-A13.

In one instance, the kinase inhibitor is a BTK inhibitor, e.g., ibrutinib (PCI-32765), and the ibrutinib is administered at a dose of about 250 mg, 300 mg, 350 mg, 400 mg, 420 mg, 440 mg, 460 mg, 480 mg, 500 mg, 520 mg, 540 mg, 560 mg, 580 mg, 600 mg (e.g., 250 mg, 420 mg or 560 mg) daily for a period of time, e.g., daily for 21 day cycle cycle, or daily for 28 day cycle. In one instance, 1, 2, 3, 4, 5, 6, 7, 8, 9, 10, 11, 12 or more cycles of ibrutinib are administered.

In some instances of the methods, uses, and compositions herein, the BTK inhibitor is a BTK inhibitor described in International Application WO/2015/079417. For instance, in some instances, the BTK inhibitor is a compound of formula (I) or a pharmaceutically acceptable salt thereof; 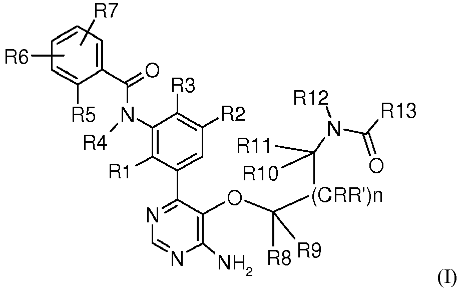 wherein,
R1 is hydrogen, C1-C6 alkyl optionally substituted by hydroxy;
R2 is hydrogen or halogen;
R3 is hydrogen or halogen;
R4 is hydrogen;
R5 is hydrogen or halogen;
or R4 and R5 are attached to each other and stand for a bond, -CH2-, -CH2-CH2- , -CH=CH-, -CH=CH-CH2-; -CH2-CH=CH-; or -CH2-CH2-CH2-;
R6 and R7 stand independently from each other for H, C1-C6 alkyl optionally substituted by hydroxyl, C3-C6 cycloalkyl optionally substituted by halogen or hydroxy, or halogen;
R8, R9, R, R', R10 and R11 independently from each other stand for H, or C1-C6 alkyl optionally substituted by C1-C6 alkoxy; or any two of R8, R9, R, R', R10 and R11 together with the carbon atom to which they are bound may form a 3 - 6 membered saturated carbocyclic ring;
R12 is hydrogen or C1-C6 alkyl optionally substituted by halogen or C1-C6 alkoxy;
or R12 and any one of R8, R9, R, R', R10 or R11 together with the atoms to which they are bound may form a 4, 5, 6 or 7 membered azacyclic ring, which ring may optionally be substituted by halogen, cyano, hydroxyl, C1-C6 alkyl or C1-C6 alkoxy;
n is 0 or 1; and
R13 is C2-C6 alkenyl optionally substituted by C1-C6 alkyl, C1-C6 alkoxy or N,N-di-C1-C6 alkyl amino; C2-C6 alkynyl optionally substituted by C1-C6 alkyl or C1-C6 alkoxy; or C2-C6 alkylenyl oxide optionally substituted by C1-C6 alkyl.

In some instances, the BTK inhibitor of Formula I is chosen from: N-(3-(5-((1-Acryloylazetidin-3-yl)oxy)-6-aminopyrimidin-4-yl)-5-fluoro-2-methylphenyl)-4-cyclopropyl-2-fluorobenzamide; (E)-N-(3-(6-Amino-5-((1-(but-2-enoyl)azetidin-3-yl)oxy)pyrimidin-4-yl)-5-fluoro-2-methylphenyl)-4-cyclopropyl-2-fluorobenzamide; N-(3-(6-Amino-5-((1-propioloylazetidin-3-yl)oxy)pyrimidin-4-yl)-5-fluoro-2-methylphenyl)-4-cyclopropyl-2-fluorobenzamide; N-(3-(6-Amino-5-((1-(but-2-ynoyl)azetidin-3-yl)oxy)pyrimidin-4-yl)-5-fluoro-2-methylphenyl)-4-cyclopropyl-2-fluorobenzamide; N-(3-(5-((1-Acryloylpiperidin-4-yl)oxy)-6-aminopyrimidin-4-yl)-5-fluoro-2-methylphenyl)-4-cyclopropyl-2-fluorobenzamide; N-(3-(6-Amino-5-(2-(N-methylacrylamido)ethoxy)pyrimidin-4-yl)-5-fluoro-2-methylphenyl)-4-cyclopropyl-2-fluorobenzamide; (E)-N-(3-(6-Amino-5-(2-(N-methylbut-2-enamido)ethoxy)pyrimidin-4-yl)-5-fluoro-2-methylphenyl)-4-cyclopropyl-2-fluorobenzamide; N-(3-(6-Amino-5-(2-(N-methylpropiolamido)ethoxy)pyrimidin-4-yl)-5-fluoro-2-methylphenyl)-4-cyclopropyl-2-fluorobenzamide; (E)-N-(3-(6-Amino-5-(2-(4-methoxy-N-methylbut-2-enamido)ethoxy)pyrimidin-4-yl)-5-fluoro-2-methylphenyl)-4-cyclopropyl-2-fluorobenzamide; N-(3-(6-Amino-5-(2-(N-methylbut-2-ynamido)ethoxy)pyrimidin-4-yl)-5-fluoro-2-methylphenyl)-4-cyclopropyl-2-fluorobenzamide; N-(2-((4-Amino-6-(3-(4-cyclopropyl-2-fluorobenzamido)-5-fluoro-2-methylphenyl)pyrimidin-5-yl)oxy)ethyl)-N-methyloxirane-2-carboxamide; N-(2-((4-Amino-6-(3-(6-cyclopropyl-8-fluoro-1-oxoisoquinolin-2(1H)-yl)phenyl)pyrimidin-5-yl)oxy)ethyl)-N-methylacrylamide; N-(3-(5-(2-Acrylamidoethoxy)-6-aminopyrimidin-4-yl)-5-fluoro-2-methylphenyl)-4-cyclopropyl-2-fluorobenzamide; N-(3-(6-Amino-5-(2-(N-ethylacrylamido)ethoxy)pyrimidin-4-yl)-5-fluoro-2-methylphenyl)-4-cyclopropyl-2-fluorobenzamide; N-(3-(6-Amino-5-(2-(N-(2-fluoroethyl)acrylamido)ethoxy)pyrimidin-4-yl)-5-fluoro-2-methylphenyl)-4-cyclopropyl-2-fluorobenzamide; N-(3-(5-((1-Acrylamidocyclopropyl)methoxy)-6-aminopyrimidin-4-yl)-5-fluoro-2-methylphenyl)-4-cyclopropyl-2-fluorobenzamide; (S)-N-(3-(5-(2-Acrylamidopropoxy)-6-aminopyrimidin-4-yl)-5-fluoro-2-methylphenyl)-4-cyclopropyl-2-fluorobenzamide; (S)-N-(3-(6-Amino-5-(2-(but-2-ynamido)propoxy)pyrimidin-4-yl)-5-fluoro-2-methylphenyl)-4-cyclopropyl-2-fluorobenzamide; (S)-N-(3-(6-Amino-5-(2-(N-methylacrylamido)propoxy)pyrimidin-4-yl)-5-fluoro-2-methylphenyl)-4-cyclopropyl-2-fluorobenzamide; (S)-N-(3-(6-Amino-5-(2-(N-methylbut-2-ynamido)propoxy)pyrimidin-4-yl)-5-fluoro-2-methylphenyl)-4-cyclopropyl-2-fluorobenzamide; N-(3-(6-Amino-5-(3-(N-methylacrylamido)propoxy)pyrimidin-4-yl)-5-fluoro-2-methylphenyl)-4-cyclopropyl-2-fluorobenzamide; (S)-N-(3-(5-((1-Acryloylpyrrolidin-2-yl)methoxy)-6-aminopyrimidin-4-yl)-5-fluoro-2-methylphenyl)-4-cyclopropyl-2-fluorobenzamide; (S)-N-(3-(6-Amino-5-((1-(but-2-ynoyl)pyrrolidin-2-yl)methoxy)pyrimidin-4-yl)-5-fluoro-2-methylphenyl)-4-cyclopropyl-2-fluorobenzamide; (S)-2-(3-(5-((1-Acryloylpyrrolidin-2-yl)methoxy)-6-aminopyrimidin-4-yl)-5-fluoro-2-(hydroxymethyl)phenyl)-6-cyclopropyl-3,4-dihydroisoquinolin-1(2H)-one; N-(2-((4-Amino-6-(3-(6-cyclopropyl-1-oxo-3,4-dihydroisoquinolin-2(1H)-yl)-5-fluoro-2-(hydroxymethyl)phenyl)pyrimidin-5-yl)oxy)ethyl)-N-methylacrylamide; N-(3-(5-(((2S,4R)-1-Acryloyl-4-methoxypyrrolidin-2-yl)methoxy)-6-aminopyrimidin-4-yl)-5-fluoro-2-methylphenyl)-4-cyclopropyl-2-fluorobenzamide; N-(3-(6-Amino-5-(((2S,4R)-1-(but-2-ynoyl)-4-methoxypyrrolidin-2-yl)methoxy)pyrimidin-4-yl)-5-fluoro-2-methylphenyl)-4-cyclopropyl-2-fluorobenzamide; 2-(3-(5-(((2S,4R)-1-Acryloyl-4-methoxypyrrolidin-2-yl)methoxy)-6-aminopyrimidin-4-yl)-5-fluoro-2-(hydroxymethyl)phenyl)-6-cyclopropyl-3,4-dihydroisoquinolin-1(2H)-one; N-(3-(5-(((2S,4S)-1-Acryloyl-4-methoxypyrrolidin-2-yl)methoxy)-6-aminopyrimidin-4-yl)-5-fluoro-2-methylphenyl)-4-cyclopropyl-2-fluorobenzamide; N-(3-(6-Amino-5-(((2S,4S)-1-(but-2-ynoyl)-4-methoxypyrrolidin-2-yl)methoxy)pyrimidin-4-yl)-5-fluoro-2-methylphenyl)-4-cyclopropyl-2-fluorobenzamide; N-(3-(5-(((2S,4R)-1-Acryloyl-4-fluoropyrrolidin-2-yl)methoxy)-6-aminopyrimidin-4-yl)-5-fluoro-2-methylphenyl)-4-cyclopropyl-2-fluorobenzamide; N-(3-(6-Amino-5-(((2S,4R)-1-(but-2-ynoyl)-4-fluoropyrrolidin-2-yl)methoxy)pyrimidin-4-yl)-5-fluoro-2-methylphenyl)-4-cyclopropyl-2-fluorobenzamide; (S)-N-(3-(5-((1-Acryloylazetidin-2-yl)methoxy)-6-aminopyrimidin-4-yl)-5-fluoro-2-methylphenyl)-4-cyclopropyl-2-fluorobenzamide; (S)-N-(3-(6-Amino-5-((1-propioloylazetidin-2-yl)methoxy)pyrimidin-4-yl)-5-fluoro-2-methylphenyl)-4-cyclopropyl-2-fluorobenzamide; (S)-2-(3-(5-((1-Acryloylazetidin-2-yl)methoxy)-6-aminopyrimidin-4-yl)-5-fluoro-2-(hydroxymethyl)phenyl)-6-cyclopropyl-3,4-dihydroisoquinolin-1(2H)-one; (R)-N-(3-(5-((1-Acryloylazetidin-2-yl)methoxy)-6-aminopyrimidin-4-yl)-5-fluoro-2-methylphenyl)-4-cyclopropyl-2-fluorobenzamide; (R)-N-(3-(5-((1-Acryloylpiperidin-3-yl)methoxy)-6-aminopyrimidin-4-yl)-5-fluoro-2-methylphenyl)-4-cyclopropyl-2-fluorobenzamide; N-(3-(5-(((2R,3S)-1-Acryloyl-3-methoxypyrrolidin-2-yl)methoxy)-6-aminopyrimidin-4-yl)-5-fluoro-2-methylphenyl)-4-cyclopropyl-2-fluorobenzamide; N-(3-(5-(((2S,4R)-1-Acryloyl-4-cyanopyrrolidin-2-yl)methoxy)-6-aminopyrimidin-4-yl)-5-fluoro-2-methylphenyl)-4-cyclopropyl-2-fluorobenzamide; or N-(3-(5-(((2S,4S)-1-Acryloyl-4-cyanopyrrolidin-2-yl)methoxy)-6-aminopyrimidin-4-yl)-5-fluoro-2-methylphenyl)-4-cyclopropyl-2-fluorobenzamide.

Unless otherwise provided, the chemical terms used above in describing the BTK inhibitor of Formula I are used according to their meanings as set out in International Application WO/2015/079417.

In one instance, the kinase inhibitor is an mTOR inhibitor selected from temsirolimus; ridaforolimus (1*R*,2*R*,4*S*)-4-[(2*R*)-2[(1*R*,9*S*,12*S*,15*R*,16*E*,18*R*,19*R*,21*R*, 23*S*,24*E*,26*E*,28*Z*,30*S*,32*S*,35*R*)-1,18-dihydroxy-19,30-dimethoxy-15,17,21,23, 29,35-hexamethyl-2,3,10,14,20-pentaoxo-11,36-dioxa-4-azatricyclo[30.3.1.0^{4,9}] hexatriaconta-16,24,26,28-tetraen-12-yl]propyl]-2-methoxycyclohexyl dimethylphosphinate, also known as AP23573 and MK8669; everolimus (RAD001); rapamycin (AY22989); simapimod; (5-{2,4-bis[(3*S*)-3-methylmorpholin-4-yl]pyrido[2,3-*d*]pyrimidin-7-yl}-2-methoxyphenyl)methanol (AZD8055); 2-mmino-8-[*trans*-4-(2-hydroxyethoxy)cyclohexyl]-6-(6-methoxy-3-pyridinyl)-4-methyl-pyrido[2,3-*d*]pyrimidin-7(8*H*)-one (PF04691502); and *N*²-[1,4-dioxo-4-[[4-(4-oxo-8-phenyl-4*H*-1-benzopyran-2-yl)morpholinium-4-yl]methoxy]butyl]-L-arginylglycyl-L-α-aspartylL-serine- (SEQ ID NO: 84), inner salt (SF1126); and XL765.

In one instance, the kinase inhibitor is an mTOR inhibitor, e.g., rapamycin, and the rapamycin is administered at a dose of about 3 mg, 4 mg, 5 mg, 6 mg, 7 mg, 8 mg, 9 mg, 10 mg (e.g., 6 mg) daily for a period of time, e.g., daily for 21 day cycle cycle, or daily for 28 day cycle. In one instance, 1, 2, 3, 4, 5, 6, 7, 8, 9, 10, 11, 12 or more cycles of rapamycin are administered. In one instance, the kinase inhibitor is an mTOR inhibitor, e.g., everolimus and the everolimus is administered at a dose of about 2 mg, 2.5 mg, 3 mg, 4 mg, 5 mg, 6 mg, 7 mg, 8 mg, 9 mg, 10 mg, 11 mg, 12 mg, 13 mg, 14 mg, 15 mg (e.g., 10 mg) daily for a period of time, e.g., daily for 28 day cycle. In one instance, 1, 2, 3, 4, 5, 6, 7, 8, 9, 10, 11, 12 or more cycles of everolimus are administered.

In one instance, the kinase inhibitor is an MNK inhibitor selected from CGP052088; 4-amino-3-(p-fluorophenylamino)-pyrazolo[3,4-*d*]pyrimidine (CGP57380); cercosporamide; ETC-1780445-2; and 4-amino-5-(4-fluoroanilino)-pyrazolo[3,4-*d*]pyrimidine.

Drugs that inhibit either the calcium dependent phosphatase calcineurin (cyclosporine and FK506) or inhibit the p70S6 kinase that is important for growth factor induced signaling (rapamycin). (Liu et al., Cell 66:807-815, 1991; Henderson et al., Immun. 73:316-321, 1991; Bierer et al., Curr. Opin. Immun. 5:763-773, 1993) can also be used. In a further aspect, the cell compositions of the present invention may be administered to a patient in conjunction with (e.g., before, simultaneously or following) bone marrow transplantation, T cell ablative therapy using chemotherapy agents such as, fludarabine, external-beam radiation therapy (XRT), cyclophosphamide, and/or antibodies such as OKT3 or CAMPATH. In one aspect, the cell compositions of the present invention are administered following B-cell ablative therapy such as agents that react with CD20, e.g., Rituxan. For example, in one embodiment, subjects may undergo standard treatment with high dose chemotherapy followed by peripheral blood stem cell transplantation. In certain instances, following the transplant, subjects receive an infusion of the expanded immune cells of the present disclosure. In an additional instance, expanded cells are administered before or following surgery.

In some instances, a CAR-expressing cell described herein is administered in combination with an oncolytic virus. In instances, oncolytic viruses are capable of selectively replicating in and triggering the death of or slowing the growth of a cancer cell. In some cases, oncolytic viruses have no effect or a minimal effect on non-cancer cells. An oncolytic virus includes but is not limited to an oncolytic adenovirus, oncolytic Herpes Simplex Viruses, oncolytic retrovirus, oncolytic parvovirus, oncolytic vaccinia virus, oncolytic Sinbis virus, oncolytic influenza virus, or oncolytic RNA virus (e.g., oncolytic reovirus, oncolytic Newcastle Disease Virus (NDV), oncolytic measles virus, or oncolytic vesicular stomatitis virus (VSV)).

In some instances, the oncolytic virus is a virus, e.g., recombinant oncolytic virus, described in US2010/0178684 A1. In some instances, a recombinant oncolytic virus comprises a nucleic acid sequence (e.g., heterologous nucleic acid sequence) encoding an inhibitor of an immune or inflammatory response, e.g., as described in US2010/0178684 A1. In instances, the recombinant oncolytic virus, e.g., oncolytic NDV, comprises a pro-apoptotic protein (e.g., apoptin), a cytokine (e.g., GM-CSF, interferon-gamma, interleukin-2 (IL-2), tumor necrosis factor-alpha), an immunoglobulin (e.g., an antibody against ED-B firbonectin), tumor associated antigen, a bispecific adapter protein (e.g., bispecific antibody or antibody fragment directed against NDV HN protein and a T cell co-stimulatory receptor, such as CD3 or CD28; or fusion protein between human IL-2 and single chain antibody directed against NDV HN protein). See, e.g., Zamarin et al. Future Microbiol. 7.3(2012):347-67. In some instances, the oncolytic virus is a chimeric oncolytic NDV described in US 8591881 B2, US 2012/0122185 A1, or US 2014/0271677 A1.

In some instances, the oncolytic virus comprises a conditionally replicative adenovirus (CRAd), which is designed to replicate exclusively in cancer cells. See, e.g., Alemany et al. Nature Biotechnol. 18(2000):723-27. In some instances, an oncolytic adenovirus comprises one described in Table 1 on page 725 of Alemany et al..

Exemplary oncolytic viruses include but are not limited to the following:
Group B Oncolytic Adenovirus (ColoAd1) (PsiOxus Therapeutics Ltd.) (see, e.g., Clinical Trial Identifier: NCT02053220);
ONCOS-102 (previously called CGTG-102), which is an adenovirus comprising granulocyte-macrophage colony stimulating factor (GM-CSF) (Oncos Therapeutics) (see, e.g., Clinical Trial Identifier: NCT01598129);
VCN-01, which is a genetically modified oncolytic human adenovirus encoding human PH20 hyaluronidase (VCN Biosciences, S.L.) (see, e.g., Clinical Trial Identifiers: NCT02045602 and NCT02045589);
Conditionally Replicative Adenovirus ICOVIR-5, which is a virus derived from wild-type human adenovirus serotype 5 (Had5) that has been modified to selectively replicate in cancer cells with a deregulated retinoblastoma/E2F pathway (Institut Català d'Oncologia) (see, e.g., Clinical Trial Identifier: NCT01864759);
Celyvir, which comprises bone marrow-derived autologous mesenchymal stem cells (MSCs) infected with ICOVIR5, an oncolytic adenovirus (Hospital Infantil Universitario Niño Jesus, Madrid, Spain/ Ramon Alemany) (see, e.g., Clinical Trial Identifier: NCT01844661);
CG0070, which is a conditionally replicating oncolytic serotype 5 adenovirus (Ad5) in which human E2F-1 promoter drives expression of the essential E1a viral genes, thereby restricting viral replication and cytotoxicity to Rb pathway-defective tumor cells (Cold Genesys, Inc.) (see, e.g., Clinical Trial Identifier: NCT02143804); or
DNX-2401 (formerly named Delta-24-RGD), which is an adenovirus that has been engineered to replicate selectively in retinoblastoma (Rb)-pathway deficient cells and to infect cells that express certain RGD-binding integrins more efficiently (Clinica Universidad de Navarra, Universidad de Navarra/ DNAtrix, Inc.) (see, e.g., Clinical Trial Identifier: NCT01956734).

In some instances, an oncolytic virus described herein is administering by injection, e.g., subcutaneous, intra-arterial, intravenous, intramuscular, intrathecal, or intraperitoneal injection. In instances, an oncolytic virus described herein is administered intratumorally, transdermally, transmucosally, orally, intranasally, or via pulmonary administration.

In one embodiment, the subject can be administered an agent which reduces or ameliorates a side effect associated with the administration of the CAR-expressing CD4⁺ T cells and CD8⁺ T cells. Side effects associated with the administration of the CAR-expressing CD4⁺ T cells and CD8⁺ T cells include, but are not limited to CRS, and hemophagocytic lymphohistiocytosis (HLH), also termed Macrophage Activation Syndrome (MAS). Symptoms of CRS include high fevers, nausea, transient hypotension, hypoxia, and the like. CRS may include clinical constitutional signs and symptoms such as fever, fatigue, anorexia, myalgias, arthalgias, nausea, vomiting, and headache. CRS may include clinical skin signs and symptoms such as rash. CRS may include clinical gastrointestinal signs and symsptoms such as nausea, vomiting and diarrhea. CRS may include clinical respiratory signs and symptoms such as tachypnea and hypoxemia. CRS may include clinical cardiovascular signs and symptoms such as tachycardia, widened pulse pressure, hypotension, increased cardac output (early) and potentially diminished cardiac output (late). CRS may include clinical coagulation signs and symptoms such as elevated d-dimer, hypofibrinogenemia with or without bleeding. CRS may include clinical renal signs and symptoms such as azotemia. CRS may include clinical hepatic signs and symptoms such as transaminitis and hyperbilirubinemia. CRS may include clinical neurologic signs and symptoms such as headache, mental status changes, confusion, delirium, word finding difficulty or frank aphasia, hallucinations, tremor, dymetria, altered gait, and seizures.

Accordingly, the methods described herein can comprise administering a CAR-expressing cell described herein to a subject and further administering one or more agents to manage elevated levels of a soluble factor resulting from treatment with the CAR-expressing CD4⁺ T cells and CD8⁺ T cells. In one -instance, the soluble factor elevated in the subject is one or more of IFN-γ, TNFα, IL-2 and IL-6. In an instance, the factor elevated in the subject is one or more of IL-1, GM-CSF, IL-10, IL-8, IL-5 and fraktalkine. Therefore, an agent administered to treat this side effect can be an agent that neutralizes one or more of these soluble factors. In one embodiment, the agent that neutralizes one or more of these soluble forms is an antibody or antigen binding fragment thereof. Examples of such agents include, but are not limited to a steroid (e.g., corticosteroid), an inhibitor of TNFα, and an inhibitor of IL-6. An example of a TNFα inhibitor is an anti-TNFa antibody molecule such as, infliximab, adalimumab, certolizumab pegol, and golimumab. Another example of a TNFα inhibitor is a fusion protein such as entanercept. Small molecule inhibitor of TNFα include, but are not limited to, xanthine derivatives (e.g. pentoxifylline) and bupropion. An example of an IL-6 inhibitor is an anti-IL-6 antibody molecule such as tocilizumab (toc), sarilumab, elsilimomab, CNTO 328, ALD518/BMS-945429, CNTO 136, CPSI-2364, CDP6038, VX30, ARGX-109, FE301, and FM101. In one embodiment, the anti-IL-6 antibody molecule is tocilizumab. An example of an IL-1R based inhibitor is anakinra.

In instances, a lymphodepleting chemotherapy is administered to the subject prior to, concurrently with, or after administration (e.g., infusion) of CAR cells, e.g., CD4+ or CD8+ cells described herein. In an example, the lymphodepleting chemotherapy is administered to the subject prior to administration of CAR cells. For example, the lymphodepleting chemotherapy ends 1-4 days (e.g., 1, 2, 3, or 4 days) prior to CAR cell infusion. In instances, multiple doses of CAR cells are administered, e.g., as described herein. For example, a single dose comprises about 5 x 10⁸ CAR cells. In instances, a lymphodepleting chemotherapy is administered to the subject prior to, concurrently with, or after administration (e.g., infusion) of a CAR-expressing cell described herein.

In some instances, CARX cells described herein (e.g., CAR^{CD4+} cells and/or CAR^{CD8+} cells), is administered to a subject in combination with a CD19 CAR-expressing cell, e.g., CTL019, e.g., as described in WO2012/079000, for treatment of a disease associated with the expression of cancer antigen, e.g., a cancer described herein. Without being bound by theory, it is believed that administering a CD19 CAR-expressing cell in combination with another CAR-expressing cell improves the efficacy of a CAR-expressing cell described herein by targeting early lineage cancer cells, e.g., cancer stem cells, modulating the immune response, depleting regulatory B cells, and/or improving the tumor microenvironment. For example, a CD19 CAR-expressing cell targets cancer cells that express early lineage markers, e.g., cancer stem cells and CD19-expressing cells, while the other CAR-expressing cell described herein targets cancer cells that express later lineage markers, e.g., CD33. This preconditioning approach can improve the efficacy of the CAR-expressing cell described herein. In such instances, the CD19 CAR-expressing cell is administered prior to, concurrently with, or after administration (e.g., infusion) of the second CAR-expressing cell.

In embodiments, a CAR-expressing cell which expresses a CAR targeting a cancer antigen other than CD19 also expresses a CAR targeting CD19, e.g., a CD19 CAR. In an embodiment, the cell expressing a non-CD 19 CAR and a CD19 CAR is administered to a subject for treatment of a cancer described herein, e.g., AML. In an embodiment, the configurations of one or both of the CAR molecules comprise a primary intracellular signaling domain and a costimulatory signaling domain. In another embodiment, the configurations of one or both of the CAR molecules comprise a primary intracellular signaling domain and two or more, e.g., 2, 3, 4, or 5 or more, costimulatory signaling domains. In such embodiments, the non-CD 19 CAR molecule and the CD19 CAR may have the same or a different primary intracellular signaling domain, the same or different costimulatory signaling domains, or the same number or a different number of costimulatory signaling domains. Alternatively, the non-CD19 CAR and the CD19 CAR are configured as a split CAR, in which one of the CAR molecules comprises an antigen binding domain and a costimulatory domain (e.g., 4-1BB), while the other CAR molecule comprises an antigen binding domain and a primary intracellular signaling domain (e.g., CD3 zeta).

In one instance, the subject can be administered an agent which enhances the activity of the CAR-expressing CD4⁺ T cells and CD8⁺ T cells described herein. For example, in one instance, the agent can be an agent which inhibits an inhibitory molecule, e.g., the agent is a checkpoint inhibitor. Inhibitory molecules, e.g., Programmed Death 1 (PD-1), can, in some instances, decrease the ability of a CAR-expressing cell to mount an immune effector response. Examples of inhibitory molecules include PD-1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta, e.g., as described herein. Inhibition of an inhibitory molecule, e.g., by inhibition at the DNA, RNA or protein level, can optimize performance of the CAR-expressing CD4⁺ T cells and CD8⁺ T cells. In embodiments, an inhibitory nucleic acid, e.g., an inhibitory nucleic acid, e.g., a dsRNA, e.g., an siRNA or shRNA, a clustered regularly interspaced short palindromic repeats (CRISPR), a transcription-activator like effector nuclease (TALEN), or a zinc finger endonuclease (ZFN), e.g., as described herein, can be used to inhibit expression of an inhibitory molecule in the CAR-expressing CD4⁺ T cells and CD8⁺ T cells. In an embodiment the inhibitor is an shRNA. In an embodiment, the inhibitory molecule is inhibited within the CAR-expressing CD4⁺ T cells and CD8⁺ T cells. In these embodiments, a dsRNA molecule that inhibits expression of the inhibitory molecule is linked to the nucleic acid that encodes a component, e.g., all of the components, of the CAR.

In an instance, a nucleic acid molecule that encodes a dsRNA molecule that inhibits expression of the molecule that modulates or regulates, e.g., inhibits, T-cell function is operably linked to a promoter, e.g., a H1- or a U6-derived promoter such that the dsRNA molecule that inhibits expression of the molecule that modulates or regulates, e.g., inhibits, T-cell function is expressed, e.g., is expressed within a CAR-expressing cell. See e.g., Tiscornia G., "Development of Lentiviral Vectors Expressing siRNA," Chapter 3, in Gene Transfer: Delivery and Expression of DNA and RNA (eds. Friedmann and Rossi). Cold Spring Harbor Laboratory Press, Cold Spring Harbor, NY, USA, 2007; Brummelkamp TR, et al. (2002) Science 296: 550-553; Miyagishi M, et al. (2002) Nat. Biotechnol. 19: 497-500. In an instance the nucleic acid molecule that encodes a dsRNA molecule that inhibits expression of the molecule that modulates or regulates, e.g., inhibits, T-cell function is present on the same vector, e.g., a lentiviral vector, that comprises a nucleic acid molecule that encodes a component, e.g., all of the components, of the CAR. In such an instance, the nucleic acid molecule that encodes a dsRNA molecule that inhibits expression of the molecule that modulates or regulates, e.g., inhibits, T-cell function is located on the vector, e.g., the lentiviral vector, 5'- or 3'- to the nucleic acid that encodes a component, e.g., all of the components, of the CAR. The nucleic acid molecule that encodes a dsRNA molecule that inhibits expression of the molecule that modulates or regulates, e.g., inhibits, T-cell function can be transcribed in the same or different direction as the nucleic acid that encodes a component, e.g., all of the components, of the CAR. In an instance the nucleic acid molecule that encodes a dsRNA molecule that inhibits expression of the molecule that modulates or regulates, e.g., inhibits, T-cell function is present on a vector other than the vector that comprises a nucleic acid molecule that encodes a component, e.g., all of the components, of the CAR. In an embodiment, the nucleic acid molecule that encodes a dsRNA molecule that inhibits expression of the molecule that modulates or regulates, e.g., inhibits, T-cell function it transiently expressed within a CAR-expressing cell. In an embodiment, the nucleic acid molecule that encodes a dsRNA molecule that inhibits expression of the molecule that modulates or regulates, e.g., inhibits, T-cell function is stably integrated into the genome of a CAR-expressing cell. In an embodiment, the the molecule that modulates or regulates, e.g., inhibits, T-cell function is PD-1.

In one instance, the inhibitor of an inhibitory signal can be, e.g., an antibody or antibody fragment that binds to an inhibitory molecule. For example, the agent can be an antibody or antibody fragment that binds to PD-1, PD-L1, PD-L2, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, TGFR beta, or CTLA4 (e.g., ipilimumab (also referred to as MDX-010 and MDX-101, and marketed as Yervoy®; Bristol-Myers Squibb; Tremelimumab (IgG2 monoclonal antibody available from Pfizer, formerly known as ticilimumab, CP-675,206).). In an instance, the agent is an antibody or antibody fragment that binds to TIM3. In an instance, the agent is an antibody or antibody fragment that binds to LAG3. In instances, the agent that enhances the activity of a CAR-expressing cell, e.g., inhibitor of an inhibitory molecule, is administered in combination with an allogeneic CAR, e.g., an allogeneic CAR described herein (e.g., described in the Allogeneic CAR section herein).

PD-1 is an inhibitory member of the CD28 family of receptors that also includes CD28, CTLA-4, ICOS, and BTLA. PD-1 is expressed on activated B cells, T cells and myeloid cells (Agata et al. 1996 Int. Immunol 8:765-75). Two ligands for PD-1, PD-L1 and PD-L2 have been shown to downregulate T cell activation upon binding to PD-1 (Freeman et a. 2000 J Exp Med 192:1027-34; Latchman et al. 2001 Nat Immunol 2:261-8; Carter et al. 2002 Eur J Immunol 32:634-43). PD-L1 is abundant in human cancers (Dong et al. 2003 J Mol Med 81:281-7; Blank et al. 2005 Cancer Immunol. Immunother 54:307-314; Konishi et al. 2004 Clin Cancer Res 10:5094). Immune suppression can be reversed by inhibiting the local interaction of PD-1 with PD-L1. Antibodies, antibody fragments, and other inhibitors of PD-1, PD-L1 and PD-L2 are available in the art and may be used combination with a CAR of the present invention described herein. For example, nivolumab (also referred to as BMS-936558 or MDX1106; Bristol-Myers Squibb) is a fully human IgG4 monoclonal antibody which specifically blocks PD-1. Nivolumab (clone 5C4) and other human monoclonal antibodies that specifically bind to PD-1 are disclosed in US 8,008,449 and WO2006/121168. Pidilizumab (CT-011; Cure Tech) is a humanized IgGlk monoclonal antibody that binds to PD-1. Pidilizumab and other humanized anti-PD-1 monoclonal antibodies are disclosed in WO2009/101611. Lambrolizumab (also referred to as MK03475; Merck) is a humanized IgG4 monoclonal antibody that binds to PD-1. Lambrolizumab and other humanized anti-PD-1 antibodies are disclosed in US 8,354,509 and WO2009/114335. MEDI4736 (Medimmune) is a human monoclonal antibody that binds to PDL1, and inhibits interaction of the ligand with PD1. MDPL3280A (Genentech / Roche) is a human Fc optimized IgG1 monoclonal antibody that binds to PD-L1. MDPL3280A and other human monoclonal antibodies to PD-L1 are disclosed in U.S. Patent No.: 7,943,743 and U.S Publication No.: 20120039906. Other anti-PD-L1 binding agents include YW243.55.S70 (heavy and light chain variable regions are shown in SEQ ID NOs 20 and 21 in WO2010/077634) and MDX-1 105 (also referred to as BMS-936559, and, e.g., anti-PD-Ll binding agents disclosed in WO2007/005874). AMP-224 (B7-DCIg; Amplimmune; e.g., disclosed in WO2010/027827 and WO2011/066342), is a PD-L2 Fc fusion soluble receptor that blocks the interaction between PD-1 and B7-H1. Other anti-PD-1 antibodies include AMP 514 (Amplimmune), among others, e.g., anti-PD-1 antibodies disclosed in US 8,609,089, US 2010028330, and/or US 20120114649.

TIM3 (T cell immunoglobulin-3) also negatively regulates T cell function, particularly in IFN-g-secreting CD4+ T helper 1 and CD8+ T cytotoxic 1 cells, and plays a critical role in T cell exhaustion. Inhibition of the interaction between TIM3 and its ligands, e.g., galectin-9 (Gal9), phosphotidylserine (PS), and HMGB1, can increase immune response. Antibodies, antibody fragments, and other inhibitors of TIM3 and its ligands are available in the art and may be used combination with a CD19 CAR described herein. For example, antibodies, antibody fragments, small molecules, or peptide inhibitors that target TIM3 binds to the IgV domain of TIM3 to inhibit interaction with its ligands. Antibodies and peptides that inhibit TIM3 are disclosed in WO2013/006490 and US20100247521. Other anti-TIM3 antibodies include humanized versions of RMT3-23 (disclosed in Ngiow et al., 2011, Cancer Res, 71:3540-3551), and clone 8B.2C12 (disclosed in Monney et al., 2002, Nature, 415:536-541). Bi-specific antibodies that inhibit TIM3 and PD-1 are disclosed in US20130156774.

In other instances, the agent which enhances the activity of a CAR-expressing cell is a CEACAM inhibitor (e.g., CEACAM-1, CEACAM-3, and/or CEACAM-5 inhibitor). In one instance, the inhibitor of CEACAM is an anti-CEACAM antibody molecule. Exemplary anti-CEACAM-1 antibodies are described in WO 2010/125571, WO 2013/082366 WO 2014/059251 and WO 2014/022332, e.g., a monoclonal antibody 34B1, 26H7, and 5F4; or a recombinant form thereof, as described in, e.g., US 2004/0047858, US 7,132,255 and WO 99/052552. In other instances, the anti-CEACAM antibody binds to CEACAM-5 as described in, e.g., Zheng et al. PLoS One. 2010 Sep 2;5(9). pii: e12529 (DOI:10:1371/journal.pone.0021146), or crossreacts with CEACAM-1 and CEACAM-5 as described in, e.g., WO 2013/054331 and US 2014/0271618.

Without wishing to be bound by theory, carcinoembryonic antigen cell adhesion molecules (CEACAM), such as CEACAM-1 and CEACAM-5, are believed to mediate, at least in part, inhibition of an anti-tumor immune response (see e.g., Markel et al. J Immunol. 2002 Mar 15;168(6):2803-10; Markel et al. J Immunol. 2006 Nov 1;177(9):6062-71; Markel et al. Immunology. 2009 Feb;126(2):186-200; Markel et al. Cancer Immunol Immunother. 2010 Feb;59(2):215-30; Ortenberg et al. Mol Cancer Ther. 2012 Jun;11(6):1300-10; Stern et al. J Immunol. 2005 Jun 1;174(11):6692-701; Zheng et al. PLoS One. 2010 Sep 2;5(9). pii: e12529). For example, CEACAM-1 has been described as a heterophilic ligand for TIM-3 and as playing a role in TIM-3-mediated T cell tolerance and exhaustion (see e.g., WO 2014/022332; Huang, et al. (2014) Nature doi:10.1038/nature13848). In instances, co-blockade of CEACAM-1 and TIM-3 has been shown to enhance an anti-tumor immune response in xenograft colorectal cancer models (see e.g., WO 2014/022332; Huang, et al. (2014), supra). In other instances, co-blockade of CEACAM-1 and PD-1 reduce T cell tolerance as described, e.g., in WO 2014/059251. Thus, CEACAM inhibitors can be used with the other immunomodulators described herein (e.g., anti-PD-1 and/or anti-TIM-3 inhibitors) to enhance an immune response against a cancer, e.g., a melanoma, a lung cancer (e.g., NSCLC), a bladder cancer, a colon cancer an ovarian cancer, and other cancers as described herein.

LAG3 (lymphocyte activation gene-3 or CD223) is a cell surface molecule expressed on activated T cells and B cells that has been shown to play a role in CD8+ T cell exhaustion. Antibodies, antibody fragments, and other inhibitors of LAG3 and its ligands are available in the art and may be used combination with a CD19 CAR described herein. For example, BMS-986016 (Bristol-Myers Squib) is a monoclonal antibody that targets LAG3. IMP701 (Immutep) is an antagonist LAG3 antibody and IMP731 (Immutep and GlaxoSmithKline) is a depleting LAG3 antibody. Other LAG3 inhibitors include IMP321 (Immutep), which is a recombinant fusion protein of a soluble portion of LAG3 and Ig that binds to MHC class II molecules and activates antigen presenting cells (APC). Other antibodies are disclosed, e.g., in WO2010/019570.

In some instances, the agent which enhances the activity of a CAR-expressing CD4⁺ T cells and/or a CAR-expressing CD8⁺ T cells, e.g., a fusion protein comprising a first domain and a second domain, wherein the first domain is an inhibitory molecule, or fragment thereof, and the second domain is a polypeptide that is associated with a positive signal, e.g., a polypeptide comrpsing an antracellular signaling domain as described herein. In some instances, the polypeptide that is associated with a positive signal can include a costimulatory domain of CD28, CD27, ICOS, e.g., an intracellular signaling domain of CD28, CD27 and/or ICOS, and/or a primary signaling domain, e.g., of CD3 zeta, e.g., described herein. In one embodiment, the fusion protein is expressed by the same cell that expressed the CAR. In another instance, the fusion protein is expressed by a cell, e.g., a T cell that does not express a CAR of the present disclosure.

In one instance, the agent which enhances activity of the CAR-expressing CD4⁺ T cells and CD8⁺ T cells described herein is miR-17-92.

In one instance, the agent which enhances activity of CARX cells described herein is a cytokine. Cytokines have important functions related to T cell expansion, differentiation, survival, and homeostatis. Cytokines that can be administered to the subject receiving CARX cells described herein include: IL-2, IL-4, IL-7, IL-9, IL-15, IL-18, and IL-21, or a combination thereof. In preferred instances, the cytokine administered is IL-7, IL-15, or IL-21, or a combination thereof. The cytokine can be administered once a day or more than once a day, e.g., twice a day, three times a day, or four times a day. The cytokine can be administered for more than one day, e.g. the cytokine is administered for 2 days, 3 days, 4 days, 5 days, 6 days, 1 week, 2 weeks, 3 weeks, or 4 weeks. For example, the cytokine is administered once a day for 7 days.

In instances, the cytokine is administered in combination with CAR-expressing cells. The cytokine can be administered simultaneously or concurrently with the CAR-expressing cells, e.g., administered on the same day. The cytokine may be prepared in the same pharmaceutical composition as the CAR-expressing cells, or may be prepared in a separate pharmaceutical composition. Alternatively, the cytokine can be administered shortly after administration of the CAR-expressing cells, e.g., 1 day, 2 days, 3 days, 4 days, 5 days, 6 days, or 7 days after administration of the CAR-expressing cells. In instances where the cytokine is administered in a dosing regimen that occurs over more than one day, the first day of the cytokine dosing regimen can be on the same day as administration with the CAR-expressing cells, or the first day of the cytokine dosing regimen can be 1 day, 2 days, 3 days, 4 days, 5 days, 6 days, or 7 days after administration of the CAR-expressing cells. In one instance, on the first day, the CAR-expressing cells are administered to the subject, and on the second day, a cytokine is administered once a day for the next 7 days. In a preferred instance, the cytokine to be administered in combination with the CAR-expressing cells is IL-7, IL-15, or IL-21, or a combination thereof.

In other instances, the cytokine is administered a sufficient period of time after administration of the CAR-expressing cells, e.g., at least 2 weeks, 3 weeks, 4 weeks, 6 weeks, 8 weeks, 10 weeks, 12 weeks, 4 months, 5 months, 6 months, 7 months, 8 months, 9 months, 10 months, 11 months, or 1 year or more after administration of CAR-expressing cells. In one instance, the cytokine is administered after assessment of the subject's response to the CAR-expressing cells. For example, the subject is administered CAR-expressing cells according to the dosage and regimens described herein. The response of the subject to CART therapy is assessed at 2 weeks, 3 weeks, 4 weeks, 6 weeks, 8 weeks, 10 weeks, 12 weeks, 4 months, 5 months, 6 months, 7 months, 8 months, 9 months, 10 months, 11 months, or 1 year or more after administration of CAR-expressing cells, using any of the methods described herein, including inhibition of tumor growth, reduction of circulating tumor cells, or tumor regression. Subjects that do not exhibit a sufficient response to CART therapy can be administered a cytokine. Administration of the cytokine to the subject that has sub-optimal response to the CART therapy improves CART efficacy or anti-tumor activity. In an instance, the cytokine administered after administration of CAR-expressing cells is IL-7.

### COMBINATION WITH A LOW, IMMUNE ENHANCING DOSE OF AN MTOR INHIBITOR

CD4⁺ T cells expressing a CAR^{CD4+} and/or CD8⁺ T cells expressing a CAR^{CD8+} described herein, can be administered in combination with a low, immune enhancing dose of an mTOR inhibitor. Methods described herein use low, immune enhancing, doses of mTOR inhibitors, e.g., allosteric mTOR inhibitors, including rapalogs such as RAD001. Administration of a low, immune enhancing, dose of an mTOR inhibitor (e.g., a dose that is insufficient to completely suppress the immune system, but sufficient to improve immune function) can optimize the performance of immune effector cells, e.g., T cells or CAR-expressing cells, in the subject. Methods for measuring mTOR inhibition, dosages, treatment regimens, and suitable pharmaceutical compositions are described in U.S. Patent Application No. 2015/01240036.

In an instance, administration of a low, immune enhancing, dose of an mTOR inhibitor can result in one or more of the following:
i) a decrease in the number of PD-1 positive immune effector cells;
ii) an increase in the number of PD-1 negative immune effector cells;
iii) an increase in the ratio of PD-1 negative immune effector cells / PD-1 positive immune effector cells;
iv) an increase in the number of naive T cells;
v) an increase in the expression of one or more of the following markers: CD62L^{high}, CD127^{high}, CD27⁺, and BCL2, e.g., on memory T cells, e.g., memory T cell precursors;
vi) a decrease in the expression of KLRG1, e.g., on memory T cells, e.g., memory T cell precursors; or
vii) an increase in the number of memory T cell precursors, e.g., cells with any one or combination of the following characteristics: increased CD62L^{high}, increased CD127^{high}, increased CD27+, decreased KLRG1, and increased BCL2;
and wherein any of the foregoing, e.g., i), ii), iii), iv), v), vi), or vii), occurs e.g., at least transiently, e.g., as compared to a non-treated subject.

In another instance, administration of a low, immune enhancing, dose of an mTOR inhibitor results in increased or prolonged proliferation or persistence of CAR-expressing cells, e.g., in culture or in a subject, e.g., as compared to non-treated CAR-expressing cells or a non-treated subject. In instances, increased proliferation or persistence is associated with in an increase in the number of CAR-expressing cells. Methods for measuring increased or prolonged proliferation are described in Examples 8 and 9. In another instance, administration of a low, immune enhancing, dose of an mTOR inhibitor results in increased killing of cancer cells by CAR-expressing cells, e.g., in culture or in a subject, e.g., as compared to non-treated CAR-expressing cells or a non-treated subject. In instances, increased killing of cancer cells is associated with in a decrease in tumor volume. Methods for measuring increased killing of cancer cells are described in Example 6.

In one instance, the cells expressing a CAR molecule, e.g., a CAR molecule described herein, are administered in combination with a low, immune enhancing dose of an mTOR inhibitor, e.g., an allosteric mTOR inhibitor, e.g., RAD001, or a catalytic mTOR inhibitor. For example, administration of the low, immune enhancing, dose of the mTOR inhibitor can be initiated prior to administration of a CAR-expressing cell described herein; completed prior to administration of a CAR-expressing cell described herein; initiated at the same time as administration of a CAR-expressing cell described herein; overlapping with administration of a CAR-expressing cell described herein; or continuing after administration of a CAR-expressing cell described herein.

Alternatively or in addition, administration of a low, immune enhancing, dose of an mTOR inhibitor can optimize immune effector cells to be engineered to express a CAR molecule described herein. In such instances, administration of a low, immune enhancing, dose of an mTOR inhibitor, e.g., an allosteric inhibitor, e.g., RAD001, or a catalytic inhibitor, is initiated or completed prior to harvest of immune effector cells, e.g., T cells or NK cells, to be engineered to express a CAR molecule described herein, from a subject.

In another instance, immune effector cells, e.g., T cells or NK cells, to be engineered to express a CAR molecule described herein, e.g., after harvest from a subject, or CAR-expressing immune effector cells, e.g., T cells or NK cells, e.g., prior to administration to a subject, can be cultured in the presence of a low, immune enhancing, dose of an mTOR inhibitor.

In an instance, administering to the subject a low, immune enhancing, dose of an mTOR inhibitor comprises administering, e.g., once per week, e.g., in an immediate release dosage form, 0.1 to 20, 0.5 to 10, 2.5 to 7.5, 3 to 6, or about 5, mgs of RAD001, or a bioequivalent dose thereof. In an instance, administering to the subject a low, immune enhancing, dose of an mTOR inhibitor comprises administering, e.g., once per week, e.g., in a sustained release dosage form, 0.3 to 60, 1.5 to 30, 7.5 to 22.5, 9 to 18, or about 15 mgs of RAD001, or a bioequivalent dose thereof.

In an instance, a dose of an mTOR inhibitor is associated with, or provides, mTOR inhibition of at least 5 but no more than 90%, at least 10 but no more than 90%, at least 15, but no more than 90%, at least 20 but no more than 90%, at least 30 but no more than 90%, at least 40 but no more than 90%, at least 50 but no more than 90%, at least 60 but no more than 90%, at least 70 but no more than 90%, at least 5 but no more than 80%, at least 10 but no more than 80%, at least 15, but no more than 80%, at least 20 but no more than 80%, at least 30 but no more than 80%, at least 40 but no more than 80%, at least 50 but no more than 80%, at least 60 but no more than 80%, at least 5 but no more than 70%, at least 10 but no more than 70%, at least 15, but no more than 70%, at least 20 but no more than 70%, at least 30 but no more than 70%, at least 40 but no more than 70%, at least 50 but no more than 70%, at least 5 but no more than 60%, at least 10 but no more than 60%, at least 15, but no more than 60%, at least 20 but no more than 60%, at least 30 but no more than 60%, at least 40 but no more than 60%, at least 5 but no more than 50%, at least 10 but no more than 50%, at least 15, but no more than 50%, at least 20 but no more than 50%, at least 30 but no more than 50%, at least 40 but no more than 50%, at least 5 but no more than 40%, at least 10 but no more than 40%, at least 15, but no more than 40%, at least 20 but no more than 40%, at least 30 but no more than 40%, at least 35 but no more than 40%, at least 5 but no more than 30%, at least 10 but no more than 30%, at least 15, but no more than 30%, at least 20 but no more than 30%, or at least 25 but no more than 30%.

The extent of mTOR inhibition can be conveyed as, or corresponds to, the extent of P70 S6 kinase inhibition, e.g., the extent of mTOR inhibition can be determined by the level of decrease in P70 S6 kinase activity, e.g., by the decrease in phosphorylation of a P70 S6 kinase substrate. The level of mTOR inhibition can be evaluated by various methods, such as measuring P70 S6 kinase activity by the Boulay assay, as described in U.S. Patent Application No. 2015/01240036, or as described in U.S. Patent No. 7,727,950; measuring the level of phosphorylated S6 by western blot; or evaluating a change in the ratio of PD1 negative immune effector cells to PD1 positive immune effector cells.

As used herein, the term "mTOR inhibitor" refers to a compound or ligand, or a pharmaceutically acceptable salt thereof, which inhibits the mTOR kinase in a cell. In an instance, an mTOR inhibitor is an allosteric inhibitor. Allosteric mTOR inhibitors include the neutral tricyclic compound rapamycin (sirolimus), rapamycin-related compounds, that is compounds having structural and functional similarity to rapamycin including, e.g., rapamycin derivatives, rapamycin analogs (also referred to as rapalogs) and other macrolide compounds that inhibit mTOR activity. In an instance, an mTOR inhibitor is a catalytic inhibitor.

Rapamycin is a known macrolide antibiotic produced by Streptomyces hygroscopicus having the structure shown in Formula A.

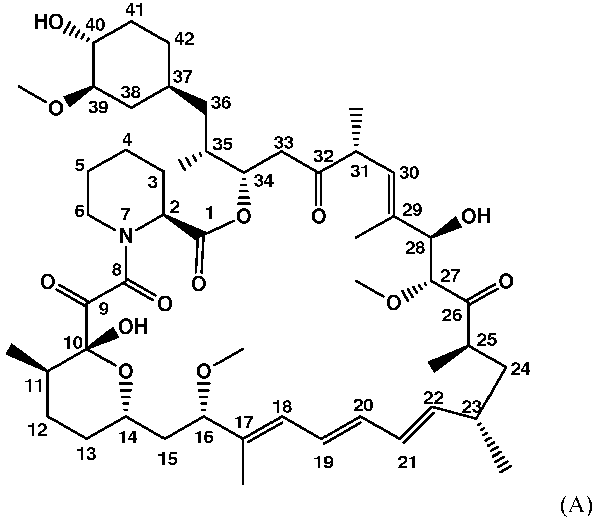

See, e.g., McAlpine, J.B., et al., J. Antibiotics (1991) 44: 688; Schreiber, S.L., et al., J. Am. Chem. Soc. (1991) 113: 7433; U.S. Patent No. 3,929,992. There are various numbering schemes proposed for rapamycin. To avoid confusion, when specific rapamycin analogs are named herein, the names are given with reference to rapamycin using the numbering scheme of formula A.

Rapamycin analogs useful in the invention are, for example, O-substituted analogs in which the hydroxyl group on the cyclohexyl ring of rapamycin is replaced by OR₁ in which R₁ is hydroxyalkyl, hydroxyalkoxyalkyl, acylaminoalkyl, or aminoalkyl; e.g. RAD001, also known as everolimus, as described in US 5,665,772 and WO94/09010.

Other suitable rapamycin analogs include those substituted at the 26- or 28-position. The rapamycin analog may be an epimer of an analog mentioned above, particularly an epimer of an analog substituted in position 40, 28 or 26, and may optionally be further hydrogenated, e.g. as described in US 6,015,815, WO95/14023 and WO99/15530, e.g. ABT578 also known as zotarolimus or a rapamycin analog described in US 7,091,213, WO98/02441 and WO01/14387, e.g. AP23573 also known as ridaforolimus.

Examples of rapamycin analogs suitable for use in the present invention from US 5,665,772 include, but are not limited to, 40-O-benzyl-rapamycin, 40-O-(4'-hydroxymethyl)benzyl-rapamycin, 40-O-[4'-(1,2-dihydroxyethyl)]benzyl-rapamycin, 40-0-allyl-rapamycin, 40-O-[3'-(2,2-dimethyl-1,3-dioxolan-4(S)-yl)-prop-2'-en-1'-yl]-rapamycin, (2'E,4'S)-40-O-(4',5'-dihydroxypent-2'-en-1'-yl)-rapamycin, 40-0-(2-hydroxy)ethoxycarbonylmethyl-rapamycin, 40-O-(2-hydroxy)ethyl-rapamycin, 40-0-(3-hydroxy)propyl-rapamycin, 40-O-(6-hydroxy)hexyl-rapamycin, 40-O-[2-(2-hydroxy)ethoxy]ethyl-rapamycin, 40-O-[(3S)-2,2-dimethyldioxolan-3-yl]methyl-rapamycin, 40-O-[(2S)-2,3-dihydroxyprop-1-yl]-rapamycin, 40-O-(2-acetoxy)ethyl-rapamycin, 40-0-(2-nicotinoyloxy)ethyl-rapamycin, 40-O-[2-(N-morpholino)acetoxy]ethyl-rapamycin, 40-O-(2-N-imidazolylacetoxy)ethyl-rapamycin, 40-O-[2-(N-methyl-N'-piperazinyl)acetoxy]ethyl-rapamycin, 39-O-desmethyl-39,40-O,O-ethylene-rapamycin, (26R)-26-dihydro-40-O-(2-hydroxy)ethyl-rapamycin, 40-O-(2-aminoethyl)-rapamycin, 40-O-(2-acetaminoethyl)-rapamycin, 40-O-(2-nicotinamidoethyl)-rapamycin, 40-O-(2-(N-methyl-imidazo-2'-ylcarbethoxamido)ethyl)-rapamycin, 40-O-(2-ethoxycarbonylaminoethyl)-rapamycin, 40-0-(2-tolylsulfonamidoethyl)-rapamycin and 40-O-[2-(4',5'-dicarboethoxy-1',2',3'-triazol-1'-yl)-ethyl] -rapamycin.

Other rapamycin analogs useful in the present invention are analogs where the hydroxyl group on the cyclohexyl ring of rapamycin and/or the hydroxy group at the 28 position is replaced with an hydroxyester group are known, for example, rapamycin analogs found in US RE44,768, e.g. temsirolimus.

Other rapamycin analogs useful in the preset invention include those wherein the methoxy group at the 16 position is replaced with another substituent, preferably (optionally hydroxy-substituted) alkynyloxy, benzyl, orthomethoxybenzyl or chlorobenzyl and/or wherein the mexthoxy group at the 39 position is deleted together with the 39 carbon so that the cyclohexyl ring of rapamycin becomes a cyclopentyl ring lacking the 39 position methyoxy group; e.g. as described in WO95/16691 and WO96/41807. The analogs can be further modified such that the hydroxy at the 40-position of rapamycin is alkylated and/or the 32-carbonyl is reduced.

Rapamycin analogs from WO95/16691 include, but are not limited to, 16-demthoxy-16-(pent-2-ynyl)oxy-rapamycin, 16-demthoxy-16-(but-2-ynyl)oxy-rapamycin, 16-demthoxy-16-(propargyl)oxy-rapamycin, 16-demethoxy-16-(4-hydroxy-but-2-ynyl)oxy-rapamycin, 16-demthoxy-16-benzyloxy-40-O-(2-hydroxyethyl)-rapamycin, 16-demthoxy-16-benzyloxy-rapamycin, 16-demethoxy-16-ortho-methoxybenzyl-rapamycin, 16-demethoxy-40-O-(2-methoxyethyl)-16-pent-2-ynyl)oxy-rapamycin, 39-demethoxy-40-desoxy-39-formyl-42-norrapamycin, 39-demethoxy-40-desoxy-39-hydroxymethyl-42-nor-rapamycin, 39-demethoxy-40-desoxy-39-carboxy-42-nor-rapamycin, 39-demethoxy-40-desoxy-39-(4-methyl-piperazin-1-yl)carbonyl-42-nor-rapamycin, 39-demethoxy-40-desoxy-39-(morpholin-4-yl)carbonyl-42-norrapamycin, 39-demethoxy-40-desoxy-39-[N-methyl, N-(2-pyridin-2-yl-ethyl)]carbamoyl-42-nor-rapamycin and 39-demethoxy-40-desoxy-39-(p-toluenesulfonylhydrazonomethyl)-42-norrapamycin.

Rapamycin analogs from WO96/41807 include, but are not limited to, 32-deoxorapamycin, 16-O-pent-2-ynyl-32-deoxo-rapamycin, 16-O-pent-2-ynyl-32-deoxo-40-O-(2-hydroxy-ethyl)-rapamycin, 16-O-pent-2-ynyl-32-(S)-dihydro-40-O-(2-hydroxyethyl)-rapamycin, 32(S)-dihydro-40-O-(2-methoxy)ethyl-rapamycin and 32(S)-dihydro-40-O-(2-hydroxyethyl)-rapamycin.

Another suitable rapamycin analog is umirolimus as described in US2005/0101624.

RAD001, otherwise known as everolimus (Afinitor®), has the chemical name (1R,9S,12S,15R,16E,18R,19R,21R,23S,24E,26E,28E,30S,32S,35R)-1,18-dihydroxy-12-{(1R)-2-[(1S,3R,4R)-4-(2-hydroxyethoxy)-3-methoxycyclohexyl]-1-methylethyl}-19,30-dimethoxy-15,17,21,23,29,35-hexamethyl-11,36-dioxa-4-aza-tricyclo[30.3.1.04,9]hexatriaconta-16,24,26,28-tetraene-2,3,10,14,20-pentaone, as described in US 5,665,772 and WO94/09010.

Further examples of allosteric mTOR inhibitors include sirolimus (rapamycin, AY-22989), 40-[3-hydroxy-2-(hydroxymethyl)-2-methylpropanoate]-rapamycin (also called temsirolimus or CCI-779) and ridaforolimus (AP-23573/MK-8669). Other examples of allosteric mTor inhibtors include zotarolimus (ABT578) and umirolimus.

Alternatively or additionally, catalytic, ATP-competitive mTOR inhibitors have been found to target the mTOR kinase domain directly and target both mTORC1 and mTORC2. These are also more effective inhibitors of mTORC1 than such allosteric mTOR inhibitors as rapamycin, because they modulate rapamycin-resistant mTORC1 outputs such as 4EBP1-T37/46 phosphorylation and cap-dependent translation.

Catalytic inhibitors include: BEZ235 or 2-methyl-2-[4-(3-methyl-2-oxo-8-quinolin-3-yl-2,3-dihydro-imidazo[4,5-c]quinolin-1-yl)-phenyl]-propionitrile, or the monotosylate salt form (the synthesis of BEZ235 is described in WO2006/122806); CCG168 (otherwise known as AZD-8055, Chresta, C.M., et al., Cancer Res, 2010, 70(1), 288-298) which has the chemical name {5-[2,4-bis-((S)-3-methyl-morpholin-4-yl)-pyrido[2,3d]pyrimidin-7-yl]-2-methoxyphenyl}-methanol; 3-[2,4-bis[(3S)-3-methylmorpholin-4-yl]pyrido[2,3-d]pyrimidin-7-yl]-N-methylbenzamide (WO09104019); 3-(2-aminobenzo[d]oxazol-5-yl)-1-isopropyl-1H-pyrazolo[3,4-d]pyrimidin-4-amine (WO10051043 and WO2013023184); A N-(3-(N-(3-((3,5-dimethoxyphenyl)amino)quinoxaline-2-yl)sulfamoyl)phenyl)-3-methoxy-4-methylbenzamide (WO07044729 and WO12006552); PKI-587 (Venkatesan, A.M., J. Med.Chem., 2010, 53, 2636-2645) which has the chemical name 1-[4-[4-(dimethylamino)piperidine-1-carbonyl]phenyl]-3-[4-(4,6-dimorpholino-1,3,5-triazin-2-yl)phenyl]urea; GSK-2126458 (ACS Med. Chem. Lett., 2010, 1, 39-43) which has the chemical name 2,4-difluoro-N-{2-methoxy-5-[4-(4-pyridazinyl)-6-quinolinyl]-3-pyridinyl}benzenesulfonamide; 5-(9-isopropyl-8-methyl-2-morpholino-9H-purin-6-yl)pyrimidin-2-amine (WO10114484); and (E)-N-(8-(6-amino-5-(trifluoromethyl)pyridin-3-yl)-1-(6-(2-cyanopropan-2-yl)pyridin-3-yl)-3-methyl-1H-imidazo[4,5-c]quinolin-2(3H)-ylidene)cyanamide(WO12007926).

Further examples of catalytic mTOR inhibitors include 8-(6-methoxy-pyridin-3-yl)-3-methyl-1-(4-piperazin-1-yl-3-trifluoromethyl-phenyl)-1,3-dihydro-imidazo[4,5-c]quinolin-2-one (WO2006/122806) and Ku-0063794 (Garcia-Martinez JM, et al.,Biochem J., 2009, 421(1), 29-42. Ku-0063794 is a specific inhibitor of the mammalian target of rapamycin (mTOR).) WYE-354 is another example of a catalytic mTOR inhibitor (Yu K, et al. (2009). Biochemical, Cellular, and In vivo Activity of Novel ATP-Competitive and Selective Inhibitors of the Mammalian Target of Rapamycin. Cancer Res. 69(15): 6232-6240).

mTOR inhibitors useful according to the present invention also include prodrugs, derivatives, pharmaceutically acceptable salts, or analogs thereof of any of the foregoing. mTOR inhibitors, such as RAD001, may be formulated for delivery based on well-established methods in the art based on the particular dosages described herein. In particular, US Patent No. 6,004,973 provides examples of formulations useable with the mTOR inhibitors described herein.

### METHODS AND BIOMARKERS FOR EVALUATING CAR-EFFECTIVENESS OR SAMPLE SUITABILITY

In another aspect, the present disclosure features a method of evaluating or monitoring the effectiveness of a CAR-expressing cell therapy, in a subject (e.g., a subject having a cancer), or the suitability of a sample (e.g., an apheresis sample) for a CAR therapy, e.g., therapy including administration of a low, immune-enhancing dose of an mTOR inhibitor. The method includes acquiring a value of effectiveness to the CAR therapy, or sample suitability, wherein said value is indicative of the effectiveness or suitability of the CAR-expressing cell therapy.

In instances, the value of effectiveness to the CAR therapy, or sample suitability, comprises a measure of one, two, three, four, five, six or more (all) of the following:
(i) the level or activity of one, two, three, or more (e.g., all) of resting T_{EFF} cells, resting T_{REG} cells, younger T cells (e.g., younger CD4 or CD8 cells, or gamma/delta T cells), or early memory T cells, or a combination thereof, in a sample (e.g., an apheresis sample or a manufactured CAR-expressing cell product sample);
(ii) the level or activity of one, two, three, or more (e.g., all) of activated T_{EFF} cells, activated T_{REG} cells, older T cells (e.g., older CD4 or CD8 cells), or late memory T cells, or a combination thereof, in a sample (e.g., an apheresis sample or a manufactured CAR-expressing cell product sample);
(iii) the level or activity of an immune cell exhaustion marker, e.g., one, two or more immune checkpoint inhibitors (e.g., PD-1, PD-L1, TIM-3 and/or LAG-3) in a sample (e.g., an apheresis sample or a manufactured CAR-expressing cell product sample). In one instance, an immune cell has an exhausted phenotype, e.g., co-expresses at least two exhaustion markers, e.g., co-expresses PD-1 and TIM-3. In other instances, an immune cell has an exhausted phenotype, e.g., co-expresses at least two exhaustion markers, e.g., co-expresses PD-1 and LAG-3;
(iv) the level or activity of CD27 and/or CD45RO- (e.g., CD27+ CD45RO-) immune effector cells, e.g., in a CD4+ or a CD8+ T cell population, in a sample (e.g., an apheresis sample or a manufactured CAR-expressing cell product sample);
(v) the level or activity of one, two, three, four, five, ten, twelve or more of the biomarkers chosen from CCL20, IL-17a and/or IL-6, PD-1, PD-L1, LAG-3, TIM-3, CD57, CD27, CD122, CD62L, KLRG1;
(vi) a cytokine level or activity (e.g., quality of cytokine reportoire) in a CAR-expressing cell product sample; or
(vii) a transduction efficiency of a CAR-expressing cell in a manufactured CAR-expressing cell product sample.

In some instances of any of the methods disclosed herein, the CAR-expressing cell therapy comprises a plurality (e.g., a population) of CAR-expressing immune effector cells, e.g., a plurality (e.g., a population) of T cells or NK cells, or a combination thereof. In one instance, the CAR-expressing cell therapy includes administration of a low, immune-enhancing dose of an mTOR inhibitor.

In some instances of any of the methods disclosed herein, the measure of one or more of (i)-(vii) is obtained from an apheresis sample acquired from the subject. The apheresis sample can be evaluated prior to infusion or re-infusion.

In some instances of any of the methods disclosed herein, the measure of one or more of (i)-(vii) is obtained from a manufactured CAR-expressing cell product sample. The manufactured CAR-expressing cell product can be evaluated prior to infusion or re-infusion.

In some instances of any of the methods disclosed herein, the subject is evaluated prior to receiving, during, or after receiving, the CAR-expressing cell therapy.

In some instances of any of the methods disclosed herein, the measure of one or more of (i)-(vii) evaluates a profile for one or more of gene expression, flow cytometry or protein expression.

In some instances of any of the methods disclosed herein, the method further comprises identifying the subject as a responder, a non-responder, a relapser or a non-relapser, based on a measure of one or more of (i)-(vii).

In some instances of any of the methods disclosed herein, a responder (e.g., a complete responder) has, or is identified as having, a greater level or activity of one, two, or more (all) of GZMK, PPF1BP2, or naive T cells as compared to a non-responder.

In some instances of any of the methods disclosed herein, a non-responder has, or is identified as having, a greater level or activity of one, two, three, four, five, six, seven, or more (e.g., all) of IL22, IL-2RA, IL-21, IRF8, IL8, CCL17, CCL22, effector T cells, or regulatory T cells, as compared to a responder.

In an instance, a relapser is a patient having, or who is identified as having, an increased level of expression of one or more of (e.g., 2, 3, 4, or all of) the following genes, compared to non relapsers: MIR199A1, MIR1203, uc021ovp, ITM2C, and HLA-DQB1 and/or a decreased levels of expression of one or more of (e.g., 2, 3, 4, 5, 6, 7, 8, 9, 10, 11, or all of) the following genes, compared to non relapsers: PPIAL4D, TTTY10, TXLNG2P, MIR4650-1, KDM5D, USP9Y, PRKY, RPS4Y2, RPS4Y1, NCRNA00185, SULT1E1, and EIF1AY.

In some instances of any of the methods disclosed herein, a complete responder has, or is identified as having, a greater, e.g., a statistically significant greater, percentage of CD8+ T cells compared to a reference value, e.g., a non-responder percentage of CD8+ T cells.

In some instances of any of the methods disclosed herein, a complete responder has, or is identified as having, a greater percentage of CD27+ CD45RO- immune effector cells, e.g., in the CD8+ population, compared to a reference value, e.g., a non-responder number of CD27+ CD45RO- immune effector cells.

In some instances of any of the methods disclosed herein, a complete responder or a partial responder has, or is identified as having, a greater, e.g., a statistically significant greater, percentage of CD4+ T cells compared to a reference value, e.g., a non-responder percentage of CD4+ T cells.

In some instances of any of the methods disclosed herein, a complete responder has, or is identified as having, a greater percentage of one, two, three, or more (e.g., all) of resting T_{EFF} cells, resting T_{REG} cells, younger T cells (e.g., younger CD4 or CD8 cells, or gamma/delta T cells), or early memory T cells, or a combination thereof, compared to a reference value, e.g., a non-responder number of resting T_{EFF} cells, resting T_{REG} cells, younger T cells (e.g., younger CD4 or CD8 cells), or early memory T cells.

In some instances of any of the methods disclosed herein, a non-responder has, or is identified as having, a greater percentage of one, two, three, or more (e.g., all) of activated T_{EFF} cells, activated T_{REG} cells, older T cells (e.g., older CD4 or CD8 cells), or late memory T cells, or a combination thereof, compared to a reference value, e.g., a responder number of activated T_{EFF} cells, activated T_{REG} cells, older T cells (e.g., older CD4 or CD8 cells), or late memory T cells.

In some instances of any of the methods disclosed herein, a non-responder has, or is identified as having, a greater percentage of an immune cell exhaustion marker, e.g., one, two or more immune checkpoint inhibitors (e.g., PD-1, PD-L1, TIM-3 and/or LAG-3). In one instance, a non-responder has, or is identified as having, a greater percentage of PD-1, PD-L1, or LAG-3 expressing immune effector cells (e.g., CD4+ T cells and/or CD8+ T cells) (e.g., CAR-expressing CD4+ cells and/or CD8+ T cells) compared to the percentage of PD-1 or LAG-3 expressing immune effector cells from a responder.

In one instance, a non-responder has, or is identified as having, a greater percentage of immune cells having an exhausted phenotype, e.g., immune cells that co-express at least two exhaustion markers, e.g., co-expresses PD-1, PD-L1 and/or TIM-3. In other instances, a non-responder has, or is identified as having, a greater percentage of immune cells having an exhausted phenotype, e.g., immune cells that co-express at least two exhaustion markers, e.g., co-expresses PD-1 and LAG-3.

In some instances of any of the methods disclosed herein, a non-responder has, or is identified as having, a greater percentage of PD-1/ PD-L1+/LAG-3+ cells in the CAR-expressing cell population compared to a responder (e.g., a complete responder) to the CAR-expressing cell therapy.

In some instances of any of the methods disclosed herein, a partial responder has, or is identified as having, a higher percentages of PD-1/ PD-L1+/LAG-3+ cells, than a responder, in the CAR-expressing cell population.

In some instances of any of the methods disclosed herein, a non-responder has, or is identified as having, an exhausted phenotype of PD1/ PD-L1+ CAR+ and co-expression of LAG3 in the CAR-expressing cell population.

In some instances of any of the methods disclosed herein, a non-responder has, or is identified as having, a greater percentage of PD-1/ PD-L1+/TIM-3+ cells in the CAR-expressing cell population compared to the responder (e.g., a complete responder).

In some instances of any of the methods disclosed herein, a partial responders has, or is identified as having, a higher percentage of PD-1/ PD-L1+/TIM-3+ cells, than responders, in the CAR-expressing cell population.

In some instances of any of the methods disclosed herein, the presence of CD8+ CD27+ CD45RO- T cells in an apheresis sample is a positive predictor of the subject response to a CAR-expressing cell therapy.

In some instances of any of the methods disclosed herein, a high percentage of PD1+ CAR+ and LAG3+ or TIM3+ T cells in an apheresis sample is a poor prognostic predictor of the subject response to a CAR-expressing cell therapy.

In some instances of any of the methods disclosed herein, the responder (e.g., the complete or partial responder) has one, two, three or more (or all) of the following profile:
(i) has a greater number of CD27+ immune effector cells compared to a reference value, e.g., a non-responder number of CD27+ immune effector cells;
(ii) has a greater number of CD8+ T cells compared to a reference value, e.g., a non-responder number of CD8+ T cells;
(iii) has a lower number of immune cells expressing one or more checkpoint inhibitors, e.g., a checkpoint inhibitor chosen from PD-1, PD-L1, LAG-3, TIM-3, or KLRG-1, or a combination, compared to a reference value, e.g., a non-responder number of cells expressing one or more checkpoint inhibitors; or
(iv) has a greater number of one, two, three, four or more (all) of resting TEFF cells, resting T_{REG} cells, naive CD4 cells, unstimulated memory cells or early memory T cells, or a combination thereof, compared to a reference value, e.g., a non-responder number of resting T_{EFF} cells, resting T_{REG} cells, naive CD4 cells, unstimulated memory cells or early memory T cells.

In some instances of any of the methods disclosed herein, the cytokine level or activity of (vi) is chosen from one, two, three, four, five, six, seven, eight, or more (or all) of cytokine CCL20/MIP3a, IL17A, IL6, GM-CSF, IFNγ, IL10, IL13, IL2, IL21, IL4, IL5, IL9 or TNFα, or a combination thereof. The cytokine can be chosen from one, two, three, four or more (all) of IL-17a, CCL20, IL2, IL6, or TNFa. In one instance, an increased level or activity of a cytokine is chosen from one or both of IL-17a and CCL20, is indicative of increased responsiveness or decreased relapse.

In some instances of any of the methods disclosed herein, a transduction efficiency of 15% or higher in (vii) is indicative of increased responsiveness or decreased relapse.

In some instances of any of the methods disclosed herein, a transduction efficiency of less than 15% in (vii) is indicative of decreased responsiveness or increased relapse.

In instances, the responder, a non-responder, a relapser or a non-relapser identified by the methods herein can be further evaluated according to clinical criteria. For example, a complete responder has, or is identified as, a subject having a disease, e.g., a cancer, who exhibits a complete response, e.g., a complete remission, to a treatment. A complete response may be identified, e.g., using the NCCN Guidelines®, as described herein. A partial responder has, or is identified as, a subject having a disease, e.g., a cancer, who exhibits a partial response, e.g., a partial remission, to a treatment. A partial response may be identified, e.g., using the NCCN Guidelines®, as described herein. A non-responder has, or is identified as, a subject having a disease, e.g., a cancer, who does not exhibit a response to a treatment, e.g., the patient has stable disease or progressive disease. A non-responder may be identified, e.g., using the NCCN Guidelines®, as described herein.

Alternatively, or in combination with the methods disclosed herein, responsive to said value, performing one, two, three, four or more of:
administering e.g., to a responder or a non-relapser, a CAR-expressing cell therapy;
administered an altered dosing of a CAR-expressing cell therapy;
altering the schedule or time course of a CAR-expressing cell therapy;
administering, e.g., to a non-responder or a partial responder, an additional agent in combination with a CAR-expressing cell therapy, e.g., a checkpoint inhibitor, e.g., a checkpoint inhibitor described herein;
administering to a non-responder or partial responder a therapy that increases the number of younger T cells in the subject prior to treatment with a CAR-expressing cell therapy;
modifying a manufacturing process of a CAR-expressing cell therapy, e.g., enriching for younger T cells prior to introducing a nucleic acid encoding a CAR, or increasing the transduction efficiency, e.g., for a subject identified as a non-responder or a partial responder;
administering an alternative therapy, e.g., for a non-responder or partial responder or relapser; or
if the subject is, or is identified as, a non-responder or a relapser, decreasing the TREG cell population and/or T_{REG} gene signature, e.g., by one or more of CD25 depletion, administration of cyclophosphamide, anti-GITR antibody, or a combination thereof.

In certain instances, the subject is pre-treated with an anti-GITR antibody. In certain instance, the subject is treated with an anti-GITR antibody prior to infusion or re-infusion.

### BIOPOLYMER DELIVERY METHODS

In some instances, one or more CAR-expressing cells as disclosed herein, optionally in combination with a low, immune-enhancing dose of an mTOR inhibitor (e.g., an mTOR inhibitor described herein) can be administered or delivered to the subject via a biopolymer scaffold, e.g., a biopolymer implant. Biopolymer scaffolds can support or enhance the delivery, expansion, and/or dispersion of the CAR-expressing cells described herein. A biopolymer scaffold comprises a biocompatible (e.g., does not substantially induce an inflammatory or immune response) and/or a biodegradable polymer that can be naturally occurring or synthetic.

Examples of suitable biopolymers include, but are not limited to, agar, agarose, alginate, alginate/calcium phosphate cement (CPC), beta-galactosidase (β-GAL), (1 ,2,3,4,6-pentaacetyl α-D-galactose), cellulose, chitin, chitosan, collagen, elastin, gelatin, hyaluronic acid collagen, hydroxyapatite, poly(3-hydroxybutyrate-co-3-hydroxy-hexanoate) (PHBHHx), poly(lactide), poly(caprolactone) (PCL), poly(lactide-co-glycolide) (PLG), polyethylene oxide (PEO), poly(lactic-co-glycolic acid) (PLGA), polypropylene oxide (PPO), polyvinyl alcohol) (PVA), silk, soy protein, and soy protein isolate, alone or in combination with any other polymer composition, in any concentration and in any ratio. The biopolymer can be augmented or modified with adhesion- or migration-promoting molecules, e.g., collagen-mimetic peptides that bind to the collagen receptor of lymphocytes, and/or stimulatory molecules to enhance the delivery, expansion, or function, e.g., anti-cancer activity, of the cells to be delivered. The biopolymer scaffold can be an injectable, e.g., a gel or a semi-solid, or a solid composition.

In some instances, CAR-expressing cells described herein are seeded onto the biopolymer scaffold prior to delivery to the subject. In instances, the biopolymer scaffold further comprises one or more additional therapeutic agents described herein (e.g., another CAR-expressing cell, an antibody, or a small molecule) or agents that enhance the activity of a CAR-expressing cell, e.g., incorporated or conjugated to the biopolymers of the scaffold. In instances, the biopolymer scaffold is injected, e.g., intratumorally, or surgically implanted at the tumor or within a proximity of the tumor sufficient to mediate an anti-tumor effect. Additional examples of biopolymer compositions and methods for their delivery are described in Stephan et al., Nature Biotechnology, 2015, 33:97-101; and WO2014/110591.

### EXAMPLES

The invention is further described in detail by reference to the following experimental examples. These examples are provided for purposes of illustration only, and are not intended to be limiting unless otherwise specified. Thus, the invention should in no way be construed as being limited to the following examples, but rather, should be construed to encompass any and all variations which become evident as a result of the teaching provided herein.

Without further description, it is believed that one of ordinary skill in the art can, using the preceding description and the following illustrative examples, make and utilize the compounds of the present disclosure and practice the disclosed methods. The following working examples specifically point out various aspects of the present invention, and are not to be construed as limiting in any way the remainder of the disclosure.

### Example 1: Analyzing combinations of CD4⁺ and CD8⁺ T cells redirected with CARs containing different costimulatory domains

The following materials and methods were used to test whether CD4⁺ and CD8⁺ T cells require distinct cytokine and costimulation signals for optimal persistence, and whether the proper redirection of CD4⁺ T cells may be key to sustain CD8⁺ T cell persistence and killing, as described in further detail in Examples 2-6.

### Isolation, Transduction, and Expansion of Primary Human T Lymphocytes.

Blood samples were obtained from the Human Immunology Core of the University of Pennsylvania. Peripheral blood CD4⁺ and CD8⁺ T cells were negatively isolated using RosetteSep Kits (Stem cell Technologies). Cells were cultured in RPMI 1640 media supplemented with 10% FCS, 100-U/ml penicillin, 100 µg/ml streptomycin sulfate, 10 mM Hepes in a 37°C and 5% CO2 incubator. For stimulation, CD4⁺ and CD8⁺ T cells were cultured with activating beads coated with antibodies to CD3 and CD28 at a 1:2 cell to bead ratio. Approximately 24 h after activation, T cells were transduced with lentiviral vectors at an MOI of 5. For CD8⁺ T cells, human IL-2 (Chiron) was added every other day to a final concentration of 30 IU/ml. Cells were counted and fed every 2 days and once T cells appeared to rest down, as determined by both decreased growth kinetics and cell size, they were either used for functional assays or cryopreserved. All T cell functional assays were performed in media without cytokines.

### Polarization of T_{H}17 cells

For T_{H}17 polarization, CD4⁺ T cells were stimulated with activating beads coated with antibodies to CD3 and ICOS at a 1:3 cell to bead ratio and cultured in the presence of IL-1β (10 ng/ml), IL-6 (10 ng/ml), IL-23 (20 ng/ml), and neutralizing antibodies (10 µg/ml) against IL-4 and IFN-γ (eBioscience). All experiments were conducted with fetal calf serum containing endogenous sources of TGF-β. Human IL-2 (Chiron) was added 3 days after activation to a final concentration of 50 IU/ml.

### Cytotoxicity assays

For the cell-based bioluminescence assays, 10 x 10⁴ firefly Luciferase (fLuc)-expressing tumor cells (L55) were cultured with R10 media in the presence of different ratios of transduced T cells with the use of a 96-well Microplate (BD Biosciences). After incubation for ∼18 hours at 37°C, cells were lysed with luciferase assay lysis buffer and luciferase reagent was add into each well as described in the manufacture's instructions. Specific lysis was calculated as the mean luminescence of the experimental sample minus spontaneous lysis divided by the mean luminescence of the maximum lysis minus spontaneous lysis times 100. All data are represented as a mean of triplicate wells.

### Cytokine Production of Restimulated T Cells.

Cryopreserved T cells expressing the SS1 CARs were thawed, washed, and placed in culture for 16 h. T cells (4 x 10⁵) were then cocultured with 2 x 10⁵ mesothelin-expressing tumor cells and supernatants were harvested 24 h later. T cell cultures were normalized to equivalent CAR expression by addition of mock transduced T cells as required. Concentrations of IL-2, IFN-γ, TNF-α, were determined using the DuoSet® ELISA Development Systems.

### Flow cytometry

The following conjugated antibodies were purchased from eBioscience: anti-CD8 APC, anti-CD4-PE, anti-CD45 PerCp-Cy5.5. Expression of the various SS1 scFv fusion proteins on T cells was detected using biotinylated goat anti-mouse IgG (specific for scFvs of murine origin) from Jackson ImmunoResearch. Streptavidin (PE) and streptavidin (APC) were purchased from BD Biosciences. Samples were analyzed in the LSRII flow cytometer using the DiVa software (BD Biosciences), and results were evaluated using the FlowJo software (TreeStar).

### Mice

The University of Pennsylvania Institutional Animal Care and Use Committee approved all animal experiments. NSG mice were purchased from The Jackson Laboratory and bred in the vivarium at the University of Pennsylvania. The mice were housed under specific pathogen-free conditions in microisolator cages and given ad libitum access to autoclaved food and acidified water.

### In vivo assessment of anti-mesothelin CAR T cells.

Xenograft tumors were established by subcutaneous injection of 5x10⁶ L55, SKOV3 or Capan-2 cells in the presence of a 50% solution of Matrigel (BD Biosciences) in PBS. L55 and SKOV3 tumors were allowed to grow in NSG mice for 2 or 4 weeks and Capan-2 tumors grew for 2 weeks. The route, dose and timing of T-cell injections is indicated in the individual figure legends. Tumor dimensions were measured with calipers, and tumor volumes calculated using the formula V= 1/2 x L x W x W, where L is length (longest dimension) and W is width (shortest dimension). Peripheral blood was obtained from retro-orbital bleeding or intracardiac puncture and stained for the presence of human CD45, CD4, and CD8 T cells. After gating on the human CD45⁺ population, the CD4⁺ and CD8⁺ subsets were quantified using TruCount tubes (BD Biosciences). All experiments were performed in a blinded, randomized fashion.

### Example 2: In vitro assessment of CD4⁺ and CD8⁺ T cells redirected with SS1-CARs containing different costimulatory domains

The *in vitro* antitumor potential of CD4⁺ and CD8⁺ T cells redirected with CARs that contain the SS1 scFv that recognizes human mesothelin (SS1 CARs) was examined. The scFv was fused to the TCR-ζ signal transduction domain (z) with the CD28, 4-1BB or ICOS costimulatory signaling domains in tandem. The SS1 CAR containing a TCR-ζ domain and a CD28 costimulatory domain is referred to as 28z; SS1 CAR containing a TCR-ζ domain and a 4-1BB costimulatory domain is referred to as BBz; and SS1 CAR containing a TCR-ζ domain and an ICOS costimulatory domain is referred to as ICOSz. Diagrams of the mesothelin CAR constructs are shown in Figure 1A. As negative control for signal transduction, a chimeric receptor containing a truncated form of the TCR-ζ intracellular domain (Δz) can be used.

CD4⁺ and CD8⁺ T cells redirected with the SS1-CARs were each cocultured with firefly Luciferase (fLuc)-expressing non-small cell lung carcinoma cells (L55) (which express mesothelin) for 18 hours at the indicated effector-target (E:T) ratios. Specific cytolysis was determined using a cell-based bioluminescence assay, as described in further detail in Example 1. As shown in Figures 2A and 2B, both CD4⁺ and CD8⁺ redirected T cells efficiently lysed the non-small cell lung carcinoma cells (L55), although CD8⁺ T cells (Fig. 2B) killed tumor cells at a lower E:T ratio than CD4⁺ T cells (Fig. 2A). No differences in cytotoxicity were observed between the different CAR signaling domains (e.g., CD28, 41-BB, and ICOS).

*In vitro* cytokine release of the redirected CD4⁺ and CD8⁺ T cells was measured after exposure to tumor cells that express mesothelin: L55 (non-small cell lung tumor cells), ASPC1 (pancreatic tumor cells), Capan2 (pancreatic tumor cells), and SKOV3 (ovarian tumor cells). CD4⁺ T cells and CD8⁺ T cells redirected with SS1 CARs (28z, BBz, ICOSz) were cocultured with 2x10⁵ of the mesothelin-expressing tumor cells for 24 hours. Media supernatant was harvested and concentrations of TNF-α, IL-2, and IFN-γ were determined using the DuoSet® ELISA Development Systems (R&D Systems). T cells redirected with the 28z CAR secreted greater levels of IL-2 (Fig. 3B and 3D) and TNF-α (Fig. 3A) compared to control (CD3z), while CD8⁺ T cells redirected with 28z and ICOSz CARs showed similar levels of IFN-γ (Fig. 3E).

### Example 3: In vivo assessment of T cell persistence of CD4⁺ and CD8⁺ T cells redirected with SS1-CARs containing different costimulatory domains

The *in vivo* T cell persistence of CD4⁺ and CD8⁺ T cells redirected with the same CAR (z, 28z, BBz, ICOSz) or with different CARs that recognize mesothelin (SS1 CAR) was analyzed. CD4⁺ and CD8⁺ T cells were redirected with the same CARs (e.g., CARs containing the same costimulatory domains z, 28z, BBz, or ICOSz; see Fig. 4A) or different CARs (e.g., CD8⁺ T cells were redirected with CAR containing BBz, while CD4⁺ T cells were redirected with different CARs containing 28z, BBz, or ICOSz, see Fig. 4B; or CD4⁺ T cells were redirected with CAR containing ICOSz, while CD8⁺ T cells were redirected with different CARs containing 28z, BBz, or ICOSz, see Fig. 4C) NSG mice bearing subcutaneous non-small cell lung tumors (L55) were treated 30 days after tumor implantation with two doses of redirected CD4⁺ and CD8⁺ T cells (10x10⁶ T cells per dose, 60-70% CAR⁺). The second dose was administered 8 days after the first dose. Blood was obtained 22 days after the first T cell injection and quantified for the presence of CD4⁺ and CD8⁺ T cells to determine CD4⁺ or CD8⁺ T cell persistence. *In vivo* T cell persistence of CD4⁺ T cells is shown in Figure 4, while the *in vivo* T cell persistence of CD8⁺ T cells is shown in Figure 5.

Results from the experiments described above indicated that redirection of CD4⁺ T cells with an ICOS-based CAR enhanced persistence of CD4⁺ T cells when compared to cells redirected with a CAR without any costimulatory domain (z) (Fig. 4A). By contrast, CD4⁺ T cells redirected with CD28 or 4-1BB-based CARs showed impaired persistence (Fig. 4B). The enhanced persistence of CD4⁺ T cells with ICOS-based CAR was independent of the CAR used to redirect CD8⁺ T cells, as enhanced T cells persistence of CD4⁺ T cells was demonstrated by using a CAR with any costimulatory domain.

By contrast, persistence of CD8⁺ T cells was highly dependent on the CAR used to redirect CD4⁺ T cells. Redirection of CD4⁺ T cells with an ICOS-based CAR significantly increased the persistence of CD8⁺ T cells redirected with CD28 (Fig. 5C) or 4-1BB-based CAR (Fig. 5B). The group of mice that showed a better CD8+ T cell persistence was those that received CD4⁺ T cells redirected with ICOSz and CD8⁺ T cells redirected with BBz (ICOSz-BBz group) (Fig. 5B compared to Fig. 5C). The average number of T cells per µl of blood on mice treated with ICOSz-BBz T cells was 7-fold, 2800-fold, 17-fold and 5-fold higher when compared with z-z, 28z-28z, BBz-BBz, or ICOSz-ICOSz groups respectively (when both CD4⁺ and CD8⁺ T cells were redirected with the same CAR) (Fig. 5B compared to Fig. 5A).

In addition, the *in vivo* persistence CD4⁺ and CD8⁺ T cells when culturing the CD4⁺ T cells under Th17-polarizing conditions was examined. CD4⁺ T cells redirected with ICOS-based CAR were grown under Th17-polarizing conditions, as described in Example 1, to produce Th17 cells redirected with ICOS-based CAR, or were grown under standard expansion conditions. NSG mice bearing subcutaneous non-small cell lung tumors (L55) were treated 30 days after tumor implantation with two doses of CD8⁺ T cells redirected with SS1-CAR containing BBz, and either CD4⁺ T cells redirected with SS1-CAR containing ICOSz or Th17 cells redirected with SS1-CAR containing ICOSz. T cell persistence of the CD4⁺ T cells and CD8⁺ T cells was measured by quantifying the number of CD4⁺ and CD8⁺ T cells in the blood of treated animals 22 days after T cell injection. The results demonstrate that culture of CD4⁺ T cells under Th17-polarizing conditions further enhanced the persistence of ICOS-redirected CD4⁺ T cells (Fig. 6A). Importantly, Th17-polarized CD4⁺ T cells expressing an ICOS-based CAR also significantly increased the circulatory persistence of bulk CD8⁺ T cells expressing a 4-1BB-based CAR when compared to non-polarized CD4⁺ T cells (Fig. 6B).

### Example 4: In vivo assessment of antitumor activity and tumor infiltration of CD4⁺ and CD8⁺ T cells redirected with SS1-CARs containing different costimulatory domains

Next, we did another in vivo experiment were all CD8⁺ T cells were redirected with a 28z CAR and CD4⁺ T cells were redirected with 28z, BBz, ICOSz or left untransduced. NSG mice bearing L55 tumors were treated 16 days after tumor implantation with two doses of redirected T cells (10x10⁶ T cells per dose, CD4⁺ were 40% CAR⁺ and CD8⁺ cells were 80% CAR⁺). A significant antitumor effect was observed in groups where CD4⁺ T cells were redirected with 28z or ICOSz when compared with non-treated animals or animals treated with UTD CD4⁺ and 28z CD8⁺ (UTD-28z), indicating that redirection of CD4⁺ T cells with a proper CAR is critical for anti-tumor effect (Fig. 7). As previously, redirection of CD4⁺ T cells with an ICOS-based CAR enhanced the persistence of both CD4⁺ and CD8⁺ T cells. Importantly, mice treated with CD4⁺ T cells expressing an ICOS-based CAR showed greater CD4⁺ T cells infiltration in tumors, an effect that was enhanced when CD4⁺ T cells were cultured under Th17-polarizing conditions (Fig. 8).

### Example 5: In vivo assessment of CD4⁺ and CD8⁺ T cells redirected with MOv19-CARs containing different costimulatory domains

To confirm the results obtained with the SS1-CARs (e.g., the results described in Examples 2-4), T cells engineered to express a MOv19-CAR (a CAR specific for Folate Receptor-α) were examined in experiments similar to those described above. For these experiments, MOv19 CAR constructs with the stimulatory and costimulatory domains as shown in Figure 1B were used.

All CD8⁺ T cells were engineered to express a MOv19-BBz CAR, while CD4⁺ T cells were left untransduced (UTD) or engineered to express z, 28z , BBz or ICOSz MOv19 CARs. NSG mice bearing ovarian tumors (SKOV3) were treated 30 days after tumor implantation with two doses of redirected T cells (10x10⁶ T cells per dose, 80% CAR⁺). The second dose was administered 8 days after the first dose. All mice treated with redirected T cells showed great antitumor responses (Fig. 9A), e.g., significant decrease in tumor volume or regression of the tumor.

Persistence of CD4⁺ T cells and CD8⁺ T cells was also measured for each of the redirected CD4⁺ and CD8⁺ T cell combinations by quantifying the number of CD4⁺ and CD8⁺ T cells circulating in the blood of the treated animals. CD4⁺ T cells redirected with an ICOS-based CAR showed enhanced persistence in comparison to combinations with CD4⁺ T cells redirected with CARs containing other costimulatory domains (other than ICOS) (Fig. 9B). This enhanced persistence of CD4⁺ T cells correlated with an enhanced persistence of accompanying CD8⁺ T cells engineered to express MOv19-BBz (Fig. 9C).

### Example 6: Combination of CD4⁺ T cells redirected with ICOS-based CAR with a CD8⁺ T cells redirected with two CARs containing different costimulatory domains

Overall, the results described above (e.g., in Examples 2-5) demonstrate that CD4⁺ and CD8⁺ T cells require to be redirected with CARs with different intracellular domains. Redirection of CD4⁺ T cells with an ICOS-based CAR increased the persistence of CD4⁺ T cells as well as the persistence of accompanying CD8⁺ T cells (Fig. 4). By contrast, CD8⁺ T cells showed an enhanced persistence when redirected with CARs containing the 4-1BB costimulatory domain (Fig. 5). T cells redirected with CD28-based CAR showed a slightly better cytokine release and antitumor effect, but very poor in vivo persistence (Fig. 3).

Thus, a combination of CD4⁺ T cells redirected with ICOSz and CD8⁺ T cells redirected with a mix of 28z and BBz CARs was examined for anti-tumor activity and T cell persistence. NSG mice bearing ovarian (SKOV3), pancreatic (Capan-2) or lung (L55) tumors were treated with two intratumoral injections of the combination therapy. This therapy was able to control or eradicate large established tumors from different origins, with 86% of tumors responding to the treatment (Fig. 10A, 10B, and 10C). In addition, the combination therapy showed a great T cell persistence in all mice treated (Fig. 10D).

### Example 7: In vivo assessment of CD4⁺ and CD8⁺ T cell subsets expressing CAR containing a mutant ICOS domain

In the following experiment, the *in vivo* anti-tumor effect and T cell persistence of an ICOSz CAR with a mutation (Y to F) in the YMFM motif (SEQ ID NO: 85) of the ICOS intracellular domain is examined. The cytoplasmic tail of this ICOS mutant, named ICOS(FMFM)z ("FMFM" disclosed as SEQ ID NO: 47), has a mutation at the site that interacts with P13K (Fig. 11A), and cannot activate PI3K. For this experiment, all CD8⁺ T cells were redirected with a BBz CAR and CD4⁺ T cells were redirected with delz, BBz, ICOSz or ICOS(FMFM)z ("FMFM" disclosed as SEQ ID NO: 47). The CAR constructs utilized in this example have an antigen binding domain that targets mesothelin (SS1 antigen binding domain), and schematic representations of the constructs are shown in Figure 1A. The CAR construct delz (Δz) lacking intracellular signaling domains (see Fig. 1A) was used as a negative control for signaling. NSG mice bearing Capan2 pancreatic tumors were treated 15 days after tumor implantation with two doses of redirected T cells (10x10⁶ T cells per dose, 50% CAR⁺). The second dose was administered 8 days after the first dose.

A significant antitumor effect was observed in groups where CD4⁺ T cells were redirected with ICOSz or ICOS(FMFM)z ("FMFM" disclosed as SEQ ID NO: 47) when compared with non-treated animals or animals treated with delz (Δz) and BBz CD4⁺, indicating that redirection of CD4⁺ T cells with a proper CAR is critical for anti-tumor effect (Fig. 11B). The treatment of CD4⁺ T cells expressing ICOS(FMFM)z ("FMFM" disclosed as SEQ ID NO: 47) with the CD8⁺ T cells expressing BBz showed the best antitumor effect. Tumor persistence was also analyzed, as described in the previous Examples, and it was found that CD4⁺ T cells expressing ICOS(FMFM)z ("FMFM" disclosed as SEQ ID NO: 47) persisted at a similar number in tumor-bearing mice as CD4⁺ T cells expressing ICOSz cells (Fig. 11C). These results indicate that PI3K-independent ICOS signaling mechanisms may contribute to T cell costimulation during tumor rejection.

### Example 8: Low dose RAD001 stimulates CART proliferation in a cell culture model

The effect of low doses of RAD001 on CAR T cell proliferation in vitro was evaluated by co-culturing CART-expressing cells with target cells in the presence of different concentrations of RAD001.

### Materials and Methods

### Generation of CAR-transduced T cells

A humanized, anti-human CD19 CAR (huCART19) lentiviral transfer vector was used to produce the genomic material packaged into VSVg pseudotyped lentiviral particles. The amino acid and nucleotide sequence of the humanized anti-human CD19 CAR (huCART19) is CAR 1, ID 104875, described in PCT publication, WO2014/153270, filed March 15, 2014, and is designated SEQ ID NOs. 85 and 31 therein.

Lentiviral transfer vector DNA is mixed with the three packaging components VSVg env, gag/pol and rev in combination with lipofectamine reagent to transfect Lenti-X 293T cells. Medium is changed after 24h and 30h thereafter, the virus-containing media is collected, filtered and stored at -80°C. CARTs are generated by transduction of fresh or frozen naive T cells obtained by negative magnetic selection of healthy donor blood or leukopak. T cells are activated by incubation with anti-CD3/anti-CD28 beads for 24h, after which viral supernatant or concentrated virus (MOI=2 or 10, respectively) is added to the cultures. The modified T cells are allowed to expand for about 10 days. The percentage of cells transduced (expressing the CARs on the cell surface) and the level of CAR expression (relative fluorescence intensity, Geo Mean) are determined by flow cytometric analysis between days 7 and 9. The combination of slowing growth rate and T cell size approaching ∼350 fL determines the state for T cells to be cryopreserved for later analysis.

### Evaluating proliferation of CARTs

To evaluate the functionality of CARTs, the T cells are thawed and counted, and viability is assessed by Cellometer. The number of CAR-positive cells in each culture is normalized using non-transduced T cells (UTD). The impact of RAD001 on CARTs was tested in titrations with RAD001, starting at 50nM. The target cell line used in all co-culture experiments is NALM6 (Nalm-6), a human pre-B cell acute lymphoblastic leukemia (ALL) cell line expressing CD 19 and transduced to express luciferase.

For measuring the proliferation of CARTs, T cells are cultured with target cells at a ratio of 1:1. The assay is run for 4 days, when cells are stained for CD3, CD4, CD8 and CAR expression. The number of T cells is assessed by flow cytometry using counting beads as reference.

### Results

The proliferative capacity of CART cells was tested in a 4 day co-culture assay. The number of CAR-positive CD3-positive T cells (dark bars) and total CD3-positive T cells (light bars) was assessed after culturing the CAR-transduced and non-transduced T cells with NALM6 (Nalm-6) (Fig. 12). huCART19 cells expanded when cultured in the presence of less than 0.016 nM of RAD001, and to a lesser extent at higher concentrations of the compound. Importantly, both at 0.0032 and 0.016 nM RAD001 the proliferation was higher than observed without the addition of RAD001. The non-transduced T cells (UTD) did not show detectable expansion.

### Example 9: Low dose RAD001 stimulates CART expansion in vivo

This example evaluates the ability of huCAR19 cells to proliferate in vivo with different concentrations of RAD001.

### Materials and Methods:

NALM6-luc cells: The NALM6 human acute lymphoblastic leukemia (ALL) cell line was developed from the peripheral blood of a patient with relapsed ALL. The cells were then tagged with firefly luciferase. These suspension cells grow in RPMI supplemented with 10% heat inactivated fetal bovine serum.

Mice: 6 week old NSG (NOD.Cg-PrkdcscidI12rgtm1 Wj1/SzJ) mice were received from the Jackson Laboratory (stock number 005557).

Tumor implantation: NALM6-luc cells were grown and expanded in vitro in RPMI supplemented with 10% heat inactivated fetal bovine serum. The cells were then transferred to a 15 ml conical tube and washed twice with cold sterile PBS. NALM6-luc cells were then counted and resuspended at a concentration of 10x106 cells per milliliter of PBS. The cells were placed on ice and immediately (within one hour) implanted in the mice. NALM6-luc cells were injected intravenously via the tail vein in a 100 µl volume, for a total of 1x10⁶ cells per mouse.

CAR T cell dosing: Mice were administered 5x10⁶ CAR T cells 7 days after tumor implantation. Cells were partially thawed in a 37 degree Celsius water bath and then completely thawed by the addition of 1 ml of cold sterile PBS to the tube containing the cells. The thawed cells were transferred to a 15 ml falcon tube and adjusted to a final volume of 10 mls with PBS. The cells were washed twice at 1000 rpm for 10 minutes each time and then counted on a hemocytometer. T cells were then resuspended at a concentration of 50x10⁶ CAR T cells per ml of cold PBS and kept on ice until the mice were dosed. The mice were injected intravenously via the tail vein with 100 µl of the CAR T cells for a dose of 5x10⁶ CAR T cells per mouse. Eight mice per group were treated either with 100 µl of PBS alone (PBS), or humanized CD19 CAR T cells.

RAD001 dosing: A concentrated micro-emulsion of 50mg equal to 1mg RAD001 was formulated and then resuspended in D5W (dextrose 5% in water) at the time of dosing. Mice were orally dosed daily (via oral gavage) with 200 µl of the desired doses of RAD001.

PK analysis: Mice were dosed daily with RAD001 starting 7 days post tumor implantation. Dosing groups were as follows: 0.3 mg/kg, 1 mg/kg, 3 mg/kg, and 10 mg/kg. Mice were bled on days 0 and 14 following the first and last dose of RAD001, at the following time points for PK analysis: 15 minutes, 30 minutes, 1 hour, 2 hours, 4 hours, 8 hours, 12 hours, and 24 hours.

### Results:

The expansion and pharmacokinetics of RAD001 was tested in NSG mice with NALM6-luc tumors. Daily oral dosing of RAD001 alone did not have an impact on the growth of NALM6-luc tumors (Figure 13). The pharmacokinetic analysis of RAD001 shows that it is fairly stable in the blood of tumor bearing mice (Figures 14A and 14B). Both the day 0 and day 14 PK analyses show that the RAD001 concentrations in the blood is above 10nm even 24 hours after dosing at the lowest dose tested (0.3 mg/kg).

Based on these doses, huCAR19 CAR T cells were dosed with and without RAD001 to determine the proliferative ability of these cells. The highest dose used was 3 mg/kg based on the levels of RAD001 in the blood 24 hours after dosing. As the concentration of RAD001 was above 10nM 24 hours after the final dose of RAD001, several lower doses of RAD001 were used in the in vivo study with CAR T cells. The CAR T cells were dosed IV one day prior to the start of the daily oral RAD001 dosing. Mice were monitored via FACS for T cell expansion.

The lowest doses of RAD001 show an enhanced proliferation of the CAR T cells (Figures 15A and 15B). This enhanced proliferation is more evident and prolonged with the CD4+ CAR T cells than the CD8+ CAR T cells. However, with the CD8+ CAR T cells, enhanced proliferation can be seen at early time points following the CAR T cell dose.

## Claims

1. A composition or set of compositions comprising the following components:
1) a CD4+ T cell comprising a CAR (the CAR^{CD4+}) comprising:
an antigen binding domain;
a transmembrane domain; and
an intracellular signaling domain; and
2) a CD8+ T cell comprising a CAR (the CAR^{CD8+}) comprising:
an antigen binding domain;
a transmembrane domain; and
an intracellular signaling domain; and,
wherein the intracellular signaling domain of CAR^{CD4+} and the intracellular signaling domain of CAR^{CD8+} differ from one another, further wherein the CAR^{CD4+} comprises an ICOS domain.

2. The composition, or set of compositions, of claim 1, wherein the CAR^{CD8+} does not comprise an ICOS domain.

3. The composition, or set of compositions, of any of claim 1 or 2, further comprising:
3) a second CD8+ T cell comprising a CAR (the second CAR^{CD8+}) comprising:
an antigen binding domain;
a transmembrane domain; and
an intracellular signaling domain,
wherein the second CAR^{CD8+} differs from the CAR^{CD4+} and the CAR^{CD8+}, and
wherein the second CAR^{CD8+} does not comprise an ICOS signaling domain and comprises an intracellular signaling domain not present on the CAR^{CD8+}.

4. The composition, or set of compositions, of any of claims 1-3, which is a pharmaceutically acceptable composition or pharmaceutically acceptable set of compositions.

5. The composition, or set of compositions, of any of claims 1-4, wherein the CAR^{CD4+} does not include a 4-1BB domain, a CD28 domain and/or a costimulatory domain other than the ICOS domain.

6. The composition, or set of compositions, of any of claims 1-5, wherein the CAR^{CD4+} comprises a primary intracellular signaling domain, which is optionally a primary signal domain from FcR gamma, FcR beta, CD3 gamma, CD3 delta, CD3 epsilon, CD3 zeta, CD5, CD22, CD79a, CD79b, CD66d, DAP10, DAP12, or CD32, optionally wherein the primary signal domain is a CD3zeta domain.

7. The composition, or set of compositions, of any of claims 1-6, wherein the CAR^{CD4+} comprises a second costimulatory signaling domain, which is optionally from CD27, CD28, 4-1BB (CD137), OX40, CD30, CD40, ICOS (CD278), ICAM-1, CD2, CD7, LIGHT, NKG2C, B7-H3, a ligand that specifically binds with CD83, CDS, GITR, BAFFR, HVEM (LIGHTR), SLAMf7, NKP80 (KLRF1), CD160 (BY55), CD19, CD4, CD8 alpha, CD8 beta, IL2R beta, IL2R gamma, IL7R alpha, ITGA4, VLA1, CD49a, ITGA4, IA4, CD49D, ITGA6, VLA-6, C49f, ITGAD, CD11d, ITGAE, CD103, ITGAL, CD11a, LFA-1, ITGAM, CD11b, ITGAX, CD11c, ITGB1, CD29, ITGB2, CD18, ITGB7, TNFR2, TRANCE/RANKL, DNAM1 (CD226), SLAMF4 (C244, 2B4), CD84, CD96 (Tactile), CEACAM1, CRTAM, Ly9 (CD229), PSGL1, C100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, or PAG/Cbp.

8. The composition, or set of compositions, of any of claims 1-7, wherein the CAR^{CD8+} comprises:
a primary intracellular signaling domain which is optionally a primary signal domain from FcR gamma, FcR beta, CD3 gamma, CD3 delta, CD3 epsilon, CD3 zeta, CD5, CD22, CD79a, CD79b, CD66d, DAP10, DAP12, or CD32, e.g. wherein the primary signal domain is a CD3zeta domain, and
a costimulatory signaling domain other than ICOS which is optionally from CD27, CD28, 4-1BB (CD137), OX40, CD30, CD40, ICOS (CD278), ICAM-1, CD2, CD7, LIGHT, NKG2C, B7-H3, a ligand that specifically binds with CD83, CDS, GITR, BAFFR, HVEM (LIGHTR), SLAMf7, NKP80 (KLRF1), CD160 (BY55), CD19, CD4, CD8 alpha, CD8 beta, IL2R beta, IL2R gamma, IL7R alpha, ITGA4, VLA1, CD49a, ITGA4, IA4, CD49D, ITGA6, VLA-6, C49f, ITGAD, CD11d, ITGAE, CD103, ITGAL, CD11a, LFA-1, ITGAM, CD11b, ITGAX, CD11c, ITGB1, CD29, ITGB2, CD18, ITGB7, TNFR2, TRANCE/RANKL, DNAM1 (CD226), SLAMF4 (C244, 2B4), CD84, CD96 (Tactile), CEACAM1, CRTAM, Ly9 (CD229), PSGL1, C100 (SEMA4D), CD69, SLAMF6 (NTB-A, Ly108), SLAM (SLAMF1, CD150, IPO-3), BLAME (SLAMF8), SELPLG (CD 162), LTBR, LAT, GADS, or PAG/Cbp, e.g. wherein the costimulatory signaling domain other than ICOS is from 4-1BB or CD28.

9. The composition, or set of compositions, of any of claims 1-8, wherein the CAR^{CD8+} comprises a second costimulatory signaling domain which is optionally from CD27, CD28, 4-1BB (CD137), OX40, CD30, CD40, ICOS (CD278), ICAM-1, CD2, CD7, LIGHT, NKG2C, B7-H3, a ligand that specifically binds with CD83, CDS, GITR, BAFFR, HVEM (LIGHTR), SLAMf7, NKP80 (KLRF1), CD160 (BY55), CD19, CD4, CD8 alpha, CD8 beta, IL2R beta, IL2R gamma, IL7R alpha, ITGA4, VLA1, CD49a, ITGA4, IA4, CD49D, ITGA6, VLA-6, C49f, ITGAD, CD11d, ITGAE, CD103, ITGAL, CD11a, LFA-1, ITGAM, CD11b, ITGAX, CD11c, ITGB1, CD29, ITGB2, CD18, ITGB7, TNFR2, TRANCE/RANKL, DNAM1 (CD226), SLAMF4 (C244, 2B4), CD84, CD96 (Tactile), CEACAM1, CRTAM, Ly9 (CD229), PSGL1, C100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, or PAG/Cbp.

10. The composition, or set of compositions, of any of claims 1-9, wherein
(1) at least 10, 20, 30, 40, 50, 60, 70, or 80% of the T cells in the composition are CD4+ T cells comprising a CAR^{CD4+}; and/or
(2) at least 10, 20, 30, 40, 50, 60, 70, or 80% of the T cells in the composition are CD8+ T cells comprising a CAR^{CD8+}.

11. The composition, or set of compositions, of any of claims 1-10, wherein the CD4+ cell is a Thl7 polarized cell.

12. The composition, or set of compositions, of any of claims 1-11, wherein the CAR^{CD4+} and the CAR^{CD8+} each comprises an antibody variable domain, an scFv, a nanobody, or an antigen binding fragment thereof.

13. The composition, or set of compositions, of any of claims 1-12, wherein the CAR^{CD4+} and the CAR^{CD8+} each comprises an antigen binding domain which:
(1) is specific for a tumor antigen selected from CD19, CD20, CD22, ROR1, mesothelin, CD33/IL3Ra, c-Met, PSMA, Glycolipid F77, EGFRvIII, GD-2, NY-ESO-1, MAGE A3, and any combination thereof; or
(2) binds an antigen chosen from CD19, CD123, EGFRvIII, mesothelin, or Folate Receptor α.

14. The composition, or set of compositions, of any of claims 1-13, wherein the CAR^{CD4+} and the CAR^{CD8+} each comprises an antigen binding domain comprising an amino acid sequence chosen from SEQ ID NOs: 48-81.

15. The composition or set of compositions of any of claims 1-14, wherein the CAR^{CD4+} comprises an ICOS domain comprising the sequence of SEQ ID NO: 46 or SEQ ID NO: 40.

16. A kit comprising:
(1) the composition or set of compositions of any of claims 1-15; or
(2) the following components:
(a) a nucleic acid comprising a sequence encoding a CAR^{CD4+}; and
(b) a nucleic acid comprising a sequence encoding a CAR^{CD8+};
wherein said CAR^{CD4+} and CAR^{CD8+} are as defined in any one of claims 1-15.

17. The composition or set of compositions of any of claims 1-15, or the kit of claim 16, for use in the treatment of cancer.

18. The composition, set of compositions or kit, for use of claim 17, wherein
(i) the CD4⁺ T cell comprising the CAR^{CD4+} is an autologous T cell or an allogeneic T cell; and/or
(ii) the CD8⁺ T cell comprising the CAR^{CD8+} is an autologous T cell or an allogeneic T cell.

## Patentansprüche

1. Eine Zusammensetzung oder ein Satz von Zusammensetzungen, die folgende Bestandteile beinhalten:
1) eine CD4+-T-Zelle, die einen CAR (CAR^{CD4+}) beinhaltet, der Folgendes beinhaltet:
eine Antigenbindungsdomäne;
eine Transmembrandomäne; und
eine intrazelluläre Signalgebungsdomäne; und
2) eine CD8+-T-Zelle, die einen CAR (CAR^{CD8+}) beinhaltet, der Folgendes beinhaltend:
eine Antigenbindungsdomäne;
eine Transmembrandomäne; und
eine intrazelluläre Signalgebungsdomäne; und,
wobei die intrazelluläre Signalgebungsdomäne des CAR^{CD4+} und die intrazelluläre Signalgebungsdomäne des CAR^{CD8+} sich voneinander unterscheiden, wobei der CAR^{CD4+} ferner eine ICOS-Domäne beinhaltet.

2. Zusammensetzung oder Satz von Zusammensetzungen nach Anspruch 1, wobei der CAR^{CD8+} keine ICOS-Domäne beinhaltet.

3. Zusammensetzung oder Satz von Zusammensetzungen nach einem der Ansprüche 1 oder 2, die ferner Folgendes beinhalten:
3) eine zweite CD8+-T-Zelle, die einen CAR (den zweiten CAR^{CD8+}) beinhaltet, der Folgendes beinhaltet:
eine Antigenbindungsdomäne;
eine Transmembrandomäne; und
eine intrazelluläre Signalgebungsdomäne,
wobei der zweite CAR^{CD8+} sich von dem CAR^{CD4+} und dem CAR^{CD8+} unterscheidet und wobei der zweite CAR^{CD8+} keine ICOS-Signalgebungsdomäne beinhaltet und eine intrazelluläre Signalgebungsdomäne beinhaltet, die nicht auf dem CAR^{CD8+} vorhanden ist.

4. Zusammensetzung oder Satz von Zusammensetzungen nach einem der Ansprüche 1-3, die eine pharmazeutisch akzeptable Zusammensetzung ist oder der ein pharmazeutisch akzeptabler Satz von Zusammensetzungen ist.

5. Zusammensetzung oder Satz von Zusammensetzungen nach einem der Ansprüche 1-4, wobei der CAR^{CD4+} keine 4-1BB-Domäne, keine CD28-Domäne und/oder keine costimulatorische Domäne abgesehen von der ICOS-Domäne umfasst.

6. Zusammensetzung oder Satz von Zusammensetzungen nach einem der Ansprüche 1-5, wobei der CAR^{CD4+} eine primäre intrazelluläre Signalgebungsdomäne beinhaltet, die optional eine primäre Signaldomäne von FcR-gamma, FcR-beta, CD3-gamma, CD3-delta, CD3-epsilon, CD3-zeta, CD5, CD22, CD79a, CD79b, CD66d, DAP10, DAP12 oder CD32 ist, wobei die primäre Signaldomäne optional eine CD3-zeta-Domäne ist.

7. Zusammensetzung oder Satz von Zusammensetzungen nach einem der Ansprüche 1-6, wobei der CAR^{CD4+} eine zweite costimulatorische Signalgebungsdomäne beinhaltet, die optional von den Folgenden ist: CD27, CD28, 4-1BB (CD137), OX40, CD30, CD40, ICOS (CD278), ICAM-1, CD2, CD7, LIGHT, NKG2C, B7-H3, ein Ligand, der spezifisch an CD83 bindet, CDS, GITR, BAFFR, HVEM (LIGHTR), SLAMf7, NKP80 (KLRF1), CD160 (BY55), CD19, CD4, CD8-alpha, CD8-beta, IL2R-beta, IL2R-gamma, IL7R-alpha, ITGA4, VLA1, CD49a, ITGA4, IA4, CD49D, ITGA6, VLA-6, C49f, ITGAD, CD11d, ITGAE, CD103, ITGAL, CD11a, LFA-1, ITGAM, CD11b, ITGAX, CD11c, ITGB1, CD29, ITGB2, CD18, ITGB7, TNFR2, TRANCE/RANKL, DNAM1 (CD226), SLAMF4 (C244, 2B4), CD84, CD96 (Tactile), CEACAM1, CRTAM, Ly9 (CD229), PSGL1, C100 (SEMA4D), CD69, SLAMF6 (NTB-A, Ly108), SLAM (SLAMF1, CD150, IPO-3), BLAME (SLAMF8), SELPLG (CD162), LTBR, LAT, GADS oder PAG/Cbp.

8. Zusammensetzung oder Satz von Zusammensetzungen nach einem der Ansprüche 1-7, wobei der CAR^{CD8+} Folgendes beinhaltet:
eine primäre intrazelluläre Signalgebungsdomäne, die optional eine primäre Signaldomäne von FcR-gamma, FcR-beta, CD3-gamma, CD3-delta, CD3-epsilon, CD3-zeta, CD5, CD22, CD79a, CD79b, CD66d, DAP10, DAP12 oder CD32 ist, wobei die primäre Signaldomäne z. B. eine CD3-zeta-Domäne ist, und
eine costimulatorische Signalgebungsdomäne abgesehen von der ICOS, die optional von Folgendem ist: CD27, CD28, 4-1BB (CD137), OX40, CD30, CD40, ICOS (CD278), ICAM-1, CD2, CD7, LIGHT, NKG2C, B7-H3, ein Ligand, der spezifisch an CD83 bindet, CDS, GITR, BAFFR, HVEM (LIGHTR), SLAMf7, NKP80 (KLRF1), CD160 (BY55), CD19, CD4, CD8-alpha, CD8-beta, IL2R-beta, IL2R-gamma, IL7R-alpha, ITGA4, VLA1, CD49a, ITGA4, IA4, CD49D, ITGA6, VLA-6, C49f, ITGAD, CD11d, ITGAE, CD103, ITGAL, CD11a, LFA-1, ITGAM, CD11b, ITGAX, CD11c, ITGB1, CD29, ITGB2, CD18, ITGB7, TNFR2, TRANCE/RANKL, DNAM1 (CD226), SLAMF4 (C244, 2B4), CD84, CD96 (Tactile), CEACAM1, CRTAM, Ly9 (CD229), PSGL1, C100 (SEMA4D), CD69, SLAMF6 (NTB-A, Ly108), SLAM (SLAMF1, CD150, IPO-3), BLAME (SLAMF8), SELPLG (CD162), LTBR, LAT, GADS oder PAG/Cbp, wobei z. B. die costimulatorische Signalgebungsdomäne abgesehen von der ICOS von 4-1BB oder CD28 ist.

9. Zusammensetzung oder Satz von Zusammensetzungen nach einem der Ansprüche 1-8, wobei der CAR^{CD8+} eine zweite costimulatorische Signalgebungsdomäne beinhaltet, die optional von Folgendem ist: CD27, CD28, 4-1BB (CD137), OX40, CD30, CD40, ICOS (CD278), ICAM-1, CD2, CD7, LIGHT, NKG2C, B7-H3, ein Ligand, der spezifisch an CD83 bindet, CDS, GITR, BAFFR, HVEM (LIGHTR), SLAMf7, NKP80 (KLRF1), CD160 (BY55), CD19, CD4, CD8-alpha, CD8-beta, IL2R-beta, IL2R-gamma, IL7R-alpha, ITGA4, VLA1, CD49a, ITGA4, IA4, CD49D, ITGA6, VLA-6, C49f, ITGAD, CD11d, ITGAE, CD103, ITGAL, CD11a, LFA-1, ITGAM, CD11b, ITGAX, CD11c, ITGB1, CD29, ITGB2, CD18, ITGB7, TNFR2, TRANCE/RANKL, DNAM1 (CD226), SLAMF4 (C244, 2B4), CD84, CD96 (Tactile), CEACAM1, CRTAM, Ly9 (CD229), PSGL1, C100 (SEMA4D), CD69, SLAMF6 (NTB-A, Ly108), SLAM (SLAMF1, CD150, IPO-3), BLAME (SLAMF8), SELPLG (CD162), LTBR, LAT, GADS oder PAG/Cbp.

10. Zusammensetzung oder Satz von Zusammensetzungen nach einem der Ansprüche 1-9, wobei
(1) mindestens 10, 20, 30, 40, 50, 60, 70 oder 80 % der T-Zellen in der Zusammensetzung CD4+-T-Zellen sind, die einen CAR^{CD4+} beinhalten; und/oder
(2) mindestens 10, 20, 30, 40, 50, 60, 70 oder 80 % der T-Zellen in der Zusammensetzung CD8+-T-Zellen sind, die einen CAR^{CD8+} beinhalten.

11. Zusammensetzung oder Satz von Zusammensetzungen nach einem der Ansprüche 1-10, wobei die CD4+-Zelle eine polarisierte Th17-Zelle ist.

12. Zusammensetzung oder Satz von Zusammensetzungen nach einem der Ansprüche 1-11, wobei der CAR^{CD4+} und der CAR^{CD8+} jeweils eine variable Antikörperdomäne, ein scFv, einen Nanokörper oder ein Antigenbindungsfragment davon beinhalten.

13. Zusammensetzung oder Satz von Zusammensetzungen nach einem der Ansprüche 1-12, wobei der CAR^{CD4+} und der CAR^{CD8+} jeweils eine Antigenbindungsdomäne beinhalten, die:
(1) spezifisch für ein Tumorantigen ist, das aus CD19, CD20, CD22, ROR1, Mesothelin, CD33/IL3Ra, c-Met, PSMA, Glycolipid F77, EGFRvIII, GD-2, NY-ESO-1, MAGE A3 und einer beliebigen Kombination davon ausgewählt ist; oder
(2) ein aus CD19, CD123, EGFRvIII, Mesothelin oder Folatrezeptor-a ausgewähltes Antigen bindet.

14. Zusammensetzung oder Satz von Zusammensetzungen nach einem der Ansprüche 1-13, wobei der CAR^{CD4+} und der CAR^{CD8+} jeweils eine Antigenbindungsdomäne beinhalten, die eine Aminosäuresequenz beinhaltet, die aus SEQ ID NOs: 48-81 ausgewählt ist.

15. Zusammensetzung oder Satz von Zusammensetzungen nach einem der Ansprüche 1-14, wobei der CAR^{CD4+} eine ICOS-Domäne beinhaltet, die die Sequenz von SEQ ID NO: 46 oder SEQ ID NO: 40 beinhaltet.

16. Ein Kit, der Folgendes beinhaltet:
(1) die Zusammensetzung oder den Satz von Zusammensetzungen nach einem der Ansprüche 1-15; oder
(2) die folgenden Bestandteile:
(a) eine Nukleinsäure, die eine Sequenz beinhaltet, die für einen CAR^{CD4+} kodiert; und
(b) eine Nukleinsäure, die eine Sequenz beinhaltet, die für einen CAR^{CD8+} kodiert;
wobei der genannte CAR^{CD4+} und CAR^{CD8+} wie in einem der Ansprüche 1-15 definiert sind.

17. Zusammensetzung oder Satz von Zusammensetzungen nach einem der Ansprüche 1-15 oder Kit nach Anspruch 16 zur Verwendung bei der Behandlung von Krebs.

18. Zusammensetzung, Satz von Zusammensetzungen oder Kit zur Verwendung nach Anspruch 17, wobei
(i) die CD4⁺-T-Zelle, die den CAR^{CD4+} beinhaltet, eine autologe T-Zelle oder eine allogene T-Zelle ist; und/oder
(ii) die CD8⁺-T-Zelle, die den CAR^{CD8+} beinhaltet, eine autologe T-Zelle oder eine allogene T-Zelle ist.

## Revendications

1. Composition ou ensemble de compositions comprenant les constituants suivants :
1) un lymphocyte T CD4+ comprenant un CAR (le CAR^{CD4+}) comprenant :
un domaine de liaison à un antigène ;
un domaine transmembranaire ; et
un domaine de signalisation intracellulaire ; et
2) un lymphocyte T CD8+ comprenant un CAR (le CAR^{CD8+}) comprenant :
un domaine de liaison à un antigène ;
un domaine transmembranaire ; et
un domaine de signalisation intracellulaire ; et
dans laquelle ou dans lequel le domaine de signalisation intracellulaire de CAR^{CD4+} et le domaine de signalisation intracellulaire de CAR^{CD8+} diffèrent l'un de l'autre, en outre dans laquelle ou dans lequel le CAR^{CD4+} comprend un domaine ICOS.

2. Composition, ou ensemble de compositions, selon la revendication 1, dans laquelle ou dans lequel le CAR^{CD8+} ne comprend pas de domaine ICOS.

3. Composition, ou ensemble de compositions, selon l'une quelconque des revendications 1 ou 2, comprenant en outre :
3) un deuxième lymphocyte T CD8+ comprenant un CAR (le deuxième CAR^{CD8+}) comprenant :
un domaine de liaison à un antigène ;
un domaine transmembranaire ; et
un domaine de signalisation intracellulaire,
dans laquelle ou dans lequel le deuxième CAR^{CD8+} diffère du CAR^{CD4+} et du CAR ^{CD8+}, et dans laquelle ou dans lequel le deuxième CAR^{CD8+} ne comprend pas de domaine de signalisation ICOS et comprend un domaine de signalisation intracellulaire qui n'est pas présent sur le CAR^{CD8+}.

4. Composition, ou ensemble de compositions, selon l'une quelconque des revendications 1 à 3, laquelle ou lequel est une composition pharmaceutiquement acceptable ou un ensemble de compositions pharmaceutiquement acceptable.

5. Composition, ou ensemble de compositions, selon l'une quelconque des revendications 1 à 4, dans laquelle ou dans lequel le CAR^{CD4+} ne comprend pas de domaine 4-1BB, de domaine CD28 et/ou de domaine de costimulation autre que le domaine ICOS.

6. Composition, ou ensemble de compositions, selon l'une quelconque des revendications 1 à 5, dans laquelle ou dans lequel le CAR^{CD4+} comprend un domaine de signalisation intracellulaire primaire, qui est optionnellement un domaine de signalisation primaire parmi FcR gamma, FcR bêta, CD3 gamma, CD3 delta, CD3 epsilon, CD3 zêta, CD5, CD22, CD79a, CD79b, CD66d, DAP10, DAP12, ou CD32, optionnellement dans laquelle ou dans lequel le domaine de signalisation primaire est un domaine CD3 zêta.

7. Composition, ou ensemble de compositions, selon l'une quelconque des revendications 1 à 6, dans laquelle ou dans lequel le CAR^{CD4+} comprend un deuxième domaine de signalisation de costimulation, qui est optionnellement parmi CD27, CD28, 4-1BB (CD137), OX40, CD30, CD40, ICOS (CD278), ICAM-1, CD2, CD7, LIGHT, NKG2C, B7-H3, un ligand qui se lie spécifiquement à CD83, CDS, GITR, BAFFR, HVEM (LIGHTR), SLAMf7, NKP80 (KLRF1), CD160 (BY55), CD19, CD4, CD8 alpha, CD8 bêta, IL2R bêta, IL2R gamma, IL7R alpha, ITGA4, VLA1, CD49a, ITGA4, IA4, CD49D, ITGA6, VLA-6, C49f, ITGAD, CD11d, ITGAE, CD103, ITGAL, CD11a, LFA-1, ITGAM, CD11b, ITGAX, CD11c, ITGB1, CD29, ITGB2, CD18, ITGB7, TNFR2, TRANCE/RANKL, DNAM1 (CD226), SLAMF4 (C244, 2B4), CD84, CD96 (Tactile), CEACAM1, CRTAM, Ly9 (CD229), PSGL1, C100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, ou PAG/Cbp.

8. Composition, ou ensemble de compositions, selon l'une quelconque des revendications 1 à 7, dans laquelle ou dans lequel le CAR^{CD8+} comprend :
un domaine de signalisation intracellulaire primaire qui est optionnellement un domaine de signalisation primaire parmi FcR gamma, FcR bêta, CD3 gamma, CD3 delta, CD3 epsilon, CD3 zêta, CD5, CD22, CD79a, CD79b, CD66d, DAP10, DAP12, ou CD32, le domaine de signalisation primaire étant par exemple un domaine CD3 zêta, et
un domaine de signalisation de costimulation autre qu'ICOS qui est optionnellement parmi CD27, CD28, 4-1BB (CD137), OX40, CD30, CD40, ICOS (CD278), ICAM-1, CD2, CD7, LIGHT, NKG2C, B7-H3, un ligand qui se lie spécifiquement à CD83, CDS, GITR, BAFFR, HVEM (LIGHTR), SLAMf7, NKP80 (KLRF1), CD160 (BY55), CD19, CD4, CD8 alpha, CD8 bêta, IL2R bêta, IL2R gamma, IL7R alpha, ITGA4, VLA1, CD49a, ITGA4, IA4, CD49D, ITGA6, VLA-6, C49f, ITGAD, CD11d, ITGAE, CD103, ITGAL, CD11a, LFA-1, ITGAM, CD11b, ITGAX, CD11c, ITGB1, CD29, ITGB2, CD18, ITGB7, TNFR2, TRANCE/RANKL, DNAM1 (CD226), SLAMF4 (C244, 2B4), CD84, CD96 (Tactile), CEACAM1, CRTAM, Ly9 (CD229), PSGL1, C100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, ou PAG/Cbp, le domaine de signalisation de costimulation autre qu'ICOS étant par exemple parmi 4-1BB ou CD28.

9. Composition, ou ensemble de compositions, selon l'une quelconque des revendications 1 à 8, dans laquelle ou dans lequel le CAR^{CD8+} comprend un deuxième domaine de signalisation de costimulation, qui est optionnellement parmi CD27, CD28, 4-1BB (CD137), OX40, CD30, CD40, ICOS (CD278), ICAM-1, CD2, CD7, LIGHT, NKG2C, B7-H3, un ligand qui se lie spécifiquement à CD83, CDS, GITR, BAFFR, HVEM (LIGHTR), SLAMf7, NKP80 (KLRF1), CD160 (BY55), CD19, CD4, CD8 alpha, CD8 bêta, IL2R bêta, IL2R gamma, IL7R alpha, ITGA4, VLA1, CD49a, ITGA4, IA4, CD49D, ITGA6, VLA-6, C49f, ITGAD, CD11d, ITGAE, CD103, ITGAL, CD11a, LFA-1, ITGAM, CD11b, ITGAX, CD11c, ITGB1, CD29, ITGB2, CD18, ITGB7, TNFR2, TRANCE/RANKL, DNAM1 (CD226), SLAMF4 (C244, 2B4), CD84, CD96 (Tactile), CEACAM1, CRTAM, Ly9 (CD229), PSGL1, C100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, ou PAG/Cbp.

10. Composition, ou ensemble de compositions, selon l'une quelconque des revendications 1 à 9, dans laquelle ou dans lequel :
(1) au moins 10, 20, 30, 40, 50, 60, 70 ou 80 % des lymphocytes T dans la composition sont des lymphocytes T CD4+ comprenant un CAR^{CD4+} ; et/ou
(2) au moins 10, 20, 30, 40, 50, 60, 70 ou 80 % des lymphocytes T dans la composition sont des lymphocytes T CD8+ comprenant un CAR^{CD8+}.

11. Composition, ou ensemble de compositions, selon l'une quelconque des revendications 1 à 10, dans laquelle ou dans lequel le lymphocyte CD4+ est un lymphocyte Thl7 polarisé.

12. Composition, ou ensemble de compositions, selon l'une quelconque des revendications 1 à 11, dans laquelle ou dans lequel le CAR^{CD4+} et le CAR^{CD8+} comprennent chacun un domaine variable d'anticorps, un fragment scFv, un nanobody, ou un fragment de ceux-ci se liant à un antigène.

13. Composition, ou ensemble de compositions, selon l'une quelconque des revendications 1 à 12, dans laquelle ou dans lequel le CAR^{CD4+} et le CAR^{CD8+} comprennent chacun un domaine de liaison à un antigène qui :
(1) est spécifique d'un antigène tumoral sélectionné parmi CD19, CD20, CD22, ROR1, la mésothéline, CD33/IL3Ra, c-Met, PSMA, le glycolipide F77, EGFRvIII, GD-2, NY-ESO-1, MAGE A3, et n'importe quelle combinaison de ceux-ci ; ou
(2) se lie un antigène choisi parmi CD19, CD123, EGFRvIII, la mésothéline, ou le récepteur alpha des folates.

14. Composition, ou ensemble de compositions, selon l'une quelconque des revendications 1 à 13, dans laquelle ou dans lequel le CAR^{CD4+} et le CAR^{CD8+} comprennent chacun un domaine de liaison à un antigène comprenant une séquence d'acides aminés choisie parmi les SÉQ. ID n^{os} 48 à 81.

15. Composition, ou ensemble de compositions, selon l'une quelconque des revendications 1 à 14, dans laquelle ou dans lequel le CAR^{CD4+} comprend un domaine ICOS comprenant la séquence SÉQ. ID n° 46 ou SÉQ. ID n° 40.

16. Trousse comprenant :
(1) la composition ou l'ensemble des compositions selon l'une quelconque des revendications 1 à 15 ; ou
(2) les constituants suivants :
(a) un acide nucléique comprenant une séquence codant un CAR^{CD4+} ; et
(b) un acide nucléique comprenant une séquence codant un CAR^{CD8+} ; lesdits CAR^{CD4+} et CAR^{CD8+} étant tels que définis dans l'une quelconque des revendications 1 à 15.

17. Composition ou ensemble de compositions selon l'une quelconque des revendications 1 à 15, ou trousse selon la revendication 16, destiné(e) à une utilisation dans le traitement du cancer.

18. Composition, ensemble de compositions ou trousse, destiné(e) à une utilisation selon la revendication 17, dans laquelle ou dans lequel
(i) le lymphocyte CD4⁺ comprenant le CAR^{CD4+} est un lymphocyte T autologue ou un lymphocyte T allogénique ; et/ou
(i) le lymphocyte CD8⁺ comprenant le CAR^{CD8+} est un lymphocyte T autologue ou un lymphocyte T allogénique.
